# EUROPEAN PATENT APPLICATION

(11) **EP 4 342 892 A1**
(43) Date of publication of application: **27.03.2024**
(21) Application number: 22832202.0
(22) Date of filing: 30.06.2022
(51) Int. Cl.: C07D 401/14, C07D 413/14, C07D 417/14, C07D 471/04, A61P 35/00, A61K 31/4427

(54) **BRUTON'S TYROSINE KINASE AND MUTANT DEGRADER, COMPOSITION AND APPLICATION THEREOF**

(30) Priority: 01.07.2021 CN 202110743897; 27.01.2022 CN 202210103330
(71) Applicant: Hangzhou Healzen Therapeutics Co., Ltd., Hangzhou, Zhejiang 310018 (CN); Shanghai Institute of Materia Medica, Chinese Academy of Sciences, Pudong, Shanghai 201203 (CN)
(72) Inventor: ZHOU, Xinglu, Hangzhou, Zhejiang 310018 (CN); LI, Jia, Shanghai 201203 (CN); LIU, Xingguo, Hangzhou, Zhejiang 310018 (CN); ZHOU, Yubo, Shanghai 201203 (CN); HU, Miao, Hangzhou, Zhejiang 310018 (CN); LUO, Xiaomin, Shanghai 201203 (CN); XIE, Jiangfeng, Hangzhou, Zhejiang 310018 (CN); KAN, Weijuan, Shanghai 201203 (CN); SU, Mingbo, Shanghai 201203 (CN); WU, Yizhe, Hangzhou, Zhejiang 310018 (CN)
(74) Representative: Ostertag & Partner Patentanwälte mbB
(86) International application number: PCT/CN2022/103155
(87) International publication number: WO 2023/274390

(57) **Abstract**

Disclosed are a Bruton's tyrosine kinase and a mutant degrader thereof, or a stereoisomer thereof, or a stereoisomer mixture thereof or a pharmaceutically acceptable salt thereof, and an application thereof in the preparation of a drug for treating diseases, disorders or conditions that would benefit from the degradation of the Bruton's tyrosine kinase and the mutant thereof. The compound of the present invention can degrade BTK protein, can degrade BTKC481S protein, has an anti-proliferation inhibiting effect on tumor cell strains Mino and OCI-LY10, shows good anti-tumor activity in an OCI-LY10 subcutaneous transplantation tumor model, has an inhibiting effect on B cell activation, and can be applied to B cell or plasma cell proliferative diseases and autoimmune diseases. The compound of the present invention has good oral absorption properties, and can be applied to oral treatment of human or animal B cell or plasma cell proliferative diseases and autoimmune diseases.

## Description

### TECHNICAL FIELD

The invention belongs to the technical field of pharmaceutical synthesis, and specifically relates to a degrader of Bruton's tyrosine kinase and Bruton's tyrosine kinase mutant, and its composition and application thereof.

### BACKGROUND

Bruton's tyrosine kinase (Btk), a member of the Tec family of non-receptor tyrosine kinases, is a key signaling enzyme expressed in all hematopoietic cell types except T lymphocytes and natural killer cells. Btk plays a critical role in the B cell signaling pathway linking cell surface B-cell receptor (BCR) stimulation to downstream intracellular responses. Btk is a key regulator of B cell development, activation, signaling and survival. In addition, Bkt plays a role in numerous other hematopoietic cell signaling pathways, such as Toll like receptor (TLR) and cytokine receptor-mediated TNF-α production in macrophages, and immunoglobulin E receptor (FcεR1) signaling in mast cells, inhibition of Fas/APO-1 cell apoptosis signaling and collagen-stimulated platelet aggregation in B-lineage lymphoid cells. See, for example, C.A. Jeffries et al., J. Bio. Chem. (2003) 278: 26258-26264, N. J. Horwood et al., J. Exp. Med. (2003) 197: 1603-1611. Research in recent years has shown that the Btk signaling pathway is a new hot spot in the clinical treatment research of non-Hodgkin lymphoma (NHL), especially chronic lymphocytic leukemia (CLL), B-cell lymphoma and autoimmune diseases. By acting on the BCR signaling pathway, small molecule Btk inhibitors bind to Btk to inhibit Btk autophosphorylation, prevent Btk activation, and thereby block cell conduction and induce apoptosis.

As BTK inhibition is used in the clinical setting, some CLL patients have developed acquired resistance to covalent BTK drugs caused by BTK C481 mutations. A new generation of BTK drugs is urgently needed to solve this acquired resistance.

In recent years, significant progress has been made in the targeted protein degradation technology (PROTAC), which achieves target protein degradation by inducing ubiquitination of target proteins. Drugs using this technology do not need to continuously occupy the target proteins, and the catalytic concentration can achieve target degradation. Therefore, the development of targeting degradation molecules for BTK is expected to solve the problem of acquired resistance to BTK.

### SUMMARY

The purpose of the present invention is to provide a novel, unreported BTK degradation molecule with excellent properties, its optical isomer or pharmaceutically acceptable salt thereof, having high degradation activity against BTK and position 481 cysteine mutation-type BTK, having anti-proliferation inhibitory effect on Mino and OCI-LY10 tumor cell lines, showing good anti-tumor activity in OCI-LY10 subcutaneous transplanted tumor model, having inhibitory effect on B cell activation, and having excellent oral PK property.

The present invention also provides a pharmaceutical composition comprising the above compound and its optical isomer or pharmaceutically acceptable salt thereof. The present invention relates to a class of drugs or pharmaceutically acceptable salts that can degrade BTK or position 481 cysteine mutation-type BTK protein, and can be used as medicaments to treat hyperproliferative diseases, such as cancer and inflammation, as well as immune and autoimmune diseases.

A first aspect of the present invention provides a compound as shown in the formula (I), or its pharmaceutically acceptable salt thereof: 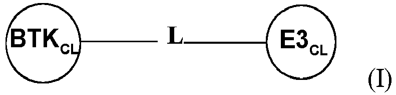
or its stereoisomer thereof, or its stereoisomer mixture thereof, or its pharmaceutically acceptable salt thereof;
where:
   BTK_{CL} represents a chemical ligand that can bind to BTK kinase.
   E3_{CL} represents a chemical ligand that can bind to E3 ubiquitin ligase.
   L represents a class of chemical groups or chemical bonds that connect BTK_{CL} and E3_{CL} at the same time.

As a preferred embodiment, E3_{CL} is selected from: chemical fragments that bind to the E3 ubiquitin ligase CRBN.

L has the following structure:

-X1-L1-X2-Cyc1-X3-L2-X4-Cyc2-X5-L3-X6-Cyc3-X7-L4-X8-

wherein:
X1, X2, X3, X4, X5, X6, X7 and X8 are each independently selected from: absent (or chemical bonds), -O-, -S-, or -N(Ra)-.
L1, L2, L3, and L4 are each independently selected from: absent, chemical bonds, C1-C4 alkyl, C2-C4 alkenyl, or C2-C4 alkynyl. The above-mentioned alkyl, alkenyl, and alkynyl can be substituted with oxo, alkyl, halogen, cyano, or haloalkyl.
Cyc1, Cyc2, and Cyc3 are each independently selected from: absent, 3-12 membered heterocyclic ring, a 5-12 membered aromatic ring (such as benzene ring), a 5-12 membered heteroaromatic ring, a 3-12 membered cycloalkane, a 4-12 membered cycloalkyne, a 3-12 membered cycloalkene, wherein the heterocyclic ring, aromatic ring, heteroaromatic ring, cycloalkane, cycloalkyne, and cycloalkene can be substituted with oxo, alkyl, halogen, cyano, haloalkyl.
Ra is selected from: H, C1-C4 alkyl.

As a preferred embodiment, BTK_{CL} is selected from the following structures: 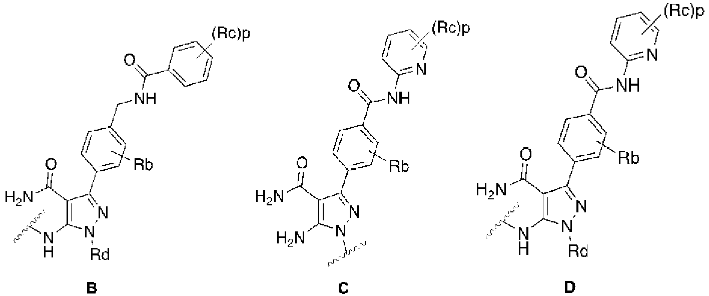 wherein:
when BTK_{CL} is selected from structure B, C, D:
Rb is selected from: H, halogen, cyano, methyl, -CF₃, C1-C3 alkoxy;
Rc can be one or more, each independently selected from: H, CN, halogen, C1-C4 alkyl, C1-C4 cycloalkyl, C1-C4 haloalkyl, C1-C4 alkoxy, C1-C4 alkylamino;
Rd is selected from: C1-C6 alkyl, C1-C6 haloalkyl, C3-C8 cycloalkyl, C3-C8 heterocyclyl; the C1-C6 alkyl, cycloalkyl, and heterocyclyl can be further substituted with one or more substituents selected from: halogen, C1-C4 alkyl, C1-C4 haloalkyl, C1-C4 alkoxy, cyano, oxo, carboxyl, ester group, amido, hydroxyl;
and p is selected from: an integer of 0-4.

As a preferred embodiment, BTK_{CL} is selected from the following structures: 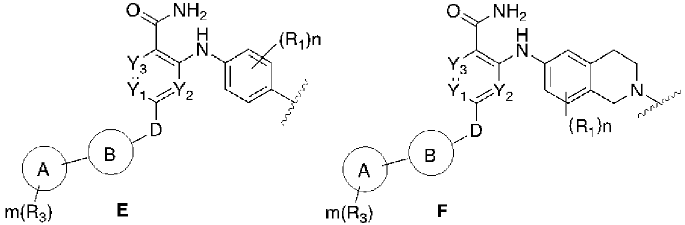
Yi is selected from: N, CR₂;
Y₂ and Y₃ are independently selected from: N, CH;
ring A is selected from: 3-12 membered heterocyclic ring;
ring B is selected from: 5-6 membered heteroaromatic ring, a 5-10 membered heterocyclic ring, wherein the heteroaromatic ring and heterocyclic ring can be further substituted with one or more substituents selected from: halogen, C1-C4 alkyl, C1-C4 haloalkyl, C1-C4 alkoxy, cyano, oxo, carboxyl, ester group, amido, hydroxyl, wherein two substituents of ring B can be connected to form a bridged ring, a spiro ring, a fused ring;
D is selected from: chemical bonds, C1-C3 alkyl, -O-, -NH-, -S-;
R₁ can be one or more, each R₁ is independently selected from: halogen, C1-C6 alkyl, C1-C6 haloalkyl, -CN, -COOH, -NH₂;
R₂ is selected from: hydrogen, cyano, halogen, C1-C4 alkyl, C3-C8 cycloalkyl, C1-C4 haloalkyl, C1-C4 alkoxy;
R₃ can be one or more, each R₃ is independently selected from: 3-12 membered heterocyclyl, 5-12 membered heteroaryl, 5-12 membered aryl (such as phenyl), 3-12 membered cycloalkyl, halogen, C1-C4 alkyl, C1-C4 haloalkyl, C1-C4 alkoxy, cyano, oxo, carboxyl, ester group, amido, or hydroxyl, wherein the heterocyclyl, heteroaryl, and cycloalkyl can be further substituted with substituents selected from: halogen, C1-C4 alkyl, C1-C4 haloalkyl, C1-C4 alkoxy, cyano, oxo, carboxyl, ester group, amido, or hydroxyl. Wherein two R3s of ring A can be connected to form a spiro ring, a bridged ring, a fused ring;
m is selected from: an integer of 0-3;
n is selected from: an integer of 0-2;
represents that the atom connected thereto is connected to L;
when Yi is selected from CH, ring A is a monocyclic ring, and ring B is a benzene ring, a 5-6 membered heteroaromatic ring, a 4-6 membered alkane heterocyclic ring or an 8-10 membered spiro ring, R₃ is not C1-C4 alkyl, C1-C4 haloalkyl.

Further, a preferred compound of the present invention has the structure of the general formula II(b) 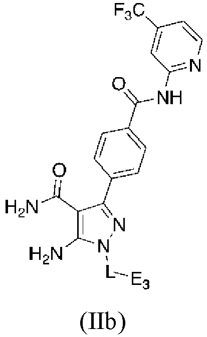 or its stereoisomer thereof, or its stereoisomer mixture thereof, or its pharmaceutically acceptable salt thereof; Further, a preferred compound of the present invention has the structure of general formula III(a) or III(b): 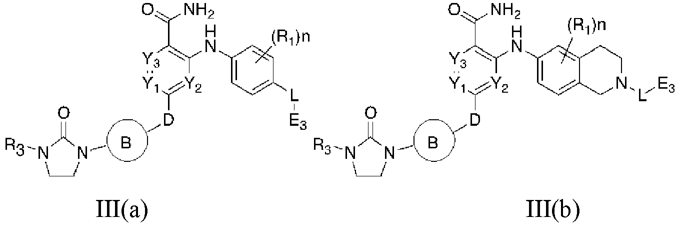
or a stereoisomer thereof, or a stereoisomer mixture thereof, or a pharmaceutically acceptable salt thereof;
ring B is not a bridged ring;
R₃ is selected from: C1-C4 alkyl, C1-C4 haloalkyl, 3-6 membered cycloalkyl, 3-6 membered heterocyclyl, 5-6 membered aryl (such as phenyl), 5-6 membered heteroaryl, wherein the cycloalkyl, heterocyclyl, aryl (phenyl), and the heteroaryl can be further substituted with one or more substituents selected from: halogen, C1-C4 alkyl, C1-C4 haloalkyl, C1-C4 alkoxy, cyano, oxo, carboxyl, ester group, amide, hydroxyl, two substituents on the cycloalkyl, heterocyclyl, aryl (phenyl), and heteroaryl can be connected to form a fused ring, a bridged ring, or a spiro ring;
when Y1 is selected from CH, R₃ is not C1-C4 alkyl, C1-C4 haloalkyl.

Still further, a preferred compound of the present invention has the structure of the general formula IV(a), IV(b), IV(c), and IV(d): 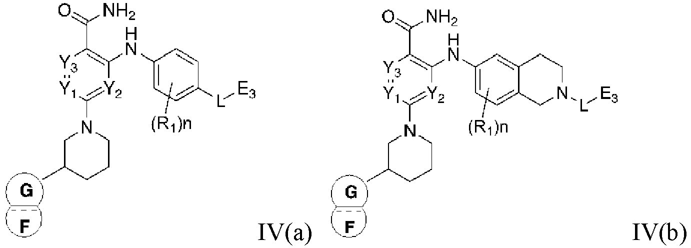 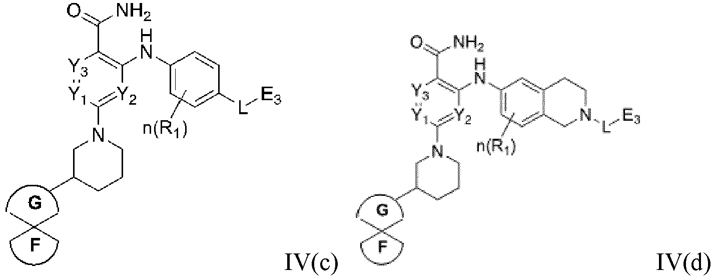
or its stereoisomer thereof, or its stereoisomer mixture thereof, or its pharmaceutically acceptable salt thereof;
wherein the fused ring or spiro ring structure composed of ring G and ring F is selected from:

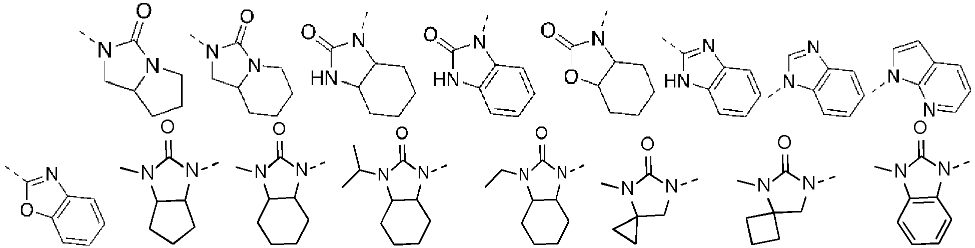

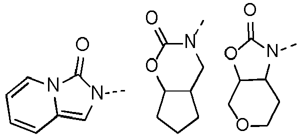

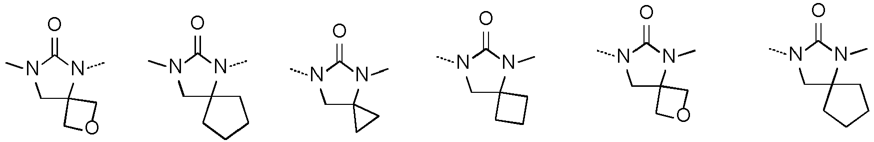

The above-mentioned fused ring, spiro ring structure can be further substituted with one or more substituents selected from: halogen, C1-C4 alkyl, C1-C4 haloalkyl, C1-C4 alkoxy, cyano, oxo, carboxyl, ester group, amido, hydroxyl.

Further, a preferred compound of the present invention has the structure of the general formula V(a) or V(b): 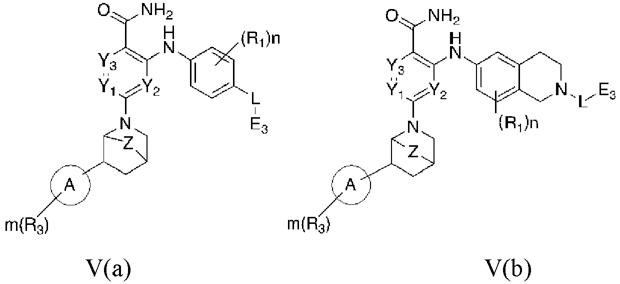
or its stereoisomer thereof, or its stereoisomer mixture thereof, or its pharmaceutically acceptable salt thereof;
Z is selected from: -CH₂-, -CH₂CH₂-, -O-.

As a further preferred embodiment, in the general formulas III(a) and III(b)
R3 is preferably selected from: C1-C4 alkyl, ring D;
ring D is selected from: 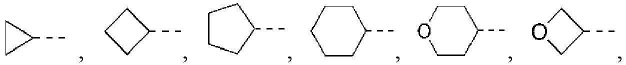 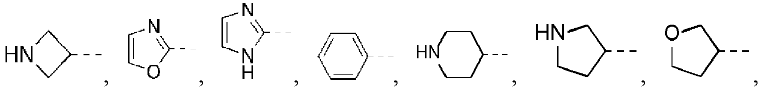 ring D can be further substituted with one or more substituents which can be on C atom or N atom and are selected from: halogen, C1-C4 alkyl, C1-C4 haloalkyl, C1-C4 alkoxy, cyano, oxo, carboxyl, ester group, amide, hydroxyl. Two substituents on ring A can be connected to form a spiro ring, a bridged ring or a fused ring;
when Yi is CH, R₃ is selected from ring D.

Further, a preferred compound of the present invention has the structure of the general formula VI: 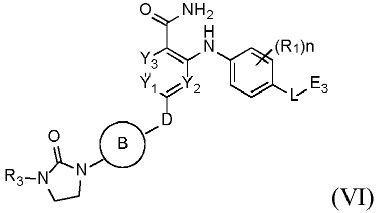
the definition of "E₃" in general formulas (IIb), III(a), III(b), IV(a), IV(b), IV(c), IV(d), V(a), V(b) and VI is the same as that of "E3_{CL}" mentioned in the previous contents, that is, it represents a chemical ligand that can bind to E3 ubiquitin ligase,
or its stereoisomer thereof, or its stereoisomer mixture thereof, or its pharmaceutically acceptable salt, prodrug thereof; where:
   L represents a chemical group or a chemical bond connecting benzene ring to E₃;
   E₃ represents a chemical ligand that can bind to E₃ ubiquitin ligase;
   Yi is selected from: N, CR₂;
   Y₂ and Y₃ are each independently selected from: N, CH;
   D is selected from: chemical bonds;
   one or more R₁ₛ are each selected from: H, halogen, C₁-C₆ alkyl, C₁-C₆ haloalkyl, -CN, -COOH, -NH₂;
   R₂ is selected from: H, cyano, halogen, C₁-C₄ alkyl, C₃-C₈ cycloalkyl, C₁-C₄ haloalkyl, C₁-C₄ alkoxy;
   ring B is selected from: 5-6 membered heteroaryl, 5-10 membered heterocyclyl, wherein the heteroaryl and heterocyclyl can be further substituted with one or more substituents selected from: halogen, C₁-C₄ alkyl, C₁-C₄ haloalkyl, C₁-C₄ alkoxy, cyano, oxo, carboxyl, ester group, amido, hydroxyl, wherein two substituents occurring silmutaneously on ring B can be connected to form a bridged ring, a spiro ring, a fused ring;
   R₃ is selected from: C₁-C₄ alkyl, C₁-C₄ haloalkyl, 3-6 membered cycloalkyl, 3-6 membered heterocyclyl, 5-6 membered aryl, or 5-6 membered heteroaryl; wherein the cycloalkyl, the heterocyclyl, the aryl, and the heteroaryl can be further substituted with one or more substituents selected from: halogen, C₁-C₄ alkyl, C₁-C₄ haloalkyl, C₁-C₄ alkoxy, cyano, oxo, carboxyl, ester group, amide, hydroxyl, two substituents on the cycloalkyl, heterocyclyl, aryl and the heteroaryl can be connected to form a fused ring, a bridged ring or a spiro ring;
   n is selected from: an integer of 0-2;
   when Yi is selected from CH, and ring B is a benzene ring, a 5-6 membered heteroaromatic ring, a 4-6 membered heterocyclic ring or an 8-10 membered spiro ring, R₃ is not C₁-C₄ alkyl, C₁-C₄ haloalkyl.

As a further preferred embodiment, in the general formula VI,
In some embodiments, ring B is preferably selected from: 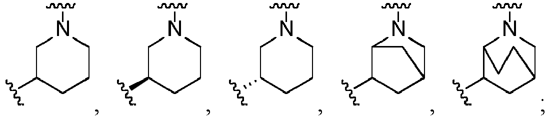 still further, as a preferred embodiment:
L is selected from: 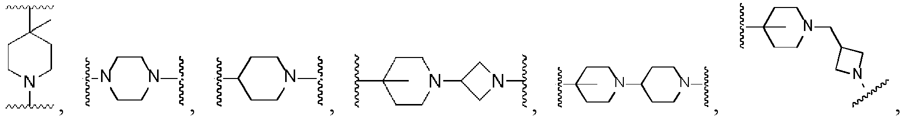 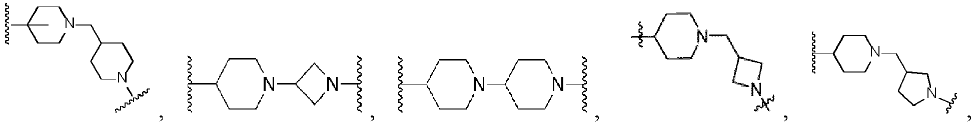 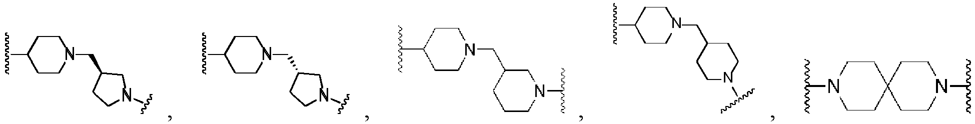 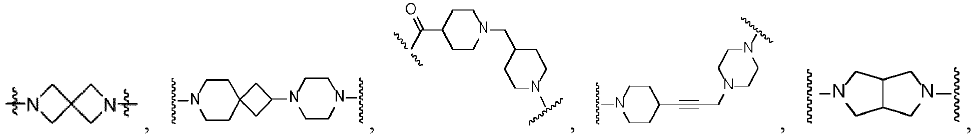 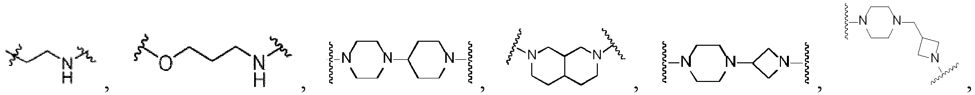 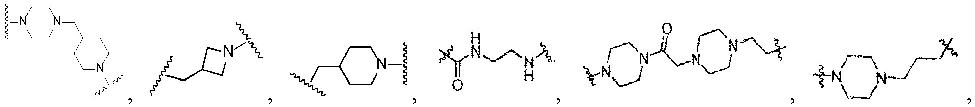 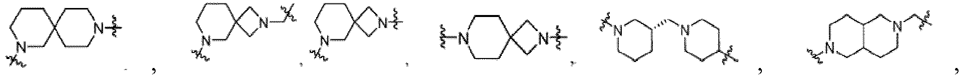 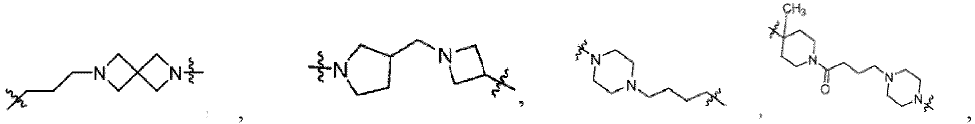 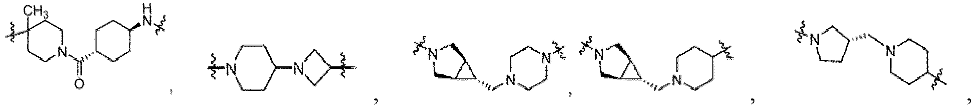 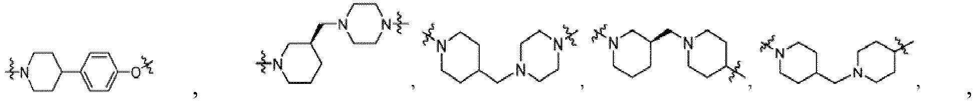 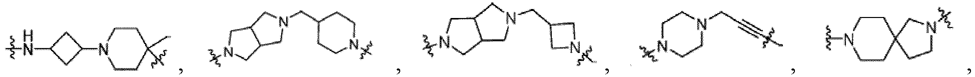 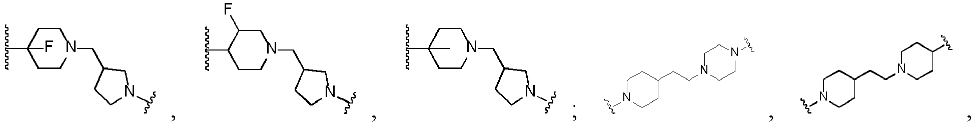 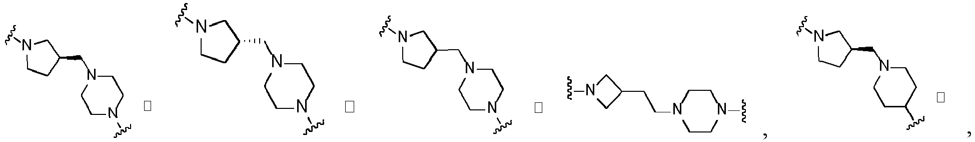 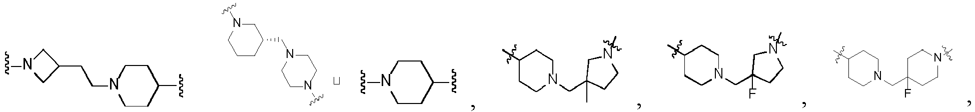 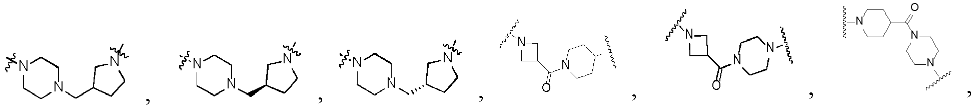 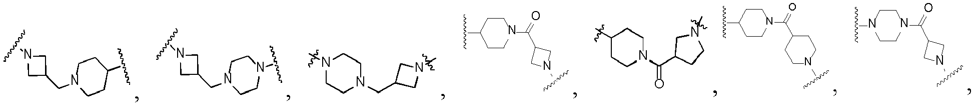 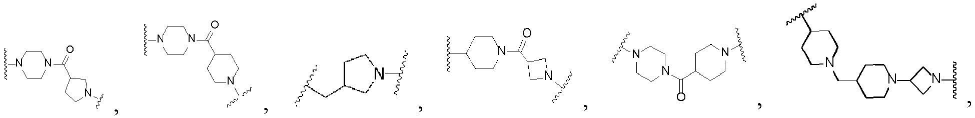 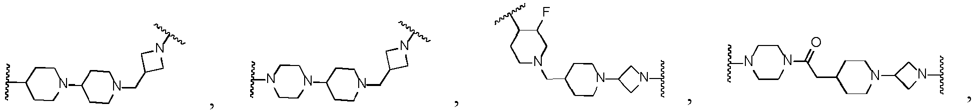 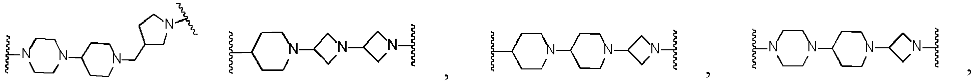 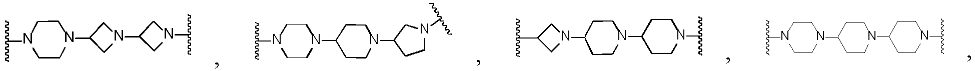 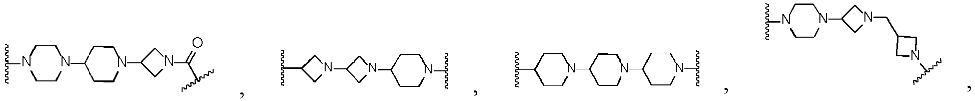 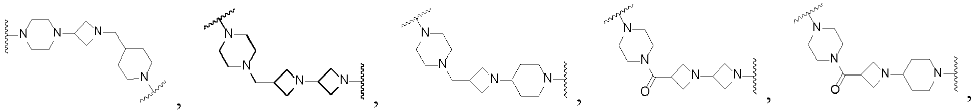 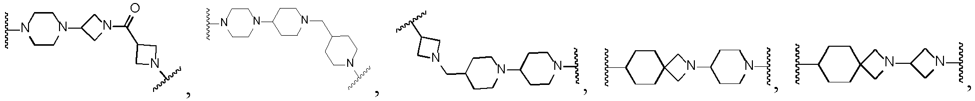 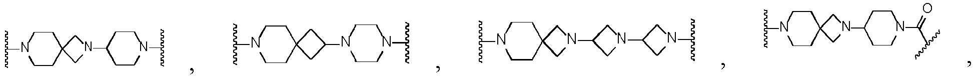 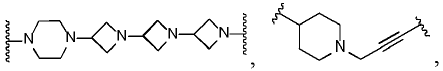 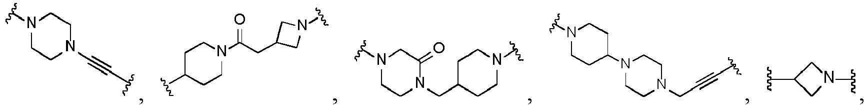
E3_{CL} is selected from: 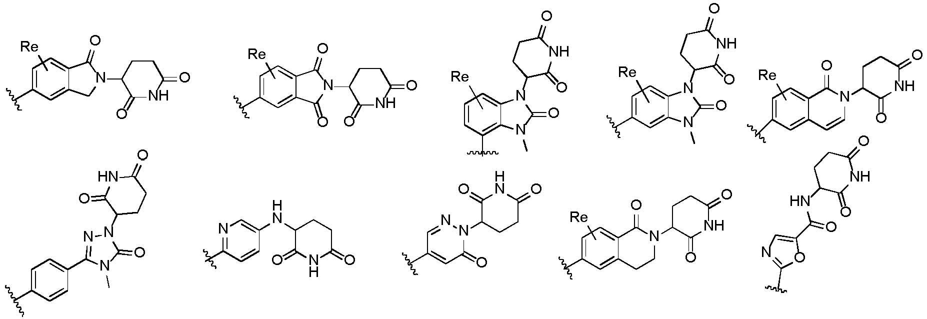 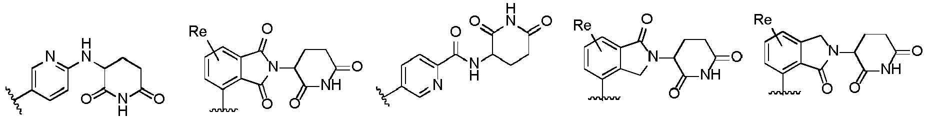 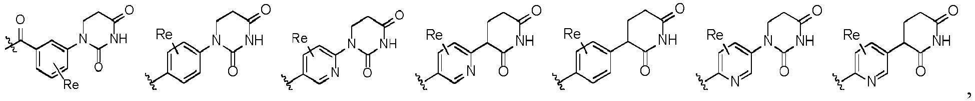 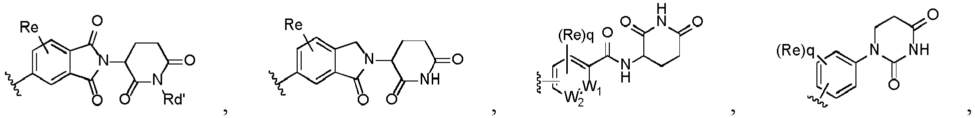 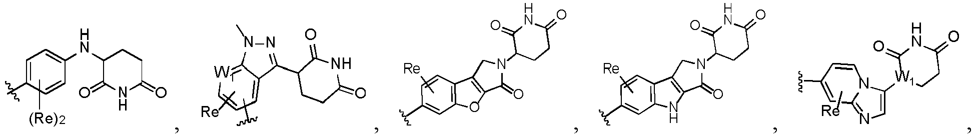 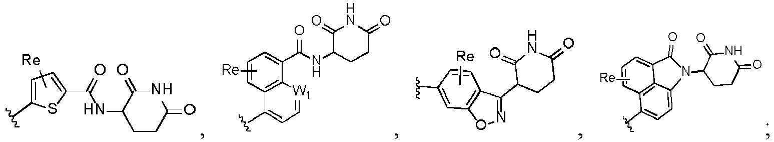
Re is selected from: H, F, Cl, -CH₃, -OMe, -CN, -CF₃, -CHF₂, -CH(CH₃)₂, -cyclopropyl, -C(O)NH₂, -S(O)₂Rf, or -P(O)(Rf)₂;
Rd' is selected from: H, Rf OCORg, RfOCOORg, ROCONRgRh, COORf, CONRfRg;
Rf, Rg, and Rh are each independently selected from: H, Ci-Cs alkyl, 3-8 membered cycloalkyl, 3-8 membered heterocyclyl, C₁-C₆ alkyl, 3-8 membered cycloalkyl, C₁-C₆ alkyl, 3-8 membered heterocyclyl;
or two adjacent Res on the benzene ring form a 7-10 membered benzo ring together with the benzene ring; the C atom on the benzo ring can be further substituted with one or more heteroatoms selected from N, O, S; the benzo ring is optionally substituted with H, F, Cl, -CH₃, -OMe, -CN, -CF₃, -CHF₂, -CH(CH₃)₂, -cyclopropyl, -C(O)NH₂;
W₁ and W₂ are selected from: CH, N;
q is selected from: 0, 1, 2, 3.

Still further, E3 (*i.e.,* E3_{CL}) is selected from:

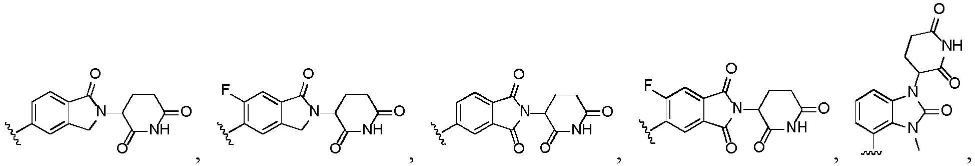

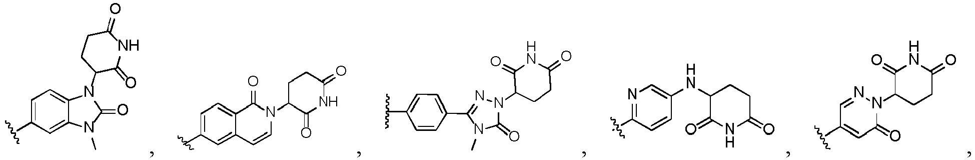

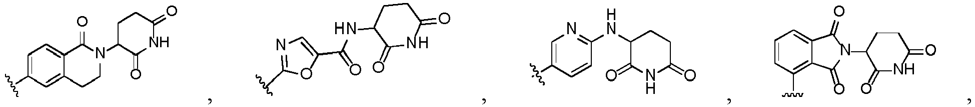

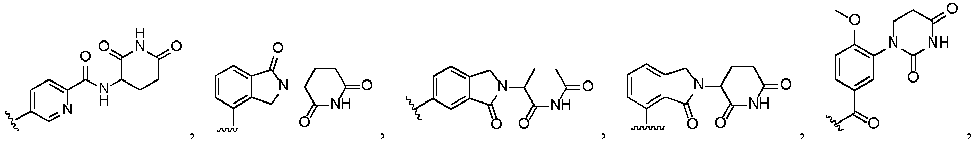

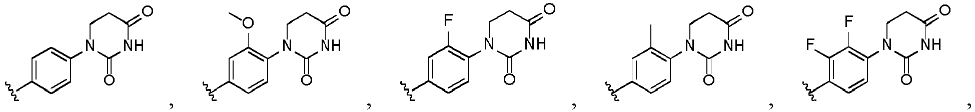

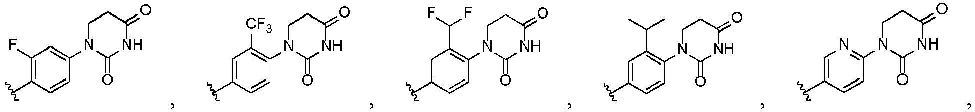

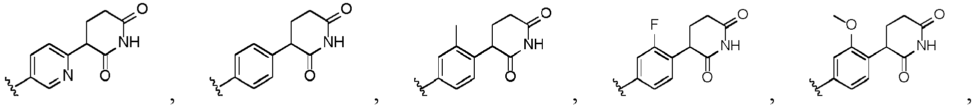

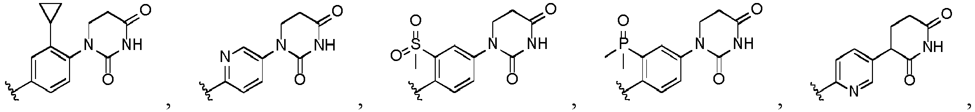

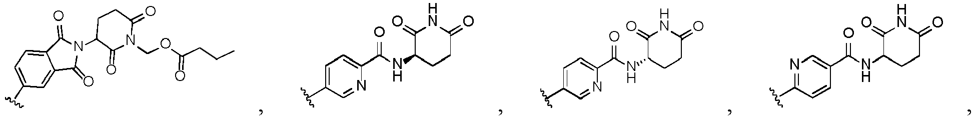

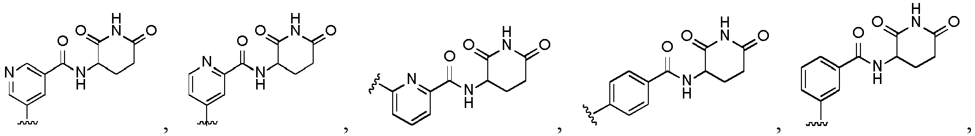

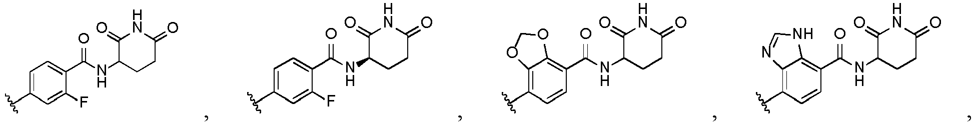

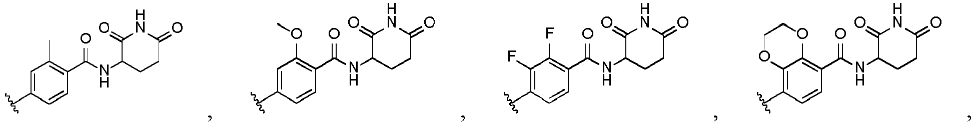

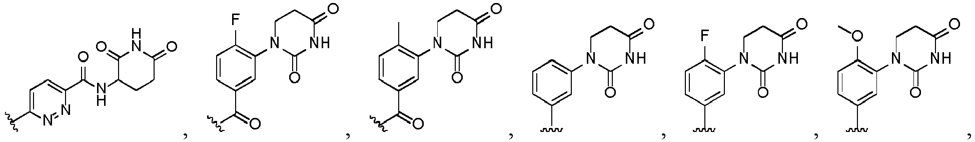

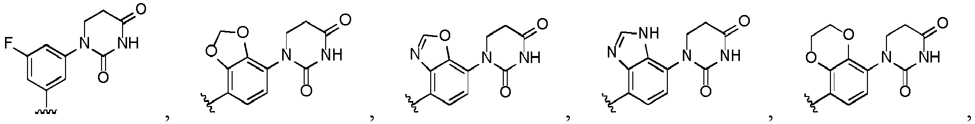

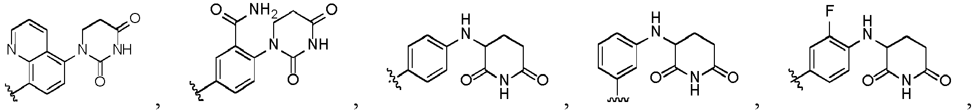

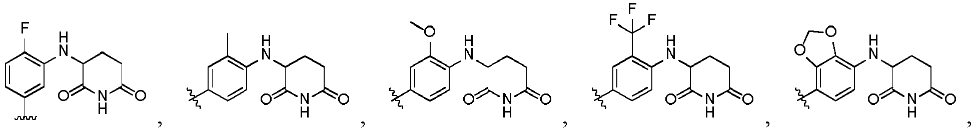

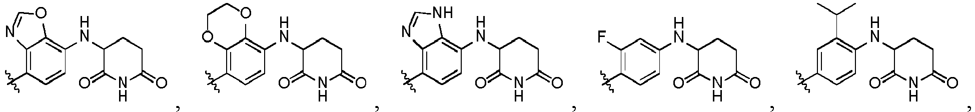

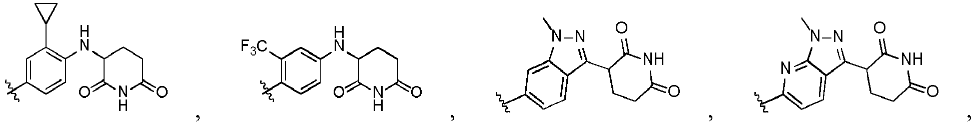

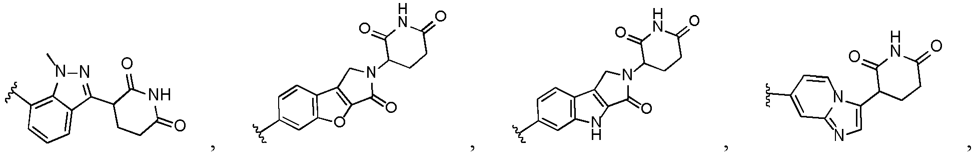

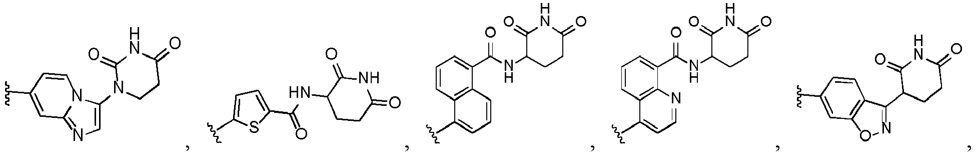

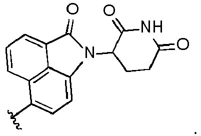

Preferably, the compound or the stereoisomer thereof, or the stereoisomer mixture thereof, or the pharmaceutically acceptable salt thereof is selected from the following compounds:

| No. | Structure | No. | Structure |
|---|---|---|---|
| 001 | 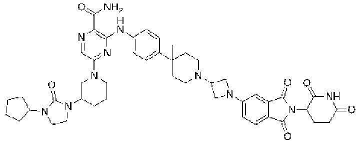 | 002 | 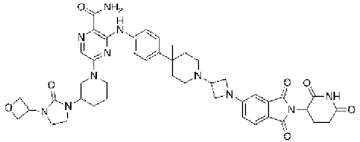 |
| 003 | 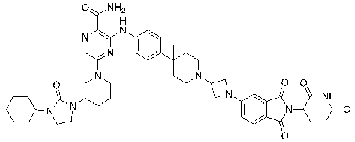 | 004 | 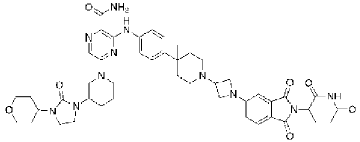 |
| 005 | 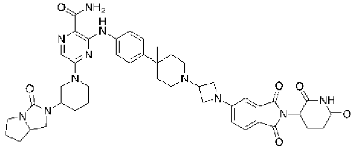 | 006 | 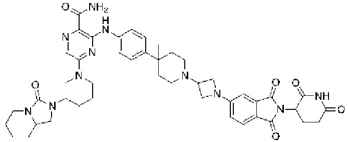 |
| 007 | 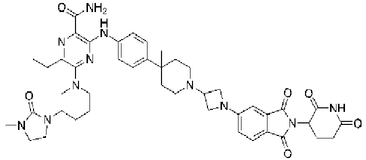 | 008 | 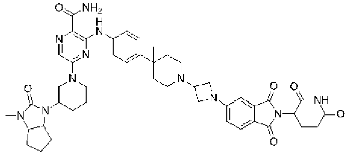 |
| 009 | 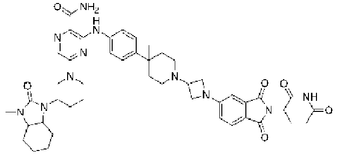 | 010 | 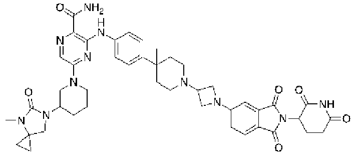 |
| 011 | 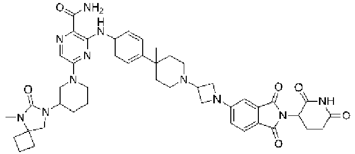 | 012 | 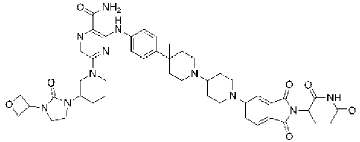 |
| 013 | 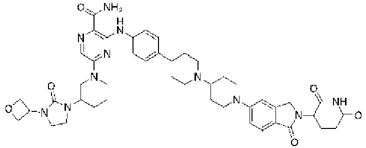 | 014 | 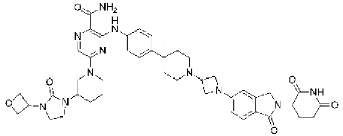 |
| 015 | 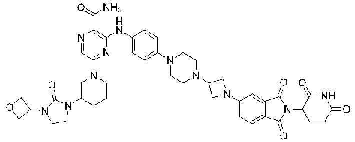 | 016 | 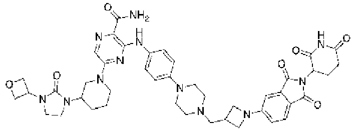 |
| 017 | 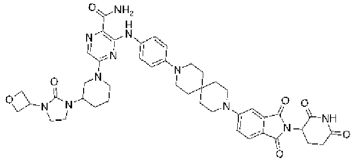 | 018 | 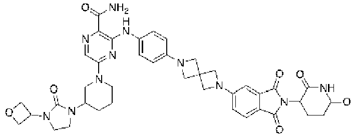 |
| 019 | 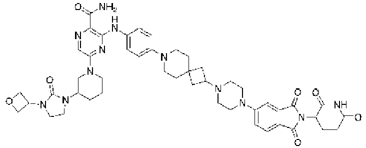 | 020 | 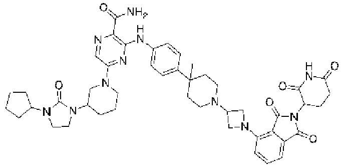 |
| 021 | 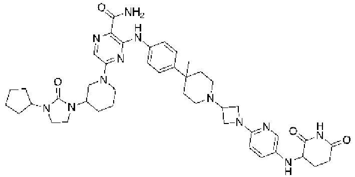 | 022 | 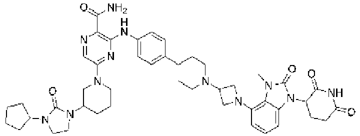 |
| 023 | 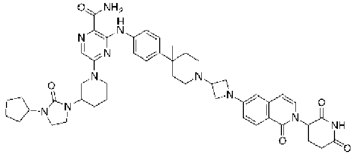 | 024 | 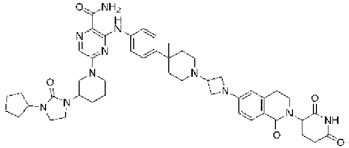 |
| 025 | 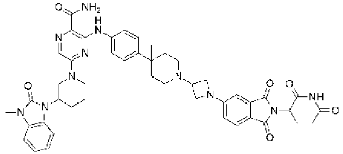 | 026 | 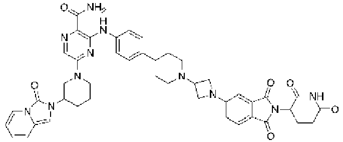 |
| 027 | 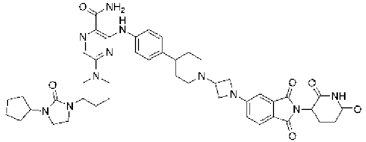 | 028 | 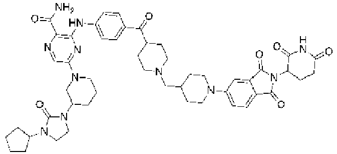 |
| 029 | 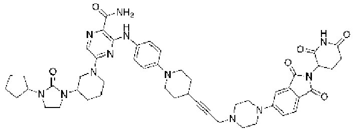 | 030 | 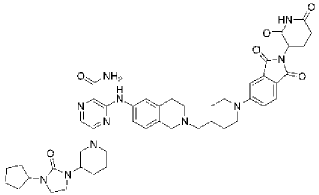 |
| 031 | 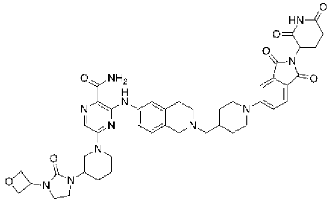 | 032 | 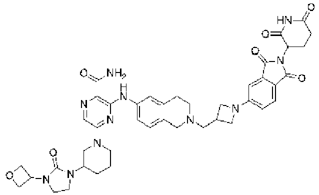 |
| 033 | 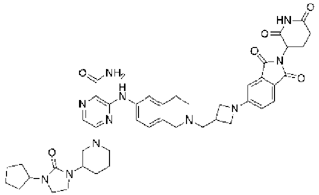 | 034 | 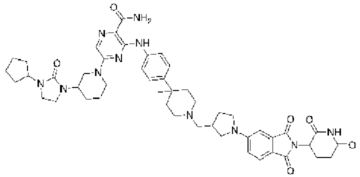 |
| 035 | 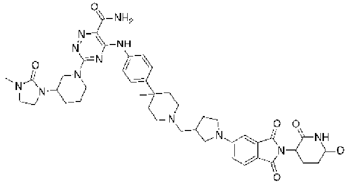 | 036 | 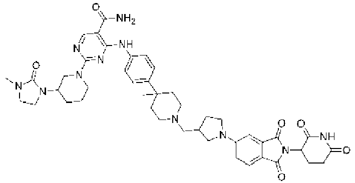 |
| 037 | 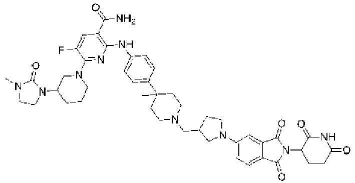 | 038 | 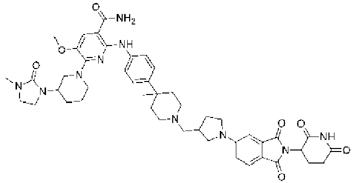 |
| 039 | 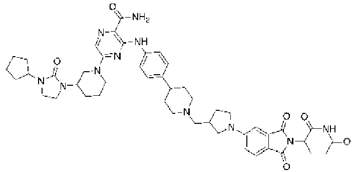 | 040 | 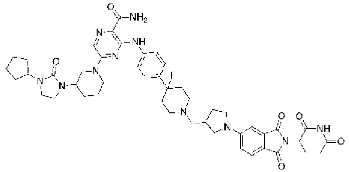 |
| 041 | 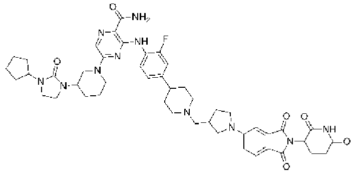 | 042 | 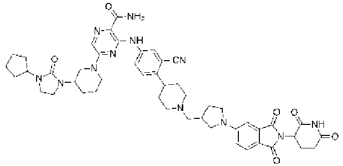 |
| 043 | 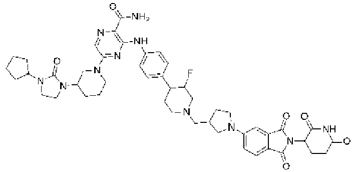 | 044 | 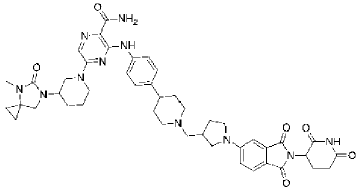 |
| 045 | 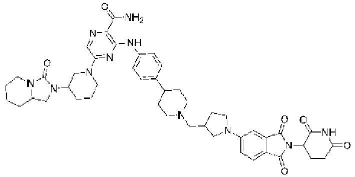 | 046 | 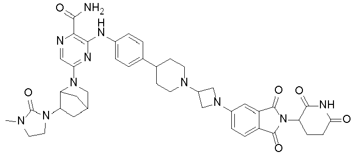 |
| 047 | 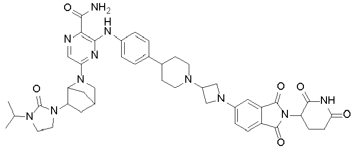 | 048 | 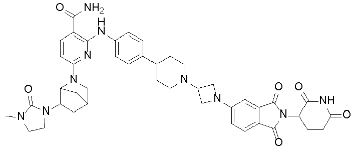 |
| 049 | 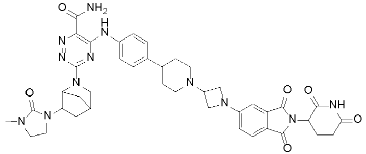 | 050 | 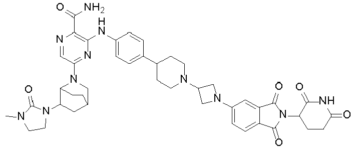 |
| 051 | 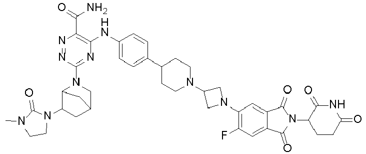 | 052 | 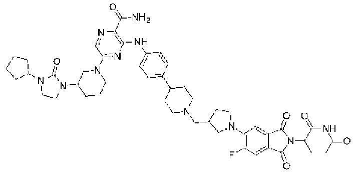 |
| 053 | 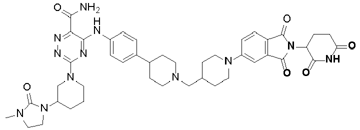 | 054 | 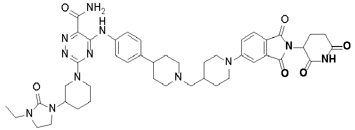 |
| 055 | 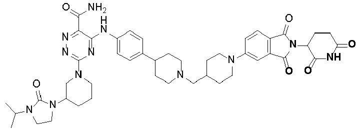 | 056 | 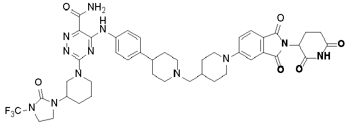 |
| 057 | 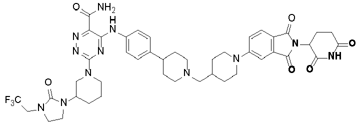 | 058 | 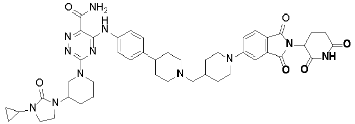 |
| 059 | 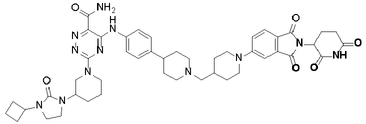 | 060 | 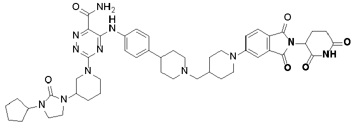 |
| 061 | 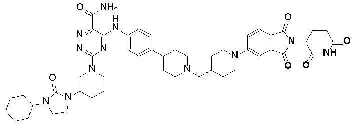 | 062 | 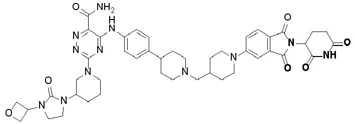 |
| 063 | 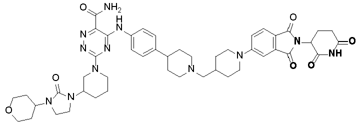 | 064 | 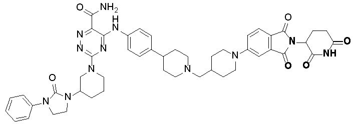 |
| 065 | 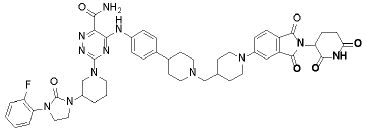 | 066 | 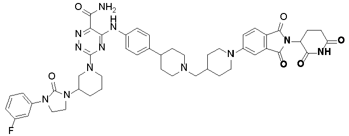 |
| 067 | 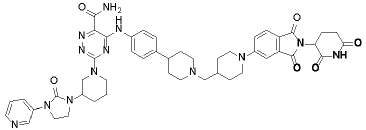 | 068 | 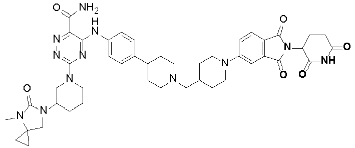 |
| 069 | 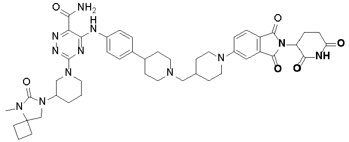 | 070 | 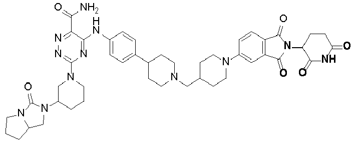 |
| 071 | 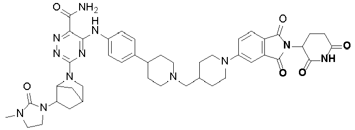 | 072 | 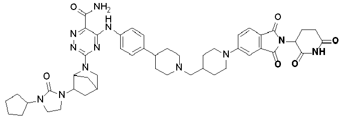 |
| 073 | 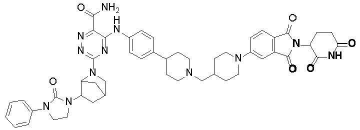 | 074 | 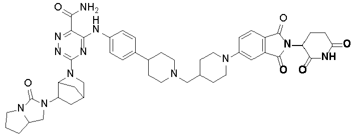 |
| 075 | 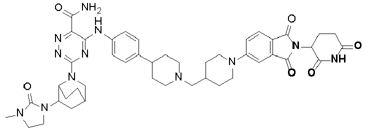 | 076 | 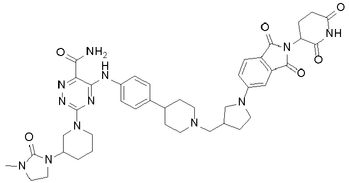 |
| 077 | 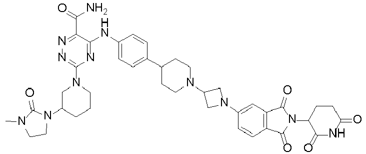 | 078 | 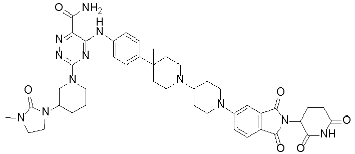 |
| 079 | 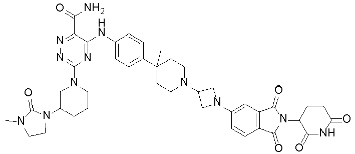 | 080 | 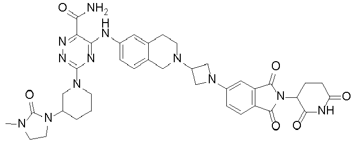 |
| 081 | 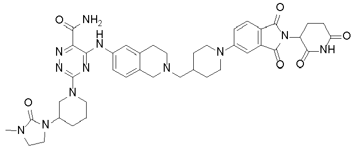 | 082 | 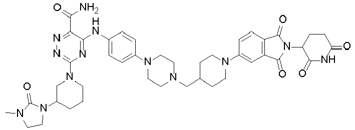 |
| 083 | 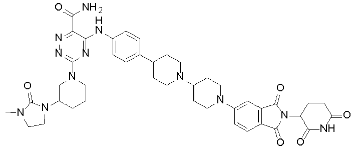 | 084 | 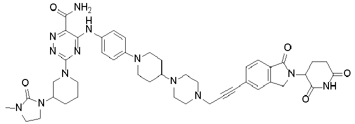 |
| 085 | 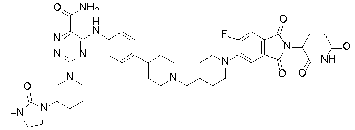 | 086 | 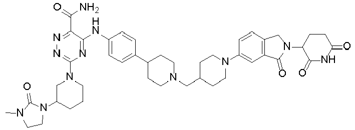 |
| 087 | 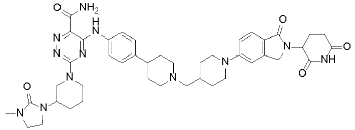 | 088 | 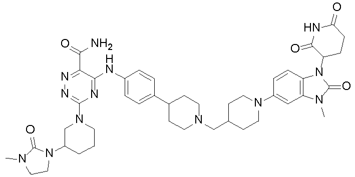 |
| 089 | 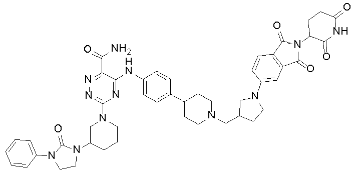 | 090 | 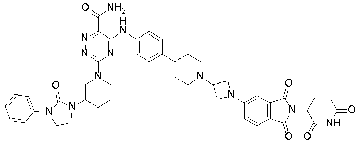 |
| 091 | 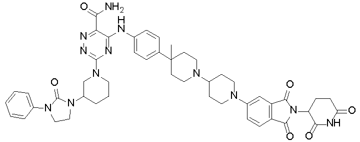 | 092 | 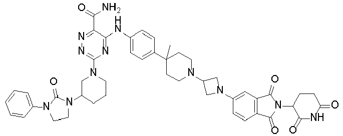 |
| 093 | 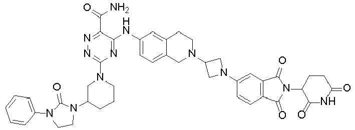 | 094 | 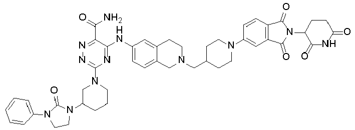 |
| 095 | 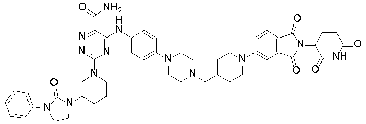 | 096 | 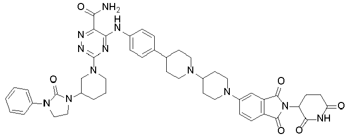 |
| 097 | 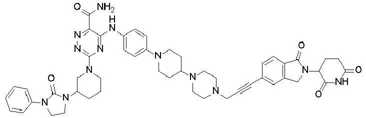 | 098 | 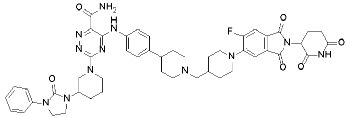 |
| 099 | 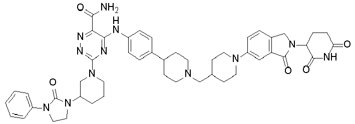 | 100 | 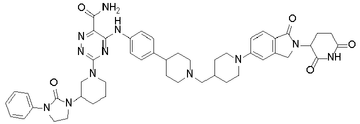 |
| 101 | 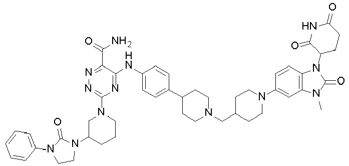 | 102 | 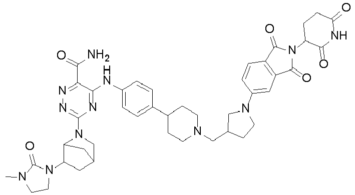 |
| 103 | 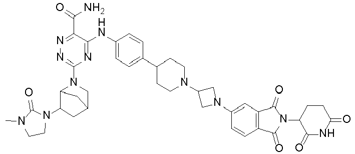 | 104 | 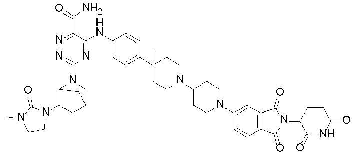 |
| 105 | 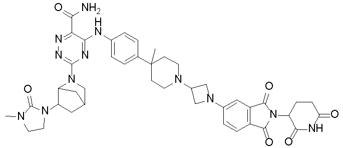 | 106 | 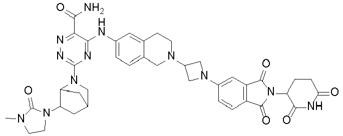 |
| 107 | 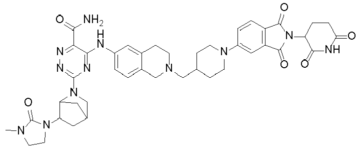 | 108 | 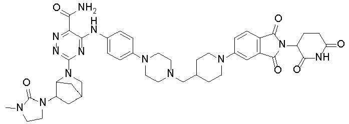 |
| 109 | 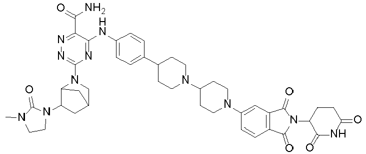 | 110 | 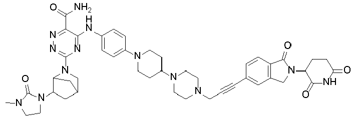 |
| 111 | 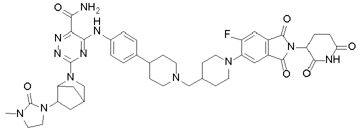 | 112 | 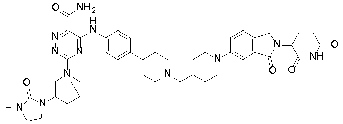 |
| 113 | 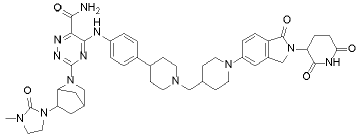 | 114 | 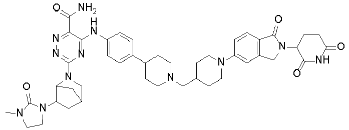 |
| 115 | 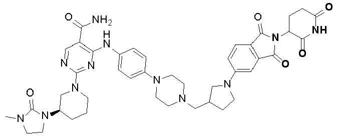 | 116 | 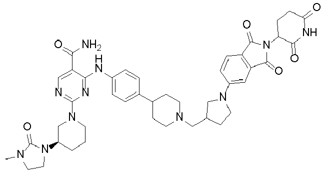 |
| 117 | 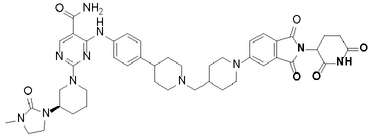 | 118 | 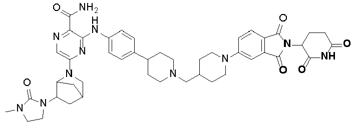 |
| 119 | 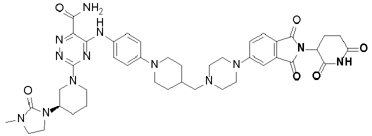 | 120 | 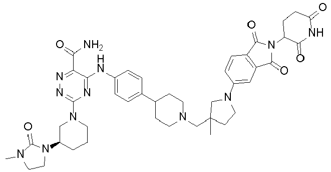 |
| 121 | 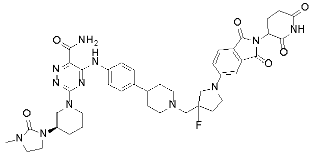 | 122 | 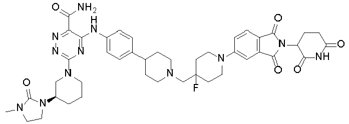 |
| 123 | 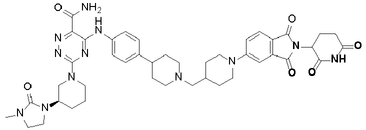 | 124 | 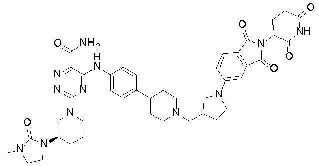 |
| 125 | 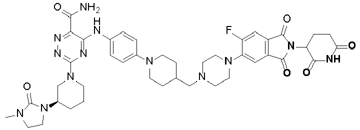 | 126 | 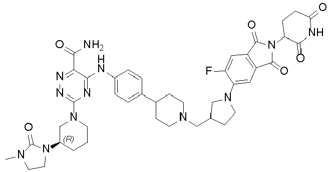 |
| 127 | 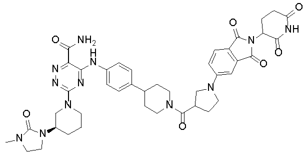 | 128 | 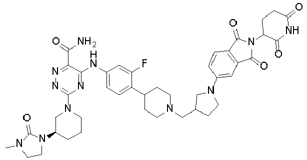 |
| 129 | 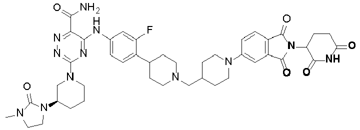 | 130 | 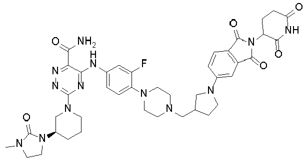 |
| 131 | 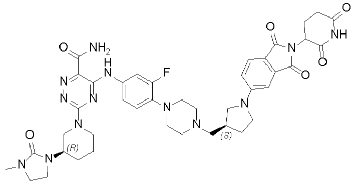 | 132 | 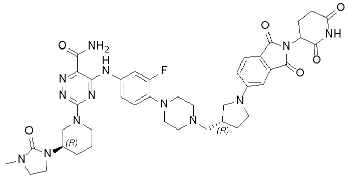 |
| 133 | 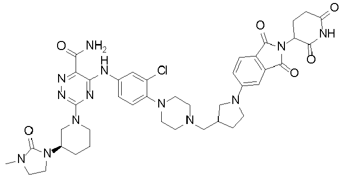 | 134 | 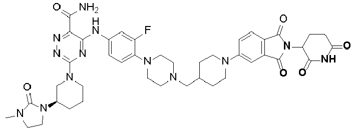 |
| 135 | 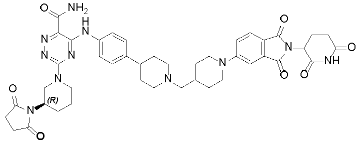 | 136 | 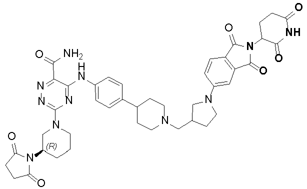 |
| 137 | 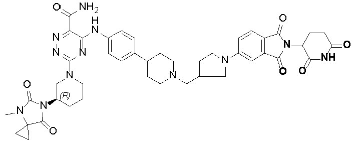 | 138 | 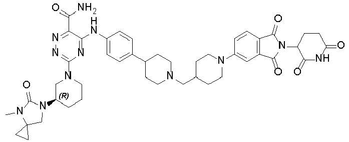 |
| 139 | 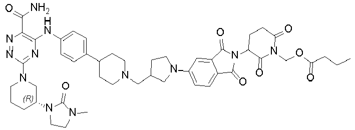 | 140 | 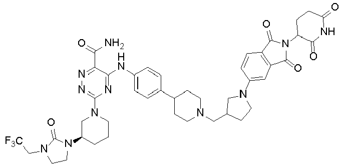 |
| 141 | 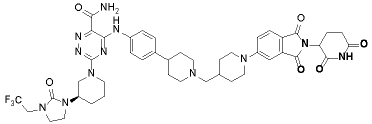 | 142 | 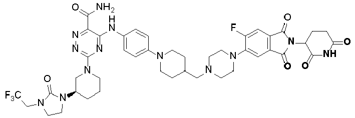 |
| 143 | 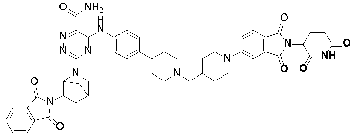 | 144 | 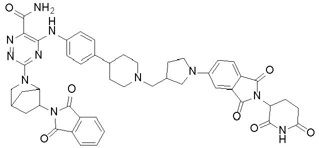 |
| 145 | 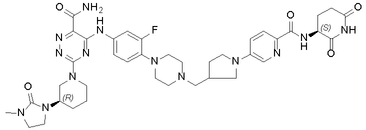 | 146 | 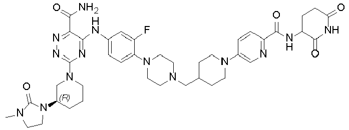 |
| 147 | 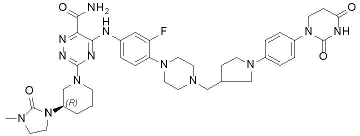 | 148 | 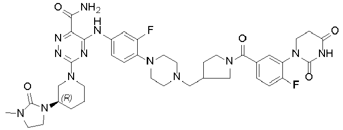 |
| 149 | 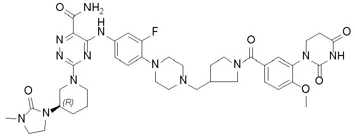 | 150 | 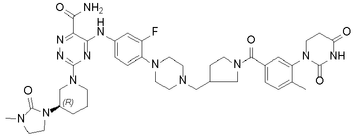 |
| 151 | 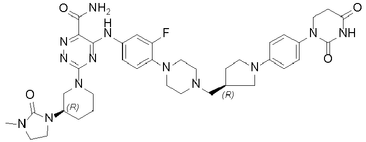 | 152 | 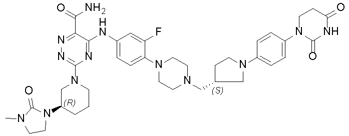 |
| 153 | 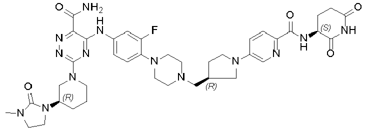 | 154 | 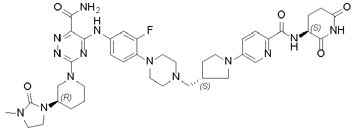 |
| 155 | 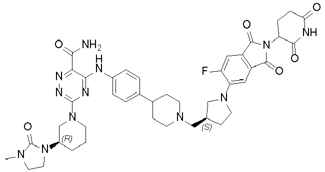 | 156 | 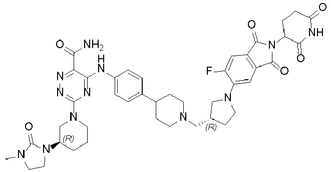 |
| 157 | 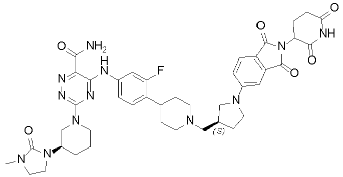 | 158 | 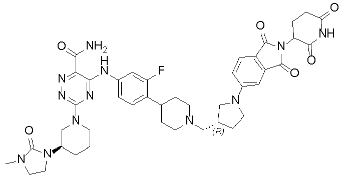 |
| 159 | 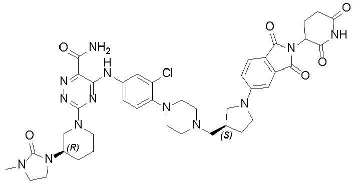 | 160 | 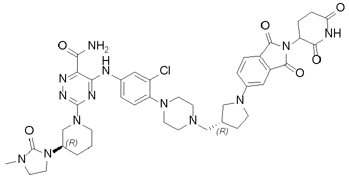 |
| 161 | 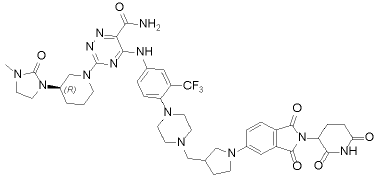 | 162 | 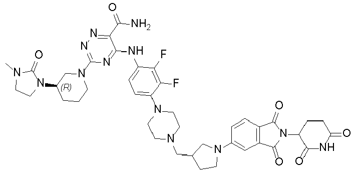 |
| 163 | 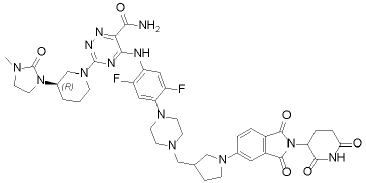 | 164 | 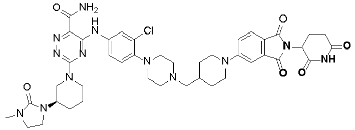 |
| 165 | 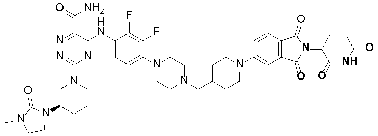 | 166 | 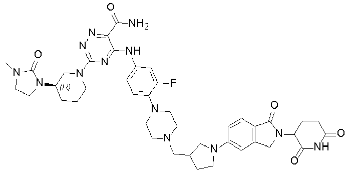 |
| 167 | 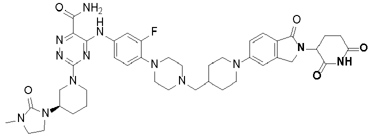 | 168 | 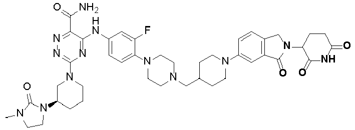 |
| 169 | 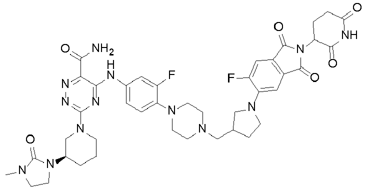 | 170 | 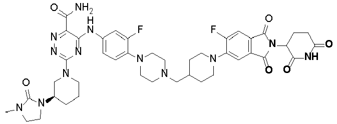 |
| 171 | 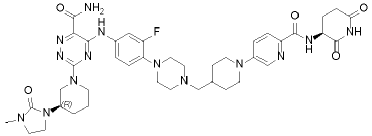 | 172 | 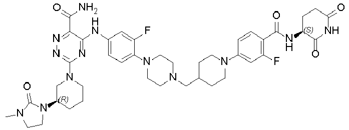 |
| 173 | 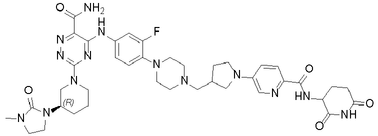 | 174 | 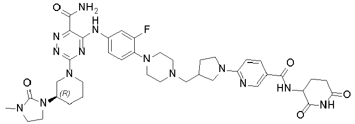 |
| 175 | 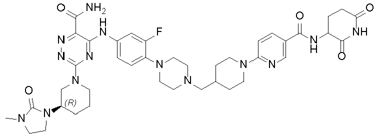 | 176 | 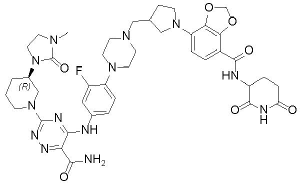 |
| 177 | 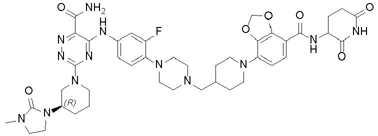 | 178 | 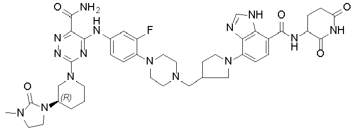 |
| 179 | 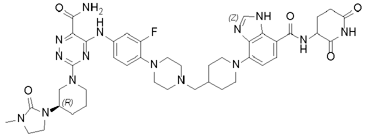 | 180 | 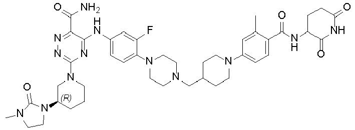 |
| 181 | 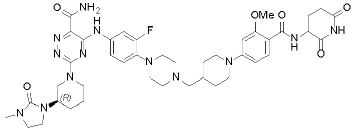 | 182 | 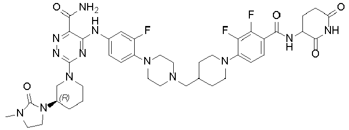 |
| 183 | 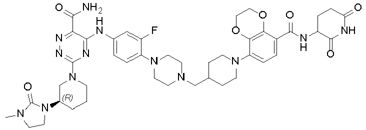 | 184 | 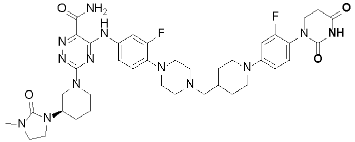 |
| 185 | 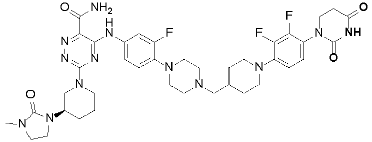 | 186 | 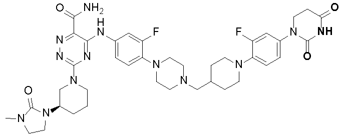 |
| 187 | 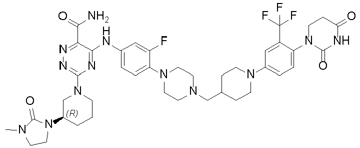 | 188 | 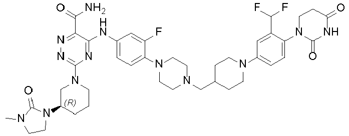 |
| 189 | 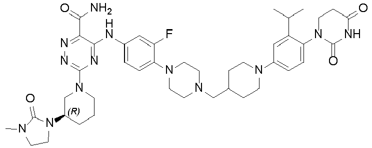 | 190 | 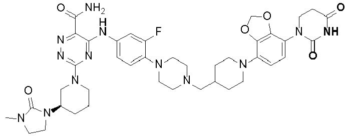 |
| 191 | 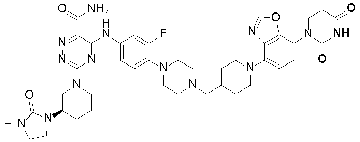 | 192 | 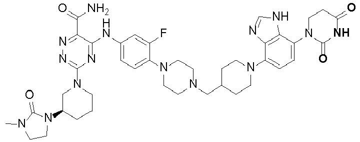 |
| 193 | 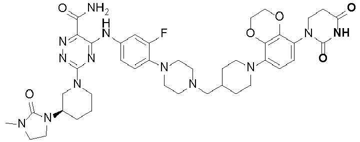 | 194 | 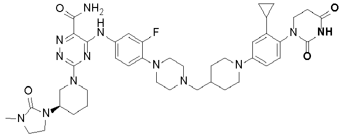 |
| 195 | 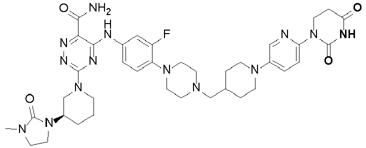 | 196 | 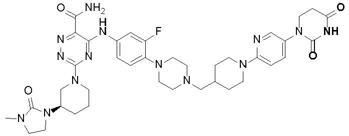 |
| 197 | 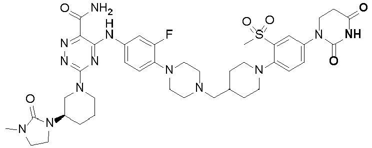 | 198 | 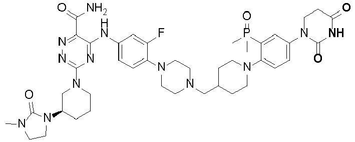 |
| 199 | 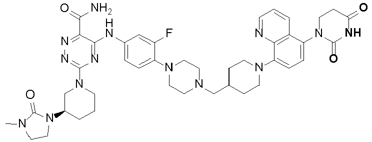 | 200 | 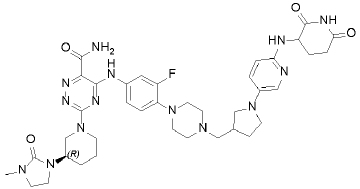 |
| 201 | 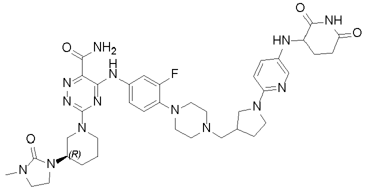 | 202 | 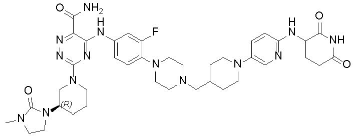 |
| 203 | 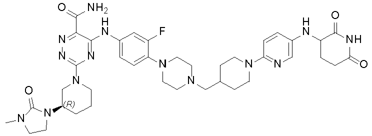 | 204 | 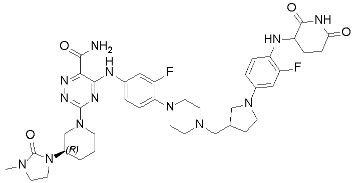 |
| 205 | 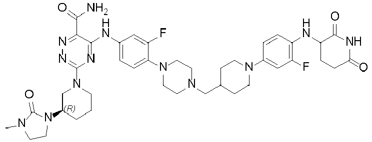 | 206 | 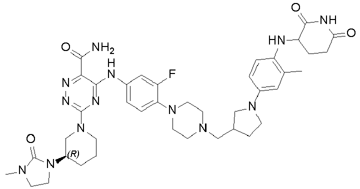 |
| 207 | 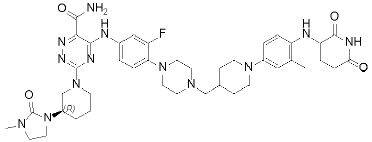 | 208 | 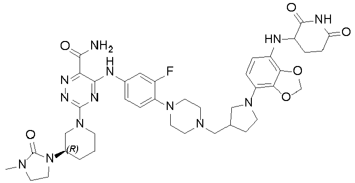 |
| 209 | 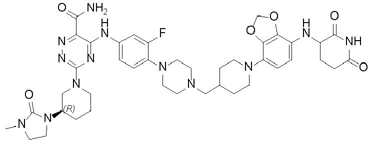 | 210 | 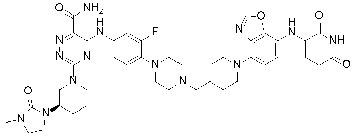 |
| 211 | 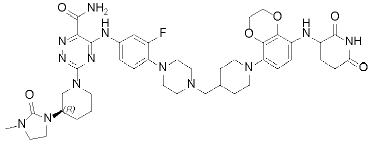 | 212 | 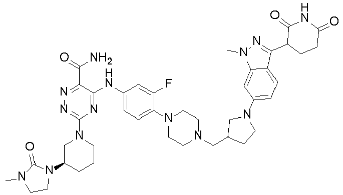 |
| 213 | 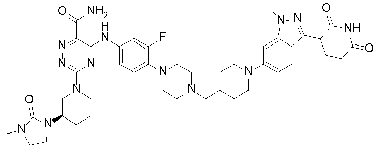 | 214 | 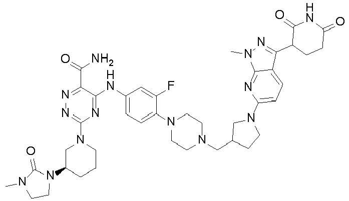 |
| 215 | 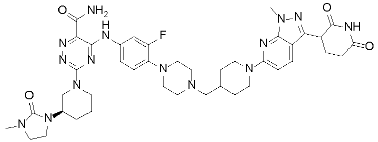 | 216 | 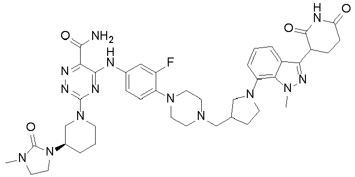 |
| 217 | 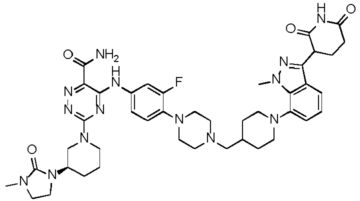 | 218 | 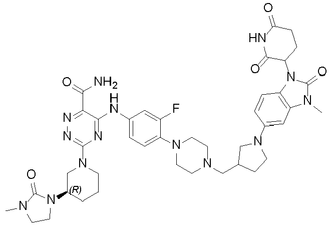 |
| 219 | 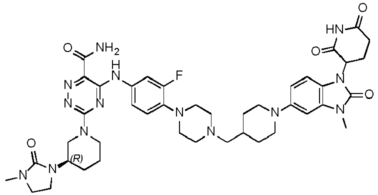 | 220 | 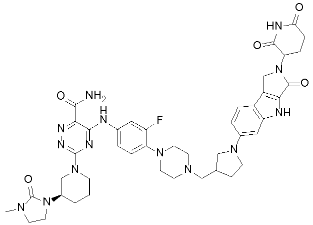 |
| 221 | 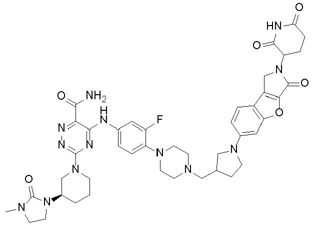 | 222 | 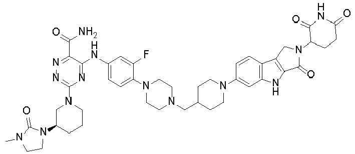 |
| 223 | 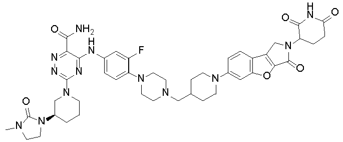 | 224 | 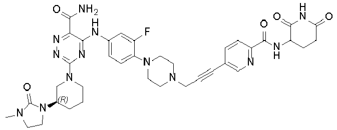 |
| 225 | 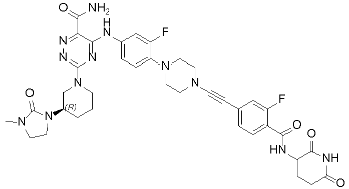 | 226 | 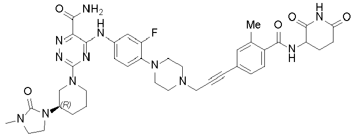 |
| 227 | 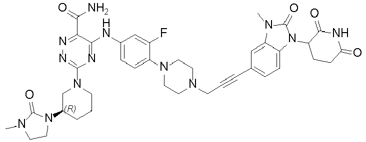 | 228 | 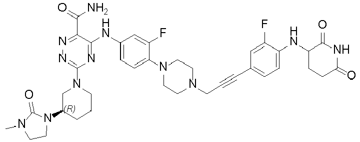 |
| 229 | 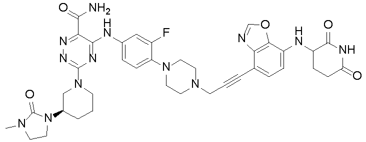 | 230 | 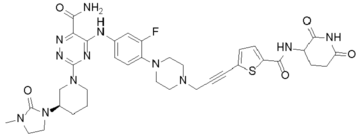 |
| 231 | 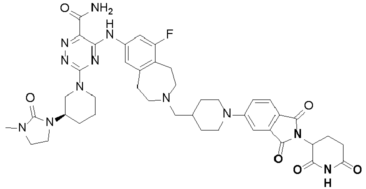 | 232 | 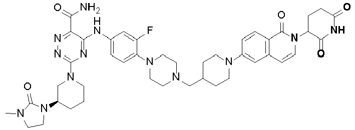 |
| 233 | 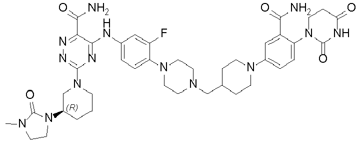 | 234 | 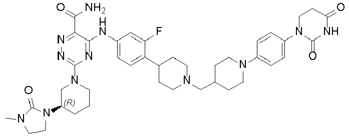 |
| 235 | 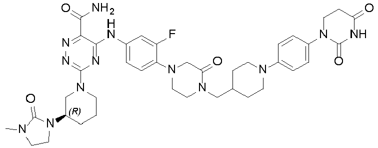 | 236 | 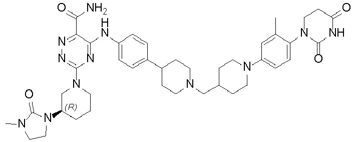 |
| 237 | 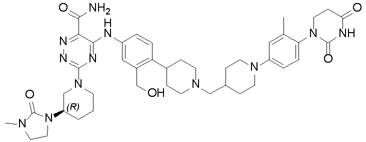 | 238 | 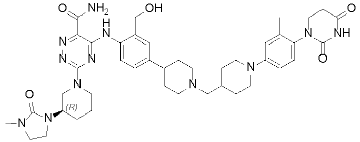 |
| 239 | 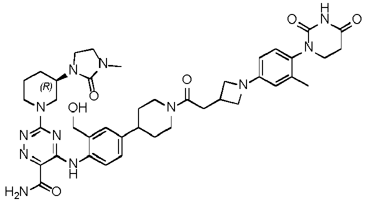 | 240 | 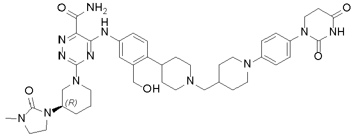 |
| 241 | 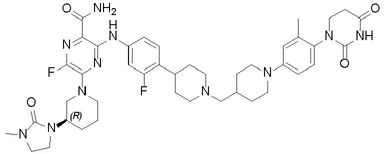 | 242 | 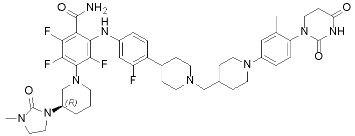 |
| 243 | 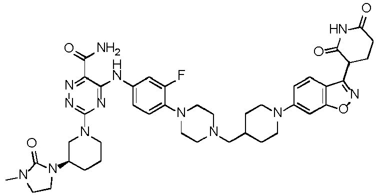 | 244 | 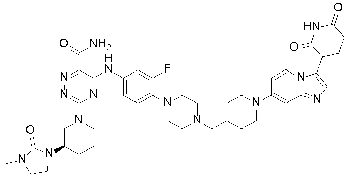 |
| 245 | 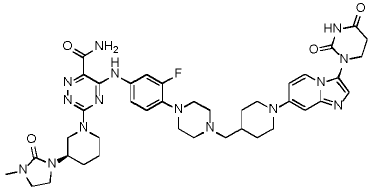 | 246 | 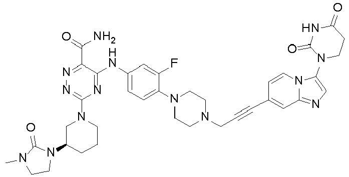 |
| 247 | 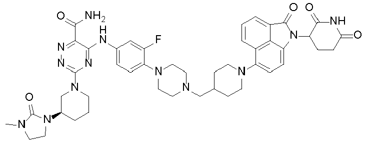 | 248 | 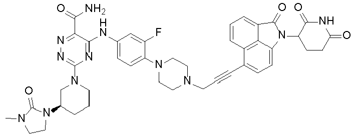 |
| 249 | 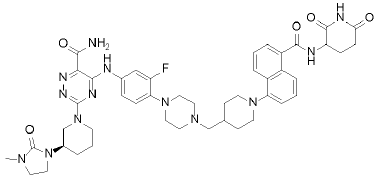 | 250 | 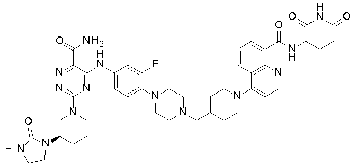 |
| 251 | 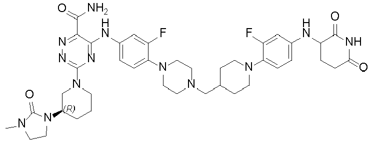 | 252 | 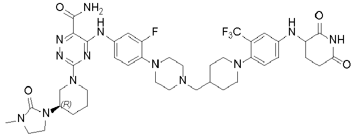 |
| 253 | 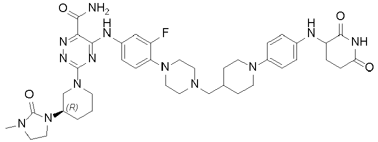 | 254 | 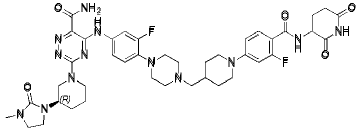 |
| 255 | 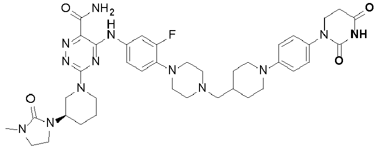 | 256 | 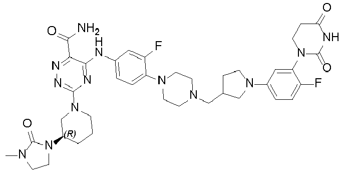 |
| 257 | 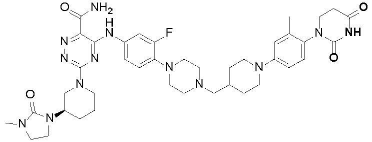 | 258 | 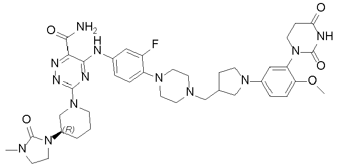 |
| 259 | 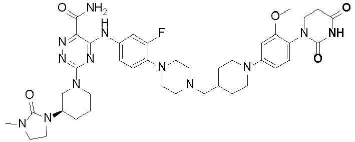 | | |

or its stereoisomer thereof, or its stereoisomer mixture thereof, or its pharmaceutically acceptable salt thereof. Preferably, the compound is the following compound:
5-(3-(3-cyclopentyl-2-oxoimidazolin-1-yl)piperidin-1-yl)-3-((4-(1-(1-(2-(2,6-dioxopiperi din-3-yl)-1,3-dioxoisoindol-5-yl)azetidin-3-yl)-4-methylpiperidin-4-yl)phenyl)amino)pyrazin-2-carboxamide **001**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(3-(oxetan-3-yl)-2-oxoimidazolin-1-yl)piperidin-1-yl)pyrazin-2 -carboxamide **002**
5-(3-(3-cyclohexyl-2-oxoimidazolin-1-yl)piperidin-1-yl)-3-((4-(1-(1-(2-(2,6-dioxopiperid in-3-yl)-1,3-dioxoisoindol-5-yl)azetidin-3-yl)-4-methylpiperidin-4-yl)phenyl)amino)pyrazin-2-ca rboxamide **003**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(2-oxo-3-(tetrahydro-2H-pyran-4-yl)imidazolin-1-yl)piperidin-1-yl)pyrazin-2-carboxamide **004**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(3-oxotetrahydro-1H-pyrrolo[1,2-c]imidazol-2(3H)-yl)piperidin -1-yl)pyrazin-2-carboxamide **005**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(3-oxohexahydroimidazo[1,5-a]pyridin-2(3H)-yl)piperidin-1-yl )pyrazin-2-carboxamide **006**
3-((4-(1-(1-(2-(2,6-dioxopiperidin-3-yl)-1,3-dioxoisoindol-5-yl)azetidin-3-yl)-4-methylpi peridin-4-yl)phenyl)amino)-6-ethyl-5-(3-(3-(3-methyl-2-oxyimidazolin-1-yl)piperidin-1-yl)pyraz in-2-carboxamide **007**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(3-methyl-2-oxohexahydrocyclopenta[d]imidazol-1(2H)-yl)pip eridin-1-yl)pyrazin-2-carboxamide **008**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(3-methyl-2-oxooctahydro-1H-benzo[d]imidazol-1-yl)piperidin -1-yl)pyrazin-2-carboxamide **009**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(4-methyl-5-oxo-4,6-diazaspirocyclo[2.4]heptan-6-yl)piperidin-1-yl)pyrazin-2-carboxamide **010**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(5-methyl-6-oxo-5,7-diazaspirocyclo[3.4]octan-7-yl)piperidin-1 -yl)pyrazin-2-carboxamide **011**
3-((4-(1'-(2-(2,6-dioxopiperidin-3-yl)-1,3-dioxoisoindol-5-yl)-4-methyl-[1,4'-bipiperidin] -4-yl)phenyl)amino)-5-(3-(3-(oxetan-3-yl)-2-oxoimidazolin-1-yl)piperidin-1-yl)pyrazin-2-carbox amide **012**
3-((4-(1'-(2-(2,6-dioxopiperidin-3-yl)-1-oxoisoindol-5-yl)-4-methyl-[1,4'-bipiperidin]-4-y l)phenyl)amino)-5-(3-(3-(oxetan-3-yl)-2-oxoimidazolin-1-yl)piperidin-1-yl)pyrazin-2-carboxami de **013**
3-((4-(1-(1-(2-(2,6-dioxopiperidin-3-yl)-1-oxoisoindol-5-yl)azetidin-3-yl)-4-methylpiperi din-4-yl)phenyl)amino)-5-(3-(3-(oxetan-3-yl)-2-oxoimidazolin-1-yl)piperidin-1-yl)pyrazin-2-car boxamide **014**
3-((4-(4-(1-(2-(2,6-dioxopiperidin-3-yl)-1,3-dioxoisoindol-5-yl)azetidin-3-yl)piperazin-1-yl)phenyl)amino)-5-(3-(3-(oxetan-3-yl)-2-oxoimidazolin-1-yl)piperidin-1-yl)pyrazin-2-carboxa mide **015**
3-((4-(4-((1-(2-(2,6-dioxopiperidin-3-yl)-1,3-dioxoisoindol-5-yl)azetidin-3-yl)methyl)pip erazin-1-yl)phenyl)amino)-5-(3-(3-(oxetan-3-yl)-2-oxoimidazolin-1-yl)piperidin-1-yl)pyrazin-2-carboxamide **016**
3-((4-(9-(2-(2,6-dioxopiperidin-3-yl)-1,3-dioxoisoindol-5-yl)-3,9-diazaspirocyclo[5.5]un dec-3-yl)phenyl)amino)-5-(3-(3-(oxetan-3-yl)-2-oxoimidazolin-1-yl)piperidin-1-yl)pyrazin-2-car boxamide **017**
3-((4-(6-(2-(2,6-dioxopiperidin-3-yl)-1,3-dioxoisoindol-5-yl)-2,6-diazaspirocyclo[3.3]he ptan-2-yl)phenyl)amino)-5-(3-(3-(oxetan-3-yl)-2-oxoimidazolin-1-yl)piperidin-1-yl)pyrazin-2-ca rboxamide **018**
3-((4-(2-(4-(2,6-dioxopiperidin-3-yl)-1,3-dioxoisoindol-5-yl)piperazin-1-yl)-7-azaspiro[3 .5]non-7-yl)phenyl)amino)-5-(3-(3-(oxetan-3-yl)-2-oxoimidazolin-1-yl)piperidin-1-yl)pyrazin-2-carboxamide **019**
5-(3-(3-cyclopentyl-2-oxoimidazolin-1-yl)piperidin-1-yl)-3-((4-(1-(1-(2-(2,6-dioxopiperi din-3-yl)-1,3-dioxoisoindol-4-yl)azetidin-3-yl)-4-methylpiperidin-4-yl)phenyl)amino)pyrazin-2-carboxamide **020**
5-(3-(3-cyclopentyl-2-oxoimidazolin-1-yl)piperidin-1-yl)-3-((4-(1-(5-((2,6-dioxopiperidi n-3-yl)amino)pyridin-2-yl)azetidin-3-yl)-4-methylpiperidin-4-yl)phenyl)amino)pyrazin-2-carbox amide **021**
5-(3-(3-cyclopentyl-2-oxoimidazolin-1-yl)piperidin-1-yl)-3-((4-(1-(1-(2,6-dioxopiperidin -3-yl)-3-methyl-2-oxo-2,3-dihydro-1H-benzo[d]imidazol-4-yl)azetidin-3-yl)-4-methylpiperidin-4 -yl)phenyl)amino)pyrazin-2-carboxamide **022**
5-(3-(3-cyclopentyl-2-oxoimidazolin-1-yl)piperidin-1-yl)-3-((4-(1-(1-(2-(2,6-dioxopiperi din-3-yl)-1-oxo-1,2-dihydroisoquinolin-6-yl)azetidin-3-yl)-4-methylpiperidin-4-yl)phenyl)amino )pyrazin-2-carboxamide **023**
5-(3-(3-cyclopentyl-2-oxoimidazolin-1-yl)piperidin-1-yl)-3-((4-(1-(1-(2-(2,6-dioxopiperi din-3-yl)-1-oxo-1,2,3,4-tetrahydroisoquinolin-6-yl)azetidin-3-yl)-4-methylpiperidin-4-yl)phenyl) amino)pyrazin-2-carboxamide **024**
3-((4-(1-(1-(2-(2,6-dioxopiperidin-3-yl)-1,3-dioxoisoindol-5-yl)azetidin-3-yl)-4-methylpi peridin-4-yl)phenyl)amino)-5-(3-(3-methyl-2-oxo-2,3-dihydro-1H-benzo[d]imidazol-1-yl)piperi din-1-yl)pyrazin-2-carboxamide **025**
3-((4-(1-(1-(2-(2,6-dioxopiperidin-3-yl)-1,3-dioxoisoindolin-5-yl)azetidin-3-yl)-4-methyl piperidin-4-yl)phenyl)amino)-5-(3-(3-oxoimidazo[1,5-*a*]pyridin-2(3*H*)-yl)piperidin-1-yl)pyrazin -2-carboxamide **026**
5-(3-(3-cyclopentyl-2-oxyimidazolin-1-yl)piperidin-1-yl)-3-((4-(1-(1-(2-(2,6-dioxopiperi din-3-yl)-1,3-dioxoisoindol-5-yl)azetidin-3-yl)piperidin-4-yl)phenyl)amino)pyrazin-2-carboxami de **027**
5-(3-(3-cyclopentyl-2-oxoimidazolin-1-yl)piperidin-1-yl)-3-((4-(1-((1-(2-(2,6-dioxopiperi din-3-yl)-1,3-dioxoisoindolin-5-yl)piperidin-4-yl)methyl)piperidin-4-formyl)phenyl)amino)pyraz in-2-carboxamide **028**
5-(3-(3-cyclopentyl-2-oxoimidazolin-1-yl)piperidin-1-yl)-3-((4-(4-(3-(4-(2-(2,6-dioxopip eri din-3 -yl)-1,3 -dioxoisoindolin-5-yl)piperazin-1 -yl)propan-1 -yne-1 -yl)piperidin-1 -yl)phenyl)am ino)pyrazin-2-carboxamide **029**
5-(3-(3-cyclopentyl-2-oxoimidazolin-1-yl)piperidin-1-yl)-3-((2-((1-(2-(2,6-dioxopiperidi n-3-yl)-1,3-dioxoisoindol-5-yl)piperidin-4-yl)methyl)-1,2,3,4-tetrahydroisoquinolin-6-yl)amino) pyrazin-2-carboxamide **030**
3-((2-((1-(2-(2,6-dioxopiperidin-3-yl)-1,3-dioxoisoindol-5-yl)piperidin-4-yl)methyl)-1,2, 3,4-tetrahydroisoquinolin-6-yl)amino)-5-(3-(3-(oxetan-3-yl)-2-oxoimidazolin-1-yl)piperidin-1-yl )pyrazin-2-carboxamide **031**
3-((2-((1-(2-(2,6-dioxopiperidin-3-yl)-1,3-dioxoisoindol-5-yl)azetidin-3-yl)methyl)-1,2,3, 4-tetrahydroisoquinolin-6-yl)amino)-5-(3-(3-(oxetan-3-yl)-2-oxyimidazolin-1-yl)piperidin-1-yl)p yrazin-2-carboxamide **032**
5-(3-(3-cyclopentyl-2-oxoimidazolin-1-yl)piperidin-1-yl)-3-((2-((1-(2-(2,6-dioxopiperidi n-3-yl)-1,3-dioxoisoindol-5-yl)azetidin-3-yl)methyl)-1,2,3,4-tetrahydroisoquinolin-6-yl)amino)p yrazin-2-carboxamide **033**
5-(3-(3-cyclopentyl-2-oxoimidazolin-1-yl)piperidin-1-yl)-3-((4-(1-((1-(2-(2,6-dioxopiperi din-3-yl)-1,3-dioxoisoindolin-5-yl)pyrrolidin-3-yl)methyl)-4-methylpiperidin-4-yl)phenyl)amino )pyrazin-2-carboxamide **034**
5-((4-(1-((1-(2-(2,6-dioxopiperidin-3-yl)-1,3-dioxoisoindolin-5-yl)pyrrolidin-3-yl)methyl )-4-methylpiperidin-4-yl)phenyl)amino)-3-(3-(3-methyl-2-oxoimidazolin-1-yl)piperidin-1-yl)-1, 2,4-triazin-6-carboxamide **035**
4-((4-(1-((1-(2-(2,6-dioxopiperidin-3-yl)-1,3-dioxoisoindolin-5-yl)pyrrolidin-3-yl)methyl )-4-methylpiperidin-4-yl)phenyl)amino)-2-(3-(3-methyl-2-oxoimidazolin-1-yl)piperidin-1-yl)pyr imidin-5-carboxamide **036**
2-((4-(1-((1-(2-(2,6-dioxopiperidin-3-yl)-1,3-dioxoisoindolin-5-yl)pyrrolidin-3-yl)methyl )-4-methylpiperidin-4-yl)phenyl)amino)-5-fluoro-6-(3-(3-methyl-2-oxoimidazolin-1-yl)piperidin -1-yl)nicotinamide **037**
2-((4-(1-((1-(2-(2,6-dioxopiperidin-3-yl)-1,3-dioxoisoindolin-5-yl)pyrrolidin-3-yl)methyl )-4-methylpiperidin-4-yl)phenyl)amino)-5-methoxy-6-(3-(3-methyl-2-oxoimidazolin-1-yl)piperi din-1-yl)nicotinamide **038**
5-(3-(3-cyclopentyl-2-oxoimidazolin-1-yl)piperidin-1-yl)-3-((4-(1-((1-(2-(2,6-dioxopiperi din-3-yl)-1,3-dioxoisoindolin-5-yl)pyrrolidin-3-yl)methyl)piperidin-4-yl)phenyl)amino)pyrazin-2-carboxamide **039**
5-(3-(3-cyclopentyl-2-oxoimidazolin-1-yl)piperidin-1-yl)-3-((4-(1-((1-(2-(2,6-dioxopiperi din-3-yl)-1,3-dioxoisoindolin-5-yl)pyrrolidin-3-yl)methyl)-4-fluoropiperidin-4-yl)phenyl)amino) pyrazin-2-carboxamide **040**
5-(3-(3-cyclopentyl-2-oxoimidazolin-1-yl)piperidin-1-yl)-3-((4-(1-((1-(2-(2,6-dioxopiperi din-3-yl)-1,3-dioxoisoindolin-5-yl)pyrrolidin-3-yl)methyl)piperidin-4-yl)-2-fluorophenyl)amino) pyrazin-2-carboxamide **041**
3-((3-cyano-4-(1-((1-(2-(2,6-dioxopiperidin-3-yl)-1,3-dioxoisoindolin-5-yl)pyrrolidin-3-y 1)methyl)piperidin-4-yl)phenyl)amino)-5-(3-(3-cyclopentyl-2-oxoimidazolin-1-yl)piperidin-1-yl) pyrazin-2-carboxamide **042**
5-(3-(3-cyclopentyl-2-oxoimidazolin-1-yl)piperidin-1-yl)-3-((4-(1-((1-(2-(2,6-dioxopiperi din-3-yl)-1,3-dioxoisoindolin-5-yl)pyrrolidin-3-yl)methyl)-3-fluoropiperidin-4-yl)phenyl)amino) pyrazin-2-carboxamide **043**
3-((4-(1-((1-(2-(2,6-dioxopiperidin-3-yl)-1,3-dioxoisoindolin-5-yl)pyrrolidin-3-yl)methyl )piperidin-4-yl)phenyl)amino)-5-(3-(4-methyl-5-oxo-4,6-diazaspiro[2.4]cycloheptan-6-yl)piperid in-1-yl)pyrazin-2-carboxamide **044**
3-((4-(1-((1-(2-(2,6-dioxopiperidin-3-yl)-1,3-dioxoisoindolin-5-yl)pyrrolidin-3-yl)methyl )piperidin-4-yl)phenyl)amino)-5-(3-(3-oxohexahydroimidazo[1,5-*a*]pyridin-2(3*H*)-yl)piperidin-1 -yl)pyrazin-2-carboxamide **045**
3-((4-(1-(1-(2-(2,6-dioxopiperidin-3-yl)-1,3-dioxoisoindolin-5-yl)azetidin-3-yl)piperidin-4-yl)phenyl)amino)-5-(-6-(3-methyl-2-oxoimidazolin-1-yl)-2-azabicyclo[2.2.1]heptan-2-yl)pyraz in-2-carboxamide **046**
3-((4-(1-(1-(2-(2,6-dioxopiperidin-3-yl)-1,3-dioxoisoindolin-5-yl)azetidin-3-yl)piperidin-4-yl)phenyl)amino)-5-(-6-(3-isopropyl-2-oxoimidazolin-1-yl)-2-azabicyclo[2.2.1]heptan-2-yl)py razin-2-carboxamide **047**
2-((4-(1-(1-(2-(2,6-dioxopiperidin-3-yl)-1,3-dioxoisoindolin-5-yl)azetidin-3-yl)piperidin-4-yl)phenyl)amino)-6-(-6-(3-methyl-2-oxoimidazolin-1-yl)-2-azabicyclo[2.2.1]heptan-2-yl)nicot inamide **048**
5-((4-(1-(1-(2-(2,6-dioxopiperidin-3-yl)-1,3-dioxoisoindolin-5-yl)azetidin-3-yl)piperidin-4-yl)phenyl)amino)-3-(-6-(3-methyl-2-oxoimidazolin-1-yl)-2-azabicyclo[2.2.1]heptan-2-yl)-1,2, 4-triazin-6-carboxamide **049**
3-((4-(1-(1-(2-(2,6-dioxopiperidin-3-yl)-1,3-dioxoisoindolin-5-yl)azetidin-3-yl)piperidin-4-yl)phenyl)amino)-5-(-6-(3-methyl-2-oxoimidazolin-1-yl)-2-azabicyclo[2.2.2]octan-2-yl)pyrazi n-2-carboxamide **050**
5-((4-(1-(1-(2-(2,6-dioxopiperidin-3-yl)-6-fluoro-1,3-dioxoisoindolin-5-yl)azetidin-3-yl)p iperidin-4-yl)phenyl)amino)-3-(-6-(3-methyl-2-oxoimidazolin-1-yl)-2-azabicyclo[2.2.1]heptan-2 -yl)-1,2,4-triazin-6-carboxamide **051**
5-(3-(3-cyclopentyl-2-oxoimidazolin-1-yl)piperidin-1-yl)-3-((4-(1-((1-(2-(2,6-dioxopiperi din-3-yl)-6-fluoro-1,3-dioxoisoindolin-5-yl)pyrrolidin-3-yl)methyl)piperidin-4-yl)phenyl)amino) pyrazin-2-carboxamide **052**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(3-methyl-2-oxoimidazolin-1-yl)piperidin-1-yl)-1,2,4-triazin-6-carboxamide **053**
5-((4-(1-((1-(2-(2,6-dioxopiperidin-3-yl)-1,3-dioxoindol-5-yl)piperidin-4-yl)methyl)piper idin-4-yl)phenyl)amino)-3-(3-(3-ethyl-2-oxyimidazolin-1-yl)piperidin-1-yl)-1,2,4-triazin-6-carbo xamide **054**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(3-isopropyl-2-oxoimidazolin-1-yl)piperidin-1-yl)-1,2,4-triazin-6-carboxamide **055**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(2-oxo-3-(trifluoromethyl)imidazolin-1-yl)piperidin-1-yl)-1,2,4 -triazin-6-carboxamide **056**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(2-oxo-3-(2,2,2-trifluoroethyl)imidazolin-1-yl)piperidin-1-yl)-1 ,2,4-triazin-6-carboxamide **057**
3-(3-(3-cyclopropyl-2-oxoimidazolin-1-yl)piperidin-1-yl)-5-((4-(1-((1-(2-(2,6-dioxopiper idin-3-yl)-1,3-dioxoisoindol-5-yl)piperidin-4-yl)methyl)piperidin-4-yl)phenyl)amino)-1,2,4-triaz in-6-carboxamide **058**
3-(3-(3-cyclobutyl-2-oxoimidazolin-1-yl)piperidin-1-yl)-5-((4-(1-((1-(2-(2,6-dioxopiperi din-3-yl)-1,3-dioxoisoindol-5-yl)piperidin-4-yl)methyl)piperidin-4-yl)phenyl)amino)-1,2,4-triazi n-6-carboxamide **059**
3-(3-(3-cyclopentyl-2-oxoimidazolin-1-yl)piperidin-1-yl)-5-((4-(1-((1-(2-(2,6-dioxopiperi din-3-yl)-1,3-dioxoisoindol-5-yl)piperidin-4-yl)methyl)piperidin-4-yl)phenyl)amino)-1,2,4-triazi n-6-carboxamide **060**
3-(3-(3-cyclohexyl-2-oxoimidazolin-1-yl)piperidin-1-yl)-5-((4-(1-((1-(2-(2,6-dioxopiperi din-3-yl)-1,3-dioxoisoindol-5-yl)piperidin-4-yl)methyl)piperidin-4-yl)phenyl)amino)-1,2,4-triazi n-6-carboxamide **061**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(3-(oxetan-3-yl)-2-oxoimidazolin-1-yl)piperidin-1-yl)-1,2,4-tria zin-6-carboxamide **062**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(2-oxo-3-(tetrahydro-2H-pyran-4-yl)imidazolin-1-yl)piperidin-1-yl)-1,2,4-triazin-6-carboxamide **063**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(2-oxo-3-phenylimidazolin-1-yl)piperidin-1-yl)-1,2,4-triazin-6-carboxamide **064**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(3-(2-fluorophenyl)-2-oxoimidazolin-1-yl)piperidin-1-yl)-1,2,4-triazin-6-carboxamide **065**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(3-(3-fluorophenyl)-2-oxoimidazolin-1-yl)piperidin-1-yl)-1,2,4-triazin-6-carboxamide **066**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(2-oxo-3-(pyridin-3-yl)imidazolin-1-yl)piperidin-1-yl)-1,2,4-tri azin-6-carboxamide **067**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(4-methyl-5-oxo-4,6-diazaspirocyclo[2.4]heptan-6-yl)piperidin-1-yl)-1,2,4-triazin-6-carboxamide **068**
5-((4-(1-((1-(2-(2,6-dioxopiperidin-3-yl)-1,3-dioxoisoindol-5-yl)piperidin-4-yl)methyl)pi peri din-4-yl)phenyl)amino)-3-(3-(5-methyl-6-oxo-5,7-diazaspirocyclo[3.4]octan-7-yl)piperi din-1 -yl)-1,2,4-triazin-6-carboxamide **069**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(3-(3-oxytrihydro-1H-pyrrolo[1,2-c]imidazol-2(3H)-yl)piperidin-1 -yl)-1,2,4-triazin-6-carboxamide **070**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(6-(3-methyl-2-oxoimidazolin-1-yl)-2-azabicyclo[2.2.1]heptan-2-y 1)-1,2,4-triazin-6-carboxamide **071**
3-(6-(3-cyclopentyl-2-oxoimidazolin-1-yl)-2-azabicyclo[2.2.1]heptan-2-yl)-5-((4-(1-((1-( 2-(2,6-dioxopiperidin-3-yl)-1,3-dioxoisoindol-5-yl)piperidin-4-yl)methyl)piperidin-4-yl)phenyl) amino)-1,2,4-triazin-6-carboxamide **072**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(6-(2-oxo-3-phenylimidazolin-1-yl)-2-azabicyclo[2.2.1]heptan-2-y 1)-1,2,4-triazin-6-carboxamide **073**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(6-(3-oxotetrahydro-1H-pyrrolo[1,2-c]imidazol-2(3H)-yl)-2-azabi cyclo[2.2.1]heptan-2-yl)-1,2,4-triazin-6-carboxamide **074**
5-((4-(1-((1-(2-(2,6-dioxopiperidin-3-yl)-1,3-dioxoisoindol-5-yl)piperidin-4-yl)methyl)pi peridin-4-yl)phenyl)amino)-3-(6-(3-methyl-2-oxoimidazolin-1-yl)-2-azabicyclo[2.2.2]octan-2-yl )-1,2,4-triazin-6-carboxamide **075**
5-((4-(1-((1-(2-(2,6-dioxopiperidin-3-yl)-1,3-dioxoisooctyl-5-yl)pyrrolidin-3-yl)methyl)p iperidin-4-yl)phenyl)amino)-3-(3-(3-methyl-2-oxoimidazolin-1-yl)piperidin-1-yl)-1,2,4-triazin-6 -carboxamide **076**
5-((4-(1-(1-(2-(2,6-dioxopiperidin-3-yl)-1,3-dioxoisoindol-5-yl)azetidin-3-yl)piperidin-4-yl)phenyl)amino)-3-(3-(3-methyl-2-oxoimidazolin-1-yl)piperidin-1-yl)-1,2,4-triazin-6-carboxam ide **077**
5-((4-(1'-(2-(2,6-dioxopiperidin-3-yl)-1,3-dioxoisoindol-5-yl)-4-methyl-[1,4'-bipiperidin] -4-yl)phenyl)amino)-3-(3-(3-methyl-2-oxoimidazolidin-1-yl)piperidin-1-yl)-1,2,4-triazin-6-carbo xamide **078**
5-((4-(1-(1-(2-(2,6-dioxopiperidin-3-yl)-1,3-dioxoisoindol-5-yl)azetidin-3-yl)-4-methylpi peridin-4-yl)phenyl)amino)-3-(3-(3-methyl-2-oxoimidazolin-1-yl)piperidin-1-yl)-1,2,4-triazin-6-carboxamide **079**
5-((2-(1-(2,6-dioxopiperidin-3-yl)-1,3-dioxoisoindol-5-yl)azetidin-3-yl)-1,2,3,4-tetrahydr oisoquinolin-6-yl)amino)-3-(3-(3-methyl-2-oxoimidazolin-1-yl)piperidin-1-yl)-11,2,4-triazin-6-ca rboxamide **080**
5-((2-((1-(2-(2,6-dioxopiperidin-3-yl)-1,3-dioxoisoindol-5-yl)piperidin-4-yl)methyl)-1,2, 3,4-tetrahydroisoquinolin-6-yl)amino)-3-(3-(3-methyl-2-oxoimidazolin-1-yl)piperidin-1-yl)-1,2, 4-triazin-6-carboxamide **081**
5-((4-(4-((1-(2-(2,6-dioxopiperidin-3-yl)-1,3-dioxoisoindol-5-yl)piperidin-4-yl)methyl)pi perazin-1-yl)phenyl)amino)-3-(3-(3-methyl-2-oxoimidazolin-1-yl)piperidin-1-yl)-1,2,4-triazin-6-carboxamide **082**
5-((4-(1'-(2-(2,6-dioxopiperidin-3-yl)-1,3-dioxoisoindol-5-yl)-[1,4'-bipiperidin]-4-yl)phe nyl)amino)-3-(3-(3-methyl-2-oxoimidazolin-1-yl)piperidin-1-yl)-1,2,4-triazin-6-carboxamide **083**
5-((4-(4-(3-(2-(2,6-dioxopiperidin-3-yl)-1-oxoisoindol-5-yl)propan-2-yn-1-yl)piperazin-1 -yl)piperidin-1-yl)phenyl)amino)-3-(3-(3-methyl-2-oxoimidazolin-1-yl)piperidin-1-yl)-11,2,4-tria zin-6-carboxamide **084**
5-((4-(1-((1-(2-(2,6-dioxopiperidin-3-yl)-6-fluoro-1,3-dioxoisoindol-5-yl)piperidin-4-yl) methyl)piperidin-4-yl)phenyl)amino)-3-(3-(3-methyl-2-oxoimidazolin-1-yl)piperidin-1-yl)-1,2,4-triazin-6-carboxamide **085**
5-((4-(1-((1-(2-(2,6-dioxopiperidin-3-yl)-3-oxoisoindol-5-yl)piperidin-4-yl)methyl)piperi din-4-yl)phenyl)amino)-3-(3-(3-methyl-2-oxoimidazolin-1-yl)piperidin-1-yl)-1,2,4-triazin-6-carb oxamide **086**
5-((4-(1-((1-(2-(2,6-dioxopiperidin-3-yl)-1-oxoisoindol-5-yl)piperidin-4-yl)methyl)piperi din-4-yl)phenyl)amino)-3-(3-(3-methyl-2-oxyimidazolin-1-yl)piperidin-1-yl)-1,2,4-triazin-6-carb oxamide **087**
5-((4-(1-((1-(1-(2,6-dioxopiperidin-3-yl)-3-methyl-2-oxo-2,3-dihydro-1H-benzo[d]imida zol-5-yl)piperidin-4-yl)methyl)piperidin-4-yl)phenyl)amino)-3-(3-(3-methyl-2-oxoimidazolin-1-yl)piperidin-1-yl)-1,2,4-triazin-6-carboxamide **088**
5-((4-(1-((1-(2-(2,6-dioxopiperidin-3-yl)-1,3-dioxoisoindol-5-yl)pyrrolidin-3-yl)methyl)p iperidin-4-yl)phenyl)amino)-3-(3-(2-oxo-3-phenylimidazolin-1-yl)piperidin-1-yl)-1,2,4-triazin-6 -carboxamide **089**
5-((4-(1-(1-(2-(2,6-dioxopiperidin-3-yl)-1,3-dioxoisoindol-5-yl)azetidin-3-yl)piperidin-4-yl)phenyl)amino)-3-(3-(2-oxo-3-phenylimidazolin-1-yl)piperidin-1-yl)-1,2,4-triazin-6-carboxami de **090**
5-((4-(1'-(2-(2,6-dioxopiperidin-3-yl)-1,3-dioxoisoindol-5-yl)-4-methyl-[1,4'-bipiperidin] -4-yl)phenyl)amino)-3-(3-(2-oxo-3-phenylimidazolin-1-yl)piperidin-1-yl)-1,2,4-triazin-6-carbox amide **091**
5-((4-(1-(1-(2-(2,6-dioxopiperidin-3-yl)-1,3-dioxoisoindol-5-yl)azetidin-3-yl)-4-methylpi peridin-4-yl)phenyl)amino)-3-(3-(2-oxo-3-phenylimidazolin-1-yl)piperidin-1-yl)-1,2,4-triazin-6-carboxamide **092**
5-((2-(1-(2,6-dioxopiperidin-3-yl)-1,3-dioxoisoindol-5-yl)azetidin-3-yl)-1,2,3,4-tetrahydr oisoquinolin-6-yl)amino)-3-(3-(2-oxo-3-phenylimidazolin-1-yl)piperidin-1-yl)-11,2,4-triazin-6-ca rboxamide **093**
5-((2-((1-(2-(2,6-dioxopiperidin-3-yl)-1,3-dioxoisoindol-5-yl)piperidin-4-yl)methyl)-1,2, 3,4-tetrahydroisoquinolin-6-yl)amino)-3-(3-(2-oxo-3-phenylimidazolin-1-yl)piperidin-1-yl)-1,2,4 -triazin-6-carboxamide **094**
5-((4-(4-((1-(2-(2,6-dioxopiperidin-3-yl)-1,3-dioxoisoindol-5-yl)piperidin-4-yl)methyl)pi perazin-1-yl)phenyl)amino)-3-(3-(2-oxo-3-phenylimidazolin-1-yl)piperidin-1-yl)-1,2,4-triazin-6-carboxamide **095**
5-((4-(1'-(2-(2,6-dioxopiperidin-3-yl)-1,3-dioxoisoindol-5-yl)-[1,4'-bipiperidin]-4-yl)phe nyl)amino)-3-(3-(2-oxo-3-phenylimidazolin-1-yl)piperidin-1-yl)-1,2,4-triazin-6-carboxamide **096**
5-((4-(4-(3-(2-(2,6-dioxopiperidin-3-yl)-1-oxoisoquinolin-5-yl)propan-2-yn-1-yl)piperazi n-1-yl)piperidin-1-yl)phenyl)amino)-3-(3-(2-oxo-3-phenylimidazolin-1-yl)piperidin-1-yl)-1,2,4-t riazin-6-carboxamide **097**
5-((4-(1-((1-(2-(2,6-dioxopiperidin-3-yl)-6-fluoro-1,3-dioxoisoindol-5-yl)piperidin-4-yl) methyl)piperidin-4-yl)phenyl)amino)-3-(3-(2-oxo-3-phenylimidazolin-1-yl)piperidin-1-yl)-1,2,4-triazin-6-carboxamide **098**
5-((4-(1-((1-(2-(2,6-dioxopiperidin-3-yl)-3-oxoisoindol-5-yl)piperidin-4-yl)methyl)piperi din-4-yl)phenyl)amino)-3-(3-(2-oxo-3-phenylimidazolin-1-yl)piperidin-1-yl)-1,2,4-triazin-6-carb oxamide **099**
5-((4-(1-((1-(2-(2,6-dioxopiperidin-3-yl)-1-oxoisoindol-5-yl)piperidin-4-yl)methyl)piperi din-4-yl)phenyl)amino)-3-(3-(2-oxo-3-phenylimidazolin-1-yl)piperidin-1-yl)-1,2,4-triazin-6-carb oxamide **100**
5-((4-(1-((1-(1-(2,6-dioxopiperidin-3-yl)-3-methyl-2-oxo-2,3-dihydro-1H-benzo[d]imida zol-5-yl)piperidin-4-yl)methyl)piperidin-4-yl)phenyl)amino)-3-(3-(2-oxo-3-phenylimidazol-1-yl) piperidin-1-yl)-1,2,4-triazin-6-carboxamide **101**
5-((4-(1-((1-(2-(2,6-dioxopiperidin-3-yl)-1,3-dioxoisoindol-5-yl)pyrrolidin-3-yl)methyl)p iperidin-4-yl)phenyl)amino)-3-(6-(3-methyl-2-oxoimidazolin-1-yl)-2-azabicyclo[2.2.1]heptan-2-yl)-1,2,4-triazin-6-carboxamide **102**
5-((4-(1-(1-(2-(2,6-dioxopiperidin-3-yl)-1,3-dioxoisoindol-5-yl)azetidin-3-yl)piperidin-4-yl)phenyl)amino)-3-(6-(3-methyl-2-oxoimidazolin-1-yl)-2-azabicyclo[2.2.1]heptan-2-yl)-1,2,4-tr iazin-6-carboxamide **103**
5-((4-(1'-(2-(2,6-dioxopiperidin-3-yl)-1,3-dioxoisoindol-5-yl)-4-methyl-[1,4'-bipiperidin] -4-yl)phenyl)amino)-3-(6-(3-methyl-2-oxoimidazolin-1-yl)-2-azabicyclo[2.2.1]heptan-2-yl)-1,2, 4-triazin-6-carboxamide **104**
5-((4-(1-(1-(2-(2,6-dioxopiperidin-3-yl)-1,3-dioxoisoindol-5-yl)azetidin-3-yl)-4-methylpi peridin-4-yl)phenyl)amino)-3-(6-(3-methyl-2-oxoimidazolin-1-yl)-2-azabicyclo[2.2.1]heptan-2-y 1)-1,2,4-triazin-6-carboxamide **105**
5-((2-(1-(2-(2,6-dioxopiperidin-3-yl)-1,3-dioxoisoindol-5-yl)azetidin-3-yl)-1,2,3,4-tetrah ydroisoquinolin-6-yl)amino)-3-(6-(3-methyl-2-oxoimidazolin-1-yl)-2-azabicyclo[2.2.1]heptan-2-yl)-1,2,4-triazin-6-carboxamide **106**
5-((2-((1-(2-(2,6-dioxopiperidin-3-yl)-1,3-dioxoisoindol-5-yl)piperidin-4-yl)methyl)-1,2, 3,4-tetrahydroisoquinolin-6-yl)amino)-3-(6-(3-methyl-2-oxoimidazolin-1-yl)-2-azabicyclo[2.2.1] heptan-2-yl)-1,2,4-triazin-6-carboxamide **107**
5-((4-(4-((1-(2-(2,6-dioxopiperidin-3-yl)-1,3-dioxoisoindol-5-yl)piperidin-4-yl)methyl)pi perazin-1-yl)phenyl)amino)-3-(6-(3-methyl-2-oxoimidazolin-1-yl)-2-azabicyclo[2.2.1]heptan-2-yl)-1,2,4-triazin-6-carboxamide **108**
5-((4-(1'-(2-(2,6-dioxopiperidin-3-yl)-1,3-dioxoisoindol-5-yl)-[1,4'-bipiperidin]-4-yl)phe nyl)amino)-3-(6-(3-methyl-2-oxoimidazolin-1-yl)-2-azabicyclo[2.2.1]heptan-2-yl)-1,2,4-triazin-6-carboxamide **109**
5-((4-(4-(3-(2-(2,6-dioxopiperidin-3-yl)-1-oxoisoquinolin-5-yl)propan-2-yn-1-yl)piperazi n-1-yl)piperidin-1-yl)phenyl)amino)-3-(6-(3-methyl-2-oxoisoimidazol-1-yl)-2-azabicyclo[2.2.1] heptan-2-yl)-1,2,4-triazin-6-carboxamide **110**
5-((4-(1-((1-(2-(2,6-dioxopiperidin-3-yl)-6-fluoro-1,3-dioxoisoindol-5-yl)piperidin-4-yl) methyl)piperidin-4-yl)phenyl)amino)-3-(6-(3-methyl-2-oxoimidazolin-1-yl)-2-azabicyclo[2.2.1] heptan-2-yl)-1,2,4-triazin-6-carboxamide **111**
5-((4-(1-((1-(2-(2,6-dioxopiperidin-3-yl)-3-oxoisoindol-5-yl)piperidin-4-yl)methyl)piperi din-4-yl)phenyl)amino)-3-(6-(3-methyl-2-oxoimidazolin-1-yl)-2-azabicyclo[2.2.1]heptan-2-yl)-1, 2,4-triazin-6-carboxamide **112**
5-((4-(1-((1-(2-(2,6-dioxopiperidin-3-yl)-1-oxoisoindol-5-yl)piperidin-4-yl)methyl)piperi din-4-yl)phenyl)amino)-3-(6-(3-methyl-2-oxoimidazolin-1-yl)-2-azabicyclo[2.2.1]heptan-2-yl)-1, 2,4-triazin-6-carboxamide **113**
5-((4-(1-((1-(1-(2,6-dioxopiperidin-3-yl)-3-methyl-2-oxo-2,3-dihydro-1H-benzo[d]imida zol-5-yl)piperidin-4-yl)methyl)piperidin-4-yl)phenyl)amino)-3-(6-(3-methyl-2-oxoimidazolin-1-yl)-2-azabicyclo[2.2.1]heptan-2-yl)-1,2,4-triazin-6-carboxamide **114**
4-((4-(4-((1-(2-(2,6-dioxopiperidin-3-yl)-1,3-dioxoisoindolin-5-yl)pyrrolidin-3-yl)methyl )piperazin-1-yl)phenyl)amino)-2-((R)-3-(3-methyl-2-oxoimidazolin-1-yl)piperidin-1-yl)pyrimidi n-5-carboxamide **115**
4-((4-(1-((1-(2-(2,6-dioxopiperidin-3-yl)-1,3-dioxoisoindolin-5-yl)pyrrolidin-3-yl)methyl )piperidin-4-yl)phenyl)amino)-2-((R)-3-(3-methyl-2-oxoimidazolin-1-yl)piperidin-1-yl)pyrimidi n-5-carboxamide **116**
4-((4-(1-((1-(2-(2,6-dioxopiperidin-3-yl)-1,3-dioxoisoindolin-5-yl)piperidin-4-yl)methyl) piperidin-4-yl)phenyl)amino)-2-((R)-3-(3-methyl-2-oxoimidazolin-1-yl)piperidin-1-yl)pyrimidin -5-carboxamide **117**
3-((4-(1-((1-(2-(2,6-dioxopiperidin-3-yl)-1,3-dioxoisoindolin-5-yl)piperidin-4-yl)methyl) piperidin-4-yl)phenyl)amino)-5-(6-(3-methyl-2-oxoimidazolin-1-yl)-2-azabicyclo[2.2.1]heptan-2 -yl)pyrazin-2-carboxamide **118**
5-((4-(4-((4-(2-(2,6-dioxopiperidin-3-yl)-1,3-dioxoisoindolin-5-yl)piperazin-1-yl)methyl) piperidin-1-yl)phenyl)amino)-3-((R)-3-(3-methyl-2-oxoimidazolin-1-yl)piperidin-1-yl)-1,2,4-tria zin-6-carboxamide **119**
5-((4-(1-((1-(2-(2,6-dioxopiperidin-3-yl)-1,3-dioxoisoindolin-5-yl)-3-methylpyrrolidin-3-yl)methyl)piperidin-4-yl)phenyl)amino)-3-((R)-3-(3-methyl-2-oxoimidazolin-1-yl)piperidin-1-yl )-1,2,4-triazin-6-carboxamide **120**
5-((4-(1-((1-(2-(2,6-dioxopiperidin-3-yl)-1,3-dioxoisoindolin-5-yl)-3-fluoropyrrolidin-3-yl)methyl)piperidin-4-yl)phenyl)amino)-3-((R)-3-(3-methyl-2-oxoimidazolin-1-yl)piperidin-1-yl )-1,2,4-triazin-6-carboxamide **121**
5-((4-(1-((1-(2-(2,6-dioxopiperidin-3-yl)-1,3-dioxoisoindolin-5-yl)-4-fluoropiperidin-4-yl )methyl)piperidin-4-yl)phenyl)amino)-3-((R)-3-(3-methyl-2-oxoimidazolin-1-yl)piperidin-1-yl)-1,2,4-triazin-6-carboxamide **122**
5-((4-(1-((1-(2-(2,6-dioxopiperidin-3-yl)-1,3-dioxoisoindolin-5-yl)piperidin-4-yl)methyl) piperidin-4-yl)phenyl)amino)-3-((R)-3-(3-methyl-2-oxoimidazolin-1-yl)piperidin-1-yl)-1,2,4-tria zin-6-carboxamide **123**
5-((4-(1-((1-(2-(2,6-dioxopiperidin-3-yl)-1,3-dioxoisoindolin-5-yl)pyrrolidin-3-yl)methyl )piperidin-4-yl)phenyl)amino)-3-((R)-3-(3-methyl-2-oxoimidazolin-1-yl)piperidin-1-yl)-1,2,4-tri azin-6-carboxamide **124**
5-((4-(4-((4-(2-(2,6-dioxopiperidin-3-yl)-6-fluoro-1,3-dioxoisoindolin-5-yl)piperazin-1-y l)methyl)piperidin-1-yl)phenyl)amino)-3-((R)-3-(3-methyl-2-oxoimidazolin-1-yl)piperidin-1-yl)-1,2,4-triazin-6-carboxamide **125**
5-((4-(1-((1-(2-(2,6-dioxopiperidin-3-yl)-6-fluoro-1,3-dioxoisoindolin-5-yl)pyrrolidin-3-yl)methyl)piperidin-4-yl)phenyl)amino)-3-((R)-3-(3-methyl-2-oxoimidazolin-1-yl)piperidin-1-yl )-1,2,4-triazin-6-carboxamide **126**
5-((4-(1-(1-(2-(2,6-dioxopiperidin-3-yl)-1,3-dioxoisoindolin-5-yl)pyrrolidin-3-carbonyl)p iperidin-4-yl)phenyl)amino)-3-((R)-3-(3-methyl-2-oxoimidazolin-1-yl)piperidin-1-yl)-1,2,4-triaz in-6-carboxamide **127**
5-((4-(1-((1-(2-(2,6-dioxopiperidin-3-yl)-1,3-dioxoisoindolin-5-yl)pyrrolidin-3-yl)methyl )piperidin-4-yl)-3-fluorophenyl)amino)-3-((R)-3-(3-methyl-2-oxoimidazolin-1-yl)piperidin-1-yl) -1,2,4-triazin-6-carboxamide **128**
5-((4-(1-((1-(2-(2,6-dioxopiperidin-3-yl)-1,3-dioxoisoindolin-5-yl)piperidin-4-yl)methyl) piperidin-4-yl)-3-fluorophenyl)amino)-3-((R)-3-(3-methyl-2-oxoimidazolin-1-yl)piperidin-1-yl)-1,2,4-triazin-6-carboxamide **129**
5-((4-(4-((1-(2-(2,6-dioxopiperidin-3-yl)-1,3-dioxoisoindolin-5-yl)pyrrolidin-3-yl)methyl )piperazin-1-yl)-3-fluorophenyl)amino)-3-((R)-3-(3-methyl-2-oxoimidazolin-1-yl)piperidin-1-yl) -1,2,4-triazin-6-carboxamide **130**
5-((4-(4-(((3 S)-1-(2-(2,6-dioxopiperidin-3-yl)-1,3-dioxoisoindolin-5-yl)pyrrolidin-3-yl)m ethyl)piperazin-1-yl)-3-fluorophenyl)amino)-3-((R)-3-(3-methyl-2-oxoimidazolin-1-yl)piperidin-1-yl)-1,2,4-triazin-6-carboxamide **131**
5-((4-(4-(((3R)-1-(2-(2,6-dioxopiperidin-3-yl)-1,3-dioxoisoindolin-5-yl)pyrrolidin-3-yl)m ethyl)piperazin-1-yl)-3-fluorophenyl)amino)-3-((R)-3-(3-methyl-2-oxoimidazolin-1-yl)piperidin-1-yl)-1,2,4-triazin-6-carboxamide **132**
5-((3-chloro-4-(4-((1-(2-(2,6-dioxopiperidin-3-yl)-1,3-dioxoisoindolin-5-yl)pyrrolidin-3-yl)methyl)piperazin-1-yl)phenyl)amino)-3-((R)-3-(3-methyl-2-oxoimidazolin-1-yl)piperidin-1-yl )-1,2,4-triazin-6-carboxamide **133**
5-((4-(4-((1-(2-(2,6-dioxopiperidin-3-yl)-1,3-dioxoisoindolin-5-yl)piperidin-4-yl)methyl) piperazin-1-yl)-3-fluorophenyl)amino)-3-((R)-3-(3-methyl-2-oxoimidazolin-1-yl)piperidin-1-yl)-1,2,4-triazin-6-carboxamide **134**
5-((4-(1-((1-(2-(2,6-dioxopiperidin-3-yl)-1,3-dioxoisoindolin-5-yl)piperidin-4-yl)methyl) piperidin-4-yl)phenyl)amino)-3-((R)-3-(2,5-dioxopyrrolidin-1-yl)piperidin-1-yl)-1,2,4-triazin-6-c arboxamide **135**
5-((4-(1-((1-(2-(2,6-dioxopiperidin-3-yl)-1,3-dioxoisoindolin-5-yl)pyrrolidin-3-yl)methyl )piperidin-4-yl)phenyl)amino)-3-((R)-3-(2,5-dioxopyrrolidin-1-yl)piperidin-1-yl)-1,2,4-triazin-6-carboxamide **136**
5-((4-(1-((1-(2-(2,6-dioxopiperidin-3-yl)-1,3-dioxoisoindolin-5-yl)pyrrolidin-3-yl)methyl )piperidin-4-yl)phenyl)amino)-3-((R)-3-(4-methyl-5,7-dioxo-4,6-diazaspiro[2.4]heptan-6-yl)pipe ridin-1-yl)-1,2,4-triazin-6-carboxamide **137**
5-((4-(1-((1-(2-(2,6-dioxopiperidin-3-yl)-1,3-dioxoisoindolin-5-yl)piperidin-4-yl)methyl) piperidin-4-yl)phenyl)amino)-3-((R)-3-(4-methyl-5-oxo-4,6-diazaspiro[2.4]heptan-6-yl)piperidin -1-yl)-1,2,4-triazin-6-carboxamide **138**
Methyl (3-(5-(3-((4-(4-((6-carbamoyl-3-((R)-3-(3-methyl-2-oxoimidazolin-1-yl)piperidin -1 -yl)-1,2,4-triazin-5-yl)amino)phenyl)piperidin-1 -yl)methyl)pyrrolidin-1 -yl)-1,3-dioxoisoindoli n-2-yl)-2,6-dioxopiperidin-1-yl)butyrate **139**
5-((4-(1-((1-(2-(2,6-dioxopiperidin-3-yl)-1,3-dioxoisoindolin-5-yl)pyrrolidin-3-yl)methyl )piperidin-4-yl)phenyl)amino)-3-((R)-3-(2-oxo-3-(2,2,2-trifluoroethyl)imidazolin-1-yl)piperidin-1-yl)-1,2,4-triazin-6-carboxamide **140**
5-((4-(1-((1-(2-(2,6-dioxopiperidin-3-yl)-1,3-dioxoisoindolin-5-yl)piperidin-4-yl)methyl) piperidin-4-yl)phenyl)amino)-3-((R)-3-(2-oxo-3-(2,2,2-trifluoroethyl)imidazolin-1-yl)piperidin-1 -yl)-1,2,4-triazin-6-carboxamide **141**
5-((4-(4-((4-(2-(2,6-dioxopiperidin-3-yl)-6-fluoro-1,3-dioxoisoindolin-5-yl)piperazin-1-y l)methyl)piperidin-1-yl)phenyl)amino)-3-((R)-3-(2-oxo-3-(2,2,2-trifluoroethyl)imidazolin-1-yl)p iperidin-1-yl)-1,2,4-triazin-6-carboxamide **142**
3-(6-(1,3-dioxoisoindolin-2-yl)-2-azabicyclo[2.2.1]heptan-2-yl)-5-((4-(1-((1-(2-(2,6-diox opiperidin-3-yl)-1,3-dioxoisoindolin-5-yl)piperidin-4-yl)methyl)piperidin-4-yl)phenyl)amino)-1, 2,4-triazin-6-carboxamide **143**
3-(6-(1,3-dioxoisoindolin-2-yl)-2-azabicyclo[2.2.1]heptan-2-yl)-5-((4-(1-((1-(2-(2,6-diox opiperidin-3-yl)-1,3-dioxoisoindolin-5-yl)pyrrolidin-3-yl)methyl)piperidin-4-yl)phenyl)amino)-1, 2,4-triazin-6-carboxamide **144**
5-((4-(4-((1-(6-(((S)-2,6-dioxopiperidin-3-yl)carbamoyl)pyridin-3-yl)pyrrolidin-3-yl)met hyl)piperazin-1-yl)-3-fluorophenyl)amino)-3-((R)-3-(3-methyl-2-oxoimidazolin-1-yl)piperidin-1 -yl)-1,2,4-triazin-6-carboxamide **145**
5-((4-(4-((1-(6-((2,6-dioxopiperidin-3-yl)carbamoyl)pyridin-3-yl)piperidin-4-yl)methyl)p iperazin-1-yl)-3-fluorophenyl)amino)-3-((R)-3-(3-methyl-2-oxoimidazolin-1-yl)piperidin-1-yl)-1, 2,4-triazin-6-carboxamide **146**
5-((4-(4-((1-(4-(2,4-dioxotetrahydropyrimidin-1(2H)-yl)phenyl)pyrrolidin-3-yl)methyl)pi perazin-1-yl)-3-fluorophenyl)amino)-3-((R)-3-(3-methyl-2-oxoimidazolin-1-yl)piperidin-1-yl)-1, 2,4-triazin-6-carboxamide **147**
5-((4-(4-((1-(3-(2,4-dioxotetrahydropyrimidin-1(2H)-yl)-4-fluorobenzoyl)pyrrolidin-3-yl) methyl)piperazin-1-yl)-3 -fluorophenyl)amino)-3 -((R)-3 -(3 -methyl-2-oxoimidazolin-1-yl)piperidi n-1-yl)-1,2,4-triazin-6-carboxamide **148**
5-((4-(4-((1-(3-(2,4-dioxotetrahydropyrimidin-1(2H)-yl)-4-methoxybenzoyl)pyrrolidin-3-yl)methyl)piperazin-1-yl)-3-fluorophenyl)amino)-3-((R)-3-(3-methyl-2-oxoimidazolin-1-yl)piper idin-1-yl)-1,2,4-triazin-6-carboxamide **149**
5-((4-(4-((1-(3-(2,4-dioxotetrahydropyrimidin-1(2H)-yl)-4-methylbenzoyl)pyrrolidin-3-yl )methyl)piperazin-1-yl)-3-fluorophenyl)amino)-3-((R)-3-(3-methyl-2-oxoimidazolin-1-yl)piperid in-1-yl)-1,2,4-triazin-6-carboxamide **150**
5-((4-(4-(((R)-1-(4-(2,4-dioxotetrahydropyrimidin-1(2H)-yl)phenyl)pyrrolidin-3-yl)meth yl)piperazin-1-yl)-3-fluorophenyl)amino)-3-((R)-3-(3-methyl-2-oxoimidazolin-1-yl)piperidin-1-yl)-1,2,4-triazin-6-carboxamide **151**
5-((4-(4-(((S)-1-(4-(2,4-dioxotetrahydropyrimidin-1 (2H)-yl)phenyl)pyrrolidin-3-yl)methy l)piperazin-1-yl)-3-fluorophenyl)amino)-3-((R)-3-(3-methyl-2-oxoimidazolin-1-yl)piperidin-1-yl )-1,2,4-triazin-6-carboxamide **152**
5-((4-(4-(((R)-1-(6-(((S)-2,6-dioxopiperidin-3-yl)carbamoyl)pyridin-3-yl)pyrrolidin-3-yl) methyl)piperazin-1-yl)-3-fluorophenyl)amino)-3-((R)-3-(3-methyl-2-oxoimidazolin-1-yl)piperidi n-1-yl)-1,2,4-triazin-6-carboxamide **153**
5-((4-(4-(((S)-1-(6-(((S)-2,6-dioxopiperidin-3-yl)carbamoyl)pyridin-3-yl)pyrrolidin-3-yl) methyl)piperazin-1-yl)-3-fluorophenyl)amino)-3-((R)-3-(3-methyl-2-oxoimidazolin-1-yl)piperidi n-1-yl)-1,2,4-triazin-6-carboxamide **154**
5-((4-(1-(((3 S)-1-(2-(2,6-dioxopiperidin-3-yl)-6-fluoro-1,3-dioxoisoindolin-5-yl)pyrrolidi n-3 -yl)methyl)piperidin-4-yl)phenyl)amino)-3 -((R)-3 -(3 -methyl-2-oxoimidazolin-1-yl)piperidin-1-yl)-1,2,4-triazin-6-carboxamide **155**
5-((4-(1-(((3R)-1-(2-(2,6-dioxopiperidin-3-yl)-6-fluoro-1,3-dioxoisoindolin-5-yl)pyrrolid in-3 -yl)methyl)piperidin-4-yl)phenyl)amino)-3 -((R)-3 -(3 -methyl-2-oxoimidazolin-1-yl)piperidin -1-yl)-1,2,4-triazin-6-carboxamide **156**
5-((4-(1-(((3S)-1-(2-(2,6-dioxopiperidin-3-yl)-1,3-dioxoisoindolin-5-yl)pyrrolidin-3-yl)m ethyl)piperidin-4-yl)-3-fluorophenyl)amino)-3-((R)-3-(3-methyl-2-oxoimidazolin-1-yl)piperidin-1-yl)-1,2,4-triazin-6-carboxamide **157**
5-((4-(1-(((3R)-1-(2-(2,6-dioxopiperidin-3-yl)-1,3-dioxoisoindolin-5-yl)pyrrolidin-3-yl)m ethyl)piperidin-4-yl)-3-fluorophenyl)amino)-3-((R)-3-(3-methyl-2-oxoimidazolin-1-yl)piperidin-1-yl)-1,2,4-triazin-6-carboxamide **158**
5-((3-chloro-4-(4-(((3 S)-1-(2-(2,6-dioxopiperidin-3-yl)-1,3-dioxoisoindolin-5-yl)pyrrolid in-3-yl)methyl)piperazin-1-yl)phenyl)amino)-3-((R)-3-(3-methyl-2-oxoimidazolidin-1-yl)piperid in-1-yl)-1,2,4-triazin-6-carboxamide **159**
5-((3-chloro-4-(4-(((3R)-1-(2-(2,6-dioxopiperidin-3-yl)-1,3-dioxoisoindolin-5-yl)pyrrolid in-3-yl)methyl)piperazin-1-yl)phenyl)amino)-3-((R)-3-(3-methyl-2-oxoimidazolidin-1-yl)piperid in-1-yl)-1,2,4-triazin-6-carboxamide **160**
5-((4-(4-((1-(2-(2,6-dioxopiperidin-3-yl)-1,3-dioxoisoindolin-5-yl)pyrrolidin-3-yl)methyl )piperazin-1-yl)-3-(trifluoromethyl)phenyl)amino)-3-((R)-3-(3-methyl-2-oxoimidazolidin-1-yl)pi peridin-1-yl)-1,2,4-triazin-6-carboxamide **161**
5-((4-(4-((1-(2-(2,6-dioxopiperidin-3-yl)-1,3-dioxoisoindolin-5-yl)pyrrolidin-3-yl)methyl )piperazin-1-yl)-2,3-difluorophenyl)amino)-3-((R)-3-(3-methyl-2-oxoimidazolidin-1-yl)piperidin -1-yl)-1,2,4-triazin-6-carboxamide **162**
5-((4-(4-((1-(2-(2,6-dioxopiperidin-3-yl)-1,3-dioxoisoindolin-5-yl)pyrrolidin-3-yl)methyl )piperazin-1-yl)-2,5-difluorophenyl)amino)-3-((R)-3-(3-methyl-2-oxoimidazolidin-1-yl)piperidin -1-yl)-1,2,4-triazin-6-carboxamide **163**
5-((3-chloro-4-(4-((1-(2-(2,6-dioxopiperidin-3-yl)-1,3-dioxoisoindolin-5-yl)piperidin-4-y 1)methyl)piperazin-1-yl)phenyl)amino)-3-((R)-3-(3-methyl-2-oxoimidazolidin-1-yl)piperidin-1-y 1)-1,2,4-triazin-6-carboxamide **164**
5-((4-(4-((1-(2-(2,6-dioxopiperidin-3-yl)-1,3-dioxoisoindolin-5-yl)piperidin-4-yl)methyl) piperazin-1-yl)-2,3-difluorophenyl)amino)-3-((R)-3-(3-methyl-2-oxoimidazolidin-1-yl)piperidin-1-yl)-1,2,4-triazin-6-carboxamide **165**
5-((4-(4-((1-(2-(2,6-dioxopiperidin-3-yl)-1-oxoisoindolin-5-yl)pyrrolidin-3-yl)methyl)pip erazin-1-yl)-3-fluorophenyl)amino)-3-((R)-3-(3-methyl-2-oxoimidazolidin-1-yl)piperidin-1-yl)-1, 2,4-triazin-6-carboxamide **166**
5-((4-(4-((1-(2-(2,6-dioxopiperidin-3-yl)-1-oxoisoindolin-5-yl)piperidin-4-yl)methyl)pipe razin-1-yl)-3-fluorophenyl)amino)-3-((R)-3-(3-methyl-2-oxoimidazolidin-1-yl)piperidin-1-yl)-1, 2,4-triazin-6-carboxamide **167**
5-((4-(4-((1-(2-(2,6-dioxopiperidin-3-yl)-3-oxoisoindolin-5-yl)piperidin-4-yl)methyl)pipe razin-1-yl)-3-fluorophenyl)amino)-3-((R)-3-(3-methyl-2-oxoimidazolidin-1-yl)piperidin-1-yl)-1, 2,4-triazin-6-carboxamide **168**
5-((4-(4-((1-(2-(2,6-dioxopiperidin-3-yl)-6-fluoro-1,3-dioxoisoindolin-5-yl)pyrrolidin-3-yl)methyl)piperazin-1-yl)-3-fluorophenyl)amino)-3-((R)-3-(3-methyl-2-oxoimidazolidin-1-yl)pip eridin-1-yl)-1,2,4-triazin-6-carboxamide **169**
5-((4-(4-((1-(2-(2,6-dioxopiperidin-3-yl)-6-fluoro-1,3-dioxoisoindolin-5-yl)piperidin-4-yl )methyl)piperazin-1-yl)-3-fluorophenyl)amino)-3-((R)-3-(3-methyl-2-oxoimidazolidin-1-yl)piper idin-1-yl)-1,2,4-triazin-6-carboxamide **170**
5-((4-(4-((1-(6-(((S)-2,6-dioxopiperidin-3-yl)carbamoyl)pyridin-3-yl)piperidin-4-yl)meth yl)piperazin-1-yl)-3-fluorophenyl)amino)-3-((R)-3-(3-methyl-2-oxoimidazolidin-1-yl)piperidin-1-yl)-1,2,4-triazin-6-carboxamide **171**
5-((4-(4-((1-(4-(((S)-2,6-dioxopiperidin-3-yl)carbamoyl)-3-fluorophenyl)piperidin-4-yl) methyl)piperazin-1-yl)-3-fluorophenyl)amino)-3-((R)-3-(3-methyl-2-oxoimidazolidin-1-yl)piperi din-1-yl)-1,2,4-triazin-6-carboxamide **172**
5-((4-(4-((1-(6-(2,6-dioxopiperidin-3-yl)carbamoyl)pyridin-3-yl)pyrrolidin-3-yl)methyl)p iperazin-1-yl)-3-fluorophenyl)amino)-3-((R)-3-(3-methyl-2-oxoimidazolidin-1-yl)piperidin-1-yl) -1,2,4-triazin-6-carboxamide **173**
5-((4-(4-((1-(5-((2,6-dioxopiperidin-3-yl)carbamoyl)pyridin-2-yl)pyrrolidin-3-yl)methyl) piperazin-1-yl)-3-fluorophenyl)amino)-3-((R)-3-(3-methyl-2-oxoimidazolidin-1-yl)piperidin-1-y l)-1,2,4-triazin-6-carboxamide **174**
5-((4-(4-((1-(5-((2,6-dioxopiperidin-3-yl)carbamoyl)pyridin-2-yl)piperidin-4-yl)methyl)p iperazin-1-yl)-3-fluorophenyl)amino)-3-((R)-3-(3-methyl-2-oxoimidazolidin-1-yl)piperidin-1-yl) -1,2,4-triazin-6-carboxamide **175**
5-((4-(4-((1-(7-((2,6-dioxopiperidin-3-yl)carbamoyl)benzo[d][1,3]dioxacyclopent-4-yl)p yrrolidin-3-yl)methyl)piperazin-1-yl)-3-fluorophenyl)amino)-3-((R)-3-(3-methyl-2-oxoimidazoli din-1-yl)piperidin-1-yl)-1,2,4-triazin-6-carboxamide **176**
5-((4-(4-((1-(7-((2,6-dioxopiperidin-3-yl)carbamoyl)benzo[d][1,3]dioxacyclopent-4-yl)pi peridin-4-yl)methyl)piperazin-1-yl)-3-fluorophenyl)amino-3-((R)-3-(3-methyl-2-oxoimidazolidi n-1-yl)piperidin-1-yl)-1,2,4-triazin-6-carboxamide **177**
4-(3-((4-(4-((6-carbamoyl-3-((R)-3-(3-methyl-2-oxoimidazolidin-1-yl)piperidin-1-yl)-1,2, 4-triazin-5-yl)amino)-2-fluorophenyl)piperazin-1-yl)methyl)pyrrolidin-1-yl)-N-(2,6-dioxopiperi din-3-yl)-1Hbenzimidazol-7-carboxamide **178**
4-(4-((4-(4-((6-carbamoyl-3-((R)-3-(3-methyl-2-oxoimidazolidin-1-yl)piperidin-1-yl)-1,2, 4-triazin-5-yl)amino)-2-fluorophenyl)piperazin-1-yl)methyl)piperidin-1-yl)-N-(2,6-dioxopiperid in-3-yl)-1Hbenzimidazol-7-carboxamide **179**
5-((4-(4-((1-(4-((2,6-dioxopiperidin-3-yl)carbamoyl)-3-methylphenyl)piperidin-4-yl)met hyl)piperazin-1-yl)-3-fluorophenyl)amino)-3-((R)-3-(3-methyl-2-oxoimidazolidin-1-yl)piperidin -1-yl)-1,2,4-triazin-6-carboxamide **180**
5-((4-(4-((1-(4-((2,6-dioxopiperidin-3-yl)carbamoyl)-3-methoxyphenyl)piperidin-4-yl)me thyl)piperazin-1-yl)-3-fluorophenyl)amino)-3-((R)-3-(3-methyl-2-oxoimidazolidin-1-yl)piperidin -1-yl)-1,2,4-triazin-6-carboxamide **181**
5-((4-(4-((1-(4-((2,6-dioxopiperidin-3-yl)carbamoyl)-2,3-difluorophenyl)piperidin-4-yl) methyl)piperazin-1-yl)-3-fluorophenyl)amino)-3-((R)-3-(3-methyl-2-oxoimidazolidin-1-yl)piperi din-1-yl)-1,2,4-triazin-6-carboxamide **182**
5-((4-(4-((1-(8-((2,6-dioxopiperidin-3-yl)carbamoyl)-2,3-dihydrobenzo[b][1,4] dioxan-5-yl)piperidin-4-yl)methyl)piperazin-1-yl)-3-fluorophenyl)amino)-3-((R)-3-(3-methyl-2-oxoimidaz olidin-1-yl)piperidin-1-yl)-1,2,4-triazin-6-carboxamide **183**
(R)-5-((4-(4-((1-(4-(2,4-dioxotetrahydropyrimidin-1 (2H)-yl)-3-fluorophenyl)piperidin-4-yl)methyl)piperazin-1-yl)-3-fluorophenyl)amino)-3-(3-(3-methyl-2-oxoimidazolidin-1-yl)piperid in-1-yl)-1,2,4-triazin-6-carboxamide **184**
(R)-5-((4-(4-((1-(4-(2,4-dioxotetrahydropyrimidin-1 (2H)-yl)-2,3-difluorophenyl)piperidi n-4-yl)methyl)piperazin-1-yl)-3-fluorophenyl)amino)-3-(3-(3-methyl-2-oxoimidazolidin-1-yl)pip eridin-1-yl)-1,2,4-triazin-6-carboxamide **185**
(R)-5-((4-(4-((1-(4-(2,4-dioxotetrahydropyrimidin-1(2H)-yl)-2-fluorophenyl)piperidin-4-yl)methyl)piperazin-1-yl)-3-fluorophenyl)amino)-3-(3-(3-methyl-2-oxoimidazolidin-1-yl)piperid in-1-yl)-1,2,4-triazin-6-carboxamide **186**
(R)-5-((4-(4-((1-(4-(2,4-dioxotetrahydropyrimidin-1(2H)-yl)-3-(trifluoromethyl)phenyl)p iperidin-4-yl)methyl)piperazin-1-yl)-3-fluorophenyl)amino)-3-(3-(3-methyl-2-oxoimidazolidin-1 -yl)piperidin-1-yl)-1,2,4-triazin-6-carboxamide **187**
(R)-5-((4-(4-((1-(3-(difluoromethyl)-4-(2,4-dioxotetrahydropyrimidin-1(2H)-yl)phenyl)pi peridin-4-yl)methyl)piperazin-1-yl)-3-fluorophenyl)amino)-3-(3-(3-methyl-2-oxoimidazolidin-1-yl)piperidin-1-yl)-1,2,4-triazin-6-carboxamide **188**
(R)-5-((4-(4-((1-(4-(2,4-dioxotetrahydropyrimidin-1 (2H)-yl)-3-isopropylphenyl)piperidin -4-yl)methyl)piperazin-1-yl)-3-fluorophenyl)amino)-3-(3-(3-methyl-2-oxoimidazolidin-1-yl)pipe ridin-1-yl)-1,2,4-triazin-6-carboxamide **189**
(R)-5-((4-(4-((1-(7-(2,4-dioxotetrahydropyrimidin-1(2H)-yl)benzo[d][1,3]dioxacyclopent -4-yl)piperidin-4-yl)methyl)piperazin-1-yl)-3-fluorophenyl)amino)-3-(3-(3-methyl-2-oxoimidazo lidin-1-yl)piperidin-1-yl)-1,2,4-triazin-6-carboxamide **190**
(R)-5-((4-(4-((1-(7-(2,4-dioxotetrahydropyrimidin-1(2H)-yl)benzo[d]oxazol-4-yl)piperidi n-4-yl)methyl)piperazin-1-yl)-3-fluorophenyl)amino)-3-(3-(3-methyl-2-oxoimidazolidin-1-yl)pip eridin-1-yl)-1,2,4-triazin-6-carboxamide **191**
(R)-5-((4-(4-((1-(7-(2,4-dioxotetrahydropyrimidin-1(2H)-yl)-1H-benzo[d]imidazol-4-yl)p iperidin-4-yl)methyl)piperazin-1-yl)-3-fluorophenyl)amino)-3-(3-(3-methyl-2-oxoimidazolidin-1 -yl)piperidin-1-yl)-1,2,4-triazin-6-carboxamide **192**
(R)-5-((4-(4-((1-(8-(2,4-dioxotetrahydropyrimidin-1(2H)-yl)-2,3-dihydrobenzo[b][1,4]di oxan-5-yl)piperidin-4-yl)methyl)piperazin-1-yl)-3-fluorophenyl)amino)-3-(3-(3-methyl-2-oxoim idazolidin-1-yl)piperidin-1-yl)-1,2,4-triazin-6-carboxamide **193**
(R)-5-((4-(4-((1-(3-cyclopropyl-4-(2,4-dioxotetrahydropyrimidin-1 (2H)-yl)phenyl)piperi din-4-yl)methyl)piperazin-1-yl)-3-fluorophenyl)amino)-3-(3-(3-methyl-2-oxoimidazolidin-1-yl)p iperidin-1-yl)-1,2,4-triazin-6-carboxamide **194**
(R)-5-((4-(4-((1-(6-(2,4-dioxotetrahydropyrimidin-1(2H)-yl)pyridin-3-yl)piperidin-4-yl) methyl)piperazin-1-yl)-3-fluorophenyl)amino)-3-(3-(3-methyl-2-oxoimidazolidin-1-yl)piperidin-1-yl)-1,2,4-triazin-6-carboxamide **195**
(R)-5-((4-(4-((1-(5-(2,4-dioxotetrahydropyrimidin-1(2H)-yl)pyridin-2-yl)piperidin-4-yl) methyl)piperazin-1-yl)-3-fluorophenyl)amino)-3-(3-(3-methyl-2-oxoimidazolidin-1-yl)piperidin-1-yl)-1,2,4-triazin-6-carboxamide **196**
(R)-5-((4-(4-((1-(4-(2,4-dioxotetrahydropyrimidin-1(2H)-yl)-2-(methylsulfonyl)phenyl)pi peridin-4-yl)methyl)piperazin-1-yl)-3-fluorophenyl)amino)-3-(3-(3-methyl-2-oxoimidazolidin-1-yl)piperidin-1-yl)-1,2,4-triazin-6-carboxamide **197**
(R)-5-((4-(4-((1-(2-(dimethylphosphoryl)-4-(2,4-dioxotetrahydropyrimidin-1(2H)-yl)phe nyl)piperidin-4-yl)methyl)piperazin-1-yl)-3-fluorophenyl)amino)-3-(3-(3-methyl-2-oxoimidazoli din-1-yl)piperidin-1-yl)-1,2,4-triazin-6-carboxamide **198**
(R)-5-((4-(4-((1-(5-(2,4-dioxotetrahydropyrimidin-1(2H)-yl)quinolin-8-yl)piperidin-4-yl) methyl)piperazin-1-yl)-3-fluorophenyl)amino)-3-(3-(3-methyl-2-oxoimidazolidin-1-yl)piperidin-1-yl)-1,2,4-triazin-6-carboxamide **199**
5-((4-(4-((1-(6-((2,6-dioxopiperidin-3-yl)amino)pyridin-3-yl)pyrrolidin-3-yl)methyl)pipe razin-1-yl)-3-fluorophenyl)amino)-3-((R)-3-(3-methyl-2-oxoimidazolidin-1-yl)piperidin-1-yl)-1, 2,4-triazin-6-carboxamide **200**
5-((4-(4-((1-(5-((2,6-dioxopiperidin-3-yl)amino)pyridin-2-yl)pyrrolidin-3-yl)methyl)pipe razin-1-yl)-3-fluorophenyl)amino)-3-((R)-3-(3-methyl-2-oxoimidazolidin-1-yl)piperidin-1-yl)-1, 2,4-triazin-6-carboxamide **201**
5-((4-(4-((1-(6-((2,6-dioxopiperidin-3-yl)amino)pyridin-3-yl)piperidin-4-yl)methyl)piper azin-1-yl)-3-fluorophenyl)amino)-3-((R)-3-(3-methyl-2-oxoimidazolidin-1-yl)piperidin-1-yl)-1,2, 4-triazin-6-carboxamide **202**
5-((4-(4-((1-(5-((2,6-dioxopiperidin-3-yl)amino)pyridin-2-yl)piperidin-4-yl)methyl)piper azin-1-yl)-3-fluorophenyl)amino)-3-((R)-3-(3-methyl-2-oxoimidazolidin-1-yl)piperidin-1-yl)-1,2, 4-triazin-6-carboxamide **203**
5-((4-(4-((1-(4-((2,6-dioxopiperidin-3-yl)amino)-3-fluorophenyl)pyrrolidin-3-yl)methyl)p iperazin-1-yl)-3-fluorophenyl)amino)-3-((R)-3-(3-methyl-2-oxoimidazolidin-1-yl)piperidin-1-yl) -1,2,4-triazin-6-carboxamide **204**
5-((4-(4-((1-(4-((2,6-dioxopiperidin-3-yl)amino)-3-fluorophenyl)piperidin-4-yl)methyl)pi perazin-1-yl)-3-fluorophenyl)amino)-3-((R)-3-(3-methyl-2-oxoimidazolidin-1-yl)piperidin-1-yl)-1,2,4-triazin-6-carboxamide **205**
5-((4-(4-((1-(4-((2,6-dioxopiperidin-3-yl)amino)-3-methylphenyl)pyrrolidin-3-yl)methyl) piperazin-1-yl)-3-fluorophenyl)amino)-3-((R)-3-(3-methyl-2-oxoimidazolidin-1-yl)piperidin-1-y 1)-1,2,4-triazin-6-carboxamide **206**
5-((4-(4-((1-(4-((2,6-dioxopiperidin-3-yl)amino)-3-methylphenyl)piperidin-4-yl)methyl)p iperazin-1-yl)-3-fluorophenyl)amino)-3-((R)-3-(3-methyl-2-oxoimidazolidin-1-yl)piperidin-1-yl) -1,2,4-triazin-6-carboxamide **207**
5-((4-(4-((1-(7-((2,6-dioxopiperidin-3-yl)amino)benzo[d][1,3]dioxacyclopent-4-yl)pyrrol idin-3-yl)methyl)piperazin-1-yl)-3-fluorophenyl)amino)-3-((R)-3-(3-methyl-2-oxoimidazolidin-1 -yl)piperidin-1-yl)-1,2,4-triazin-6-carboxamide **208**
5-((4-(4-((1-(7-((2,6-dioxopiperidin-3-yl)amino)benzo[d][1,3]dioxacyclopent-4-yl)piperi din-4-yl)methyl)piperazin-1-yl)-3-fluorophenyl)amino)-3-((R)-3-(3-methyl-2-oxoimidazolidin-1 -yl)piperidin-1-yl)-1,2,4-triazin-6-carboxamide **209**
5-((4-(4-((1-(7-((2,6-dioxopiperidin-3-yl)amino)benzo[d]oxazol-4-yl)piperidin-4-yl)meth yl)piperazin-1-yl)-3-fluorophenyl)amino)-3-((R)3-(3-methyl-2-oxoimidazolidin-1-yl)piperidin-1 -yl)-1,2,4-triazin-6-carboxamide **210**
5-((4-(4-((1-(8-((2,6-dioxopiperidin-3-yl)amino)-2,3-dihydrobenzo[b][1,4]dioxan-5-yl)pi peridin-4-yl)methyl)piperazin-1-yl)-3-fluorophenyl)amino)-3-((R)-3-(3-methyl-2-oxoimidazolidi n-1-yl)piperidin-1-yl)-1,2,4-triazin-6-carboxamide **211**
5-((4-(4-((1-(3-(2,6-dioxopiperidin-3-yl)-1-methyl-1H-indazol-6-yl)pyrrolidin-3-yl)meth yl)piperazin-1-yl)-3-fluorophenyl)amino)-3-((R)-3-(3-methyl-2-oxoimidazolidin-1-yl)piperidin-1-yl)-1,2,4-triazin-6-carboxamide **212**
5-((4-(4-((1-(3-(2,6-dioxopiperidin-3-yl)-1-methyl-1H-indazol-6-yl)piperidin-4-yl)methyl )piperazin-1-yl)-3-fluorophenyl)amino)-3-((R)-3-(3-methyl-2-oxoimidazolidin-1-yl)piperidin-1-yl)-1,2,4-triazin-6-carboxamide **213**
5-((4-(4-((1-(3-(2,6-dioxopiperidin-3-yl)-1-methyl-1H-pyrazolo[3,4-b]pyridin-6-yl)pyrrol idin-3-yl)methyl)piperazin-1-yl)-3-fluorophenyl)amino)-3-((R)-3-(3-methyl-2-oxoimidazolidin-1 -yl)piperidin-1-yl)-1,2,4-triazin-6-carboxamide **214**
5-((4-(4-((1-(3-(2,6-dioxopiperidin-3-yl)-1-methyl-1H-pyrazolyl[3,4-b]pyridin-6-yl)piper idin-4-yl)methyl)piperazin-1-yl)-3-fluorophenyl)amino)-3-((R)-3-(3-methyl-2-oxoimidazolidin-1 -yl)piperidin-1-yl)-1,2,4-triazin-6-carboxamide **215**
5-((4-(4-((1-(3-(2,6-dioxopiperidin-3-yl)-1-methyl-1H-indazol-7-yl)pyrrolidin-3-yl)meth yl)piperazin-1-yl)-3-fluorophenyl)amino)-3-((R)-3-(3-methyl-2-oxoimidazolidin-1-yl)piperidin-1-yl)-1,2,4-triazin-6-carboxamide **216**
5-((4-(4-((1-(3-(2,6-dioxopiperidin-3-yl)-1-methyl-1H-indazol-7-yl)piperidin-4-yl)methyl )piperazin-1-yl)-3-fluorophenyl)amino)-3-((R)-3-(3-methyl-2-oxoimidazolidin-1-yl)piperidin-1-yl)-1,2,4-triazin-6-carboxamide **217**
5-((4-(4-((1-(1-(2,6-dioxopiperidin-3-yl)-3-methyl-2-oxo-2,3-dihydro-1H-benzo[d]imida zol-5-yl)pyrrolidin-3-yl)methyl)piperazin-1-yl)-3-fluorophenyl)amino)-3-((R)-3-(3-methyl-2-ox oimidazolidin-1-yl)piperidin-1-yl)-1,2,4-triazin-6-carboxamide **218**
5-((4-(4-((1-(1-(2,6-dioxopiperidin-3-yl)-3-methyl-2-oxo-2,3-dihydro-1H-benzo[d]imida zol-5-yl)piperidin-4-yl)methyl)piperazin-1-yl)-3-fluorophenyl)amino)-3-((R)-3-(3-methyl-2-oxoi midazolidin-1-yl)piperidin-1-yl)-1,2,4-triazin-6-carboxamide **219**
5-((4-(4-((1-(2-(2,6-dioxopiperidin-3-yl)-3-oxo-1,2,3,4-tetrahydropyrrolo[3,4-b]indol-6-y l)pyrrolidin-3-yl)methyl)piperazin-1-yl)-3-fluorophenyl)amino)-3-((R)-3-(3-methyl-2-oxoimidaz olidin-1-yl)piperidin-1-yl)-1,2,4-triazin-6-carboxamide **220**
5-((4-(4-((1-(2-(2,6-dioxopiperidin-3-yl)-3-oxo-2,3-dihydro-1H-benzofuro[2,3-c]pyrrol-6 -yl)pyrrolidin-3-yl)methyl)piperazin-1-yl)-3-fluorophenyl)amino)-3-((R)-3-(3-methyl-2-oxoimid azolidin-1-yl)piperidin-1-yl)-1,2,4-triazin-6-carboxamide **221**
5-((4-(4-((1-(2-(2,6-dioxopiperidin-3-yl)-3-oxo-1,2,3,4-tetrahydropyrrolo[3,4-b]indol-6-y l)piperidin-4-yl)methyl)piperazin-1-yl)-3-fluorophenyl)amino)-3-((R)-3-(3-methyl-2-oxoimidazo lidin-1-yl)piperidin-1-yl)-1,2,4-triazin-6-carboxamide **222**
5-((4-(4-((1-(2-(2,6-dioxopiperidin-3-yl)-3-oxo-2,3-dihydro-1H-benzofuro[2,3-c]pyrrol-6 -yl)piperidin-4-yl)methyl)piperazin-1-yl)-3-fluorophenyl)amino)-3-((R)-3-(3-methyl-2-oxoimida zolidin-1-yl)piperidin-1-yl)-1,2,4-triazin-6-carboxamide **223**
5-((4-(4-(3-(6-((2,6-dioxopiperidin-3-yl)carbamoyl)pyridin-3-yl)propan-2-yn-1-yl)pipera zin-1-yl)-3-fluorophenyl)amino)-3-((R)-3-(3-methyl-2-oxoimidazolidin-1-yl)piperidin-1-yl)-1,2, 4-triazin-6-carboxamide **224**
5-((4-(4-((4-((2,6-dioxopiperidin-3-yl)carbamoyl)-3-fluorophenyl)ethynyl)piperazin-1-yl) -3-fluorophenyl)amino)-3-((R)-3-(3-methyl-2-oxoimidazolidin-1-yl)piperidin-1-yl)-1,2,4-triazin-6-carboxamide **225**
5-((4-(4-(3-(4-((2,6-dioxopiperidin-3-yl)carbamoyl)-3-methylphenyl)propan-2-yn-1-yl)pi perazin-1-yl)-3-fluorophenyl)amino)-3-((R)-3-(3-methyl-2-oxoimidazolidin-1-yl)piperidin-1-yl)-1,2,4-triazin-6-carboxamide **226**
5-((4-(4-(3-(1-(2,6-dioxopiperidin-3-yl)-3-methyl-2-oxo-2,3-dihydro-1H-benzo[d]imidaz ol-5-yl)propan-2-yn-1-yl)piperazin-1-yl)-3-fluorophenyl)amino)-3-((R)-3-(3-methyl-2-oxoimida zolidin-1-yl)piperidin-1-yl)-1,2,4-triazin-6-carboxamide **227**
5-((4-(4-(3-(4-((2,6-dioxopiperidin-3-yl)amino)-3-fluorophenyl)propan-2-yn-1-yl)piperaz in-1-yl)-3-fluorophenyl)amino)-3-((R)-3-(3-methyl-2-oxoimidazolidin-1-yl)piperidin-1-yl)-1,2,4 -triazin-6-carboxamide **228**
5-((4-(4-(3-(7-((2,6-dioxopiperidin-3-yl)amino)benzo[d]oxazol-4-yl)propan-2-yn-1-yl)pi perazin-1-yl)-3-fluorophenyl)amino)-3-((R)-3-(3-methyl-2-oxoimidazolidin-1-yl)piperidin-1-yl)-1,2,4-triazin-6-carboxamide **229**
5-((4-(4-(3-(5-((2,6-dioxopiperidin-3-yl)carbamoyl)thiophen-2-yl)propan-2-yn-1-yl)piper azin-1-yl)-3-fluorophenyl)amino)-3-((R)-3-(3-methyl-2-oxoimidazolidin-1-yl)piperidin-1-yl)-1,2, 4-triazin-6-carboxamide **230**
5-((3-((1-(2-(2,6-dioxopiperidin-3-yl)-1,3-dioxoisoindolin-5-yl)piperidin-4-yl)methyl)-9-fluoro-2,3,4,5-tetrahydro-1H-benzo[d]azepin-7-yl)amino)-3-((R)-3-(3-methyl-2-oxoimidazolidin -1-yl)piperidin-1-yl)-1,2,4-triazin-6-carboxamide **231**
5-((4-(4-((1-(2-(2,6-dioxopiperidin-3-yl)-1-oxo-1,2-dihydroisoquinolin-6-yl)piperidin-4-yl)methyl)piperazin-1-yl)-3-fluorophenyl)amino)-3-((R)-3-(3-methyl-2-oxoimidazolidin-1-yl)pip eridin-1-yl)-1,2,4-triazin-6-carboxamide **232**
(R)-5-((4-(4-((1-(3-carbamoyl-4-(2,4-dioxotetrahydropyrimidin-1 (2H)-yl)phenyl)piperidi n-4-yl)methyl)piperazin-1-yl)-3-fluorophenyl)amino)-3-(3-(3-methyl-2-oxoimidazolidin-1-yl)pip eridin-1-yl)-1,2,4-triazin-6-carboxamide **233**
(R)-5-((4-(1-((1-(4-(2,4-dioxotetrahydropyrimidin-1(2H)-yl)phenyl)piperidin-4-yl)methyl )piperidin-4-yl)-3 -fluorophenyl)amino)-3 -(3 -(3-methyl-2-oxoimidazolidin-1 -yl)piperidin-1 -yl)-1, 2,4-triazin-6-carboxamide **234**
(R)-5-((4-(4-((1-(4-(2,4-dioxotetrahydropyrimidin-1(2H)-yl)phenyl)piperidin-4-yl)methyl )-3-oxopiperazin-1-yl)-3-fluorophenyl)amino)-3-(3-(3-methyl-2-oxoimidazolidin-1-yl)piperidin-1-yl)-1,2,4-triazin-6-carboxamide **235**
(R)-5-((4-(1-((1-(4-(2,4-dioxotetrahydropyrimidin-1(2H)-yl)-3-methylphenyl)piperidin-4 -yl)methyl)piperidin-4-yl)phenyl)amino)-3-(3-(3-methyl-2-oxoimidazolidin-1-yl)piperidin-1-yl)-1,2,4-triazin-6-carboxamide **236**
(R)-5-((4-(1-((1-(4-(2,4-dioxotetrahydropyrimidin-1(2H)-yl)-3-methylphenyl)piperidin-4 -yl)methyl)piperidin-4-yl)-3-(hydroxymethyl)phenyl)amino)-3-(3-(3-methyl-2-oxoimidazolidin-1-yl)piperidin-1-yl)-1,2,4-triazin-6-carboxamide **237**
(R)-5-((4-(1-((1-(4-(2,4-dioxotetrahydropyrimidin-1(2H)-yl)-3-methylphenyl)piperidin-4 -yl)methyl)piperidin-4-yl)-2-(hydroxymethyl)phenyl)amino)-3-(3-(3-methyl-2-oxoimidazolidin-1-yl)piperidin-1-yl)-1,2,4-triazin-6-carboxamide **238**
(R)-5-((4-(1-(2-(1-(4-(2,4-dioxotetrahydropyrimidin-1(2H)-yl)-3-methylphenyl)azetidin-3-yl)acetyl)piperidin-4-yl)-2-(hydroxymethyl)phenyl)amino)-3-(3-(3-methyl-2-oxoimidazolidin-1-yl)piperidin-1-yl)-1,2,4-triazin-6-carboxamide **239**
(R)-5-((4-(1-((1-(4-(2,4-dioxotetrahydropyrimidin-1(2H)-yl)phenyl)piperidin-4-yl)methyl )piperidin-4-yl)-3-(hydroxymethyl)phenyl)amino)-3-(3-(3-methyl-2-oxoimidazolidin-1-yl)piperi din-1-yl)-1,2,4-triazin-6-carboxamide **240**
(R)-3-((4-(1-((1-(4-(2,4-dioxotetrahydropyrimidin-1(2H)-yl)-3-methylphenyl)piperidin-4 -yl)methyl)piperidin-4-yl)-3-fluorophenyl)amino)-6-fluoro-5-(3-(3-methyl-2-oxoimidazolidin-1-yl)piperidin-1-yl)pyrazin-2-carboxamide **241**
(R)-2-((4-(1-((1-(4-(2,4-dioxotetrahydropyrimidin-1(2H)-yl)-3-methylphenyl)piperidin-4 -yl)methyl)piperidin-4-yl)-3-fluorophenyl)amino)-3,5,6-trifluoro-4-(3-(3-methyl-2-oxoimidazoli din-1-yl)piperidin-1-yl)benzamide **242**
5-((4-(4-((1-(3-(2,6-dioxopiperidin-3-yl)benzo[d]isoxazol-6-yl)piperidin-4-yl)methyl)pip erazin-1-yl)-3-fluorophenyl)amino)-3-((R)-3-(3-methyl-2-oxoimidazolidin-1-yl)piperidin-1-yl)-1, 2,4-triazin-6-carboxamide **243**
5-((4-(4-((1-(3-(2,6-dioxopiperidin-3-yl)imidazo[1,2-a]pyridin-7-yl)piperidin-4-yl)methy l)piperazin-1-yl)-3-fluorophenyl)amino)-3-((R)-3-(3-methyl-2-oxoimidazolidin-1-yl)piperidin-1-yl)-1,2,4-triazin-6-carboxamide **244**
(R)-5-((4-(4-((1-(3-(2,4-dioxotetrahydropyrimidin-1(2H)-yl)imidazo[1,2-a]pyridin-7-yl)p iperidin-4-yl)methyl)piperazin-1-yl)-3-(3-(3-methyl-2-oxoimidazolidin-1-yl)piperidin-1-yl)-1,2, 4-triazin-6-carboxamide **245**
(R)-5-((4-(4-(3-(3-(2,4-dioxotetrahydropyrimidin-1(2H)-yl)imidazo[1,2-a]pyridin-7-yl)pr opan-2-yn-1-yl)piperazin-1-yl)-3-fluorophenyl)amino)-3-(3-(3-methyl-2-oxoimidazolidin-1-yl)pi peridin-1-yl)-1,2,4-triazin-6-carboxamide **246**
5-((4-(4-((1-(1-(2,6-dioxopiperidin-3-yl)-2-oxo-1,2-dihydrobenzo[cd]indol-6-yl)piperidin -4-yl)methyl)piperazin-1-yl)-3-fluorophenyl)amino)-3-((R)-3-(3-methyl-2-oxoimidazolidin-1-yl) piperidin-1-yl)-1,2,4-triazin-6-carboxamide **247**
5-((4-(4-(3-(1-(2,6-dioxopiperidin-3-yl)-2-oxo-1,2-dihydrobenzo[cd]indol-6-yl)propan-2-yn-1-yl)piperazin-1-yl)-3-fluorophenyl)amino)-3-((R)-3-(3-methyl-2-oxoimidazolidin-1-yl)piper idin-1-yl)-1,2,4-triazin-6-carboxamide **248**
5-((4-(4-((1-(5-((2,6-dioxopiperidin-3-yl)carbamoyl)naphthalen-1-yl)piperidin-4-yl)meth yl)piperazin-1-yl)-3-fluorophenyl)amino)-3-((R)-3-(3-methyl-2-oxoimidazolidin-1-yl)piperidin-1-yl)-1,2,4-triazin-6-carboxamide **249**
4-(4-((4-(4-((6-carbamoyl-3-((R)-3-(3-methyl-2-oxoimidazolidin-1-yl)piperidin-1-yl)-1,2, 4-triazin-5-yl)amino)-2-fluorophenyl)piperazin-1-yl)methyl)piperidin-1-yl)-N-(2,6-dioxopiperid in-3-yl)quinolin-8-carboxamide **250**
5-((4-(4-((1-(4-(2,6-dioxopiperidin-3-yl)amino)-2-fluorophenyl)piperidin-4-yl)methyl)pi perazin-1-yl)-3-fluorophenyl)amino)-3-((R)-3-(3-methyl-2-oxoimidazolidin-1-yl)piperidin-1-yl)-1,2,4-triazin-6-carboxamide **251**
5-((4-(4-((1-(4-(2,6-dioxopiperidin-3-yl)amino)-2-(trifluoromethyl)phenyl)piperidin-4-yl) methyl)piperazin-1-yl)-3-fluorophenyl)amino)-3-((R)-3-(3-methyl-2-oxoimidazolidin-1-yl)piperi din-1-yl)-1,2,4-triazin-6-carboxamide **252**
5-((4-(4-((1-(4-((2,6-dioxopiperidin-3-yl)amino)phenyl)piperidin-4-yl)methyl)piperazin-1-yl)-3-fluorophenyl)amino)-3-((R)-3-(3-methyl-2-oxoimidazolidin-1-yl)piperidin-1-yl)-1,2,4-tri azin-6-carboxamide **253**
5-((4-(4-((1-(4-((2,6-dioxopiperidin-3-yl)carbamoyl)-3-fluorophenyl)piperidin-4-yl)meth yl)piperazin-1-yl)-3-fluorophenyl)amino)-3-((R)-3-(3-methyl-2-oxoimidazolidin-1-yl)piperidin-1-yl)-1,2,4-triazin-6-carboxamide **254**
(R)-5-((4-(4-((1-(4-(2,4-dioxotetrahydropyrimidin-1(2H)-yl)phenyl)piperidin-4-yl)methyl )piperazin-1-yl)-3-fluorophenyl)amino)-3-(3-(3-methyl-2-oxoimidazolidin-1-yl)piperidin-1-yl)-1, 2,4-triazin-6-carboxamide **255**
5-((4-(4-((1-(3-(2,4-dioxotetrahydropyrimidin-1 (2H)-yl)-4-fluorophenyl)pyrrolidin-3-yl) methyl)piperazin-1-yl)-3-fluorophenyl)amino)-3-((R)-3-(3-methyl-2-oxoimidazolidin-1-yl)piperi din-1-yl)-1,2,4-triazin-6-carboxamide **256**
(R)-5-((4-(4-((1-(4-(2,4-dioxotetrahydropyrimidin-1(2H)-yl)-3-methylphenyl)piperidin-4 -yl)methyl)piperazin-1-yl)-3-fluorophenyl)amino)-3-(3-(3-methyl-2-oxoimidazolidin-1-yl)piperi din-1-yl)-1,2,4-triazin-6-carboxamide **257**
5-((4-(4-((1-(3-(2,4-dioxotetrahydropyrimidin-1 (2H)-yl)-4-methoxyphenyl)pyrrolidin-3-y l)methyl)piperazin-1-yl)-3-fluorophenyl)amino)-3-((R)-3-(3-methyl-2-oxoimidazolidin-1-yl)pipe ridin-1-yl)-1,2,4-triazin-6-carboxamide **258**
(R)-5-((4-(4-((1-(4-(2,4-dioxotetrahydropyrimidin-1 (2H)-yl)-3-methoxyphenyl)piperidin-4-yl)methyl)piperazin-1-yl)-3-fluorophenyl)amino)-3-(3-(3-methyl-2-oxoimidazolidin-1-yl)piper idin-1-yl)-1,2,4-triazin-6-carboxamide **259**

### Description of Terminology

Unless otherwise defined, all technical and scientific terms used herein have the same meaning as commonly understood by those skilled in the art. Unless otherwise specified, all patent documents, publicly disclosed materials, *etc.* referred to in the present invention are incorporated herein in their entirety. If there are multiple definitions for the same term in the present invention, the definition in this section shall prevail.

It should be understood that the foregoing general description and the following detailed description are merely exemplary and explanatory, and are not interpreted as limitations on any claim. It should be noted that in the specification and the appended claims, singular references such as "a", "an" and "the" include plural references unless the context specifies otherwise. It should also be noted that "or" means "and/or" unless specified otherwise. Furthermore, "comprise", "include", and similar terms are not limiting.

"Substituted" means that a hydrogen atom is substituted with a substituent. It should be noted that the substituent on a specific atom is constrained by its valence. In the definition part, "Cᵢ-Cⱼ" means a range including the starting point and the end point, where i and j are both integers, indicating the number of carbon atoms. For example, C₁-C₄, C₁-C₈, C₃-C₈, *etc.*

C, H, O, S, N, F, Cl, Br, I, *etc.* involved in the groups and compounds of the present invention all include their isotopes. At the same time, C, H, O, S, N, F, Cl, Br, and I involved in the groups and compounds of the present invention may be optionally substituted with one or more of their corresponding isotopes, including but not limited to isotopes of carbon ¹²C, ¹³C, and ¹⁴C, isotopes of hydrogen protium (H), deuterium (D), and tritium (T), isotopes of oxygen ¹⁶O, ¹⁷O, and ¹⁸O, isotopes of sulfur ³²S, ³³S, ³⁴S, and ³⁶S, isotopes of nitrogen ¹⁴N and ¹⁵N, isotopes of fluorine ¹⁷F and ¹⁹F, isotopes of chlorine ³⁵Cl and ³⁷Cl, isotopes of bromine ⁷⁹Br and ⁸¹Br, *etc.*

The term "alkyl" used in the present invention means a straight or branched saturated hydrocarbon group containing 1 to 8 carbon atoms, including but not limited to methyl, ethyl, propyl, isopropyl, n-butyl, isobutyl, sec-butyl, tert-butyl, n-amyl, isoamyl, neoamyl, tert-amyl, n-hexyl, isohexyl, neohexyl, heptyl, isoheptyl, neoheptyl, octyl, isooctyl, *etc.* The alkyl can be substituted with one or more substituents. When there are multiple substitutions occurring, the substituents can be the same or different; the substituents are independently D (deuterium), oxo, halogen, cyano, nitro, hydroxy, amino, aminoalkyl, alkenyl, alkynyl, carboxyl, carboxylic ester group, acyl, amido, methylsulfuryl, alkylamido, Ci-Cs alkyl, Ci-Cs alkoxy, Ci-Cs hydroxyalkyl, Ci-Cs alkylamino, haloC₁-C₈ alkyl, haloC₁-C₈ alkoxy, haloCi-Cshydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂ aryl, C₅-C₁₄ heteroaryl, C₃-C₁₂ heterocyclyl.

The term "alkenyl" used in the present invention means a straight or branched hydrocarbon chain group containing 1 to 8 carbon atoms and at least one C=C double bond, including but not limited to vinyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 1-methyl-2-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 1-methyl-2-hexenyl, 2-methyl-2-hexenyl, 2-methyl-3-hexenyl, 3,5-dimethyl-2-hexenyl, 3,3-dimethyl-1-pentenyl, 3-methyl-2-ethyl-1-butenyl, 1-octenyl, 2-octenyl, *etc.* The alkenyl can be substituted with one or more substituents. When there are multiple substitutions occurring, the substituents can be the same or different; the substituents are independently D, alkyl, haloalkyl, alkoxy, haloalkoxy, hydroxyalkyl, halohydroxyalkyl, alkylamino, haloalkylamino, cycloalkyl, halocycloalkyl, heterocyclyl, aryl, heteroaryl, hydroxyl, halogen, cyano, nitro, amino, aminoalkyl, carboxyl, amido, sulfonamido.

The term "alkynyl" used in the present invention means a straight or branched hydrocarbon chain group containing 1 to 8 carbon atoms and at least one C=C triple bond, including but not limited to ethynyl, 1-propynyl, 2-propynyl, 1-butynyl, 2-butynyl, 3-butynyl, 1-pentynyl, 2-pentynyl, 3-pentynyl, 4-pentynyl, 3-methyl-1-butynyl, 4-methyl-1-butynyl, 2-methyl-3-butynyl, 1-methyl-4-butynyl, 1-hexynyl, 2-hexynyl, 3-hexynyl, 4-hexynyl, 1-methyl-2-pentynyl, 1-methyl-3-pentynyl, 1-methyl-4-pentynyl, 2-methyl-3-pentynyl, 2,2-dimethyl-4-pentynyl, 1-heptynyl, 2-heptynyl, 3-heptynyl, 4-heptynyl, 5-heptynyl, 2-methyl-3-hexynyl, 3-methyl-1-hexynyl, 3,3-dimethyl-1-hexynyl, 4-methyl-1-hexynyl, *etc.* The alkynyl can be substituted with one or more substituents. When there are multiple substitutions occurring, the substituents can be the same or different; the substituents are independently D, alkyl, haloalkyl, alkoxy, haloalkoxy, hydroxyalkyl, halohydroxyalkyl, alkylamino, haloalkylamino, cycloalkyl, halocycloalkyl, heterocyclyl, aryl, heteroaryl, hydroxyl, halogen, cyano, nitro, amino, aminoalkyl, carboxyl, amido, sulfonamido.

The term "alkylene" used in the present invention means a linear or branched divalent saturated hydrocarbon group containing 1 to 8 carbon atoms, including but not limited to methylene, ethylene, propylene, butylene, pentylene, *etc.* The alkylene can be substituted with one or more substituents. When there are multiple substitutions occurring, the substituents can be the same or different; the substituents are independently D (deuterium), oxo, halogen, cyano, nitro, hydroxy, amino, aminoalkyl, alkenyl, alkynyl, carboxyl, carboxylic ester group, acyl, amido, methylsulfuryl, alkylamido, Ci-Cs alkyl, Ci-Cs alkoxy, Ci-Cs hydroxyalkyl, Ci-Cs alkylamino, haloC₁-C₈ alkyl, haloC₁-C₈ alkoxy, haloCi-Cshydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂ aryl, C₅-C₁₄ heteroaryl, C₃-C₁₂ heterocyclyl.

The term "halogen" used in the present invention means fluorine, chlorine, bromine and iodine, preferably, fluorine, chlorine and bromine.

The term "cycloalkyl" used in the present invention means a monocyclic or polycyclic (two monocyclic rings connected by chemical bonds or bridged ring or spiro ring or fused ring) non-aromatic monovalent hydrocarbon group having 3 to 12 carbon atoms, and possibly one or more chemical bonds that are double or triple bonds. "Cycloalkyl" includes, but is not limited to, cyclopropyl, cyclobutyl, cyclopentyl, cyclohexyl, cycloheptyl, cyclooctyl, cyclononyl, cyclodecyl, decalin, cyclopropyl-fused-cyclopropyl, cyclopropyl-fused-cyclobutyl, cyclopropyl-fused-cyclopentyl, cyclopropyl-fused-cyclohexyl, cyclopropyl-fused-cycloheptyl, cyclobutyl-fused-cyclobutyl, cyclobutyl-fused-cyclopentyl, cyclobutyl-fused-cyclohexyl, cyclobutyl-fused-cycloheptyl, cyclopentyl-fused-cyclopentyl, cyclopentyl-fused-cyclohexyl, cyclopentyl-fused-cycloheptyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclopropyl-spiro-cycloheptyl, cyclobutyl-spiro-cyclobutyl, cyclobutyl-spiro-cyclopentyl, cyclobutyl-spiro-cyclohexyl, cyclobutyl-spiro-cycloheptyl, cyclopentyl-spiro-cyclopentyl, cyclopentyl-spiro-cyclohexyl, cyclobutyl-spiro-cycloheptyl, cyclohexyl-spiro-cyclohexyl, bicyclo[1.1.1]pentyl, bicyclo[2.1.1]hexyl, bicyclo[3.1.1]heptyl, bicyclo[2.2.1]heptyl, bicyclo[4.1.1]octyl, bicyclo[3.2.1]octyl, bicyclo[3.2.1]octyl, bicyclo[5.1.1]nonyl, bicyclo[4.2.1]nonyl, bicyclo[4.3.1]nonyl, bicyclo[3.2.2]nonyl, bicyclo[5.2.1]decyl, bicyclo[4.2.2]decyl, *etc.* The cycloalkyl can be substituted with one or more substituents. When there are multiple substitutions occurring, the substituents can be the same or different; the substituents are independently D (deuterium), oxo, halogen, cyano, nitro, hydroxy, amino, aminoalkyl, alkenyl, alkynyl, carboxyl, carboxylic ester group, acyl, amido, methyl sulfuryl, alkylamido, Ci-Cs alkyl, Ci-Cs alkoxy, Ci-Cs hydroxyalkyl, Ci-Cs alkylamino, haloC₁-C₈ alkyl, haloC₁-C₈ alkoxy, haloCi-Cshydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂ aryl, C₅-C₁₄ heteroaryl, C₃-C₁₂ heterocyclyl.

The term "cycloalkenyl" used in the present invention means a monocyclic or polycyclic (two monocyclic rings connected by chemical bonds or bridged ring or spiro ring or fused ring) non-aromatic monovalent hydrocarbon group having 3 to 12 carbon atoms and at least one C=C double bond, including but not limited to cyclopropenyl, cyclobutenyl, cyclopentenyl, cyclohexenyl, cycloheptenyl, cycloalkenyl, spiro[2.2]pent-1-enyl, spiro[2.2]pent-1,4-dienyl, spiro[2.3]hex-1-enyl, spiro[2.3]hex-1,4-dienyl, spiro[3.3]hept-1-enyl, spiro[3.3]hept-1,5-dienyl, spiro[3.4]oct-1-enyl, spiro[3.4]oct-1,6-dienyl, spiro[3.4]oct-5-enyl, spiro[3.4]oct-6-enyl, spiro[3.4]oct-1-enyl, bicyclo[2.1.1]hex-1-enyl, bicyclo[2.1.1]hex-2-enyl, bicyclo[3.1.1]hept-1-enyl, bicyclo[3.1.1]hept-2-enyl, bicyclo[2.2.1]hept-1-enyl, bicyclo[2.2.1]hept-2-enyl, *etc.* The cycloalkene or cycloalkenyl can be substituted with one or more substituents. When there are multiple substitutions occurring, the substituents can be the same or different; the substituents are independently D (deuterium), oxo, halogen, cyano, nitro, hydroxy, aminoalkyl, alkenyl, alkynyl, carboxyl, carboxylic ester group, acyl, amido, methylsulfuryl, alkylamido, Ci-Cs alkyl, Ci-Cs alkoxy, Ci-Cs hydroxyalkyl, Ci-Cs alkylamino, haloC₁-C₈ alkyl, haloC₁-C₈ alkoxy, haloC₁-C₈ hydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂ aryl, C₅-C₁₂ heteroaryl, C₃-C₁₂ heterocyclyl.

The term "heterocyclyl" used in the present invention means a monocyclic or polycyclic (two monocyclic rings connected by chemical bonds or bridged ring or spiro ring or fused ring) non-aromatic ring group having 3 to 12 ring atoms, one or more heteroatoms selected from N, O, S, and possibly one or more chemical bonds that are double or triple bonds. Heterocyclyl, includes but are not limited to, pyranyl, piperidinyl, piperazinyl, morpholinyl, dioxanyl, oxacyclopropyl, oxacyclobutyl, oxacyclohexyl, oxacycloheptyl, oxacyclooctyl, azacyclopropyl, azacyclobutyl, azacyclopentyl, azacycloheptyl, azacyclooctyl, thiocyclobutyl, thiocyclopropyl, azacyclooctane group, oxazepinyl, diazepinyl, thiazepinyl, dihydrofuranyl, dihydrothienyl, dihydropyranyl, tetrahydrofuranyl, tetrahydrothienyl, tetrahydropyranyl, tetrahydrothiopyranyl, tetrahydrothiazolyl, tetrahydroimidazolyl, hexahydropyridazinyl, hexahydropyrimidinyl, 1-azaspiro[2.2]pentyl, 1-azaspiro[2.3]hexyl, 4-azaspiro[2.3]hexyl, 5-azaspiro[2.3]hexyl, 2-azaspiro[3.3]heptyl, 2,6-diazaspiro[3.3]heptyl, 2-oxa-6-azaspiro[3.3]heptane, 1-azaspiro[2.5] octyl, 2-azaspiro[3.4]octyl, 6-azaspiro[3.4]octyl, 2,6-diazaspiro[3.4]octyl, 2-azaspiro[3.5]nonyl, 6-azaspiro[3.5]nonyl, 7-azaspiro[3.5]nonyl, 2,7-diazaspiro[3.5]nonyl, 2-oxa-7-azaspiro[3.5] nonyl, 1-azaspiro[4.4]nonyl, 2-azaspiro[4.4]nonyl, 8-azaspiro[4.5]decyl, 2,8-diazaspiro[4.5] decyl, 1-oxaspiro[2.2]pentyl, 1-oxaspiro[2.3]hexyl, 4-oxaspiro[2.3]hexyl, 5-oxaspiro[2.3]hexyl, 2-oxaspiro[3.3]heptyl, 1-oxaspiro[2.5]octyl, 2-oxaspiro[3.4]octyl, 6-oxaspiro[3.4]octyl, 2-oxaspiro[3.5]nonyl, 6-oxaspiro[3.5]nonyl, 7-oxaspiro[3.5]nonyl, 1-oxaspiro[4.4]nonyl, 2-oxaspiro[4.4]nonyl, 8-oxaspiro[4.5]decyl, decahydroquinolyl, decahydroisoquinolyl, 2-azabicyclo[1.1.1]pentyl, 2-oxabicyclo[1.1.1]pentyl, azabicyclo[2.1.1]hexyl, oxabicyclo[2.1.1]hexyl, azabicyclo[3.1.1]heptyl, oxabicyclo[3.1.1]heptyl, azabicyclo[2.2.1]heptyl, azabicyclo[4.1.1]octyl, azabicyclo[3.2.1]octyl, azabicyclo[3.2.1]octyl, *etc.* The heterocyclyl can be substituted with one or more substituents. When there are multiple substitutions occurring, the substituents can be the same or different; the substituents are independently D (deuterium), oxo, halogen, cyano, nitro, hydroxy, amino, aminoalkyl, alkenyl, alkynyl, carboxyl, carboxylic ester group, acyl, amido, methylsulfuryl, alkylamido, Ci-Cs alkyl, Ci-Cs alkoxy, Ci-Cs hydroxyalkyl, C₁-C₈ alkylamino, haloC₁-C₈ alkyl, haloC₁-C₈ alkoxy, haloC₁-C₈ hydroxyalkyl, haloC₁-C₈ alkylamino, C₃-C₁₂ cycloalkyl, haloC₃-C₁₂ cycloalkyl, alkoxycarbonyl, alkylthio, alkylsulfonyl, hydroxyalkylamido, sulfonamido, C₆-C₁₂ aryl, C₅-C₁₄ heteroaryl, C₃-C₁₂ heterocyclyl.

The term "aryl" used in the present invention means an all-carbon monocyclic or fused polycyclic aromatic ring group (in the case of fused polycyclic rings, one of the fused rings can be partially saturated) having 6 to 12 carbon atoms, including but not limited to benzene ring, naphthalene ring, anthracene ring, indene ring, dihydroindenyl (indanyl), dihydronaphthalene ring, tetrahydronaphthalene ring, *etc.* Aryl can be unsubstituted or substituted, can be monosubstituted (such as ortho-, meta-, or para-substituted), can also be di-substituted or tri-substituted, *etc.* When there are multiple substitutions occurring, the substituents can be the same or different; the substituents are independently D (deuterium), halogen, cyano, nitro, hydroxy, amino, aminoalkyl, alkenyl, alkynyl, carboxyl, acyl, amido, alkylamido, Ci-Cs alkyl, Ci-Cs alkoxy, Ci-Cs hydroxyalkyl, Ci-Cs alkylamino, haloC₁-C₈ alkyl, haloC₁-C₈ alkoxy, haloC₁-C₈ hydroxyalkyl, haloC₁-C₈ alkylamino, C₃-C₁₂ cycloalkyl, haloC₃-C₁₂ cycloalkyl, alkoxycarbonyl, alkylthio, alkylsulfonyl, hydroxyalkylamido, sulfonamido, C₆-C₁₂ aryl, C₅-C₁₄ heteroaryl, C₃-C₁₂ heterocyclyl.

The term "heteroaryl" used in the present invention means a monocyclic or fused polycyclic aromatic ring group (in the case of fused polycyclic rings, one of the fused rings can be partially saturated) having 5 to 14 ring atoms, which is equivalent to that one or more carbons in the above "aryl" are replaced by heteroatoms such as N, O, S, *etc.* Heteroaromatic rings can be monocyclic ring or bicyclic ring, *i.e.,* formed by the fusion of two rings. Heteroaryl includes, but are not limited to, pyridyl, pyrimidinyl, pyrazinyl, pyridazinyl, triazinyl, isoxazolyl, isothiazolyl, pyrazolyl, thiazolyl, thienyl, furanyl, triazolyl, oxazolyl, imidazolyl, indazolyl, indolizinyl, indolyl, dihydroindolyl, isoindolinyl, quinolyl, isoquinolyl, quinoxalinyl, quinazolinyl, dihydroquinolinyl, tetrahydroquinolinyl, dihydroisoquinolinyl, dihydroisoquinolinyl, tetrahydroisoquinolinyl, pteridinyl, purinyl, benzoimidazolyl, benzofuranyl, benzothienyl, benzothiazolyl, benzotriazolyl, benzotriazinyl, benzoxadiazolyl, benzoxazolyl, benzoisoxazolyl, imidazopyridyl, imidazothiazolyl, pyrrolopyridyl, thienopyrrolyl, thienothienyl, thienopyridyl, thienopyrimidinyl, pyrazolopyrimidinyl, pyrrolopyrrolyl, pyrrolopyrimidinyl, pyrrolopyridazinyl, pyrrolopyrazinyl, imidazopyrimidinyl, imidazopyrazinyl, imidazopyridazinyl, pyrazolopyridyl, pyrazolopyridazinyl, pyrazolopyrazinyl, pyrimidopyridyl, pyrimidopyrazinyl, pyrimidopyridazinyl, pyrimidopyrimidinyl, pyridopyridyl, pyridopyrazinyl, pyridopyridazinyl, pyridazinopyridazinyl, pyridazinopyrazinyl, pyrazinopyrazinyl, *etc.* Heteroaryl can be unsubstituted or monosubstituted or polysubstituted. In the case of polysubstitution, the substituents can be the same or different; the substituents are independently D (deuterium), halogen, cyano, nitro, amino, aminoalkyl, hydroxy, carboxyl, carboxylic ester group, acyl, amido, alkylamido, alkoxycarbonyl, alkylthio, alkylsulfonyl, hydroxyalkylamido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, haloC₃-C₁₂ cycloalkyl, C₃-C₁₂ heterocyclyl, C₆-C₁₂ aryl, C₅-C₁₄ heteroaryl.

The terms "spiro ring" and "spiro ring group" used in the present invention mean a polycyclic structure, in which at least two rings share one atom (usually a C atom). In this polycyclic structure, one or more chemical bonds can be double bonds or triple bonds, and one or more heteroatoms can be present. The spiro ring group can be unsubstituted or monosubstituted or polysubstituted. In the case of polysubstitution, the substituents can be the same or different; the substituents are independently D (deuterium), oxo, halogen, cyano, nitro, hydroxy, amino, aminoalkyl, alkenyl, alkynyl, carboxyl, carboxylic ester group, acyl, amido, methyl sulfuryl, alkylamido, Ci-Cs alkyl, Ci-Cs alkoxy, Ci-Cs hydroxyalkyl, Ci-Cs alkylamino, haloC₁-C₈ alkyl, haloC₁-C₈ alkoxy, haloCi-Cshydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂ aryl, C₅-C₁₄ heteroaryl, C₃-C₁₂ heterocyclyl.

The term "bridged ring group" used in the present invention means a polycyclic structure, in which at least two rings share two or more atoms. In this polycyclic structure, one or more chemical bonds can be double bonds or triple bonds, and one or more heteroatoms can be present. The bridged ring group can be unsubstituted or monosubstituted or polysubstituted. In case of polysubstitution, the substituents can be the same or different; the substituents are independently D (deuterium), oxo, halogen, cyano, nitro, hydroxy, amino, aminoalkyl, alkenyl, alkynyl, carboxyl, carboxylic ester group, acyl, amido, methylsulfuryl, alkylamido, Ci-Cs alkyl, Ci-Cs alkoxy, Ci-Cs hydroxyalkyl, Ci-Cs alkylamino, haloC₁-C₈ alkyl, haloC₁-C₈ alkoxy, haloCi-Cshydroxyalkyl, haloC₁-C₈ alkylamino, C₃-C₁₂ cycloalkyl, haloC₃-C₁₂ cycloalkyl, cycloalkenyl, cycloalkynyl, alkoxycarbonyl, alkylthio, alkylsulfonyl, hydroxyalkylamido, sulfonamido, C₆-C₁₂ aryl, C₅-C₁₄ heteroaryl, C₃-C₁₂ heterocyclyl.

The term "alkoxy" used in the present invention means alkyl-O-, in which the alkyl is as defined above. Examples of "alkoxy" used in the present invention include, but are not limited to, methoxy, ethoxy, n-propoxy, isopropoxy, n-butoxy, sec-butoxy, tert-butoxy, pentoxy, isopentyloxy, neopentyloxy, *etc.* "Alkoxy" also includes substituted alkoxy, the substituents of which may be D, halogen, oxo, amino, hydroxy, cyano, nitro, carboxyl, amido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, C₅-C₁₄ heteroaryl.

The term "hydroxyalkyl" used in the present invention means -alkyl-OH, in which the alkyl is as defined above. Examples of "hydroxyalkyl" used in the present invention include, but are not limited to, hydroxymethyl, hydroxyethyl, hydroxypropyl, hydroxyisopropyl, *etc.* "Hydroxyalkyl" also includes substituted hydroxyalkyl, the substituents of which may be D, halogen, oxo, amino, hydroxy, cyano, nitro, carboxyl, amido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, C₅-C₁₄ heteroaryl.

The term "aminoalkyl" used in the present invention means -alkyl-NH₂, in which the alkyl is as defined above. Examples of "aminoalkyl" used in the present invention include, but are not limited to, aminomethyl, aminoethyl, aminopropyl, aminoisopropyl, *etc.* "Aminoalkyl" also includes substituted aminoalkyl, the substituents of which may be D, halogen, oxo, amino, hydroxy, cyano, nitro, carboxyl, amido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, C₅-C₁₄ heteroaryl, and the substituents of which can be on alkyl or on N.

The term "alkylamino" used in the present invention means -NH-alkyl, in which the alkyl is as defined above. Examples of "alkylamino" used in the present invention include, but are not limited to, methylamino, ethylamino, propylamino, isopropylamino, *etc.* "Alkylamino" also includes substituted alkylamino, the substituents of which may be D, halogen, oxo, amino, hydroxy, cyano, nitro, carboxyl, amido, sulfonamido, Ci-Csalkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, C₅-C₁₄ heteroaryl, and the substituents of which can be on alkyl or on N.

The term "alkylcarbonyl" used in the present invention means alkyl-C(O)-, in which the alkyl is as defined above. "Alkylcarbonyl" also includes substituted alkylcarbonyl, the substituents of which may be D, halogen, oxo, amino, hydroxy, cyano, nitro, carboxyl, amido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, C₅-C₁₄ heteroaryl. The "C(O)" represents C=O.

The term "alkoxycarbonyl" used in the present invention means alkyl-O-C(O)-, in which the alkyl is as defined above. "Alkoxycarbonyl" also includes substituted alkoxycarbonyl, the substituents of which may be D, halogen, oxo, amino, hydroxy, cyano, nitro, carboxyl, amido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, and C₅-C₁₄ heteroaryl. The "C(O)" represents C=O.

The term "heterocycloalkoxy" used in the present invention means heterocycloalkyl-O-, in which the heterocycloalkyl is as defined above. "Heterocycloalkoxy" also includes substituted heterocycloalkoxy, the substituents of which may be D, halogen, oxo, amino, hydroxy, cyano, nitro, carboxyl, amido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, C₅-C₁₄ heteroaryl.

The term "cycloalkoxy" used in the present invention means cycloalkyl-O-, in which the cycloalkyl is as defined above. "Cycloalkoxy" also includes substituted cycloalkoxy, the substituents of which may be D, halogen, oxo, amino, hydroxy, cyano, nitro, carboxyl, amido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, and C₅-C₁₄ heteroaryl.

The term "cycloalkylamino" used in the present invention means cycloalkyl-NH-, in which the cycloalkyl is as defined above. "Cycloalkylamino" also includes substituted cycloalkylamino, the substituent of which may be D, halogen, oxo, amino, hydroxy, cyano, nitro, carboxyl, amido, sulfonamido, Ci-Cs alkyl, Ci-Cs hydroxyalkyl, Ci-Cs alkoxy, Ci-Cs alkylamino, haloC₁-C₈ alkyl, haloC₁-C₈ hydroxyalkyl, haloC₁-C₈ alkoxy, haloC₁-C₈ alkylamino, C₃-C₁₂ cycloalkyl, C₃-C₁₂ heterocyclyl, C₆-C₁₂ aryl, C₅-C₁₄ heteroaryl.

The term "alkylthio" used in the present invention means alkyl-S-, in which the alkyl is as defined above. Alkylthio groups include, but are not limited to, methylthio, ethylthio, propylthio, butylthio, *etc.*

The term "alkanoyl" used in the present invention means alkyl-C(O)-, in which the alkyl is as defined above.

The term "alkylsulfonyl" used in the present invention means alkyl-S(O)₂-, in which the alkyl is as defined above.

The term "haloalkyl" used in the present invention means a straight or branched alkyl group substituted with halogens (preferably fluorine, chlorine, bromine, iodine), in which the "alkyl" is as defined above. Examples of "haloalkyl" used in the present invention include, but are not limited to, fluoromethyl, difluoromethyl, trifluoromethyl, chloromethyl, dichloromethyl, trichloromethyl, 2,2,2-trifluoroethyl, 2-fluoroethyl, 2-chloroethyl, tetrafluoroethyl, pentafluoroethyl, 1,1,1-trifluoroprop-2-yl, *etc.* "Haloalkyl" can be substituted one or more times by halogens.

The term "halohydroxyalkyl" used in the present invention means a hydroxyalkyl group substituted with halogens (preferably fluorine, chlorine, bromine, iodine), in which the hydroxyalkyl is as defined above. "Halohydroxyalkyl" can be substituted one or more times by halogens.

The term "haloalkoxy" used in the present invention means a hydroxyalkyl group substituted with halogens (preferably fluorine, chlorine, bromine, iodine), in which the alkoxy is as defined above. "Haloalkoxy" can be substituted one or more times by halogens.

The term "haloalkylamino" used in the present invention means an alkylamino group substituted with halogens (preferably fluorine, chlorine, bromine, iodine), in which the alkylamino is as defined above. "Haloalkylamino" can be substituted one or more times by halogens.

To avoid ambiguity, for example: when reference is made to alkyl, cycloalkyl, heterocyclyl, aryl and/or heteroaryl substitutions, it is intended that each of these groups is substituted individually, or that a combination of these groups is substituted.

"Pharmaceutically acceptable salt" refers to a salt formed by the compound of the present invention with an acid or a base, and is suitable for use as a dug. Pharmaceutically acceptable salts include inorganic salts and organic salts. One preferred class of salts are the salts formed by the compounds of the invention with acids. Suitable salt-forming acids include, but are not limited to: inorganic acids such as hydrochloric acid, hydrobromic acid, hydrofluoric acid, hydroiodic acid, sulfuric acid, nitric acid, phosphoric acid, carbonic acid, organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, p-toluenesulfonic acid, ethanesulfonic acid, benzenesulfonic acid, camphorsulfonic acid, 2-O-β-D-glucopyranosyl-L-ascorbic acid, isonicotinic acid, salicylic acid, ascorbic acid, gentisic acid, gluconic acid, pyruvic acid, naphthalenesulfonic acid, stearic acid, phenylacetic acid, p-aminobenzenesulfonic acid, isethionic acid, pamoic acid, tannic acid; and acidic amino acids such as aspartic acid,glutamic acid. One preferred class of salts are the salts formed by the compounds of the invention with bases. Suitable salt-forming bases include, but are not limited to: inorganic bases such as sodium hydroxide, potassium hydroxide, sodium carbonate, sodium bicarbonate, and sodium phosphate, and organic bases such as ammonia, triethylamine, diethylamine, piperazine, guanidine, and diethanolamine.

The second object of the present invention is to provide a pharmaceutical composition including one or more of the compounds described in any of the above technical solutions. The pharmaceutical composition of the present invention can be composed of one or more of the compounds described in any of the above technical solutions and other compounds, or composed of one or more of the compounds described in any of the above technical solutions.

The present invention provides a pharmaceutical preparation, which comprises at least one active component, and the active component is one or more of the compounds described in any of the above technical solutions. The pharmaceutical preparation comprises at least one active component and one or more pharmaceutically acceptable carriers or excipients. The active component can be any one or more of the BTK inhibitor compound of the present invention, an optical isomer of the compound, a pharmaceutically acceptable salt of the compound or its optical isomer, a solvate of the compound or its optical isomer.

The carriers include conventional diluents, excipients, fillers, binders, wetting agents, disintegrants, absorption accelerators, surfactants, adsorption carriers, lubricants, *etc.* in the pharmaceutical field, and when necessary, flavoring agents, sweeteners, *etc.* can also be added.

The drug of the present invention can be formulated into various forms such as tablets, powders, granules, capsules, oral liquids, and injections. The drugs in each of the above dosage forms can be formulated according to conventional methods in the pharmaceutical field.

On the other hand, the present invention provides the use of the compound of the general formula VI disclosed herein and its optical isomers or pharmaceutically acceptable salts or solvates thereof to inhibit Bruton's tyrosine kinase (Btk) activity, IKZF1 activity, or to treat diseases, disorders, or conditions that would benefit from the inhibition of Bruton's tyrosine kinase (Btk) activity, IKZF1 activity.

In a further preferred embodiment, the present invention provides a method for inhibiting the Bruton's tyrosine kinase activity in a subject in need by administering a composition containing a therapeutically effective amount of at least one compound to the subject, wherein the structural formula of the compound is set forth in the general formula VI. In some embodiments, the subject in need thereof suffers from an autoimmune disease, such as inflammatory bowel disease, arthritis, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, multiple sclerosis, Guillain-Barré syndrome, acute disseminated encephalomyelitis, Addison's disease, optic opsoclonus-myoclonus syndrome, ankylosing spondylitis, antiphospholipid antibody syndrome, aplastic anemia, autoimmune hepatitis, coeliac disease, Goodpasture's syndrome, immune thrombocytopenic purpura (ITP), optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behcet's disease, chronic fatigue, familial dysautonomia, endometriosis, interstitial cystitis, neuromyotonia, scleroderma or vulvodynia and chronic graft-versus-host disease (cGvHD).

In a further embodiment, the subject in need suffers from a cancer. In one embodiment, the cancer is a B cell or plasma cell proliferative disease, such as diffuse large B cell lymphoma, follicular lymphoma, small lymphocytic lymphoma, chronic lymphocytic leukemia, B cell prolymphocytic leukemia, lymphoplasmacytic lymphoma/Waldenström macroglobulinemia, splenic marginal zone lymphoma, plasma cell myeloma, plasmacytoma, extranodal marginal zone B cell lymphoma, lymph node marginal zone B cell lymphoma, mantle cell lymphoma, mediastinum (thymus) large B cell lymphoma, intravascular large B cell lymphoma, primary effusion lymphoma, Burkitt lymphoma/leukemia, multiple myeloma, or lymphomatoid granulomatosis.

The present invention also provides the use of the compounds of the present invention or pharmaceutically acceptable salts thereof in the preparation of BTK inhibitors, especially their use in the treatment of cell proliferation diseases. The cell proliferative diseases include cancers. In other words, the invention also provides the use of the compound of the general formula VI, its optical isomers or pharmaceutically acceptable salts or solvates thereof alone or in combination with other drugs in the treatment of proliferative diseases (such as cancers). Antitumor drugs that can be used in combination with the compounds provided by the present invention or their pharmaceutically acceptable salts thereof include, but are not limited to, at least one of the following types: mitotic inhibitors (such as vinblastine, vindesine, and vinorelbine); tubulin breakdown inhibitors (such as Taxol)); alkylating reagents (such as cisplatin, carboplatin and cyclophosphamide); antimetabolites (such as 5-fluorouracil, tegafur, methotrexate, cytarabine, and hydroxyurea); antibiotics can be inserted (such as doxorubicin, mitomycin and bleomycin)); enzymes (such as aspartase); topoisomerase inhibitors (such as etoposide and camptothecin); biological response modifiers (such as interferons); immune checkpoint inhibitors (such as PD-1 inhibitors, PD-L1 inhibitors, CTLA-4 inhibitors); CD20 monoclonal antibody; BCL2 inhibitors.

The inventors of the present invention have confirmed through experiments that the compounds of the present invention can degrade BTK.

The inventors of the present invention have confirmed through experiments that the compounds of the present invention can degrade BTKC481S.

The inventors of the present invention have confirmed through experiments that the compounds of the present invention have anti-proliferation inhibitory effects on Mino, OCI-LY10 tumor cell lines.

The inventors of the present invention have confirmed through experiments that the compounds of the present invention have good oral absorption properties in mice.

The inventors of the present invention have confirmed through experiments that the compounds of the present invention have good oral absorption properties in dogs.

The inventors of the present invention have confirmed through experiments that the compounds of the present invention have a strong effect on inhibiting tumor growth *in vivo.*

### DETAILED DESCRIPTION OF EMBODIMENTS

### Intermediate 1. cyclopentyl-3-(piperidin-3-yl)imidazolin-2-one

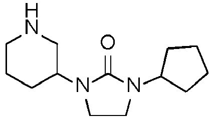

### Synthesis step 1. Tert-butyl 3-(3-(2-Chloroethyl)ureido)piperidin-1-carboxylate (1-1)

N-Boc-3-aminopiperidine (25.0 g, 125 mmol) and triethylamine (25.3 g, 250 mmol) were dissolved in dichloromethane (250 mL), and chloroethyl isocyanate (15.8 g, 150 mmol) was slowly added dropwise. After 4 h of reaction, water was added, the organic layers were combined, dried over anhydrous sodium sulfate, and the solvent was removed under reduced pressure. The residue was dissolved in ethyl acetate solution, the mixture was filtered, the solvent was removed under reduced pressure, and purification was performed by column chromatography to obtain tert-butyl 3-(3-(2-chloroethyl)ureido)piperidine-1-carboxylate (1-1) as a solid. ESI-MS: *m*/*z =* 306 [M+H]⁺.

### Synthesis step 2. Tert-butyl 3-(2-Oxoimidazolin-1-yl)piperidin-1-carboxylate (1-2)

Tert-butyl 3-(3-(2-chloroethyl)ureido)piperidine-1-carboxylate (4.03 g, 13.2 mmol) was dissolved in anhydrous tetrahydrofuran (40 mL), and 60% sodium hydride (1.06 g, 26.4 mmol) was added in ice bath, and the mixture was reacted at room temperature overnight under stirring. 60% sodium hydride (0.57 g, 14.1 mmol) was added again, the mixture was reacted for 2 h, the reaction was quenched by adding water, extracted with dichloromethane. The organic layers were combined, washed with saturated sodium chloride, dried over anhydrous sodium sulfate, and filtered with suction. The solvent was removed under pressure and purification was performed by column chromatography to obtain compound tert-butyl 3-(2-oxoimidazolin-1-yl)piperidine-1-carboxylate (1-2). ESI-MS: *m*/*z* = 270 [M+H]⁺.

### Synthesis step 3. Tert-butyl 3-(3-Cyclopentyl-2-oxoimidazolin-1-yl)piperidin-1 -carboxylate (1-3)

tert-butyl 3-(2-oxoimidazolin-1-yl)piperidine-1-carboxylate (3.38 g, 12.5 mmol) was dissolved in anhydrous tetrahydrofuran (40 mL), and 60% sodium hydride (1.01 g, 25.1 mmol) was added in ice bath, and after 5 min, the mixture was reacted at room temperature for 1 h under stirring. The mixture was placed back into the ice bath, iodocyclopentane (3.68 g, 18.8 mmol) was slowly added dropwise, and the resulting mixture was reacted at room temperature for 16 h under stirring, and then the reaction was quenched by adding water, and extracted with dichloromethane. The organic layers were combined, washed with saturated sodium chloride, dried over anhydrous sodium sulfate, and filtered with suction. The solvent was removed under pressure and purification was performed by column chromatography to obtain compound tert-butyl 3-(3-cyclopentyl-2-oxoimidazolin-1-yl)piperidine-1-carboxylate (1-3). ESI-MS: *m*/*z =* 338 [M+H]⁺.

### Synthesis step 4. 1-cyclopentyl-3-(piperidin-3-yl)imidazolin-2-one(Intermediate 1)

tert-butyl 3-(3-cyclopentyl-2-oxoimidazolin-1-yl)piperidine-1-carboxylate (3.38 g, 10 mmol) was dissolved in dioxane (35 mL), 4N HCl (31 mL) was slowly added dropwise and the mixture was reacted at room temperature for 2 h under stirring. The solvent was removed from the reaction liquid under reduced pressure to obtain 1-cyclopentyl-3-(piperidin-3-yl)imidazolin-2-one (Intermediate 1). ESI-MS: *m*/*z* = 238 [M+H]⁺.

### Intermediate 2. 1-(oxetan-3-yl)-3-(piperidin-3-yl)imidazolin-2-one

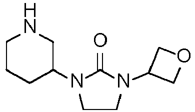

The synthesis method was the same as that for Intermediate 1, except that the iodocyclopentane in synthesis step 3 was replaced with 3-iodooxetane to obtain Intermediate 2, ESI-MS: *m*/*z* = 226 [M+H]⁺.

### Intermediate 3. 1-cyclohexyl-3-(piperidin-3-yl)imidazolin-2-one

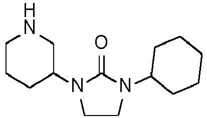

The synthesis method was the same as that for Intermediate 1, except that the iodocyclopentane in synthesis step 3 was replaced with iodocyclohexane to obtain Intermediate 3, ESI-MS: *m*/*z* = 252 [M+H]⁺.

### Intermediate 4. 1-(piperidin-3-yl)-3-(tetrahydro-2H-pyran-4-yl)imidazolin-2-one

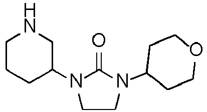

The synthesis method was the same as that for Intermediate 1, except that the iodocyclopentane in synthesis step 3 was replaced with 4-iodotetrahydropyran to obtain Intermediate 4, ESI-MS: *m*/*z* = 254 [M+H]⁺.

### Intermediate 5. 1-methyl-3-(piperidin-3-yl)imidazolin-2-one

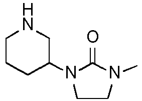

The synthesis method was the same as that for Intermediate 1, except that the iodocyclopentane in synthesis step 3 was replaced with methyl iodide to obtain Intermediate 5, ESI-MS: *m*/*z* = 184 [M+H]⁺.

### Intermediate 6. 1-(-2-azabicyclo[2.2.1]heptan-6-yl)-3-methylimidazolin-2-one

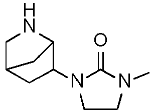

The synthesis method was the same as that for Intermediate 5, except that N-Boc-3-aminopiperidine in synthesis step 1 was replaced with tert-butyl 6-amino-2-azabicyclo[2.2.1]heptane-2-carboxylate to obtain the Intermediate 6: 1-(-2-azabicyclo[2.2.1]heptan-6-yl)-3-methylimidazolin-2-one, ESI-MS: m/z = 196 [M+H]⁺.

### Intermediate 7. 1-(-2-azabicyclo[2.2.1]heptan-6-yl)-3-isopropylimidazolin-2-one

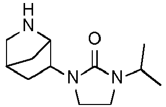

The synthesis method was the same as that for Intermediate 6, except that methyl iodide in synthesis step 3 was replaced with 2-iodopropane to obtain Intermediate 7, ESI-MS: m/z = 224 [M+H]⁺.

### Intermediate 8. 1-(-2-azabicyclo[2.2.2]octan-6-yl)-3-methylimidazolin-2-one

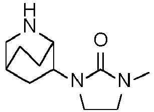

The synthesis method was the same as that for Intermediate 5, except that N-Boc-3-aminopiperidine in synthesis step 1 was replaced with tert-butyl 6-amino-2-azabicyclo[2.2.2]octane-2-carboxylate to obtain the Intermediate 8. ESI-MS: m/z = 210 [M+H]⁺.

### Intermediate 9. 3-chloro-5-(3-(3-cyclopentyl-2-oxoimidazolin-1-yl)piperidin-1-yl) pyrazin-2-carbonitrile

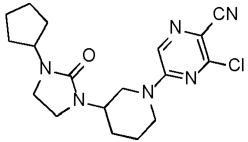

The Intermediate 1, 1-cyclopentyl-3-(piperidin-3-yl)imidazolin-2-one (2.38 g, 10 mmol), and N,N-diisopropylethylamine (7 mL) were dissolved in DMF (20 mL) solution and the mixture was placed in an ice bath. 3,5-dichloropyrazine-2-carbonitrile (1.74 g, 10 mmol) was added, the mixture was reacted for 15 min, then placed at room temperature and reacted for another 16 h. The reaction was completed, water was added, and extracted with ethyl acetate. The organic layers were combined, washed twice with water, washed with saturated sodium chloride, dried with anhydrous sodium sulfate, and filtered with suction. The solvent was removed under reduced pressure, and purification was performed by column chromatography to obtain compound 3-chloro-5-(3-(3-cyclopentyl-2-oxoimidazolin-1-yl)piperidin-1-yl)pyrazine-2 -carbonitrile. ESI-MS: *m*/*z* = 375 [M+H]⁺.

### Intermediate 10. 3-chloro-5-(3-(3-(oxetan-3-yl)-2-oxoimidazolin-1-yl)piperidin-1-yl) pyrazin-2-carbonitrile

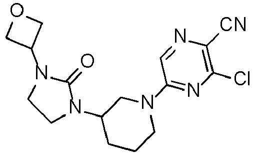

The synthesis method was the same as that for Intermediate 9, except that Intermediate 1 was replaced with Intermediate 2 to obtain Intermediate 10, ESI-MS: m/z = 363 [M+H]⁺.

### Intermediate 11. 3-chloro-5-(3-(3-cyclohexyl-2-oxoimidazolin-1-yl)piperidin-1-yl) pyrazin-2-carbonitrile

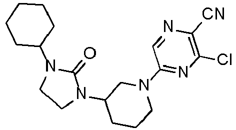

The synthesis method was the same as that for Intermediate 9, except that Intermediate 1 was replaced with Intermediate 3 to obtain Intermediate 11, ESI-MS: m/z = 389 [M+H]⁺.

### Intermediate 12. 3-chloro-5-(3-(2-oxo-3-(tetrahydro-2H-pyran-4-yl)imidazolin-1-yl) piperidin-1-yl)pyrazin-2-carbonitrile

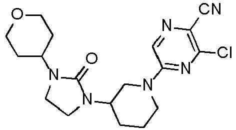

The synthesis method was the same as that for Intermediate 9, except that Intermediate 1 was replaced with Intermediate 4 to obtain Intermediate 12, ESI-MS: m/z = 391 [M+H]⁺.

### Intermediate 13. 3-chloro-5-(3-(3-oxotetrahydro-1H-pyrrolo[1,2-c]imidazol-2(3H)-yl) piperidin-1-yl)pyrazin-2-carbonitrile

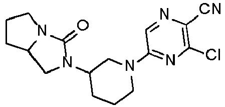

The synthesis method was the same as that for Intermediate 9, except that Intermediate 1 was replaced with 2-(piperidin-3-yl)hexahydro-3H-pyrrolo[1,2-c]imidazol-3-one to obtain Intermediate 13, ESI-MS: m/z = 347 [M+H]⁺.

### Intermediate 14. 3-chloro-5-(3-(3-oxohexahydroimidazo[1,5-a]pyridin-2(3H)-yl) piperidin-1-yl)pyrazin-2-carbonitrile

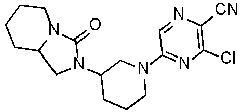

The synthesis method was the same as that for Intermediate 9, except that Intermediate 1 was replaced with 2-(piperidin-3-yl)hexahydroimidazo[1,5-a]pyridin-3(2H)-one to obtain Intermediate 14, ESI-MS: m/z = 361 [M+H]⁺.

### Intermediate 15. 3-chloro-5-(3-(3-methyl-2-oxoimidazolin-1-yl)piperidin-1-yl) pyrazin-2-carbonitrile

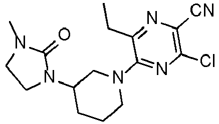

The synthesis method was the same as that for Intermediate 9, except that Intermediate 1 was replaced with Intermediate 5 and 3,5-dichloropyrazine-2-carbonitrile was replaced with 3,5-dichloro-6-ethylpyrazine-2-carbonitrile to obtain Intermediate 15, ESI-MS: m/z = 349 [M+H]⁺.

### Intermediate 16. 3-chloro-5-(3-(3-methyl-2-oxohexacyclopenta[d]imidazol-1(2H)-yl) piperidin-1-yl)pyrazin-2-carbonitrile

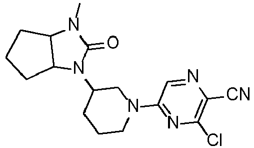

The synthesis method was the same as that for Intermediate 9, except that Intermediate 1 was replaced with 1-methyl-3-(piperidin-3-yl)hexahydrocyclopenta[d]imidazol-2(1H)-one to obtain Intermediate 16, ESI-MS: m/z = 361 [M+H]⁺.

### Intermediate 17. 3-chloro-5-(3-(3-methyl-2-oxooctahydro-1H-benzo[d]imidazol-1-yl) piperidin-1-yl)pyrazin-2-carbonitrile

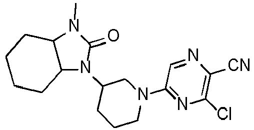

The synthesis method was the same as that for Intermediate 9, except that Intermediate 1 was replaced with 1-methyl-3-(piperidin-3-yl)octahydro-2H-benzo[d]imidazol-2-one to obtain Intermediate 17, ESI-MS: m/z = 375 [M+H]⁺.

### Intermediate 18. 3-chloro-5-(3-(4-methyl-5-oxo-4,6-diazaspirocyclo[2.4]heptan-6-yl) piperidin-1-yl)pyrazin-2-carbonitrile

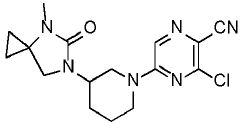

The synthesis method was the same as that for Intermediate 9, except that Intermediate 1 was replaced with 4-methyl-6-(piperidin-3-yl)-4,6-diazaspirocyclo[2.4]heptan-5-one hydrochloride to obtain Intermediate 18, ESI-MS: m/z = 347 [M+H]⁺.

### Intermediate 19. 3-chloro-5-(3-(5-methyl-6-oxo-5,7-diazaspirocyclo[3.4]octan-7-yl) piperidin-1-yl)pyrazin-2-carbonitrile

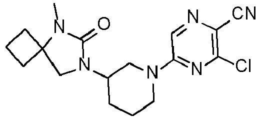

The synthesis method was the same as that for Intermediate 9, except that Intermediate 1 was replaced with 5-methyl-7-(piperidin-3-yl)-5,7-diazaspirocyclo[3.4]octane-6-one hydrochloride to obtain Intermediate 19, ESI-MS: m/z = 361 [M+H]⁺.

### Intermediate 20. 3-chloro-5-(3-(3-methyl-2-oxo-2,3-dihydro-1H-benzo[d]imidazol-1-yl)piperidin-1-yl)pyrazin-2-carbonitrile

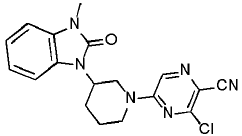

The synthesis method was the same as that for Intermediate 9, except that Intermediate 1 was replaced with 1-methyl-3-(piperidin-3-yl)-1,3-dihydro-2H-benzo[d]imidazol-2-one hydrochloride to obtain Intermediate 20, ESI-MS: m/z = 369 [M+H]⁺.

### Intermediate 21. 3-chloro-5-(3-(3-oxoimidazo[1,5-a]pyridin-2(3H)-yl)piperidin-1-yl) pyrazin-2-carbonitrile

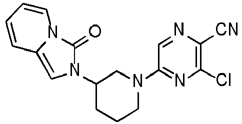

The synthesis method was the same as that for Intermediate 9, except that Intermediate 1 was replaced with 2-(piperidin-3-yl)imidazo[1,5-a]pyridin-3(2H)-one to obtain Intermediate 21, ESI-MS: m/z = 355 [M+H]⁺.

### Intermediate 22. (-5-chloro-3-(3-(3-methyl-2-oxoimidazolin-1-yl)piperidin-1-yl)-1,2,4-triazin-6-carbonitrile

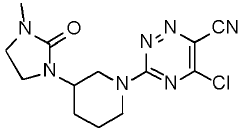

The synthesis method was the same as that for Intermediate 9, except that Intermediate 1 was replaced with Intermediate 5 and 3,5-dichloropyrazine-2-carbonitrile was replaced with 3,5-dichloro-1,2,4-triazine-6-nitrile to obtain Intermediate 22, ESI-MS: m/z = 322 [M+H]⁺.

### Intermediate 23. (-4-chloro-2-(3-(3-methyl-2-oxoimidazolin-1-yl)piperidin-1-yl) pyrimidin-5-carbonitrile

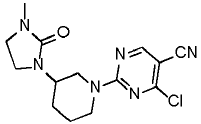

The synthesis method was the same as that for Intermediate 9, except that Intermediate 1 was replaced with Intermediate 5 and 3,5-dichloropyrazine-2-carbonitrile was replaced with 2,4-dichloro-5-cyanopyrimidine to obtain Intermediate 23, ESI-MS: m/z = 321 [M+H]⁺.

### Intermediate 24. 2-chloro-5-fluoro-6-(3-(3-methyl-2-oxoimidazolin-1-yl)piperidin-1-yl)-3-cyanopyridine

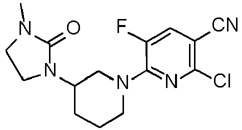

The synthesis method was the same as that for Intermediate 9, except that Intermediate 1 was replaced with Intermediate 5 and 3,5-dichloropyrazine-2-carbonitrile was replaced with 3-cyano-2,6-dichloro-5-fluoropyridine to obtain Intermediate 24, ESI-MS: m/z = 338 [M+H]⁺.

### Intermediate 25. 2-chloro-5-methoxy-6-(3-(3-methyl-2-oxoimidazolin-1-yl)piperidin -1-yl)-3-cyanopyridine

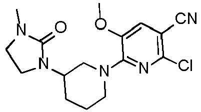

The synthesis method was the same as that for Intermediate 9, except that Intermediate 1 was replaced with Intermediate 5 and 3,5-dichloropyrazine-2-carbonitrile was replaced with 2,6-dichloro-5-methoxynicotinonitrile to obtain Intermediate 25, ESI-MS: m/z = 350 [M+H]⁺.

### Intermediate 26. 3-chloro-5-(-6-(3-methyl-2-oxoimidazolin-1-yl)-2-azabicyclo[2.2.1] heptan-2-yl)pyrazin-2-carbonitrile

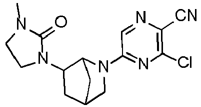

The synthesis method was the same as that for Intermediate 9, except that Intermediate 1 was replaced with Intermediate 6 to obtain Intermediate 26, ESI-MS: m/z = 333 [M+H]⁺.

### Intermediate 27. 3-chloro-5-(-6-(3-isopropyl-2-oxoimidazolin-1-yl)-2-azabicyclo [2.2.1]heptan-2-yl)pyrazin-2-carbonitrile

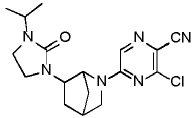

The synthesis method was the same as that for Intermediate 9, except that Intermediate 1 was replaced with Intermediate 7 to obtain Intermediate 27, ESI-MS: m/z = 361 [M+H]⁺.

### Intermediate 28. 2-chloro-6-(-6-(3-methyl-2-oxoimidazolin-1-yl)-2-azabicyclo[2.2.1] heptan-2-yl)nicotinonitrile

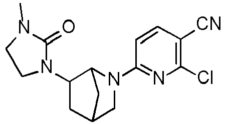

The synthesis method was the same as that for Intermediate 9, except that Intermediate 1 was replaced with Intermediate 6 and 3,5-dichloropyrazine-2-carbonitrile was replaced with 2,6-dichloronicotinonitrile to obtain Intermediate 28, ESI-MS: m/z = 332 [M+H]⁺.

### Intermediate 29. 3-chloro-5-(-6-(3-methyl-2-oxoimidazolin-1-yl)-2-azabicyclo[2.2.2] octan-2-yl)pyrazin-2-carbonitrile

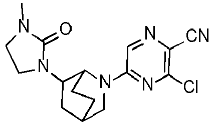

The synthesis method was the same as that for Intermediate 9, except that Intermediate 1 was replaced with Intermediate 8 to obtain Intermediate 29, ESI-MS: m/z = 347 [M+H]⁺.

### Intermediate 30. 5-chloro-3-(-6-(3-methyl-2-oxoimidazolin-1-yl)-2-azabicyclo[2.2.1] heptan-2-yl)-1,2,4-triazin-6-carbonitrile

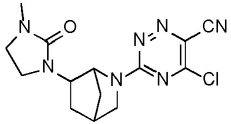

The synthesis method was the same as that for Intermediate 9, except that Intermediate 1 was replaced with Intermediate 6 and 3,5-dichloropyrazine-2-carbonitrile was replaced with 3,5-dichloro-1,2,4-triazine-6-nitrile to obtain Intermediate 30, ESI-MS: m/z = 334 [M+H]⁺.

### Intermediate 31. 5-chloro-3-(3-(3-ethyl-2-oxoimidazolin-1-yl)piperidin-1-yl) -1,2,4-triazin-6-carbonitrile

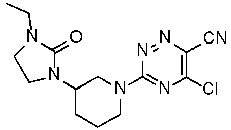

The synthesis method was the same as that for Intermediate 9, except that Intermediate 1 was replaced with 1-ethyl-3-(piperidin-3-yl)imidazolin-2-one and 3,5-dichloropyrazine-2-carbonitrile was replaced with 3,5-dichloro-1,2,4-triazine-6-nitrile to obtain Intermediate 31, ESI-MS: m/z = 336 [M+H]⁺.

### Intermediate 32. 5-chloro-3-(3-(3-isopropyl-2-oxoimidazolin-1-yl)piperidin-1-yl)-1,2,4-triazin-6-carbonitrile

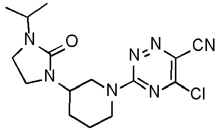

The synthesis method was the same as that for Intermediate 9, except that Intermediate 1 was replaced with 1-isopropyl-3-(piperidin-3-yl)imidazolin-2-one and 3,5-dichloropyrazine-2-carbonitrile was replaced with 3,5-dichloro-1,2,4-triazine-6-nitrile to obtain Intermediate 32, ESI-MS: m/z = 350 [M+H]⁺.

### Intermediate 33. 5-chloro-3-(3-(2-oxo-3-(trifluoromethyl)imidazolin-1-yl)piperidin-1-yl)-1,2,4-triazin-6-carbonitrile

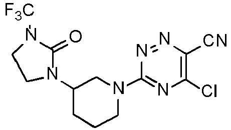

The synthesis method was the same as that for Intermediate 9, except that Intermediate 1 was replaced with 1-(piperidin-3-yl)-3-(trifluoromethyl)imidazolin-2-one and 3,5-dichloropyrazine-2-carbonitrile was replaced with 3,5-dichloro-1,2,4-triazine-6-nitrile to obtain Intermediate 33, ESI-MS: m/z = 376 [M+H]⁺.

### Intermediate 34. 5-chloro-3-(3-(2-oxo-3-(2,2,2-trifluoroethyl)imidazolin-1-yl) piperidin-1-yl)-1,2,4-triazin-6-carbonitrile

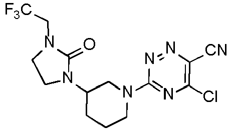

The synthesis method was the same as that for Intermediate 9, except that Intermediate 1 was replaced with 1-(piperidin-3-yl)-3-(2,2,2-trifluoroethyl)imidazolin-2-one and 3,5-dichloropyrazine-2-carbonitrile was replaced with 3,5-dichloro-1,2,4-triazine-6-nitrile to obtain Intermediate 34, ESI-MS: m/z = 390 [M+H]⁺.

### Intermediate 35. 5-chloro-3-(3-(3-cyclopropyl-2-oxoimidazolin-1-yl)piperidin-1-yl) -1,2,4-triazin-6-carbonitrile

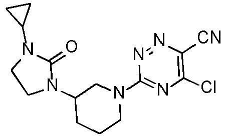

The synthesis method was the same as that for Intermediate 9, except that Intermediate 1 was replaced with 1-cyclopropyl-3-(piperidin-3-yl)imidazolin-2-one and 3,5-dichloropyrazine -2-carbonitrile was replaced with 3,5-dichloro-1,2,4-triazine-6-nitrile to obtain Intermediate 35, ESI-MS: m/z = 348 [M+H]⁺.

### Intermediate 36. 5-chloro-3-(3-(3-cyclobutyl-2-oxoimidazolin-1-yl)piperidin-1-yl) -1,2,4-triazin-6-carbonitrile

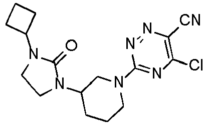

The synthesis method was the same as that for Intermediate 9, except that Intermediate 1 was replaced with 1-cyclobutyl-3-(piperidin-3-yl)imidazolin-2-one and 3,5-dichloropyrazine -2-carbonitrile was replaced with 3,5-dichloro-1,2,4-triazine-6-nitrile to obtain Intermediate 36, ESI-MS: m/z = 362 [M+H]⁺.

### Intermediate 37. 5-chloro-3-(3-(3-cyclopentyl-2-oxoimidazolin-1-yl)piperidin-1-yl) -1,2,4-triazin-6-carbonitrile

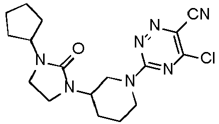

The synthesis method was the same as that for Intermediate 9, except that 3,5-dichloropyrazine-2-carbonitrile was replaced with 3,5-dichloro-1,2,4-triazine-6-nitrile to obtain Intermediate 37, ESI-MS: m/z = 376 [M+H]⁺.

### Intermediate 38. 5-chloro-3-(3-(3-cyclohexyl-2-oxoimidazolin-1-yl)piperidin-1-yl) -1,2,4-triazin-6-carbonitrile

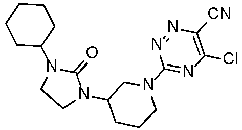

The synthesis method was the same as that for Intermediate 9, except that Intermediate 1 was replaced with Intermediate 3 and 3,5-dichloropyrazine-2-carbonitrile was replaced with 3,5-dichloro-1,2,4-triazine-6-nitrile to obtain Intermediate 38, ESI-MS: m/z = 390 [M+H]⁺.

### Intermediate 39. 5-chloro-3-(3-(3-(oxetan-3-yl)-2-oxoimidazolin-1-yl)piperidin-1-yl) -1,2,4-triazin-6-carbonitrile

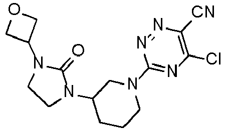

The synthesis method was the same as that for Intermediate 9, except that Intermediate 1 was replaced with Intermediate 2 and 3,5-dichloropyrazine-2-carbonitrile was replaced with 3,5-dichloro-1,2,4-triazine-6-nitrile to obtain Intermediate 39, ESI-MS: m/z = 364 [M+H]⁺.

### Intermediate 40. 5-chloro-3-(3-(2-oxo-3-(tetrahydro-2H-pyran-4-yl)imidazolin-1-yl) piperidin-1-yl)-1,2,4-triazin-6-carbonitrile

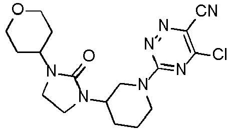

The synthesis method was the same as that for Intermediate 9, except that Intermediate 1 was replaced with Intermediate 4 and 3,5-dichloropyrazine-2-carbonitrile was replaced with 3,5-dichloro-1,2,4-triazine-6-nitrile to obtain Intermediate 40, ESI-MS: m/z = 392 [M+H]⁺.

### Intermediate 41. 5-chloro-3-(3-(2-oxo-3-phenylimidazolin-1-yl)piperidin-1-yl) -1,2,4-triazin-6-carbonitrile

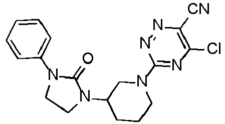

The synthesis method was the same as that for Intermediate 9, except that Intermediate 1 was replaced with 1-phenyl-3-(piperidin-3-yl)imidazolin-2-one and 3,5-dichloropyrazine -2-carbonitrile was replaced with 3,5-dichloro-1,2,4-triazine-6-nitrile to obtain Intermediate 41, ESI-MS: m/z = 384 [M+H]⁺.

### Intermediate 42. 5-chloro-3-(3-(2-fluorophenyl)-2-oxoimidazolin-1-yl)piperidin-1-yl) -1,2,4-triazin-6-carbonitrile

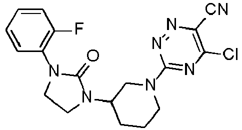

The synthesis method was the same as that for Intermediate 9, except that Intermediate 1 was replaced with 1-(2-fluorophenyl)-3-(piperidin-3-yl)imidazolin-2-one and 3,5-dichloropyrazine-2-carbonitrile was replaced with 3,5-dichloro-1,2,4-triazine-6-nitrile to obtain Intermediate 42, ESI-MS: m/z = 402 [M+H]⁺.

### Intermediate 43. 5-chloro-3-(3-(3-fluorophenyl)-2-oxoimidazolin-1-yl)piperidin-1-yl) -1,2,4-triazin-6-carbonitrile

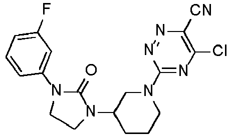

The synthesis method was the same as that for Intermediate 9, except that Intermediate 1 was replaced with 1-(3-fluorophenyl)-3-(piperidin-3-yl)imidazolin-2-one and 3,5-dichloropyrazine-2-carbonitrile was replaced with 3,5-dichloro-1,2,4-triazine-6-nitrile to obtain Intermediate 43, ESI-MS: m/z = 402 [M+H]⁺.

### Intermediate 44. 5-chloro-3-(3-(2-oxo-3-(pyridin-3-yl)imidazolin-1-yl)piperidin-1-yl) -1,2,4-triazin-6-carbonitrile

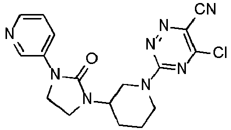

The synthesis method was the same as that for Intermediate 9, except that Intermediate 1 was replaced with 1-(piperidin-3-yl)-3-(pyridin-3-yl)imidazolin-2-one and 3,5-dichloropyrazine -2-carbonitrile was replaced with 3,5-dichloro-1,2,4-triazine-6-nitrile to obtain Intermediate 44, ESI-MS: m/z = 385 [M+H]⁺.

### Intermediate 45. 5-chloro-3-(3-(4-methyl-5-oxo-4,6-diazaspirocyclo[2.4]heptan-6-yl) piperidin-1-yl)-1,2,4-triazin-6-carbonitrile

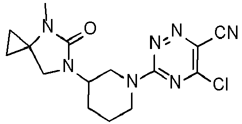

The synthesis method was the same as that for Intermediate 9, except that Intermediate 1 was replaced with 4-methyl-6-(piperidin-3-yl)-4,6-diazaspirocyclo[2.4]heptan-5-one hydrochloride and 3,5-dichloropyrazine-2-carbonitrile was replaced with 3,5-dichloro-1,2,4-triazine-6-nitrile to obtain Intermediate 45, ESI-MS: m/z = 348 [M+H]⁺.

### Intermediate 46. 5-chloro-3-(3-(5-methyl-6-oxo-5,7-diazaspirocyclo[3.4]octan-7-yl) piperidin-1-yl)-1,2,4-triazin-6-carbonitrile

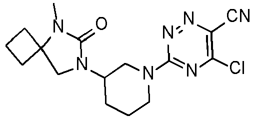

The synthesis method was the same as that for Intermediate 9, except that Intermediate 1 was replaced with 5-methyl-7-(piperidin-3-yl)-5,7-diazaspirocyclo[3.4]octane-6-one hydrochloride and 3,5-dichloropyrazine-2-carbonitrile was replaced with 3,5-dichloro-1,2,4-triazine-6-nitrile to obtain Intermediate 46, ESI-MS: m/z = 362 [M+H]⁺.

### Intermediate 47. 5-chloro-3-(3-(3-oxotrihydro-1H-pyrrolo[1,2-c]imidazol-2(3H)-yl) piperidin-1-yl)-1,2,4-triazin-6-carbonitrile

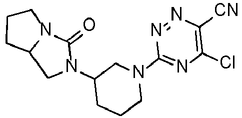

The synthesis method was the same as that for Intermediate 9, except that Intermediate 1 was replaced with 2-(piperidin-3-yl)hexahydro-3H-pyrrolo[1,2-c]imidazol-3-one and 3,5-dichloropyrazine-2-carbonitrile was replaced with 3,5-dichloro-1,2,4-triazine-6-nitrile to obtain Intermediate 47, ESI-MS: m/z = 348 [M+H]⁺.

### Intermediate 48. 5-chloro-3-(6-(3-cyclopentyl-2-oxoimidazolin-1-yl)-2-azabicyclo [2.2.1]heptan-2-yl)-1,2,4-triazin-6-carbonitrile

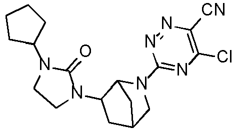

The synthesis method was the same as that for Intermediate 9, except that Intermediate 1 was replaced with 1-(2-azabicyclo[2.2.1]heptan-6-yl)-3-cyclopentylimidazolin-2-one and 3,5-dichloropyrazine-2-carbonitrile was replaced with 3,5-dichloro-1,2,4-triazine-6-nitrile to obtain Intermediate 48, ESI-MS: m/z = 388 [M+H]⁺.

### Intermediate 49. 5-chloro-3-(6-(2-oxo-3-phenylimidazolin-1-yl)-2-azabicyclo[2.2.1] heptan-2-yl)-1,2,4-triazin-6-carbonitrile

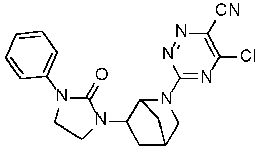

The synthesis method was the same as that for Intermediate 9, except that Intermediate 1 was replaced with 1-(2-azabicyclo[2.2.1]heptan-6-yl)-3-phenylimidazolin-2-one and 3,5-dichloropyrazine-2-carbonitrile was replaced with 3,5-dichloro-1,2,4-triazine-6-nitrile to obtain Intermediate 49, ESI-MS: m/z = 396 [M+H]⁺.

### Intermediate 50. 5-chloro-3-(6-(3-oxotrihydro-lH-pyrrolo[1,2-c]imidazol-2(3H)-yl) -2-azabicyclo[2.2.1]heptan-2-yl)-1,2,4-triazin-6-carbonitrile

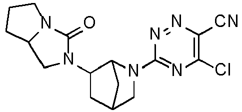

The synthesis method was the same as that for Intermediate 9, except that Intermediate 1 was replaced with 2-(2-azabicyclo[2.2.1]heptan-6-yl)hexahydro-3H-pyrrole[1,2-c] imidazole-3-one and 3,5-dichloropyrazine-2-carbonitrile was replaced with 3,5-dichloro-1,2,4-triazine-6-nitrile to obtain Intermediate 50, ESI-MS: m/z = 360 [M+H]⁺.

### Intermediate 51. 5-chloro-3-(6-(3-methyl-2-oxoimidazolin-1-yl)-2-azabicyclo[2.2.2] octan-2-yl)-1,2,4-triazin-6-carbonitrile

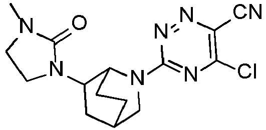

The synthesis method was the same as that for Intermediate 9, except that Intermediate 1 was replaced with Intermediate 8 and 3,5-dichloropyrazine-2-carbonitrile was replaced with 3,5-dichloro-1,2,4-triazine-6-nitrile to obtain Intermediate 51, ESI-MS: m/z = 348 [M+H]⁺.

### Intermediate 52. Tert-butyl 4-(4-aminophenyl)-4-methylpiperidin-1-carboxylate

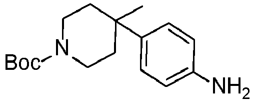

### Synthesis step 1. N-ethoxycarbonyl-4-hydroxyl-4-methylpiperidine (52-1)

N-ethoxycarbonyl-4-piperidone (10.00 g, 58.41 mmol) was dissolved in diethyl ether solution and the mixture was cooled to -30°C. 3M methylmagnesium chloride (5.24 g, 70.1 mmol) in THF was added, and the mixture was placed at 0°C and reacted for 2 h under stirring. When the reaction was completed, saturated aqueous ammonium chloride solution was added, and the mixture was filtered to remove the white solid. The aqueous layer was extracted with dichloromethane, and the organic layers were combined, dried over anhydrous sodium sulfate, and filtered with suction. The solvent was removed under reduced pressure, and purification was performed by column chromatography to obtain the target compound N-ethoxycarbonyl-4-hydroxy-4-methylpiperidine (52-1). ESI-MS: *m*/*z=* 188 [M+H]⁺.

### Synthesis step 2. N-ethoxycarbonyl-4-(4-bromophenyl)-4-methylpiperidine(52-2)

N-ethoxycarbonyl-4-hydroxy-4-methylpiperidine (3.08 g, 16.45 mmol) and 4-bromobenzene (25.83 g, 164.50 mmol) was placed in an ice bath at 0°C, and trifluoromethanesulfonic acid (24.69 g, 164.50 mmol) was slowly add dropwise and the mixture was reacted at room temperature for 3 h. After the reaction was completed, the reactant was added to ice water, alkalized by adding 1N NaOH, and extracted with dichloromethane three times. The organic layers were combined, washed with saturated sodium chloride, dried over anhydrous sodium sulfate, and filtered with suction. The solvent was removed under pressure and purification was performed by column chromatography to obtain compound N-ethoxycarbonyl-4-(4-bromophenyl)-4-methylpiperidine (52-2). ESI-MS: *m*/*z* = 326 [M+H]⁺.

### Synthesis step 3. 4-(4-bromophenyl)-4-methylpiperidine(52-3)

N-ethoxycarbonyl-4-(4-bromophenyl)-4-methylpiperidine (7.00 g, 21.46 mmol) was dissolved in ethanol (20 mL), and potassium hydroxide (24.08 g, 429.14 mmol) was added and the mixture was reacted at 80°C overnight. After the reaction was completed, the reaction liquid was distilled under reduced pressure to obtain a residue. The residue was dissolved in dichloromethane solution, washed with water, and extracted with dichloromethane. The organic layers were combined, washed with saturated sodium chloride, dried over anhydrous sodium sulfate, and filtered with suction. The solvent was removed under reduce pressure, and the resulting product was used directly in the next reaction. ESI-MS: *m*/*z* = 254 [M+H]⁺.

### Synthesis step 4. Tert-butyl 4-(4-Bromophenyl)-4-methylpiperidin-1-carboxylate (52-4)

Compound 4-(4-bromophenyl)-4-methylpiperidine (5.40 g, 21.25 mmol) was dissolved in dichloromethane (50 mL), and Boc anhydride (7.42 g, 33.99 mmol) was slowly added dropwise, and the mixture was reacted for 1 h at room temperature. After the reaction was completed, water was added, and dichloromethane was added for extraction. The organic layers were combined, washed with saturated sodium chloride, dried over anhydrous sodium sulfate, and filtered with suction. The solvent was removed under pressure and purification was performed by column chromatography to obtain compound tert-butyl 4-(4-bromophenyl)-4-methylpiperidine-1-carboxylate (52-4). ESI-MS: *m*/*z* = 354 [M+H]⁺.

### Synthesis step 5. Tert-butyl 4-(4-aminophenyl)-4-methylpiperidin-1-carboxylate (Intermediate 52)

Under nitrogen protection, compounds N-Boc-4-(4-bromophenyl)-4-methylpiperidine (2.60 g, 7.34 mmol), 2-(dicyclohexylphosphino)biphenyl (65.00 mg, 0.19 mmol), tris(dibenzylideneacetone)dipalladium (68.00 mg, 0.07 mmol), and LiHMDS (14.70 mL) were dissolved in anhydrous THF (15 mL), and the mixture was reacted at 65°C overnight. After the reaction was completed, dichloromethane was added for extraction. Then washed with water, washed with saturated sodium chloride, dried with anhydrous sodium sulfate, and filtered with suction. The solvent was removed under pressure and purification was performed by column chromatography to obtain compound tert-butyl 4-(4-aminophenyl)-4-methylpiperidine-1-carboxylate (Intermediate 52). ESI-MS: *m*/*z* = 291 [M+H]⁺.

### Intermediate 53. Tert-butyl 4-(4-aminophenyl)piperazin-1-carboxylate

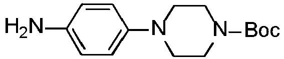

### Synthesis step 1. Tert-butyl 4-(4-nitrophenyl)piperazin-1-carboxylate (53-1)

p-fluoronitrobenzene (1.41 g, 10 mmol), N,N-diisopropylethylamine (6.46 g, 50 mmol), and 1-Boc-piperazine (1.86 g, 10 mmol) were dissolved in DMF (20 mL), the temperature was raised to 90°C for reaction overnight under stirring. Water was added, and extracted with ethyl acetate. The organic layers were combined, washed with saturated sodium chloride, dried over anhydrous sodium sulfate, and filtered with suction. The solvent was removed under pressure and purification was performed by column chromatography to obtain compound tert-butyl 4-(4-nitrophenyl) piperazine-1-carboxylate (53-1). ESI-MS: *m*/*z* = 308 [M+H]⁺.

### Synthesis step 2. Tert-butyl 4-(4-aminophenyl)piperazin-1-carboxylate (Intermediate 53)

Compound tert-butyl 4-(4-nitrophenyl)piperazine-1-carboxylate (1.54 g, 5 mmol) was dissolved in ethanol (20 mL), Pd/C (154 mg) was added, and the mixture was reacted at room temperature for 2 h under H₂. After the reaction was completed, the reaction liquid was filtered, and used ethyl acetate and methanol for the filter cake . The organic layers were combined, and the solvent was removed under reduced pressure to directly obtain the product tert-butyl 4-(4-aminophenyl)piperazine-1-carboxylate (Intermediate 53). ESI-MS: *m*/*z* = 278 [M+H]⁺.

### Intermediate 54. Tert-butyl 9-(4-aminophenyl)-3,9-diazaspirocyclo[5.5]undec-3-carboxylate

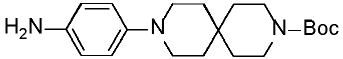

The synthesis method was the same as that for Intermediate 53, except that 1-Boc-piperazine in synthesis step 1 was replaced with tert-butyl 3,9-diazaspiro[5.5]undecane-3-carboxylate to obtain the Intermediate 54. ESI-MS: m/z = 346 [M+H]⁺.

### Intermediate 55. Tert-butyl 6-(4-aminophenyl)-2,6-diazaspirocyclo[3.3]heptan-2-carboxylate

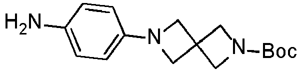

The synthesis method was the same as that for Intermediate 53, except that 1-Boc-piperazine in synthesis step 1 was replaced with tert-butyl 2,6-diazaspiro[3.3]heptane-2-carboxylate to obtain the Intermediate 55. ESI-MS: m/z = 290 [M+H]⁺.

### Intermediate 56. 7-(4-aminophenyl)-7-azaspiro[3.5]non-2-one

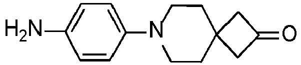

The synthesis method was the same as that for Intermediate 53, except that 1-Boc-piperazine (1 mmol) in synthesis step 1 was replaced with 7-azaspiro[3.5]nonan-2-one to obtain Intermediate 56. ESI-MS: m/z = 231 [M+H]⁺.

### Intermediate 57. Tert-butyl 4-(4-aminobenzoyl)piperidin-1-carboxylate

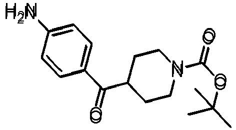

### Synthesis step 1. Tert-butyl 4-(4-bromobenzoyl)piperidin-1-carboxylate (57-1)

The compound bromobenzene (1.57 g, 10 mmol) was dissolved in tetrahydrofuran (20 mL) solution, tert-butyl 4-(chlorocarbonyl)-piperidine-1-carboxylate (2.97 g, 12 mmol) was added,aluminum trichloride (1.33 g, 10 mmol) was added, and the mixture was heated to 70°C, reacted for 10 h. After the reaction was completed, the reaction liquid was slowly added to a 3M HCl solution, extracted with ethyl acetate, washed with water, washed with saturated sodium chloride, dried over anhydrous sodium sulfate, and filtered with suction. The solvent was removed under reduced pressure, and purification was performed by column chromatography to obtain compound tert-butyl 4-(4-bromobenzoyl)piperidine-1-carboxylate (57-1). ESI-MS: *m*/*z* = 368 [M+H]⁺.

### Synthesis step 2. Tert-butyl 4-(4-aminobenzoyl)piperidin-1-carboxylate (Intermediate 57)

The synthesis method was the same as that in synthesis step 5 of Intermediate 31, except that tert-butyl 4-(4-bromophenyl)-4-methylpiperidine-1-carboxylate was replaced with tert-butyl 4-(4-bromobenzoyl)piperidine-1-carboxylate to obtain Intermediate 57. ESI-MS: *m*/*z* = 305 [M+H]⁺.

### Intermediate 58. Tert-butyl 4-(4-aminophenyl)piperidin-1-carboxylate

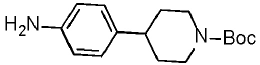

### Synthesis step 1. Tert-butyl 4-(4-nitrophenyl)-3,6-dihydropyridin-1(2H)-carboxylate (58-1)

1-Bromo-4-nitrobenzene (2.02 g, 10 mmol), N-Boc-1,2,5,6-tetrahydropyridine-4-boronic acid pinacol ester (3.71 g, 12 mmol), [1,1'-bis(diphenylphosphine)ferrocene]palladium dichloride (731.7 mg, 1 mmol), and potassium carbonate (4.15 g, 30 mmol) were dissolved in a solution of dioxane and water, and the mixture was refluxed and reacted for 2 h under nitrogen. After the reaction was completed, the reaction liquid was filtered, the solvent was removed under reduced pressure, and purification was performed by column chromatography to obtain compound (58-1). ESI-MS: *m*/*z* = 305 [M+H]⁺.

### Synthesis step 2. Tert-butyl 4-(4-aminophenyl)piperidin-1-carboxylate (Intermediate 58)

Compound tert-butyl 4-(4-nitrophenyl)-3,6-dihydropyridine-1(2H)-carboxylate (3.05 g, 10 mmol) was dissolved in a mixture solvent of ethanol and DMF (volume ratio 1:1, 20 ml), 10% palladium on carbon (305 mg) was added, and the mixture was stirred at room temperature under hydrogen gas for 24 h. After the reaction was completed, the reaction liquid was filtered, and the solvent was removed under reduced pressure, and purification was performed by column chromatography to obtain compound (Intermediate 58). ESI-MS: *m*/*z* = 277 [M+H]⁺.

### Intermediate 59. Tert-butyl 4-(4-aminophenyl)-4-fluoropiperidin-1-carboxylate

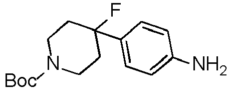

### Synthesis step 1. Tert-butyl 4-(4-bromophenyl)-4-hydroxylpiperidin-1-carboxylate (59-1)

Under nitrogen protection, p-bromoiodobenzene (2.83 g, 10 mmol) was dissolved in anhydrous THF (20 mL), and the mixture was placed at -78°C, and a solution of n-butyllithium in THF (1.92 g, 30 mmol) was added dropwise. After reacting at -78°C for 2 h, a solution of N-tert-butoxycarbonyl-4-piperidone in THF (2.40 g, 12 mmol) was added dropwise, and the mixture was reacted at -78°C for 2 h. After the reaction was completed, the reaction was quenched by adding water, and extracted by adding ethyl acetate. The organic layers were combined, the solvent was removed under reduced pressure, and purification was performed by column chromatography to obtain compound (59-1). ESI-MS: m/z = 356 [M+H]⁺.

### Synthesis step 2. Tert-butyl 4-(4-bromophenyl)-4-fluoropiperidin-1-carboxylate (59-2)

Tert-butyl 4-(4-bromophenyl)-4-hydroxypiperidine-1-carboxylate (3.56 g, 10 mmol) was dissolved in anhydrous dichloromethane (30 mL), and the mixture was placed at -78°C, and bis (2-methoxyethyl)aminosulfur trifluoride (11.06 g, 50 mmol) was added and the mixture was moved to room temperature and reacted for 72 h. After the reaction was completed, the reaction was quenched by adding saturated sodium bicarbonate solution, and extracted by adding dichloromethane. The organic phases were combined, the solvent was removed under reduced pressure, and purification was performed by column chromatography to obtain compound (59-2). ESI-MS: m/z = 358 [M+H]⁺.

### Synthesis step 3. Tert-butyl 4-(4-aminophenyl)-4-fluoropiperidin-1-carboxylate (Intermediate 59)

The synthesis method was the same as that for Intermediate 52, except that N-Boc-4-(4-bromophenyl)-4-methylpiperidine in synthesis step 5 was replaced with compound **59-2** to obtain Intermediate 59. ESI-MS: m/z = 295 [M+H]⁺.

### Intermediate 60. Tert-butyl 4-(4-amino-3-fluorophenyl)piperidin-1-carboxylate

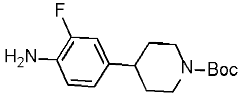

The synthesis method was the same as that for Intermediate 58, except that 1-bromo-4-nitrobenzene in synthesis step 1 was replaced with 2-fluoro-4-bromonitrobenzene to obtain Intermediate 60. ESI-MS: m/z = 295 [M+H]⁺.

### Intermediate 61. Tert-butyl 4-(4-amino-2-cyanophenyl)piperidin-1-carboxylate

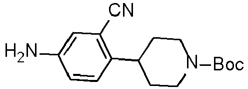

The synthesis method was the same as that for Intermediate 58, except that 1-bromo-4-nitrobenzene in synthesis step 1 was replaced with 2-bromo-5-nitrobenzonitrile to obtain Intermediate 61. ESI-MS: m/z = 302 [M+H]⁺.

### Intermediate 62. Tert-butyl 4-(4-aminophenyl)-3-fluoropiperidin-1-carboxylate

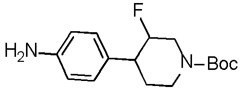

### Synthesis step 1. Tert-butyl 3-hydroxyl-4-(4-nitrophenyl)piperidin-1-carboxylate (62-1)

Under nitrogen conditions, compound **58-1** (2.44 g, 8 mmol) was dissolved in anhydrous glycol dimethyl ether (12 mL), and the mixture was placed at 0°C; sodium borohydride (484.2 mg, 12.8 mmol) was added and the mixture was reacted for 5 min under stirring. Boron trifluoride ether (2.27 g, 16 mmol) was added dropwise, and the mixture was reacted at room temperature overnight under stirring. The reaction liquid was placed at 0°C, water was added until boiling stopped, 10 M aqueous sodium hydroxide solution (5 mL) was added, hydrogen peroxide (5 mL) was added dropwise, and the reaction was warmed to room temperature and stirred for 2 h. The reaction was diluted with water, and then extracted with ethyl acetate. The organic layers were combined, dried over anhydrous sodium sulfate, and filtered. The solvent was removed under reduced pressure, and purification was performed by column chromatography to obtain compound (62-1). ESI-MS: *m*/*z* = 323 [M+H]⁺.

### Synthesis step 2. Tert-butyl 3-fluoro-4-(4-nitrophenyl)piperidin-1-carboxylate (62-2)

Compound **62-1** (2.26 g, 7 mmol) was dissolved in 1,2-dichloroethane (15 mL), and the reaction liquid was placed at 0°C. Diethylaminosulphur trifluoride (5.64 g, 35 mmol) was added and the reaction liquid was stirred at room temperature overnight. After the reaction was completed, saturated sodium bicarbonate solution was added, extraction was performed with dichloromethane, and the organic layers were combined. The organic layer was washed with water, dried over anhydrous sodium sulfate, and filtered. The solvent was removed under reduced pressure, and purification was performed by column chromatography to obtain compound (62-2). ESI-MS: *m*/*z* = 325 [M+H]⁺.

### Synthesis step 3. Tert-butyl 4-(4-aminophenyl)-3-fluoropiperidin-1- carboxylate (Intermediate 62)

Compound **62-2 (1.95 g, 6 mmol)** was dissolved in methanol (12 mL), 10% palladium on carbon (195 mg) was added, hydrogen was introduced, and the mixture was reacted overnight at a pressure of 3 atm. After the reaction was completed, the reaction liquid was filtered, and the solvent was removed under reduced pressure, and purification was performed by column chromatography to obtain compound (Intermediate 62). ESI-MS: *m*/*z* = 295 [M+H]⁺.

### Intermediate 63. 5-(3-(3-cyclopentyl-2-oxoimidazolin-1-yl)piperidin-1-yl)-3-((4-(4-methylpiperidin-4-yl)phenyl)amino)pyrazin-2-carboxamide

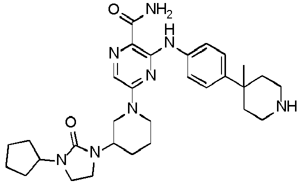

### Synthesis step 1. Tert-butyl 4-(4-((3-cyano-6-(3-(3-cyclopentyl-2-oxoimidazolin -1-yl)piperidin-1-yl)pyrazin-2-yl)amino)phenyl)-4-methylpiperidin-1-carboxylate (63-1)

Intermediate 9 (3.75 g, 10 mmol), intermediate 52 (2.91 g, 10 mmol), cesium carbonate (9.78 g, 30 mmol), BINAP (622.69 mg, 1 mmol), and Pd₂(bda)₃(1.02 g, 1 mmol) were dissolved in dioxane (25 mL), and the mixture was reacted under reflux for 3 h while stirring. After the reaction was completed, the reaction liquid was filtered, and the solid was washed with dichloromethane solution, the organic layers were combined, the solvent was removed under reduced pressure, and purification was performed by column chromatography to obtain compound (63-1). ESI-MS: *m*/*z* = 629 [M+H]⁺.

### Synthesis step 2. Tert-butyl 4-(4-((3-carbamoyl-6-(3-(3-cyclopentyl-2-oxoimidazolin -1-yl)piperidin-1-yl)pyrazin-2-yl)amino)phenyl)-4-methylpiperidin-1-carboxylate (63-2)

Tert-butyl 4-(4-((3 -cyano-6-(3 -(3 -cyclopentyl-2-oxoimidazolin-1 -yl)piperidin-1 -yl) pyrazin-2-yl) amino)phenyl)-4-methylpiperidine-1-carboxylate (6.29 g, 10 mmol) was dissolved in a mixed solvent of methanol and DMSO (1:1, 50 mL), and cesium carbonate (1.63 g, 5 mmol) was added, and 30% H₂O₂ (10 mL) was added, and the mixture was reacted at room temperature for 30 min under stirring. After the reaction was completed, acetonitrile solution was added and the mixture was stirred for 5 min, and distilled under reduced pressure, the residue was dissolved in ethyl acetate solution, and washed with water three times. The organic layers were combined, dried over anhydrous sodium sulfate, and filtered with suction. The solvent was removed under reduced pressure, and purification was performed by column chromatography to obtain compound (63-2). ESI-MS: *m*/*z* = 647 [M+H]⁺.

### Synthesis step 3. 5-(3-(3-cyclopentyl-2-oxoimidazolin-1-yl)piperidin-1-yl)-3-((4-(4-methylpiperidin-4-yl)phenyl)amino)pyrazin-2-carboxamide (Intermediate 63)

Tert-butyl 4-(4-((3-carbamoyl-6-(3-(3-cyclopentyl-2-oxoimidazolin-1-yl)piperidin-1-yl) pyrazin-2-yl)amino)phenyl)-4-methylpiperidine-1-carboxylate (3.24 g, 5 mmol) was dissolved in dioxane (15 mL), and 4 N HCl (16 mL) was added dropwise in ice bath, and the mixture was moved to room temperature and reacted for 30 min. After the reaction was completed, the reaction liquid was distilled under reduced pressure to obtain Intermediate 63. ESI-MS: *m*/*z =* 547 [M+H]⁺.

### Intermediate 64. 3-((4-(4-methylpiperidin-4-yl)phenyl)amino)-5-(3-(3-(oxetan-3-yl) -2-oxoimidazolin-1-yl)piperidin-1-yl)pyrazin-2-carboxamide

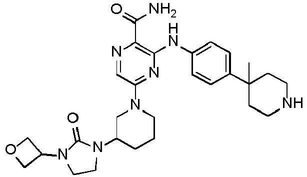

The synthesis method was the same as that for Intermediate 63, except that Intermediate 9 in synthesis step 1 was replaced with Intermediate 10 to obtain Intermediate 64. ESI-MS: m/z = 535 [M+H]⁺.

### Intermediate 65. 5-(3-(3-cyclohexyl-2-oxoimidazolin-1-yl)piperidin-1-yl)-3-((4-(4-methylpiperidin-4-yl)phenyl)amino)pyrazin-2-carboxamide

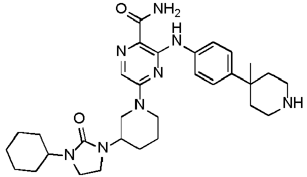

The synthesis method was the same as that for Intermediate 63, except that Intermediate 9 in synthesis step 1 was replaced with Intermediate 11 to obtain Intermediate 65. ESI-MS: *m*/*z =* 561 [M+H]⁺.

### Intermediate 66. 3-((4-(4-methylpiperidin-4-yl)phenyl)amino)-5-(3-(2-oxo-3-(tetrahydro-2H-pyran-4-yl)imidazolin-1-yl)piperidin-1-yl)pyrazin-2-carboxamide

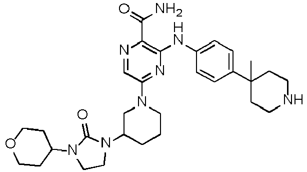

The synthesis method was the same as that for Intermediate 63, except that Intermediate 9 in synthesis step 1 was replaced with Intermediate 12 to obtain Intermediate 66. ESI-MS: *m*/*z =* 563 [M+H]⁺.

### Intermediate 67. 3-((4-(4-methylpiperidin-4-yl)phenyl)amino)-5-(3-(3-oxotetrahydro -1H-pyrrolo[1,2-c]imidazol-2(3H)-yl)piperidin-1-yl)pyrazin-2-carboxamide

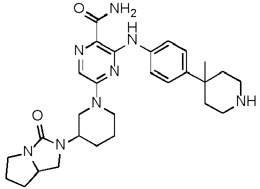

The synthesis method was the same as that for Intermediate 63, except that Intermediate 9 in synthesis step 1 was replaced with Intermediate 13 to obtain Intermediate 67. ESI-MS: *m*/*z* = 519 [M+H]⁺.

### Intermediate 68. 3-((4-(4-methylpiperidin-4-yl)phenyl)amino)-5-(3-(3-oxohexahydroimidazo[1,5-a]pyridin-2(3H)-yl)piperidin-1-yl)pyrazin-2-carboxamide

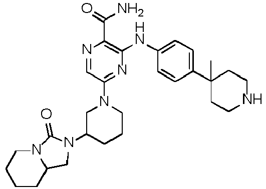

The synthesis method was the same as that for Intermediate 63, except that Intermediate 9 in synthesis step 1 was replaced with Intermediate 14 to obtain Intermediate 68. ESI-MS: *m*/*z =* 533 [M+H]⁺.

### Intermediate 69. 5-(3-(3-methyl-2-oxoimidazolin-1-yl)piperidin-1-yl)-3-((4-(4-methylpiperidin-4-yl)phenyl)amino)pyrazin-2-carboxamide

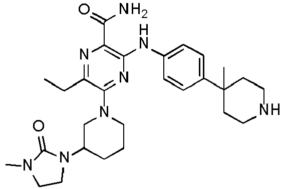

The synthesis method was the same as that for Intermediate 63, except that Intermediate 9 in synthesis step 1 was replaced with Intermediate 15 to obtain Intermediate 69. ESI-MS: *m*/*z =* 521 [M+H]⁺.

### Intermediate 70. 5-(3-(3-methyl-2-oxohexahydrocyclopenta[d]imidazol-1(2H)-yl) piperidin-1-yl)-3-((4-(4-methylpiperidin-4-yl)phenyl)amino)pyrazin-2-carboxamide)

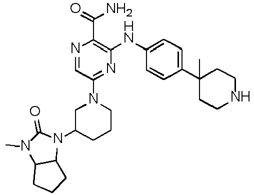

The synthesis method was the same as that for Intermediate 63, except that Intermediate 9 in synthesis step 1 was replaced with Intermediate 16 to obtain Intermediate 70. ESI-MS: *m*/*z =* 533 [M+H]⁺.

### Intermediate 71. 5-(3-(3-methyl-2-oxooctahydro-1H-benzo[d]imidazol-1-yl) piperidin-1-yl)-3-((4-(4-methylpiperidin-4-yl)phenyl)amino)pyrazin-2-carboxamide

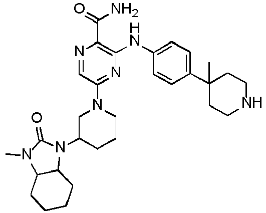

The synthesis method was the same as that for Intermediate 63, except that Intermediate 9 in synthesis step 1 was replaced with Intermediate 17 to obtain Intermediate 71. ESI-MS: m/z = 547 [M+H]⁺.

### Intermediate 72. 5-(3-(4-methyl-5-oxo-4,6-diazaspirocyclo[2.4]heptan-6-yl) piperidin-1-yl)-3-((4-(4-methylpiperidin-4-yl)phenyl)amino)pyrazin-2-carboxamide

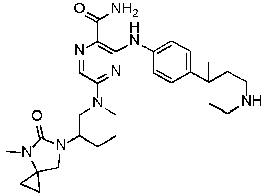

The synthesis method was the same as that for Intermediate 63, except that Intermediate 9 in synthesis step 1 was replaced with Intermediate 18 to obtain Intermediate 72. ESI-MS: *m*/*z* = 519 [M+H]⁺.

### Intermediate 73. 5-(3-(5-methyt-6-oxo-5,7-diazaspirocydo[3.4]octan-7-yl)piperidin -1-yl)-3-((4-(4-methylpiperidin-4-yl)phenyl)amino)pyrazin-2-carboxamide

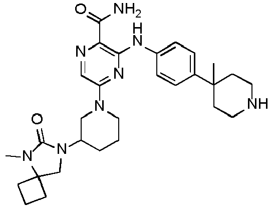

The synthesis method was the same as that for Intermediate 63, except that Intermediate 9 in synthesis step 1 was replaced with Intermediate 19 to obtain Intermediate 73. ESI-MS: *m*/*z =* 533 [M+H]⁺.

### Intermediate 74. 5-(3-(3-(oxetan-3-yl)-2-oxoimidazolin-1-yl)piperidin-1-yl)-3-((4-(piperazin-1-yl)phenyl)amino)pyrazin-2-carboxamide

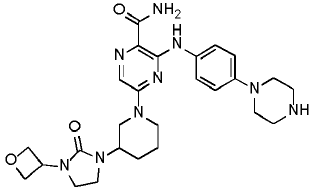

The synthesis method was the same as that for Intermediate 64, except that Intermediate 52 in synthesis step 1 was replaced with Intermediate 53 to obtain Intermediate 74. ESI-MS: *m*/*z* = 522 [M+H]⁺.

### Intermediate 75. 3-((4-(3,9-diazaspiro[5.5]undecan-3-yl)phenyl)amino)-5-(3-(3-(oxetan-3-yl)-2-oxoimidazolin-1-yl)piperidin-1-yl)pyrazin-2-carboxamide

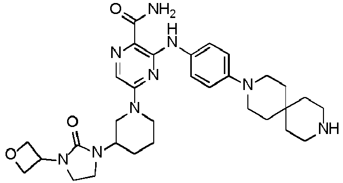

The synthesis method was the same as that for Intermediate 64, except that Intermediate 52 in synthesis step 1 was replaced with Intermediate 54 to obtain Intermediate 75. ESI-MS: *m*/*z* = 590 [M+H]⁺.

### Intermediate 76. 3-((4-(2,6-diazaspirocyclo[3.3]heptan-2-yl)phenyl)amino)-5-(3-(3-(oxetan-3-yl)-2-oxoimidazolin-1-yl)piperidin-1-yl)pyrazin-2-carboxamide

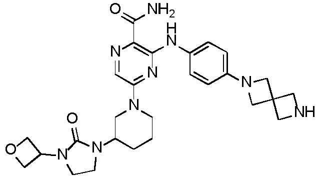

The synthesis method was the same as that for Intermediate 64, except that Intermediate 52 in synthesis step 1 was replaced with Intermediate 55 to obtain Intermediate 76. ESI-MS: m/z = 534 [M+H]⁺.

### Intermediate 77. 5-(3-(3-(oxetan-3-yl)-2-oxoimidazolin-1-yl)piperidin-1-yl)-3-((4-(2-oxo-7-azaspiro[3.5]non-7-yl)phenyl)amino)pyrazin-2-carboxamide

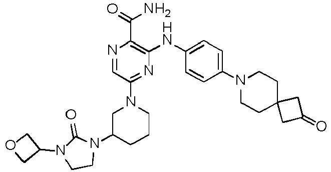

The synthesis method was the same as that for Intermediate 64, except that Intermediate 52 in synthesis step 1 was replaced with Intermediate 56 and step 3 was omitted to obtain Intermediate 77. ESI-MS: *m*/*z* = 575 [M+H]⁺.

### Intermediate 78. 5-(3-(3-methyl-2-oxo-2,3-dihydro-1H-benzo[d]imidazol-1-yl) piperidin-1-yl)-3-((4-(4-methylpiperidin-4-yl)phenyl)amino)pyrazin-2-carboxamide

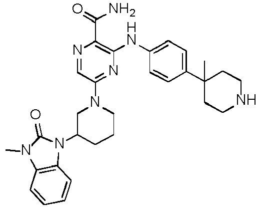

The synthesis method was the same as that for Intermediate 63, except that Intermediate 9 in synthesis step 1 was replaced with Intermediate 20 to obtain Intermediate 78. ESI-MS: *m*/*z =* 541 [M+H]⁺.

### Intermediate 79. 3-((4-(4-methylpiperidin-4-yl)phenyl)amino)-5-(3-(3-oxoimidazo [1,5-a]pyridin-2(3H)-yl)piperidin-1-yl)pyrazin-2-carboxamide

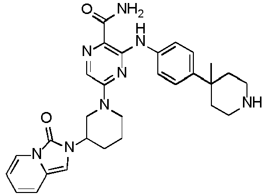

The synthesis method was the same as that for Intermediate 63, except that Intermediate 9 in synthesis step 1 was replaced with Intermediate 21 to obtain Intermediate 79. ESI-MS: *m*/*z* = 527 [M+H]⁺.

### Intermediate 80. 5-(3-(3-cyclopentyl-2-oxoimidazolin-1-yl)piperidin-1-yl)-3-((4-(piperidin-4-yl)phenyl)amino)pyrazin-2-carboxamide

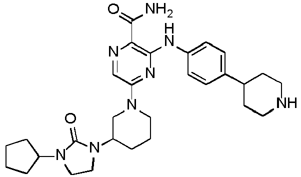

The synthesis method was the same as that for Intermediate 63, except that Intermediate 52 in synthesis step 1 was replaced with Intermediate 58 to obtain Intermediate 80. ESI-MS: *m*/*z* = 533 [M+H]⁺.

### Intermediate 81. 5-(3-(3-cyclopentyl-2-oxoimidazolin-1-yl)piperidin-1-yl)-3-((4-(piperidin-4-carbonyl)phenyl)amino)pyrazin-2-carboxamide

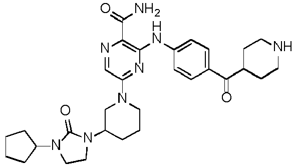

The synthesis method was the same as that for Intermediate 63, except that Intermediate 52 in synthesis step 1 was replaced with Intermediate 57 to obtain Intermediate 81. ESI-MS: *m*/*z* = 561 [M+H]⁺.

### Intermediate 82. Preparation of 5-(3-(3-cyclopentyl-2-oxoimidazolin-1-yl) piperidin -1-yl)-3-((4-fluorophenyl)amino)pyrazin-2-carboxamide

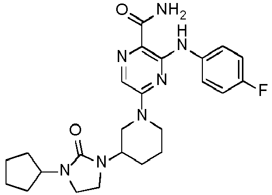

The synthesis method was the same as that for Intermediate 63, except that Intermediate 52 in synthesis step 1 was replaced with 4-fluoroaniline and step 3 was omitted to obtain Intermediate 82. ESI-MS: *m*/*z* = 468 [M+H]⁺.

### Intermediate 83. 5-(3-(3-cyclopentyl-2-oxoimidazolin-1-yl)piperidin-1-yl)-3-((1,2,3,4-tetrahydroisoquinolin-6-yl)amino)pyrazin-2-carboxamide

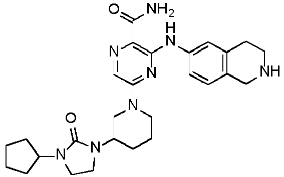

The synthesis method was the same as that for Intermediate 63, except that Intermediate 52 in synthesis step 1 was replaced with tert-butyl 6-amino-3,4-dihydroisoquinoline-2 (1H)-carboxylate to obtain Intermediate 83. ESI-MS: *m*/*z* = 505 [M+H]⁺.

### Intermediate 84. 5-(3-(3-(oxetan-3-yl)-2-oxoimidazolin-1-yl)piperidin-1-yl)-3-((1,2,3,4-tetrahydroisoquinolin-6-yl)amino)pyrazin-2-carboxamide

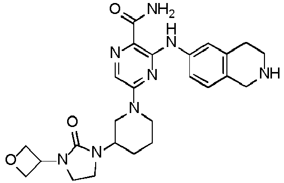

The synthesis method was the same as that for Intermediate 64, except that Intermediate 52 in synthesis step 1 was replaced with tert-butyl 6-amino-3,4-dihydroisoquinoline-2(1H)-carboxylate to obtain Intermediate 84. ESI-MS: *m*/*z* = 493 [M+H]⁺.

### Intermediate 85. 3-(3-(3-methyl-2-oxoimidazolin-1-yl)piperidin-1-yl)-5-((4-(4-methylpiperidin-4-yl)phenyl)amino)-1,2,4-triazin-6-carboxamide

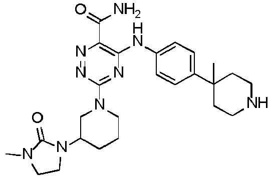

The synthesis method was the same as that for Intermediate 63, except that Intermediate 9 in synthesis step 1 was replaced with Intermediate 22 to obtain Intermediate 85. ESI-MS: *m*/*z =* 494 [M+H]⁺.

### Intermediate 86. 2-(3-(3-methyl-2-oxoimidazolin-1-yl)piperidin-1-yl)-4-((4-(4-methylpiperidin-4-yl)phenyl)amino)pyrimidin-5-carboxamide

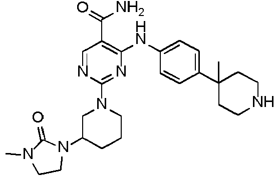

The synthesis method was the same as that for Intermediate 63, except that Intermediate 9 in synthesis step 1 was replaced with Intermediate 23 to obtain Intermediate 86. ESI-MS: *m*/*z =* 493 [M+H]⁺.

### Intermediate 87. 5-fluoro-6-(3-(3-methyl-2-oxoimidazolin-1-yl)piperidin-1-yl)-2-((4-(4-methylpiperidin-4-yl)phenyl)amino)nicotinamide

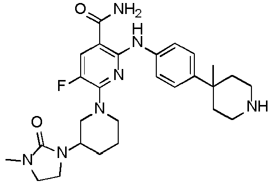

The synthesis method was the same as that for Intermediate 63, except that Intermediate 9 in synthesis step 1 was replaced with Intermediate 24 to obtain Intermediate 87. ESI-MS: *m*/*z* = 510 [M+H]⁺.

### Intermediate 88. 5-methoxy-6-(3-(3-methyl-2-oxoimidazolin-1-yl)piperidin-1-yl)-2-((4-(4-methylpiperidin-4-yl)phenyl)amino)nicotinamide

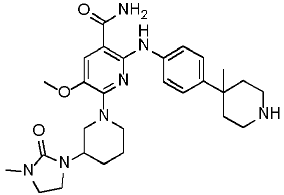

The synthesis method was the same as that for Intermediate 63, except that Intermediate 9 in synthesis step 1 was replaced with Intermediate 25 to obtain Intermediate 88. ESI-MS: *m*/*z* = 522 [M+H]⁺.

### Intermediate 89. 5-(3-(3-cyclopentyl-2-oxoimidazolin-1-yl)piperidin-1-yl)-3-((4-(4-fluoropiperidin-4-yl)phenyl)amino)pyrazin-2-carboxamide

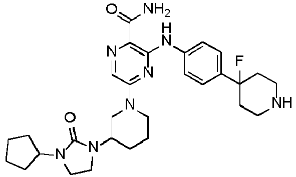

The synthesis method was the same as that for Intermediate 63, except that Intermediate 52 in synthesis step 1 was replaced with Intermediate 59 to obtain Intermediate 89. ESI-MS: *m*/*z* = 551 [M+H]⁺.

### Intermediate 90. 5-(3-(3-cyclopentyl-2-oxoimidazolin-1-yl)piperidin-1-yl)-3-((2-fluoro-4-(piperidin-4-yl)phenyl)amino)pyrazin-2-carboxamide

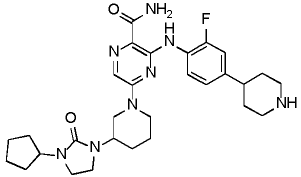

The synthesis method was the same as that for Intermediate 63, except that Intermediate 52 in synthesis step 1 was replaced with Intermediate 60 to obtain Intermediate 90. ESI-MS: *m*/*z* = 551 [M+H]⁺.

### Intermediate 91. 3-((3-cyano-4-(piperidin-4-yl)phenyl)amino)-5-(3-(3-cyclopentyl-2-oxoimidazolin-1-yl)piperidin-1-yl)pyrazin-2-carboxamide

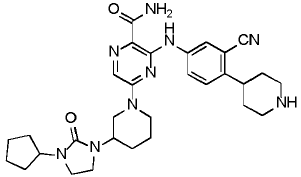

The synthesis method was the same as that for Intermediate 63, except that Intermediate 52 in synthesis step 1 was replaced with Intermediate 61 to obtain Intermediate 91. ESI-MS: *m*/*z* = 558 [M+H]⁺.

### Intermediate 92. 5-(3-(3-cyclopentyl-2-oxoimidazolin-1-yl)piperidin-1-yl)-3-((4-(3-fluoropiperidin-4-yl)phenyl)amino)pyrazin-2-carboxamide

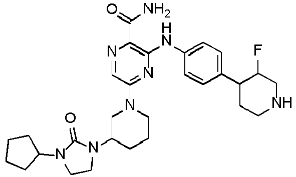

The synthesis method was the same as that for Intermediate 63, except that Intermediate 52 in synthesis step 1 was replaced with Intermediate 62 to obtain Intermediate 92. ESI-MS: *m*/*z* = 551 [M+H]⁺.

### Intermediate 93. 5-(3-(4-methyl-5-oxo-4,6-diazaspirocyclo[2.4]heptan-6-yl)piperidin -1-yl)-3-((4-(piperidin-4-yl)phenyl)amino)pyrazin-2-carboxamide

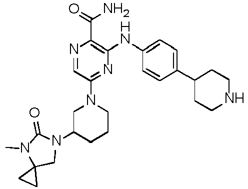

The synthesis method was the same as that for Intermediate 63, except that Intermediate 9 in synthesis step 1 was replaced with Intermediate 18 and Intermediate 52 was replaced with Intermediate 58 to obtain Intermediate 93. ESI-MS: *m*/*z* = 505 [M+H]⁺.

### Intermediate 94. 5-(3-(3-oxohexahydroimidazo[1,5-a]pyridin-2(3H)-yl)piperidin-1-yl) -3-((4-(piperidin-4-yl)phenyl)amino)pyrazin-2-carboxamide

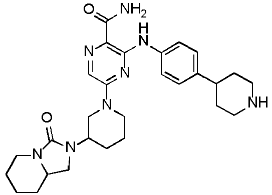

The synthesis method was the same as that for Intermediate 63, except that Intermediate 9 in synthesis step 1 was replaced with Intermediate 14 and Intermediate 52 was replaced with Intermediate 58 to obtain Intermediate 94. ESI-MS: *m*/*z* = 505 [M+H]⁺.

### Intermediate 95. 5-(-6-(3-methyl-2-oxoimidazolin-1-yl)-2-azabicyclo[2.2.1]heptan-2-yl)-3-((4-(piperidin-4-yl)phenyl)amino)pyrazin-2-carboxamide

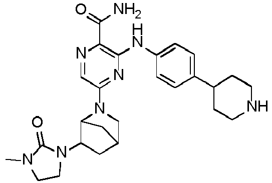

The synthesis method was the same as that for Intermediate 63, except that Intermediate 9 in synthesis step 1 was replaced with Intermediate 26 and Intermediate 52 was replaced with Intermediate 58 to obtain Intermediate 95. ESI-MS: *m*/*z* = 491 [M+H]⁺.

### Intermediate 96. 5-(-6-(3-isopropyl-2-oxoimidazolin-1-yl)-2-azabicyclo[2.2.1]heptan -2-yl)-3-((4-(piperidin-4-yl)phenyl)amino)pyrazin-2-carboxamide

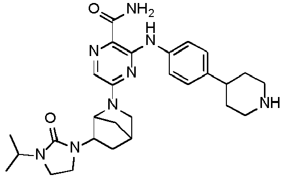

The synthesis method was the same as that for Intermediate 63, except that Intermediate 9 in synthesis step 1 was replaced with Intermediate 27 and Intermediate 52 was replaced with Intermediate 58 to obtain Intermediate 96. ESI-MS: *m*/*z* = 519 [M+H]⁺.

### Intermediate 97. 6-(-6-(3-methyl-2-oxyimidazolin-1-yl)-2-azabicyclo[2.2.1]heptan-2-yl)-2-((4-(piperidin-4-yl)phenyl)amino)nicotinamide

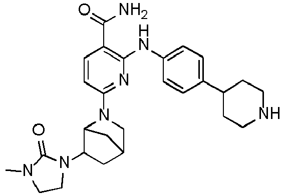

The synthesis method was the same as that for Intermediate 63, except that Intermediate 9 in synthesis step 1 was replaced with Intermediate 28 and Intermediate 52 was replaced with Intermediate 58 to obtain Intermediate 97. ESI-MS: *m*/*z* = 490 [M+H]⁺.

### Intermediate 98. 3-(-6-(3-methyl-2-oxoimidazolin-1-yl)-2-azabicyclo[2.2.1]heptan-2-yl)-5-((4-(piperidin-4-yl)phenyl)amino)-1,2,4-triazin-6-carboxamide

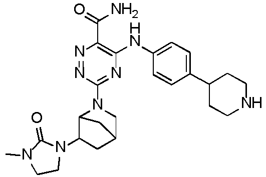

The synthesis method was the same as that for Intermediate 63, except that Intermediate

9 in synthesis step 1 was replaced with Intermediate 30 and Intermediate 52 was replaced with Intermediate 58 to obtain Intermediate 98. ESI-MS: *m*/*z* = 492 [M+H]⁺.

### Intermediate 99. 5-(-6-(3-methyl-2-oxoimidazolin-1-yl)-2-azabicyclo[2.2.2]octan-2-yl) -3-((4-(piperidin-4-yl)phenyl)amino)pyrazin-2-carboxamide

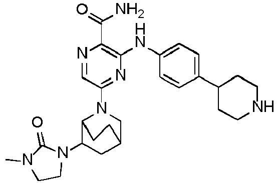

The synthesis method was the same as that for Intermediate 63, except that Intermediate 9 in synthesis step 1 was replaced with Intermediate 29 and Intermediate 52 was replaced with Intermediate 58 to obtain Intermediate 99. ESI-MS: *m*/*z* = 505 [M+H]⁺.

### Intermediate 100. 3-(3-(3-methyl-2-oxoimidazolin-1-yl)piperidin-1-yl)-5-((4-(piperidin-4-yl)phenyl)amino)-1,2,4-triazin-6-carboxamide

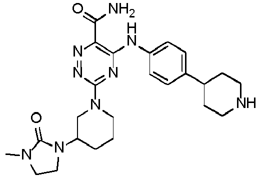

The synthesis method was the same as that for Intermediate 63, except that Intermediate 9 in synthesis step 1 was replaced with Intermediate 22 and Intermediate 52 was replaced with Intermediate 58 to obtain Intermediate 100, ESI-MS: *m*/*z* = 480 [M+H]⁺.

### Intermediate 101. 3-(3-(3-ethyl-2-oxoimidazolin-1-yl)piperidin-1-yl)-5-((4-(piperidin -4-yl)phenyl)amino)-1,2,4-triazin-6-carboxamide

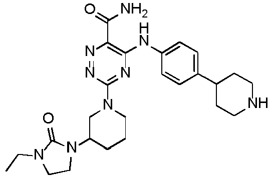

The synthesis method was the same as that for Intermediate 63, except that Intermediate 9 in synthesis step 1 was replaced with Intermediate 31 and Intermediate 52 was replaced with Intermediate 58 to obtain Intermediate 101, ESI-MS: *m*/*z* = 494 [M+H]⁺.

### Intermediate 102. 3-(3-(3-isopropyl-2-oxoimidazolin-1-yl)piperidin-1-yl)-5-((4-(piperidin-4-yl)phenyl)amino)-1,2,4-triazin-6-carboxamide

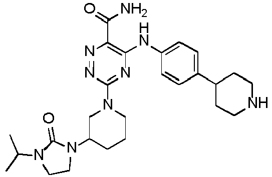

The synthesis method was the same as that for Intermediate 63, except that Intermediate 9 in synthesis step 1 was replaced with Intermediate 32 and Intermediate 52 was replaced with Intermediate 58 to obtain Intermediate 102, ESI-MS: *m*/*z* = 508 [M+H]⁺.

### Intermediate 103. 3-(3-(2-oxo-3-(trifluoromethyl)imidazolin-1-yl)piperidin-1-yl)-5-((4-(piperidin-4-yl)phenyl)amino)-1,2,4-triazin-6-carboxamide

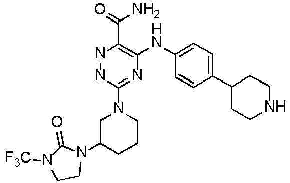

The synthesis method was the same as that for Intermediate 63, except that Intermediate 9 in synthesis step 1 was replaced with Intermediate 33 and Intermediate 52 was replaced with Intermediate 58 to obtain Intermediate 103, ESI-MS: *m*/*z* = 534 [M+H]⁺.

### Intermediate 104. 3-(3-(2-oxo-3-(2,2,2-trifluoroethyl)imidazolin-1-yl)piperidin-1-yl)-5-((4-(piperidin-4-yl)phenyl)amino)-1,2,4-triazin-6-carboxamide

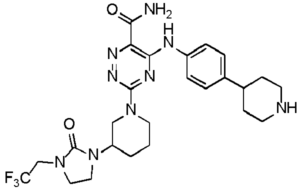

The synthesis method was the same as that for Intermediate 63, except that Intermediate 9 in synthesis step 1 was replaced with Intermediate 34 and Intermediate 52 was replaced with Intermediate 58 to obtain Intermediate 104, ESI-MS: *m*/*z* = 548 [M+H]⁺.

### Intermediate 105. 3-(3-(3-cyclopropyl-2-oxoimidazolin-1-yl)piperidin-1-yl)-5-((4-(piperidin-4-yl)phenyl)amino)-1,2,4-triazin-6-carboxamide

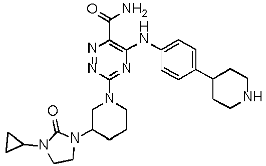

The synthesis method was the same as that for Intermediate 63, except that Intermediate 9 in synthesis step 1 was replaced with Intermediate 35 and Intermediate 52 was replaced with Intermediate 58 to obtain Intermediate 105, ESI-MS: *m*/*z* = 506 [M+H]⁺.

### Intermediate 106. 3-(3-(3-cyclobutyl-2-oxoimidazolin-1-yl)piperidin-1-yl)-5-((4-(piperidin-4-yl)phenyl)amino)-1,2,4-triazin-6-carboxamide

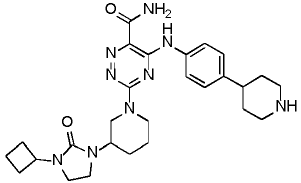

The synthesis method was the same as that for Intermediate 63, except that Intermediate 9 in synthesis step 1 was replaced with Intermediate 36 and Intermediate 52 was replaced with Intermediate 58 to obtain Intermediate 106, ESI-MS: *m*/*z* = 520 [M+H]⁺.

### Intermediate 107. 3-(3-(3-cyclopentyl-2-oxoimidazolin-1-yl)piperidin-1-yl)-5-((4-(piperidin-4-yl)phenyl)amino)-1,2,4-triazin-6-carboxamide

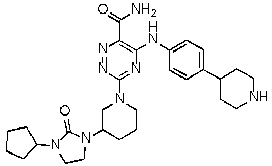

The synthesis method was the same as that for Intermediate 63, except that Intermediate 9 in synthesis step 1 was replaced with Intermediate 37 and Intermediate 52 was replaced with Intermediate 58 to obtain Intermediate 107, ESI-MS: *m*/*z* = 534 [M+H]⁺.

### Intermediate 108. 3-(3-(3-cyclohexyl-2-oxoimidazolin-1-yl)piperidin-1-yl)-5-((4-(piperidin-4-yl)phenyl)amino)-1,2,4-triazin-6-carboxamide

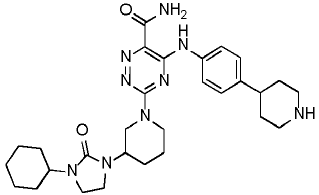

The synthesis method was the same as that for Intermediate 63, except that Intermediate 9 in synthesis step 1 was replaced with Intermediate 38 and Intermediate 52 was replaced with Intermediate 58 to obtain Intermediate 108, ESI-MS: *m*/*z* = 548 [M+H]⁺.

### Intermediate 109. 3-(3-(3-(oxetan-3-yl)-2-oxoimidazolin-1-yl)piperidin-1-yl)-5-((4-(piperidin-4-yl)phenyl)amino)-1,2,4-triazin-6-carboxamide

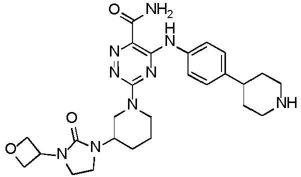

The synthesis method was the same as that for Intermediate 63, except that Intermediate 9 in synthesis step 1 was replaced with Intermediate 39 and Intermediate 52 was replaced with Intermediate 58 to obtain Intermediate 109, ESI-MS: *m*/*z* = 522 [M+H]⁺.

### Intermediate 110. 3-(3-(2-oxo-3-(tetrahydro-2H-pyran-4-yl)imidazolin-1-yl) piperidin-1-yl)-5-((4-(piperidin-4-yl)phenyl)amino)-1,2,4-triazin-6-carboxamide

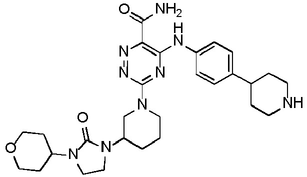

The synthesis method was the same as that for Intermediate 63, except that Intermediate 9 in synthesis step 1 was replaced with Intermediate 40 and Intermediate 52 was replaced with Intermediate 58 to obtain Intermediate 110, ESI-MS: *m*/*z* = 550 [M+H]⁺.

### Intermediate 111. 3-(3-(2-oxo-3-phenylimidazolin-1-yl)piperidin-1-yl)-5-((4-(piperidin-4-yl)phenyl)amino)-1,2,4-triazin-6-carboxamide

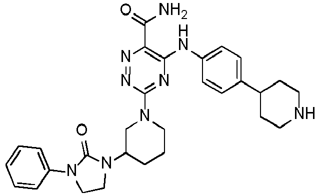

The synthesis method was the same as that for Intermediate 63, except that Intermediate 9 in synthesis step 1 was replaced with Intermediate 41 and Intermediate 52 was replaced with Intermediate 58 to obtain Intermediate 111, ESI-MS: *m*/*z* = 542 [M+H]⁺.

### Intermediate 112. 3-(3-(3-(2-fluorophenyl)-2-oxoimidazolin-1-yl)piperidin-1-yl)-5-((4-(piperidin-4-yl)phenyl)amino)-1,2,4-triazin-6-carboxamide

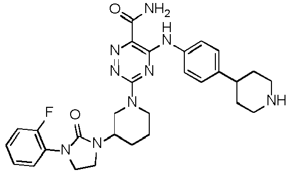

The synthesis method was the same as that for Intermediate 63, except that Intermediate 9 in synthesis step 1 was replaced with Intermediate 42 and Intermediate 52 was replaced with Intermediate 58 to obtain Intermediate 112, ESI-MS: *m*/*z* = 560 [M+H]⁺.

### Intermediate 113. 3-(3-(3-fluorophenyl)-2-oxoimidazolin-1-yl)piperidin-1-yl)-5-((4-(piperidin-4-yl)phenyl)amino)-1,2,4-triazin-6-carboxamide

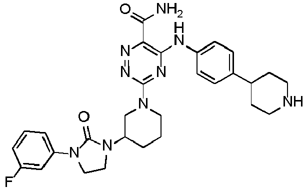

The synthesis method was the same as that for Intermediate 63, except that Intermediate 9 in synthesis step 1 was replaced with Intermediate 43 and Intermediate 52 was replaced with Intermediate 58 to obtain Intermediate 113, ESI-MS: *m*/*z* = 560 [M+H]⁺.

### Intermediate 114. 3-(3-(2-oxo-3-(pyridin-3-yl)imidazolin-1-yl)piperidin-1-yl)-5-((4-(piperidin-4-yl)phenyl)amino)-1,2,4-triazin-6-carboxamide

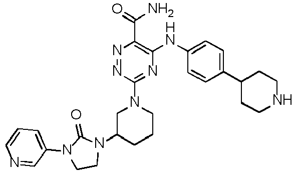

The synthesis method was the same as that for Intermediate 63, except that Intermediate 9 in synthesis step 1 was replaced with Intermediate 44 and Intermediate 52 was replaced with Intermediate 58 to obtain Intermediate 114, ESI-MS: *m*/*z* = 543 [M+H]⁺.

### Intermediate 115. 3-(3-(4-methyl-5-oxo-4,6-diazaspirocyclo[2.4]heptan-6-yl) piperidin-1-yl)-5-((4-(piperidin-4-yl)phenyl)amino)-1,2,4-triazin-6-carboxamide

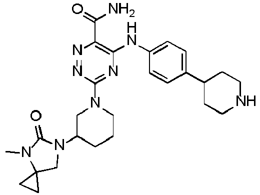

The synthesis method was the same as that for Intermediate 63, except that Intermediate 9 in synthesis step 1 was replaced with Intermediate 45 and Intermediate 52 was replaced with Intermediate 58 to obtain Intermediate 115, ESI-MS: *m*/*z* = 506 [M+H]⁺.

### Intermediate 116. 3-(3-(5-methyl-6-oxo-5,7-diazaspirocyclo[3.4]octan-7-yl) piperidin-1-yl)-5-((4-(piperidin-4-yl)phenyl)amino)-1,2,4-triazin-6-carboxamide

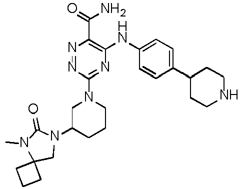

The synthesis method was the same as that for Intermediate 63, except that Intermediate 9 in synthesis step 1 was replaced with Intermediate 46 and Intermediate 52 was replaced with Intermediate 58 to obtain Intermediate 116, ESI-MS: *m*/*z* = 520 [M+H]⁺.

### Intermediate 117. 3-(3-(3-oxotrihydro-1H-pyrrolo[1,2-c]imidazol-2(3H)-yl) piperidin-1-yl)-5-((4-(piperidin-4-yl)phenyl)amino)-1,2,4-triazin-6-carboxamide

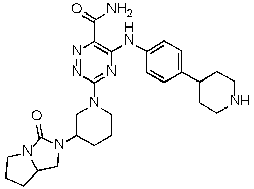

The synthesis method was the same as that for Intermediate 63, except that Intermediate 9 in synthesis step 1 was replaced with Intermediate 47 and Intermediate 52 was replaced with Intermediate 58 to obtain Intermediate 117, ESI-MS: *m*/*z* = 506 [M+H]⁺.

### Intermediate 118. 3-(6-(3-cyclopentyl-2-oxoimidazolin-1-yl)-2-azabicyclo[2.2.1] heptan-2-yl)-5-((4-(piperidin-4-yl)phenyl)amino)-1,2,4-triazin-6-carboxamide

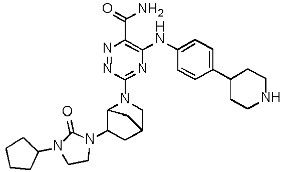

The synthesis method was the same as that for Intermediate 63, except that Intermediate 9 in synthesis step 1 was replaced with Intermediate 48 and Intermediate 52 was replaced with Intermediate 58 to obtain Intermediate 118, ESI-MS: *m*/*z* = 546 [M+H]⁺.

### Intermediate 119. 3-(6-(2-oxo-3-phenylimidazolin-1-yl)-2-azabicyclo[2.2.1]heptan -2-yl)-5-((4-(piperidin-4-yl)phenyl)amino)-1,2,4-triazin-6-carboxamide

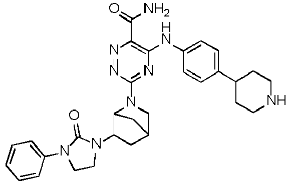

The synthesis method was the same as that for Intermediate 63, except that Intermediate

9 in synthesis step 1 was replaced with Intermediate 49 and Intermediate 52 was replaced with Intermediate 58 to obtain Intermediate 119, ESI-MS: *m*/*z* = 554 [M+H]⁺.

### Intermediate 120. 3-(6-(3-oxotrihydro-1H-pyrrolo[1,2-c]imidazol-2(3H)-yl)-2-azabicyclo[2.2.1]heptan-2-yl)-5-((4-(piperidin-4-yl)phenyl)amino)-1,2,4-triazin-6-carboxamide

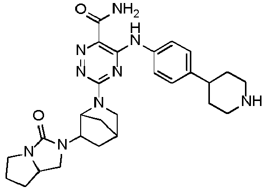

The synthesis method was the same as that for Intermediate 63, except that Intermediate 9 in synthesis step 1 was replaced with Intermediate 50 and Intermediate 52 was replaced with Intermediate 58 to obtain Intermediate 120, ESI-MS: *m*/*z* = 518 [M+H]⁺.

### Intermediate 121. 3-(6-(3-methyl-2-oxoimidazolin-1-yl)-2-azabicyclo[2.2.2] octan-2-yl)-5-((4-(piperidin-4-yl)phenyl)amino)-1,2,4-triazin-6-carboxamide

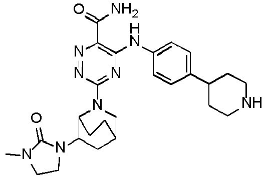

The synthesis method was the same as that for Intermediate 63, except that Intermediate 9 in synthesis step 1 was replaced with Intermediate 51 and Intermediate 52 was replaced with Intermediate 58 to obtain Intermediate 121, ESI-MS: *m*/*z* = 506 [M+H]⁺.

### Intermediate 122. 3-(3-(3-methyl-2-oxoimidazolin-1-yl)piperidin-1-yl)-5-((1,2,3,4-tetrahydroisoquinolin-6-yl)amino)-1,2,4-triazin-6-carboxamide

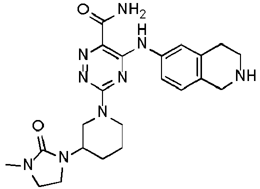

The synthesis method was the same as that for Intermediate 63, except that Intermediate 9 in synthesis step 1 was replaced with Intermediate 22 and Intermediate 52 was replaced with tert-butyl 6-amino-3,4-dihydroisoquinoline-2(1H)-carboxylate to obtain Intermediate 122, ESI-MS: *m*/*z* = 452 [M+H]⁺.

### Intermediate 123. 3-(3-(3-methyl-2-oxoimidazolin-1-yl)piperidin-1-yl)-5-((4-(piperazin-1-yl)phenyl)amino)-1,2,4-triazin-6-carboxamide

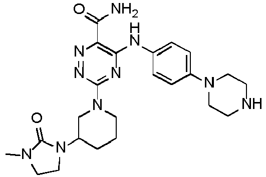

The synthesis method was the same as that for Intermediate 63, except that Intermediate 9 in synthesis step 1 was replaced with Intermediate 22 and Intermediate 52 was replaced with Intermediate 53 to obtain Intermediate 123, ESI-MS: *m*/*z* = 481 [M+H]⁺.

### Intermediate 124. 3-(3-(3-methyl-2-oxoimidazolin-1-yl)piperidin-1-yl)-5-((4-(4-oxopiperidin-1-yl)phenyl)amino)-1,2,4-triazin-6-carboxamide

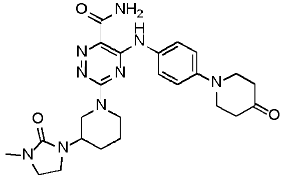

The synthesis method was the same as that for Intermediate 63, except that Intermediate 9 in synthesis step 1 was replaced with Intermediate 22 and Intermediate 52 was replaced with 1-(4-aminophenyl)piperidin-4-one to obtain Intermediate 124, ESI-MS: *m*/*z* = 494 [M+H]⁺.

### Intermediate 125. 5-((4-(4-methylpiperidin-4-yl)phenyl)amino)-3-(3-(2-oxo-3-phenylimidazolin-1-yl)piperidin-1-yl)-1,2,4-triazin-6-carboxamide

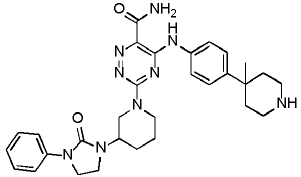

The synthesis method was the same as that for Intermediate 63, except that Intermediate 9 in synthesis step 1 was replaced with Intermediate 41 to obtain Intermediate 125, ESI-MS: m/z = 556 [M+H]⁺.

### Intermediate 126. 3-(3-(2-oxo-3-phenylimidazolin-1-yl)piperidin-1-yl)-5-((1,2,3,4-tetrahydroisoquinolin-6-yl)amino)-1,2,4-triazin-6-carboxamide

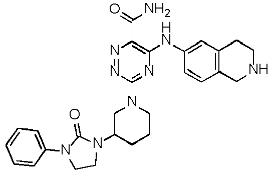

The synthesis method was the same as that for Intermediate 63, except that Intermediate 9 in synthesis step 1 was replaced with Intermediate 41 and Intermediate 52 was replaced with tert-butyl 6-amino-3,4-dihydroisoquinoline-2(1H)-carboxylate to obtain Intermediate 126, ESI-MS: *m*/*z* = 514 [M+H]⁺.

### Intermediate 127. 3-(3-(2-oxo-3-phenylimidazolin-1-yl)piperidin-1-yl)-5-((4-(piperazin-1-yl)phenyl)amino)-1,2,4-triazin-6-carboxamide

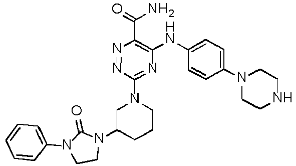

The synthesis method was the same as that for Intermediate 63, except that Intermediate 9 in synthesis step 1 was replaced with Intermediate 41 and Intermediate 52 was replaced with Intermediate 53 to obtain Intermediate 127, ESI-MS: *m*/*z* = 543 [M+H]⁺.

### Intermediate 128. 3-(3-(2-oxo-3-phenylimidazolin-1-yl)piperidin-1-yl)-5-((4-(4-oxopiperidin-1-yl)phenyl)amino)-1,2,4-triazin-6-carboxamide

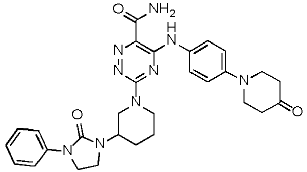

The synthesis method was the same as that for Intermediate 63, except that Intermediate 9 in synthesis step 1 was replaced with Intermediate 41 and Intermediate 52 was replaced with 1-(4-aminophenyl)piperidin-4-one to obtain Intermediate 128, ESI-MS: *m*/*z* = 556 [M+H]⁺.

### Intermediate 129. 3-(6-(3-methyl-2-oxoimidazolin-1-yl)-2-azabicyclo[2.2.1] heptan-2-yl)-5-((4-(4-methylpiperidin-4-yl)phenyl)amino)-1,2,4-triazin-6-carboxamide

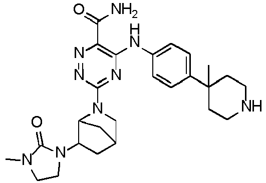

The synthesis method was the same as that for Intermediate 63, except that Intermediate 9 in synthesis step 1 was replaced with Intermediate 30 to obtain Intermediate 129, ESI-MS: m/z = 506 [M+H]⁺.

### Intermediate 130. 3-(6-(3-methyl-2-oxoimidazolin-1-yl)-2-azabicyclo[2.2.1]heptan-2 -yl)-5-((1,2,3,4-tetrahydroisoquinolin-6-yl)amino)-1,2,4-triazin-6-carboxamide

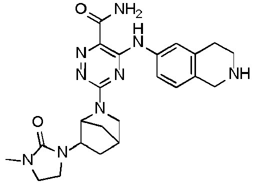

The synthesis method was the same as that for Intermediate 63, except that Intermediate 9 in synthesis step 1 was replaced with Intermediate 30 and Intermediate 52 was replaced with tert-butyl 6-amino-3,4-dihydroisoquinoline-2(1H)-carboxylate to obtain Intermediate 130, ESI-MS: *m*/*z* = 464 [M+H]⁺.

### Intermediate 131. 3-(6-(3-methyl-2-oxoimidazolin-1-yl)-2-azabicyclo[2.2.1]heptan -2-yl)-5-((4-(piperazin-1-yl)phenyl)amino)-1,2,4-triazin-6-carboxamide

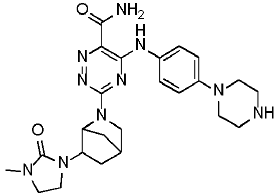

The synthesis method was the same as that for Intermediate 63, except that Intermediate 9 in synthesis step 1 was replaced with Intermediate 30 and Intermediate 52 was replaced with Intermediate 53 to obtain Intermediate 131, ESI-MS: *m*/*z* = 493 [M+H]⁺.

### Intermediate 132. 3-(6-(3-methyl-2-oxoimidazolin-1-yl)-2-azabicyclo[2.2.1]heptan-2 -yl)-5-((4-(4-oxopiperidin-1-yl)phenyl)amino)-1,2,4-triazin-6-carboxamide

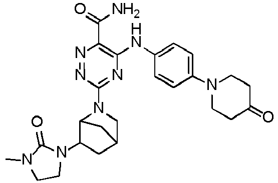

The synthesis method was the same as that for Intermediate 63, except that Intermediate 9 in synthesis step 1 was replaced with Intermediate 30 and Intermediate 52 was replaced with 1-(4-aminophenyl)piperidin-4-one to obtain Intermediate 132, ESI-MS: *m*/*z* = 506 [M+H]⁺.

### Intermediate 133. 2-(2,6-dioxopiperidin-3-yl)-5-(3-oxoazetidin-1-yl)isoindol-1,3-dione

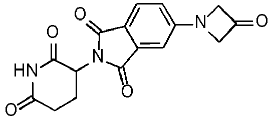

### Synthesis step 1. Synthesis of 2-(2,6-dioxo-piperidin-3-yl)-5-fluoro-isoindol-1,3-dione (133-1)

4-fluorophthalic anhydride (2.257 g, 13.59 mmol) was dissolved in 50 mL glacial acetic acid, 3-aminopiperidine-2,6-dione hydrochloride (2.46 g, 14.95 mmol) and potassium acetate (4.14 g, 42.18 mmol) were added, the mixture was heated to 90°C, and reacted overnight. After the reaction was completed, the reaction liquid was cooled to room temperature, 120 mL of water was added, and kept in ice bath for 40 min. A large amount of white solid was precipitated. Filter-suction was performed. The solid was washed with methanol. The filter cake was dried to obtain a crude product, which was purified by column chromatography to obtain a white solid (133-1) (3.05 g, yield 81.25%). ESI-MS: *m*/*z* = 277 [M+H]^{+ 1}H NMR (500 MHz, DMSO-d₆) δ 11.16 (s, 1H), 8.02 (dd, J = 8.3, 4.5 Hz, 1H), 7.86 (dd, J = 7.5, 2.3 Hz, 1H), 7.73 (ddd, J = 9.3, 8.2, 2.3 Hz, 1H), 5.18 (dd, J = 12.9, 5.4 Hz, 1H), 2.90 (ddd, J = 17.1, 13.9, 5.4 Hz, 1H), 2.70 - 2.52 (m, 2H), 2.08 (dtd, J = 13.0, 5.4, 2.3 Hz, 1H).

### Synthesis step 2. 2-(2,6-dioxopiperidin-3-yl)-5-(3-oxoazetidin-1-yl)isoindol-1,3-dione (Intermediate 133)

2-(2,6-dioxo-piperidin-3-yl)-5-fluoro-isoindole-1,3-dione (663 mg, 2.40 mmol) was dissolved in 10 mL of DMSO, azetidin-3-one hydrochloride (171 mg, 2.40 mmol) and 836 µl of DIPEA were added, and the mixture was reacted at 90°C overnight. After the reaction was completed, 10 mL of water was added, and extraction was performed with ethyl acetate. The organic layers were combined, washed with saturated sodium chloride, dried over anhydrous sodium sulfate, and filtered with suction. The solvent was removed under pressure and purification was performed by column chromatography to obtain Intermediate 79 as a yellow solid (668 mg, yield 85.0%), thus Intermediate 133 was obtained. ESI-MS: *m*/*z* = 328 [M+H]⁺ .

### Intermediate 134 2-(2,6-dioxopiperidin-3-yl)-5-(4-oxopiperidin-1-yl)isoindol-1,3-dione

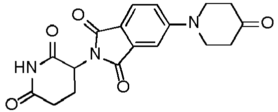

The synthesis method was the same as that for Intermediate 133, except that azetidin-3-one hydrochloride in synthesis step 1 was replaced with piperidin-4-one to obtain Intermediate 134. ESI-MS: *m*/*z* = 356[M+H]⁺.

### Intermediate 135. 3-(1-oxo-5-(4-oxopiperidin-1-yl)isoindol-2-yl)piperidin-2,6-dione

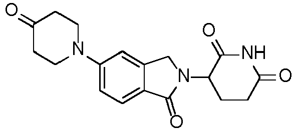

### Synthesis step 1. 5-(4-(dimethoxymethyl)piperidin-1-yl)isobenzofuran-1(3H)-one (135-1)

5-bromophthalide (1.0 g, 4.70 mmol), 4-(dimethoxymethyl)piperidine (1.20 g, 7.04mol), potassium phosphate (2.99 g, 14.09 mmol), and glycol dimethyl ether (15mL) were subjected to nitrogen replacement three times, and tris(dibenzylideneacetone)dipalladium (860 mg, 0.94 mmol) and (±)-2,2'-bis(diphenylphosphino)-1,1'-dinaphthalene (535 mg, 0.94 mmol) were added to a 100 mL double-necked flask in sequence. The mixture was reacted at 80°C for 16 h. A saturated aqueous sodium chloride (60 mL) solution was added, and extraction was performed with ethyl acetate. The organic phase was dried with anhydrous sodium sulfate, and concentrated under reduced pressure to obtain a crude product. The crude product was purified by column chromatography to obtain the product (135-1). ESI-MS: *m*/*z=* 292[M+H]⁺.

### Synthesis step 2. 4-(4-(dimethoxymethyl)piperidin-1-yl)-2-(hydroxymethyl)benzoic acid (135-2)

5-(4-(dimethoxymethyl)piperidin-1-yl)isobenzofuran-1(3H)-one (943 mg, 3.23 mmol) was added to a mixed solvent (3 mL of tetrahydrofuran, 3 mL of methanol, and 3 mL of water), and sodium hydroxide (387 mg, 9.68 mmol) was added in ice bath, and the mixture was stirred for 1 h at 25°C. After TLC monitoring showed that the reaction was completed, the reaction liquid was adjusted to pH 4-5 with 1 M dilute hydrochloric acid, and extracted with ethyl acetate. The solvent was removed under reduced pressure, and purification was performed by column chromatography to obtain the product (135-2). ESI-MS: *m*/*z* = 310[M+H]⁺.

### Synthesis step 3. Methyl 4-(4-(dimethoxymethyl)piperidin-1-yl)-2-(hydroxymethyl) benzoate (135-3)

4-(4-(dimethoxymethyl)piperidin-1-yl)-2-(hydroxymethyl)benzoic acid (738 mg, 2.38 mmol) was dissolved in a mixed solvent of 5 mL of methanol and 5 mL of ethyl acetate, and trimethylsilylated diazomethane (2 M, 3.58 ml) was added at -10°C, the reaction was stirred for 30 min at -10°C. After TLC monitoring showed that the reaction was completed, the reaction was quenched with ice water (30 mL), extracted with ethyl acetate (10 ml*3), and purification was performed by column chromatography to obtain the product (135-3). ESI-MS: *m*/*z =* 324[M+H]⁺.

### Synthesis step 4. Methyl 2-(bromomethyl)-4-(4-(dimethoxymethyl)piperidin-1-yl) benzoate (135-4)

Methyl 4-(4-(dimethoxymethyl)piperidin-1-yl)-2-(hydroxymethyl)benzoate (693 mg, 2.14 mmol) was dissolved in 10 mL of tetrahydrofuran, and triphenylphosphine (842 mg, 3.21 mmol) and carbon tetrabromide (1.06 g, 3.21 mmol) were added in sequence. The mixture was stirred for 1 h at 25°C. After TLC monitoring showed that the reaction was completed, the reaction was quenched by adding 20 mL of ice water, and extracted with ethyl acetate. The organic phases were combined, the solvent was removed under reduced pressure, and purification was performed by column chromatography to obtain the product (135-4). ESI-MS: *m*/*z* = 386[M+H]⁺.

### Synthesis step 5. 3-(5-(4-(dimethoxymethyl)piperidin-1-yl)-1-oxoisoindol-2-yl) piperidin-2,6-dione (135-5)

Methyl 2-(bromomethyl)-4-(4-(dimethoxymethyl)piperidin-1-yl)benzoate (432 mg, 1.12 mmol) was dissolved in a solvent of 10 mL of acetonitrile, and then 3-aminopiperidine-2,6-dione hydrochloride (276 mg, 1.67 mmol) and N,N-diisopropylethylamine (585 µl, 3.36 mmol) were added in sequence. The mixture was reacted at 80°C for 16 h. After TLC monitoring showed that the reaction was completed, the reaction liquid was filtered with suction in ice bath and the solid was washed with acetonitrile to obtain a crude product. ESI-MS:m/z= 402[M+H]⁺.

### Synthesis step 6. 3-(1-oxo-5-(4-oxopiperidin-1-yl)isoindol-2-yl)piperidin-2,6-dione(Intermediate 135)

3-(5-(4-(dimethoxymethyl)piperidin-1-yl)-1-oxoisoindol-2-yl)piperidine-2,6-dione (270 mg, 0.671 mmol) was dissolved in tetrahydrofuran (1 mL) and water (0.2 mL), pyridine 4-methylbenzenesulfonate (337 mg, 1.342 mmol) was added, and the mixture was stirred at 90°C, and concentrated under reduced pressure after the reaction was completed. Purification was performed by column chromatography to obtain (Intermediate 135). ESI-MS: *m*/*z* = 342[M+H]⁺.

### Intermediate 136. 3-(1-oxo-5-(3-oxoazetidin-1-yl)isoindol-2-yl)piperidin-2,6-dione

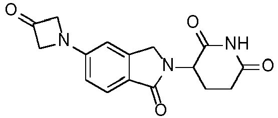

The synthesis method was the same as that for Intermediate 135, except that 4-(dimethoxymethyl)piperidine in synthesis step 1 was replaced with azetidin-3-ol to obtain Intermediate 136. ESI-MS: *m*/*z* = 314 [M+H]⁺ .

### Intermediate 137. 1-(2-(2,6-dioxopiperidin-3-yl)-1,3-dioxoisoindol-5-yl)azetidin-3-carbaldehyde

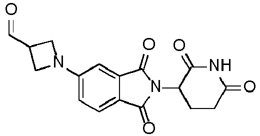

The synthesis method was the same as that for Intermediate 133, except that azetidin-3-one hydrochloride in synthesis step 2 was replaced with azetidin-3-carbaldehyde to obtain Intermediate 137. ESI-MS: *m*/*z* = 342 [M+H]⁺.

### Intermediate 138. 2-(2,6-dioxopiperidin-3-yl)-5-(piperazin-1-yl)isoindol-1,3-dione

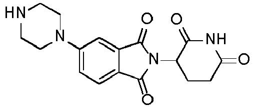

The synthesis method was the same as that for Intermediate 133, except that azetidin-3-one hydrochloride in synthesis step 2 was replaced with piperazine to obtain Intermediate 138, ESI-MS: *m*/*z* = 343 [M+H]⁺.

### Intermediate 139. 2-(2,6-dioxopiperidin-3-yl)-4-(3-oxoaza-1-yl)isoindol-1,3-dione

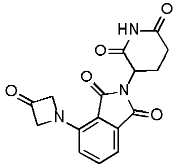

The synthesis method was the same as that for Intermediate 133, except that 4-fluorophthalic anhydride in synthesis step 1 was replaced with 3-fluorophthalic anhydride to obtain Intermediate 139. ESI-MS: m/z = 328 [M+H]⁺.

### Intermediate 140. 1-(2-(2,6-dioxopiperidin-3-yl)-1,3-dioxoisoindol-5-yl)piperidin-4-carbaldehyde

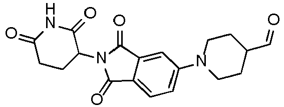

The synthesis method was the same as that for Intermediate 133, except that azetidin-3-one hydrochloride in synthesis step 2 was replaced with 4-formylpiperidine to obtain Intermediate 140, ESI-MS: *m*/*z* = 370 [M+H]⁺.

### Intermediate 141. 2-(2,6-dioxopiperidin-3-yl)-5-(4-(3-(piperidin-4-yl)propan-2-yn-1-yl)piperazin-1-yl)isoindol-1,3-dione

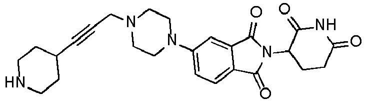

### Synthesis step 1. 2-(2,6-dioxopiperidin-3-yl)-5-(piperazin-1-yl)isoindol-1,3-dione(141-1)

The synthesis method was the same as that for Intermediate 133, except that azetidin-3-one hydrochloride in synthesis step 2 was replaced with piperazine to obtain Intermediate (141-1), ESI-MS: *m*/*z* = 343 [M+H]⁺.

### Synthesis step 2. 2-(2,6-dioxopiperidin-3-yl)-5-(4-(propan-2-yn-1-yl)piperazin-1-yl) isoindol-1,3-dione (141-2)

Compound 2-(2,6-dioxopiperidin-3-yl)-5-(piperazin-1-yl)isoindole-1,3-dione (4.12 g, 12 mmol), 3-bromopropyne (2.86 g, 24 mmol) and potassium carbonate (8.30 g, 60 mmol) were dissolved in acetonitrile (430 mL), and the mixture was stirred at room temperature for 12 h. The solvent was removed under reduced pressure, the residue was dissolved in water, and extracted with ethyl acetate. The organic layers were combined, washed with saturated aqueous sodium chloride solution, dried with anhydrous sodium sulfate, and filtered with suction. The solvent was removed under reduced pressure, and purification was performed by column chromatography to obtain compound (141-2). ESI-MS: *m*/*z* = 381 [M+H]⁺.

### Synthesis step 3. Tert-butyl 4-(3-(4-(2-(2,6-dioxopiperidin-3-yl)-1,3-dioxoisoindol -5-yl)piperazin-1-yl)propyn-1-yl)piperidin-1-carboxylate (141-3)

Under nitrogen protection, compound 2-(2,6-dioxopiperidin-3-yl)-5-(4-(prop-2-yn-1-yl) piperazin-1-yl)isoindole-1,3-dione (2.29 g, 6 mmol) was dissolved in anhydrous THF (30 mL), and the mixture was placed at -78°C. n-butyllithium (576 mg, 9 mmol) was added and the mixture was reacted for 0.5 h. A THF solution of 1-tert-butoxycarbonyl-4-iodopiperidine (2.8 g, 9 mmol) was added, and the mixture was moved to room temperature, raised to 50°C, and reacted for 24 h. The solvent was removed under reduced pressure, the residue was dissolved in water, and extracted with ethyl acetate. The organic layers were combined, washed with saturated aqueous sodium chloride solution, dried over anhydrous sodium sulfate, and filtered with suction. The solvent was removed under pressure and purification was performed by column chromatography to obtain compound (141-3). ESI-MS: *m*/*z* = 564 [M+H]⁺ .

### Synthesis step 4. 2-(2,6-dioxopiperidin-3-yl)-5-(4-(3-(piperidin-4-yl)propan-2-yn-1-yl) piperazin-1-yl)isoindol-1,3-dione(Intermediate 141)

The synthesis steps are as described in synthesis step 3 of Intermediate 63, and compound 63-2 was replaced with compound **141-3** to obtain Intermediate 141. ESI-MS: m/z = 464 [M+H]⁺.

### Intermediate 142. 1-(2-(2,6-dioxopiperidin-3-yl)-1,3-dioxoisoindol-5-yl)pyrrolidin-3-carbaldehyde

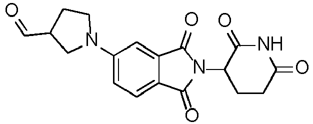

The synthesis method was the same as that for Intermediate 133, except that azetidin-3-one hydrochloride in synthesis step 2 was replaced with pyrrolidine-3-carbaldehyde. ESI-MS: *m*/*z* = 356[M+H]⁺.

### Intermediate 143. 2-(2,6-dioxopiperidin-3-yl)-5-fluoro-6-(3-oxoazetidin-1-yl) isoindolin-1,3-dione

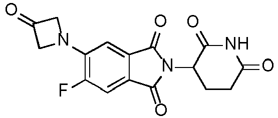

The synthesis method was the same as that for Intermediate 133, except that 4-fluorophthalic anhydride in synthesis step 1 was replaced with 4,5-difluorophthalic anhydride to obtain Intermediate 143. ESI-MS: *m*/*z* = 346[M+H]⁺.

### Intermediate 144. 1-(2-(2,6-dioxopiperidin-3-yl)-6-fluoro-1,3-dioxoisoindol-5-yl) pyrrolidin-3-carbaldehyde

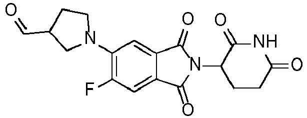

The synthesis method was the same as that for Intermediate 143, except that azetidin-3-one hydrochloride in synthesis step 2 was replaced with pyrrolidine-3-carbaldehyde. ESI-MS: *m*/*z* = 374[M+H]⁺.

### Intermediate 145. 3-(1-oxo-5-(3-(piperazin-1-yl)propan-1-yne-1-yl)isoindol-2-yl) piperidin-2,6-dione

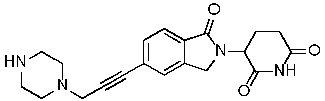

### Synthesis step 1. Methyl 4-bromo-2-bromomethyl benzoate (145-1)

Methyl 4-bromo-2-methyl-benzoate (2 g) was dissolved in carbon tetrachloride solution (25 mL), and N-bromosuccinimide (1.86 g) and dibenzoyl peroxide were added (437 mg), the mixture was refluxed and reacted for 5 h, and the reaction was monitored by TLC. After the reaction was completed, carbon tetrachloride was removed under reduced pressure, n-hexane was used for redissolution, filter-suction was performed, and the filter cake was washed with n-hexane three times, and then washed with a mixed solution of 1:4 (ethyl acetate:n-hexane). The organic phases were combined, the solvent was removed under reduced pressure, and purification was performed by column chromatography to obtain compound (145-1). ESI-MS:m/z= 307[M+H]⁺.

### Synthesis step 2. 3-(5-bromo-1-oxoisoindol-2-yl)piperidin-2,6-dione(145-2)

3-aminopiperidine-2,6-dione hydrochloride (2.41 g) was dissolved in acetonitrile (50 mL), triethylamine (3.24 mL) was added and the mixture was stirred for ten minutes, and then methyl 4-bromo-2-bromomethyl benzoate (2 g) was added dropwise, and the resulting mixture was refluxed and reacted for 5 h, and the reaction was monitored by TLC. After the reaction was completed, the solvent was removed under reduced pressure, 30 ml of methanol was added, refluxed for 30 min, and filtered with suction to obtain compound (145-2). ESI-MS:*m*/*z=* 323[M+H]⁺.

### Synthesis step 3. Tert-butyl 4-(propan-2-yn-1-yl)piperazin-1-carboxylate (145-3)

1-tert-butoxycarbonylpiperazine (5 g, 26.84 mmol) was dissolved in tetrahydrofuran (100 mL), potassium carbonate (5.57 g, 40.26 mmol) and 3-bromopropyne (3.19 g, 26.84 mmol) were added in ice bath and the mixture was reacted at room temperature for 3 h. After the reaction was completed, the reaction liquid was extracted with ethyl acetate and washed with saturated sodium chloride. The organic phases were combined, dried over anhydrous sodium sulfate. The solvent was removed under reduced pressure, and column chromatography was performed to obtain compound **(145-3),** ESI-MS: *m*/*z=* 225[M+H]⁺.

### Synthesis step 4. Tert-butyl 4-(3-(2-(2-2,6-dioxopiperidin-3-yl)-1-oxoisoindol-5-yl) propan-2-yn-1-yl)piperazin-1-carboxylate (145-4)

3-(5-bromo-1-oxoisoindol-2-yl)piperidine-2,6-dione (500 mg), copper iodide (59 mg), and triethylamine (430 ul) were added to a 25 mL double-neck flask, DMF (12mL) was added, and nitrogen replacement was performed. Tert-butyl 4-(prop-2-yn-1-yl)piperazine-1-carboxylate (347 mg) and Pd₂(pph₃)₂Cl₂ (109 mg) were added, then nitrogen replacement was performed, the mixture was reacted at 90°C overnight, and the reaction was monitored by TLC until the reaction was completed. A large amount of water was added, and the resulting reaction liquid was extracted with ethyl acetate, washed with saturated sodium chloride, and dried over anhydrous sodium sulfate, and purification was performed by column chromatography to obtain compound **(145-4).** ESI-MS: *m*/*z* = 467[M+H]⁺.

### Synthesis step 5. 3-(1-oxo-5-(3-(piperazin-1-yl)propan-1-yne-1-yl)isoindol-2-yl) piperidin-2,6-dione(145)

The synthesis method was the same as that in synthesis step 3 of Intermediate 63 to obtain Intermediate 145, ESI-MS:*m*/*z=* 367[M+H]⁺.

### Intermediate 146. 1-(2-(2,6-dioxopiperidin-3-yl)-3-oxoisoindol-5-yl)piperidin-4-carbaldehyde

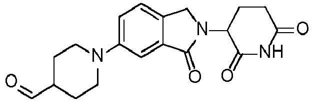

The synthesis method was the same as that for Intermediate 135, except that 4-(dimethoxymethyl)piperidine in synthesis step 1 was replaced with ethyl 4-piperidinecarboxylate, and 5-bromophthalide was replaced with 6-bromophthalide. The reaction conditions in synthesis step 6 were replaced to the following: the compound (3 mmol) was dissolved in dichloromethane (10 mL), diisobutylaluminum hydride (7.5 mmol) was added, and the mixture was stirred at room temperature overnight; and after the reaction was completed, the reaction liquid was extracted with ethyl acetate, washed with saturated sodium chloride, and dried with anhydrous sodium sulfate, and purification was performed by column chromatography to obtain Intermediate 146. ESI-MS: *m*/*z* = 356[M+H]⁺.

### Intermediate 147. 1-(2-(2,6-dioxopiperidin-3-yl)-6-fluoro-1,3-dioxoisooctanol-5-yl) piperidin-4-carboxylic acid

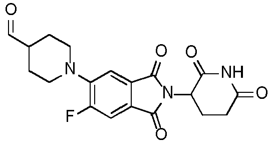

The synthesis method was the same as that for Intermediate 143, except that azetidin-3-one hydrochloride in synthesis step 2 was replaced with piperidine-4-aminocarbaldehyde. ESI-MS: *m*/*z* = 388[M+H]⁺.

### Intermediate 148. 1-(2-(2,6-dioxopiperidin-3-yl)-1-oxoisoindol-5-yl) piperidin-4-carbaldehyde

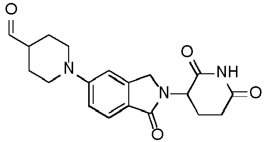

The synthesis method was the same as that for Intermediate 146, except that 6-bromophthalide was replaced with 5-bromophthalide. ESI-MS: *m*/*z* = 356 [M+H]⁺

### Intermediate 149. 1-(2-(2,6-dioxopiperidin-3-yl)-1-oxoisoindolin-5-yl) pyrrolidin-3-carbaldehyde

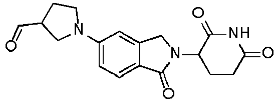

The synthesis method was the same as that for Intermediate 148, except that 4-(dimethoxymethyl)piperidine was replaced with 3-(dimethoxymethyl)pyrrolidine. ESI-MS: *m*/*z* = 342 [M+H]⁺.

### Referring to the synthetic route and method for Intermediate 133, the following intermediates were synthesized:

| Intermediate No. | Intermediate structure | LC-MS (ESI-MS) |
|---|---|---|
| | | *m*/*z* [M+H]⁺ |
| 150 | 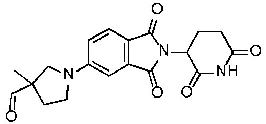 | 370.1 |
| 151 | 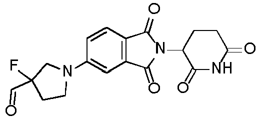 | 374.1 |
| 152 | 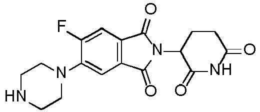 | 361.1 |
| 153 | 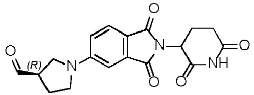 | 356.1 |
| 154 | 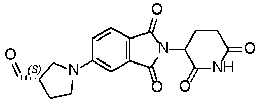 | 356.1 |
| 155 | 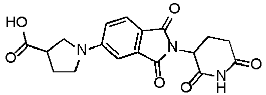 | 372.1 |

### Intermediate 156: Tert-butyl 4-(4-amino-2-fluorophenyl)piperazin-1-carboxylate

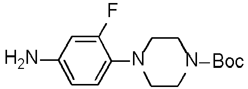

### Synthesis step 1: Tert-butyl 4-(2-fluoro-4-nitrophenyl)piperazin-1-carboxylate

3,4-difluoronitrobenzene (796 mg, 5.0 mmol) was dissolved in 20 mL of DMF, and triethylamine (1.01 g, 10.0 mmol) and tert-butyl piperazine-1-carboxylate (1.03 g, 5.5 mmol) were added to the system in sequence. The mixture was stirred, heated to 60°C and reacted for 3 h. TLC detected that the reaction was completed. Water was added. The system was cooled to room temperature, filtered, dried over anhydrous sodium sulfate, and concentrate under reduced pressure to obtain tert-butyl 4-(2-fluoro-4-nitrophenyl)piperazine-1-carboxylate (1.5 g, yield 92%). ESI-MS: *m*/*z* = 326.1 [M+H]⁺.

### Synthesis step 2: Tert-butyl 4-(4-amino-2-fluorophenyl)piperazin-1-carboxylate

Tert-butyl 4-(2-fluoro-4-nitrophenyl)piperazine-1-carboxylate (1.5 g, 4.6 mmol) was dissolved in 10 mL of ethanol, then 10 mL of water, ammonium chloride (394 mg, 7.36 mmol) and iron powder (219 mg, 3.9 mmol) were added to the system and the mixture was reacted at 50°C for 1 h. After the reaction was completed, the reaction liquid was filtered over Celite to remove iron powder and extracted three times with DCM. The organic phases were combined, dried over anhydrous sodium sulfate, concentrated under reduced pressure, and subjected to column chromatography to obtain tert-butyl 4-(4-amino-2-fluorophenyl)piperazine-1-carboxylate (1.23 g, yield 91%). ESI-MS: *m*/*z* = 296.2 [M+H]⁺.

### Referring to the synthetic route and method for Intermediate 156, the following intermediates were synthesized:

| Intermedia te No. | Intermediate structure | LC-MS (ESI-MS) |
|---|---|---|
| | | *m*/*z* [M+H]⁺ |
| 157 | 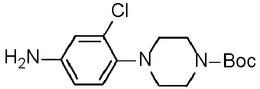 | 312.1 |
| 158 | 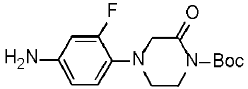 | 310.2 |
| 159 | 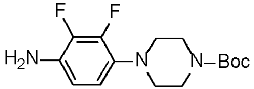 | 314.2 |
| 160 | 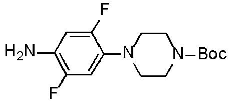 | 314.2 |
| 161 | 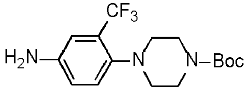 | 346.2 |

### Referring to the synthetic route and method for Intermediate 58, the following intermediates were synthesized:

| Intermediate No. | Intermediate structure | LC-MS (ESI-MS) |
|---|---|---|
| | | *m*/*z* [M+H]⁺ |
| 162 | 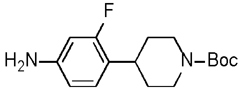 | 295.2 |
| 163 | 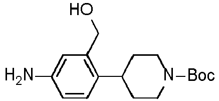 | 307.2 |
| 164 | 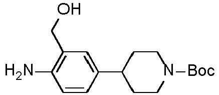 | 307.2 |

### Intermediate 165: (R)-tert-butyl 4-(4-((6-carbamoyl-3-(3-(3-methyl-2-oxoimidazolin -1-yl)piperidin-1-yl)-1,2,4-triazin-5-yl)amino)-2-fluorophenyl)piperazin-1-carboxylate

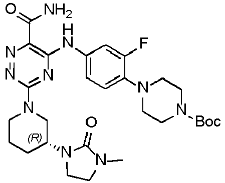

### Synthesis step 1: (R)-tert-butyl 3-(2-oxoimidazolidin-1-yl)piperidin-1-carboxylate

(R)-tert-butyl 3-aminopiperidine-1-carboxylate (2 g, 10 mmol) was dissolved in 20 mL of tetrahydrofuran, the system was cooled to 0°C, and 2-chloro ethyl isocyanate (1.17 g, 11 mmol) was slowly added and the mixture was reacted at 20°C for 1 h under stirring, then the system was cooled to 0°C, potassium tert-butoxide (1.68 g, 15 mmol) was slowly added, and the temperature was raised to room temperature for reaction for 2 h. TLC detected that the reaction was completed, water was added to quench the reaction, the system was adjusted to weak acidity, extract three times with water and DCM, and the organic phases were combined, dried over anhydrous sodium sulfate, concentrate under reduced pressure, and subjected to column chromatography to obtain tert-butyl (R)-3-(2-oxoimidazolidin-1-yl)piperidine-1-carboxylate (1.13 g, yield 42%). ESI-MS: *m*/*z* = 270.2 [M+H]⁺.

### Synthesis step 2: (R)-tert-butyl 3-(3-methyl-2-oxoimidazolidin-1-yl)piperidin-1-carboxylate

(R)-tert-butyl 3-(2-oxoimidazolidin-1-yl)piperidine-1-carboxylate (1.13 g, 4.2 mmol) was dissolved in 10 mL of tetrahydrofuran, and the system was cooled to 10°C. Potassium tert-butoxide (941 mg, 8.4 mmol) and dimethyl sulfate (635 mg, 5.04 mmol) were added in sequence. The mixture was reacted at room temperature for 3 h under stirring. After the reaction was completed, the reaction was quenched by adding water, and extracted three times with DCM, and the organic layers were combined, dried over anhydrous sodium sulfate, and concentrate under reduced pressure, and purification was performed by column chromatography to obtain (R)-tert-butyl 3-(3-methyl-2-oxoimidazolidine-1-yl)piperidine-1-carboxylate (1.13 g, yield 95%). ESI-MS: *m*/*z* = 284.2 [M+H]⁺.

### Synthesis step 3: (R)-1-methyl-3-(piperidin-3-yl)imidazolin-2-one

(R)-tert-butyl 3-(3-methyl-2-oxoimidazolidin-1-yl)piperidine-1-carboxylate (1.13 g, 4.0 mmol) was dissolved in 8 mL of DCM, and 2 mL of TFA was added and the mixture was reacted at room temperature for 1 h. After the reaction was completed, the reaction liquid was extracted three times with DCM, dried over anhydrous sodium sulfate, and concentrate under reduced pressure to obtain crude product (R)-1-methyl-3-(piperidin-3-yl)imidazolin-2-one, which was directly used in the next reaction step. ESI-MS: *m*/*z* = 184.1 [M+H]⁺.

### Synthesis step 4: Tert-butyl 4-(4-((6-carbamoyl-3-(methylthio)-1,2,4-triazin-5-yl) amino)-2-fluorophenyl)piperazin-1-carboxylate

Ethyl 5-chloro-3-(methylthio)-1,2,4-triazine-6-carboxylate (700 mg, 3.00 mmol) was dissolved in 10 mL of acetonitrile, and DIPEA (1.7 g , 12.84 mmol) and tert-butyl 4-(4-amino-2-fluorophenyl)piperazine-1-carboxylate (885 mg, 3.00 mmol) were added to the system. After reacting at room temperature for 30 min, ammonia-methanol solution (7 moL/L, 30 mL) was added, the system became turbid within 10 min, and the reaction was continued for 2 h at room temperature. After the reaction was completed, the reaction liquid was concentrated under reduced pressure, slurried with petroleum ether, and filtered to obtain tert-butyl 4-(4-((6-carbamoyl-3-(methylthio)-1,2,4-triazine-5-yl)amino)-2-fluorophenyl)piperazine-1-carbo xylate as a yellow solid (1.3 g, yield 93%). ESI-MS: *m*/*z* = 464 [M+H]⁺.

### Synthesis step 5: (R)-tert-butyl 4-(4-((6-carbamoyl-3-(3-(3-methyl-2-oxoimidazolin -1-yl)piperidin-1-yl)-1,2,4-triazin-5-yl)amino)-2-fluorophenyl)piperazin-1-carboxylate

(R)-1-methyl-3-(piperidin-3-yl)imidazolidin-2-one (1.3 g, 2.78 mmol) was dissolved in 20 mL of NMP, and mCPBA (85%, 2.4 g, 13.99 mmol) was added, and the mixture was stirred at room temperature for 2 h. After the reaction was completed, DIPEA (2 mL, 11.00 mmol) and (R)-1-methyl-3-(piperidin-3-yl)imidazolin-2-one (661 mg, 3.61 mmol) were added to the system in sequence. The mixture was reacted at 80°C for 2 h. After the reaction was completed, a solid was precipitated after adding water, and filtration was performed to obtain a yellow solid. The yellow solid was dissolved by adding DCM, dried over anhydrous sodium sulfate, concentrated under reduced pressure, and subjected to column chromatography (eluted with DCM/MeOH) to obtain (R)-tert-butyl 4-(4-((6-carbamoyl-3-(3-(3-methyl-2-oxoimidazolin -1-yl)piperidin-1-yl)-1,2,4-triazin-5-yl)amino)-2-fluorophenyl)piperazine-1-carboxylate as a yellow solid (300 mg, yield 16%). ESI-MS: *m*/*z* = 599.3 [M+H]⁺.

### Referring to the synthetic route and method for Intermediate 165, the following intermediates were synthesized:

| Intermediate No. | Intermediate structure | LC-MS (ESI-MS) |
|---|---|---|
| | | *m*/*z* [M+H]⁺ |
| 166 | 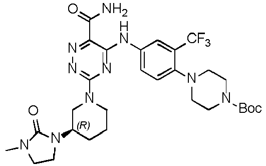 | 649.3 |
| 167 | 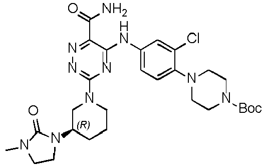 | 615.3 |
| 168 | 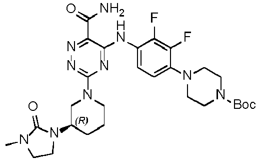 | 617.3 |
| 169 | 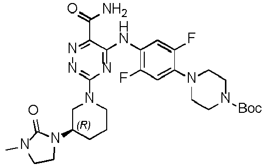 | 617.3 |
| 170 | 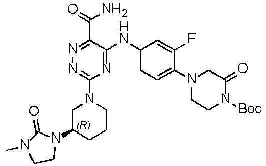 | 613.3 |
| 171 | 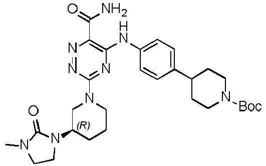 | 580.3 |
| 172 | 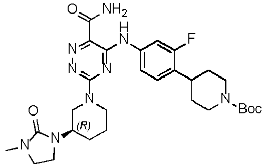 | 598.3 |
| 173 | 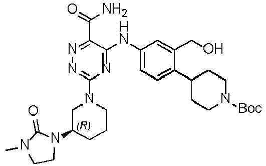 | 610.3 |
| 174 | 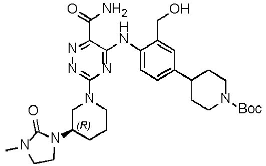 | 610.3 |
| 175 | 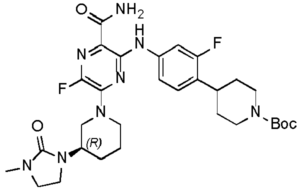 | 615.3 |
| 176 | 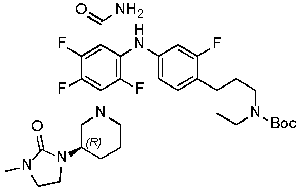 | 649.3 |
| 177 | 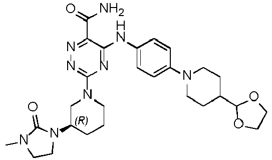 | 552.3 |
| 178 | 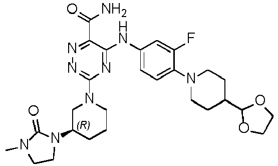 | 570.3 |
| 179 | 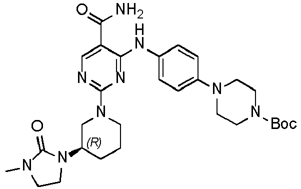 | 580.3 |
| 180 | 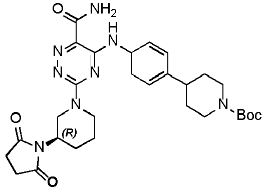 | 579.3 |
| 181 | 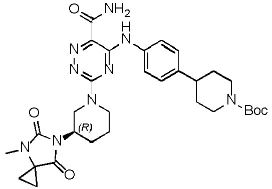 | 620.3 |
| 182 | 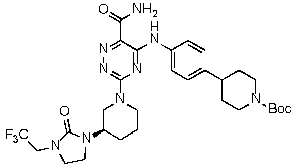 | 648.3 |
| 183 | 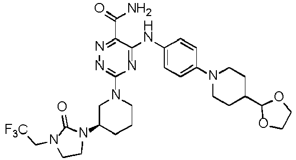 | 620.3 |
| 184 | 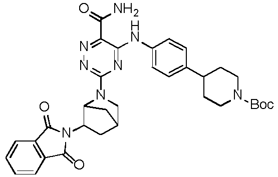 | 639.3 |

### Intermediate 185: 1-(3-(2,4-dioxotetrahydropyrimidin-1(2H)-yl)-4-fluorophenyl) pyrrolidin-3-carbaldehyde

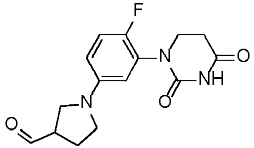

### Synthesis step 1: 3-((5-bromo-2-fluorophenyl)amino)propanoic acid

5-bromo-2-fluoroaniline (2 g, 10.58 mmol) was dissolved in 20 mL of toluene, acrylic acid (787 mg, 12.69 mmol) was added to the system, and the mixture was reacted at 100°C for 12 h. After the reaction was completed, the reaction liquid was extracted with DCM three times, and the organic phases were combined, dried over anhydrous sodium sulfate, and concentrated under reduced pressure to obtain 3-((5-bromo-2-fluorophenyl)amino)propionic acid (2.5 g, yield 90%). ESI-MS(M+H)⁺=262.0.

### Synthesis step 2: 1-(5-bromo-2-fluorophenyl)dihydropyrimidin-2,4(1H, 3H)-dione

3-((5-bromo-2-fluorophenyl)amino)propionic acid (2.5 g, 9.54 mmol) was dissolved in 30 mL of acetic acid, and urea (1.2 g, 19.08 mmol) was added to the system, and the mixture was reacted at 120°C for 12 h. After the reaction was completed, the reaction liquid was extracted with DCM three times, and the organic phases were combined, dried over anhydrous sodium sulfate, concentrated under reduced pressure and subjected to column chromatography (eluted with DCM/MeOH) to obtain 1-(5-bromo-2-fluorophenyl)dihydropyrimidin-2,4(1H,3H)-dione as a brown solid (2 g, yield 73%). ESI-MS(M+H)⁺=287.0.

### Synthesis step 3: 1-(5-(3-(1,3-dioxolan-2-yl)pyrrolidin-1-yl)-2-fluorophenyl) dihydropyrimidin-2,4(1H, 3H)-dione

1-(5-bromo-2-fluorophenyl)dihydropyrimidin-2,4(1H,3H)-dione (500 mg, 1.74 mmol) was dissolved in 10 mL of DMSO, and 3-(1,3-dioxolan-2-yl)pyrrolidine (325 mg, 2.27 mmol), t-BuONa (511 mg, 5.22 mmol), Pd₂(dba)₃ (155 mg, 0.17 mmol), and X-Phos (201 mg, 0.34 mmol) were added and the mixture was reacted at 80°C for 3h. After the reaction was completed, the reaction liquid was quenched by adding water, and extracted with DCM three times. The organic phases were combined, dried over anhydrous sodium sulfate, concentrated under reduced pressure, and subjected to column chromatography (eluted with DCM/MeOH) to obtain 1-(5-(3-(1,3-dioxolan-2-yl)pyrrolidin-1-yl)-2-fluorophenyl)dihydropyrimidin-2,4(1H,3H)-dione as a brown solid (100 mg, yield 16%). ESI-MS(M+H)⁺=350.1.

### Synthesis step 4: 1-(3-(2,4-dioxotetrahydropyrimidin-1(2H)-yl)-4-fluorophenyl) pyrrolidin-3-carbaldehyde

1-(5-(3-(1,3-dioxolan-2-yl)pyrrolidin-1-yl)-2-fluorophenyl)dihydropyrimidin-2,4(1H,3H) -dione (100 mg, 0.28 mmol) was dissolved in 2 mL of TFA and the mixture was reacted at 80°C for 1 h, then 0.1 mL of concentrated hydrochloric acid was added to the system, and the mixture was reacted at 80°C for 3 h. After the reaction was completed, the reaction liquid was adjusted to alkalinity by adding saturated aqueous sodium carbonate solution, extracted with DCM five times, and the organic phases were combined, dried over anhydrous sodium sulfate, concentrate under reduced pressure, and subjected to column chromatography to obtain 1-(3-(2,4-dioxotetrahydropyrimidin-1(2H)-yl)-4-fluorophenyl)pyrrolidin-3-carbaldehyde (80 mg, yield 93%). ESI-MS(M+H)⁺=306.1.

### Intermediate 186: 1-(3-(2,4-dioxotetrahydropyrimidin-1(2H)-yl)-4-methoxyphenyl) pyrrolidin -3-carbaldehyde

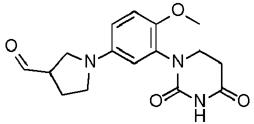

Referring to the synthetic route and method for Intermediate 185, and 5-bromo-2-fluoroaniline in synthesis step 1 was replaced with 5-bromo-2-methoxyaniline, ESI-MS(M+H)⁺=318.1.

### Intermediate 187: 3-(2,4-dioxotetrahydropyrimidin-1(2H)-yl)-4-methylbenzoic acid

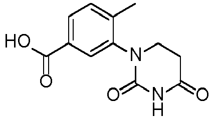

### Synthesis step 1: 3-((2-carboxyethyl)amino)-4-methylbenzoic acid

3-amino-4-methylbenzoic acid (10.0 g, 66.2 mmol) was dissolved in 30 mL of toluene, and acrylic acid (18 mL, 264.6 mmol) was added to the system and the mixture was reacted at 110°C for 4 h. After the reaction was completed, DCM was added and a solid was precipitated. Filtration was performed under reduced pressure to obtain 3-((2-carboxyethyl) amino)-4-methylbenzoic acid as a white solid (14.5 g, yield 98%). ESI-MS: *m*/*z* = 224 [M+H]⁺.

### Synthesis step 2: 3-(2,4-dioxotetrahydropyrimidin-1(2H)-yl)-4-methylbenzoic acid

3-((2-carboxyethyl)amino)-4-methylbenzoic acid (14.5 g, 65.0 mmol) was dissolved in 30 mL of acetic acid, urea (9.8 g, 162.5 mmol) was added to the system, and the mixture was reacted at 120°C for 12 h. After the reaction was completed, water was added, and a solid was precipitated in the system. The obtained system was filtered, slurried with water, then slurried with acetonitrile, and filtered to obtain 3-(2,4-dioxotetrahydropyrimidin-1(2H)-yl) -4-methylbenzoic acid as a white solid (11.5 g, yield 71%). ESI-MS: *m*/*z* = 249 [M+H]⁺.

### Referring to the synthetic route and method for Intermediate 187, the following intermediates were synthesized:

| Intermediate No. | Intermediate structure | LC-MS (ESI-MS) |
|---|---|---|
| | | *m*/*z* [M+H]⁺ |
| 188 | 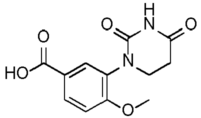 | 265.1 |
| 189 | 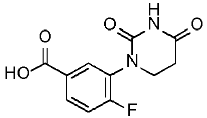 | 253.1 |

### Intermediate 190: 1-(4-(2,4-dioxotetrahydropyrimidin-1(2H)-yl)-3-fluorophenyl) piperidin-4-carbaldehyde

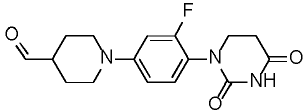

### Synthesis step 1: 5-(4-(1,3-dioxolan-2-yl)piperidin-1-yl)-2-nitroaniline

5-fluoro-2-nitroaniline (1 g, 6.41 mmol) was dissolved in 10 mL of DMF, and 4-(1,3-dioxolan-2-yl)piperidine (1.2 g, 7.69 mmol) and cesium carbonate (6.2 g, 19.23 mmol) were added to the system in sequence. The mixture was reacted at 80°C for 3 h. After the reaction was completed, the reaction liquid was quenched by adding water, and extracted with DCM three times. The organic phases were combined, dried over anhydrous sodium sulfate, concentrated under reduced pressure, and subjected to column chromatography to obtain 5-(4-(1,3-dioxolan-2-yl)piperdin-1-yl)-2-nitroaniline (1.5 g, yield 79%), ESI-MS (M+H)⁺=294.1.

### Synthesis step 2: 4-(1,3-dioxolan-2-yl)-1-(3-fluoro-4-nitrophenyl)piperidine

5-(4-(1,3-dioxolan-2-yl)piperidin-1-yl)-2-nitroaniline (1.5 g, 5.11 mmol) was dissolved in 15 mL of concentrated hydrochloric acid. HF (70%, 30 mL) was added to the system at 0°C, and the mixture was reacted at room temperature until the reactants were completely dissolved. NaNO₂ (423 mg, 6.13 mmol) was added to the system at -78°C, and the mixture was reacted at 0°C for 30 min. Then the temperature was raised to 60°C for reaction for 15-30 min. After the reaction was completed, the reaction liquid was quenched by adding water, and extracted with DCM three times. The organic phases were combined, dried over anhydrous sodium sulfate, concentrated under reduced pressure, and subjected to column chromatography to obtain 4-(1,3-dioxolan-2-yl)-1-(3-fluoro-4-nitrophenyl)piperidine (1 g, yield 66%). ESI-MS(M+H)⁺ =297.1.

### Synthesis step 3: 4-(4-(1,3-dioxolan-2-yl)piperidin-1-yl)-2-fluoroaniline

4-(1,3-dioxolan-2-yl)-1-(3-fluoro-4-nitrophenyl)piperidine (1 g, 3.37 mmol) was dissolved in 10 mL of tetrahydrofuran, and ammonium chloride (2 g, 33.78 mmol), water (10 mL), and iron powder (1.9 g, 33.78 mmol) were added to the system in sequence. The mixture was reacted at 70°C for 12 h. After the reaction was completed, the iron powder was removed by filtration with diatomaceous earth. The filtrate was extracted with DCM three times. The organic phases were combined, dried over anhydrous sodium sulfate, concentrated under reduced pressure, and subjected to purification by column chromatography (eluted with EA/PE) to obtain 4-(4-(1,3-dioxolan-2-yl)piperidin-1-yl)-2-fluoroaniline (750 mg, yield 83%). ESI-MS(M+H)⁺ =267.1.

### Synthesis step 4: 3-(4-(4-(1,3-dioxolan-2-yl)piperidin-1-yl)-2-fluorophenyl) amino) propionic acid

4-(4-(1,3-dioxolan-2-yl)piperidin-1-yl)-2-fluoroaniline (750 mg, 2.81 mmol) was dissolved in 10 mL of toluene, acrylic acid (243 mg, 3.38 mmol) was added to the system, and the mixture was reacted at 100°C for 12 h, and the reaction was completed. The reaction liquid was extracted five times with DCM, the organic phases were combined, dried over anhydrous sodium sulfate, concentrated under reduced pressure, and subjected to column chromatography to obtain 3-(4-(4-(1,3-dioxolan-2-yl)piperidin-1-yl)-2-fluorophenyl)amino) propionic acid as a yellow liquid (860 mg, yield 90%). ESI-MS(M+H)⁺=339.2.

### Synthesis step 5: 1-(4-(4-(1,3-dioxolan-2-yl)piperidin-1-yl)-2-fluorophenyl) dihydropyrimidin-2,4(1H, 3H)-dione

3-(4-(4-(1,3-dioxolan-2-yl)piperidin-1-yl)-2-fluorophenyl)amino)propionic acid (860 mg, 2.54 mmol) was dissolved in 10 mL of acetic acid, and urea (315 mg, 5.08 mmol) was added to the system, and the mixture was reacted at 120°C for 12 h. After the reaction was completed, the reaction liquid was extracted with DCM three times. The organic phases were combined, dried over anhydrous sodium sulfate, concentrated under reduced pressure, and subjected to column chromatography (eluted with DCM/MeOH) to obtain 1-(4-(4-(1,3-dioxolan-2-yl)piperidin-1-yl) -2-fluorophenyl)dihydropyrimidin-2,4(1H,3H)-dione as a brown solid (540 mg, yield 58%). ESI-MS(M+H)⁺=364.2.

### Synthesis step 6: 1-(4-(2,4-dioxotetrahydropyrimidin-1(2H)-yl)-3-fluorophenyl) piperidin-4-carbaldehyde

1-(4-(4-(1,3-dioxolan-2-yl)piperidin-1-yl)-2-fluorophenyl)dihydropyrimidin-2,4(1H,3H)-dione (540 mg, 1.68 mmol) was dissolved in 6 mL of TFA and the mixture was reacted at 80°C for 1 h, then 0.5 mL of concentrated hydrochloric acid was added to the system, and the mixture was reacted at 80°C for 3 h. After the reaction was completed, the reaction liquid was adjusted to alkalinity by adding saturated aqueous sodium carbonate solution, extracted with MeOH/DCM five times, and the organic phases were combined, dried over anhydrous sodium sulfate, concentrate under reduced pressure, and subjected to column chromatography to obtain 1-(4-(2,4-dioxotetrahydropyrimidin-1(2H)-yl)-3-fluorophenyl)piperidin-4-carbaldehyde as a yellow solid (490 mg, yield 91%). ESI-MS(M+H)⁺=320.1.

### Referring to the synthetic route and method for Intermediate 190, the following intermediates were synthesized:

| Intermediate No. | Intermediate structure | LC-MS (ESI-MS) *m*/*z* [M+H]⁺ |
|---|---|---|
| 191 | 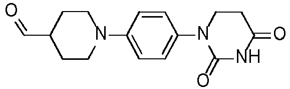 | 302.1 |
| 192 | 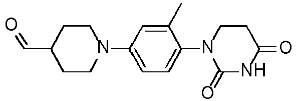 | 316.2 |
| 193 | 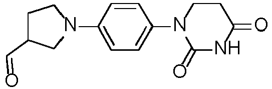 | 288.1 |
| 194 | 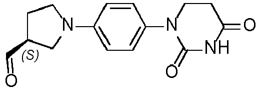 | 288.1 |
| 195 | 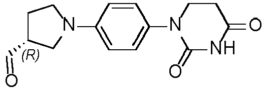 | 288.1 |
| 196 | 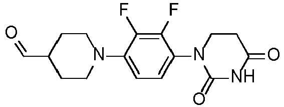 | 338.1 |
| 197 | 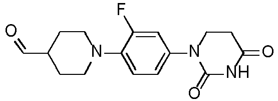 | 320.1 |
| 198 | 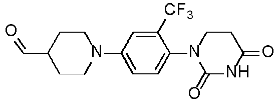 | 370.1 |
| 199 | 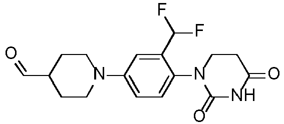 | 352.1 |
| 200 | 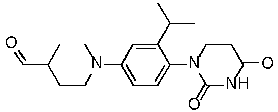 | 344.2 |
| 201 | 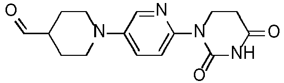 | 303.1 |
| 202 | 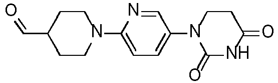 | 303.1 |
| 203 | 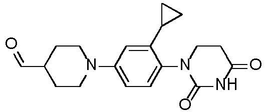 | 342.2 |
| 204 | 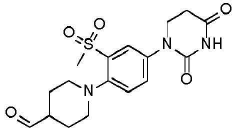 | 380.1 |
| 205 | 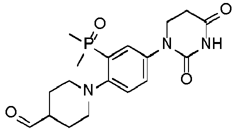 | 378.2 |
| 206 | 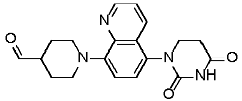 | 353.2 |
| 207 | 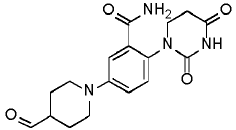 | 345.2 |
| 208 | 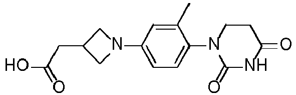 | 318.1 |
| 272 | 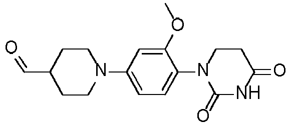 | 332.2 |

### Intermediate 209: (S)-N-(2,6-dioxopiperidin-3-yl)-2-fluoro-4-(4-formylpiperidin-1-yl) benzamide

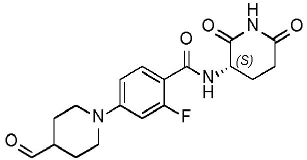

### Synthesis step 1: Methyl 4-(4-(1,3-dioxolan-2-yl)piperidin-1-yl)-2-fluoro benzoate

Methyl 4-bromo-2-fluorobenzoate (2.5 g, 10.73 mmol) was dissolved in 20 mL of DMF, and 4-(1,3-dioxolan-2-yl)piperidine (2.0 g, 12.87 mmol), cesium carbonate (8.7 g, 26.82 mmol), Sphos (880 mg, 2.15 mmol), and palladium acetate (240 mg, 1.07 mmol) were added, and the mixture was reacted at 50°C for 2 h under introduced nitrogen protection. After the reaction was completed, the reaction liquid was quenched by adding water, and extracted with DCM three times. The organic phases were combined, dried over anhydrous sodium sulfate, concentrated under reduced pressure, and subjected to column chromatography to obtain methyl 4-(4-(1,3-dioxacyclo-2-yl)piperidin-1-yl)-2-fluorobenzoate as a yellow solid (800 mg, yield 24%). ESI-MS(M+H)⁺=310.1.

### Synthesis step 2: 4-(4-(1,3-dioxolan-2-yl)piperidin-1-yl)-2-fluorobenzoic acid

Methyl 4-(4-(1,3-dioxolan-2-yl)piperidin-1-yl)-2-fluorobenzoate (240 mg, 0.78 mmol) was dissolved in 10 mL of tetrahydrofuran. Lithium hydroxide (90 mg, 3.88 mmol), water (2 mL), and methanol (4 mL) were added to the system in sequence. The mixture was reacted at room temperature for 1 h. After the reaction was completed, the reaction liquid was concentrated under reduced pressure, extracted with DCM, slurried, filtered, and concentrated under reduced pressure to obtain 4-(4-(1,3-dioxolan-2-yl)piperidin-1-yl)-2-fluorobenzoic acid as a white solid (190 mg, yield 82%). ESI-MS(M-H)⁺=296.1.

### Synthesis step 3: (S)-4-(4-(1,3-dioxolan-2-yl)piperidin-1-yl)-N-(2,6-dioxopiperidin-3-yl)-2-fluorobenzamide

(S)-tert-butyl (2,6-dioxopiperidin-3-yl)carbamate (93 mg, 0.73 mmol) was dissolved in 5 mL of DCM, and TFA (1 mL) was added to the system. The mixture was reacted at room temperature for 1 h. After the reaction was completed, the reaction liquid was concentrated under reduced pressure. 5 mL of DMF, 4-(4-(1,3-dioxolan-2-yl)piperidin-1-yl)-2-fluorobenzoic acid (180 mg, 0.61 mmol), HATU (30 mg, 0.79 mmol) and TEA (0.5 mL, 1.22 mol) were added in sequence to the above product. The mixture was reacted at room temperature for 1 h and the reaction was completed. The reaction liquid was extracted three times with DCM, the organic phases were combined, dried over anhydrous sodium sulfate, concentrated under reduced pressure, and subjected to column chromatography (eluted with DCM/MeOH) to obtain (S)-4-(4-(1,3-dioxolan-2-yl)piperidin-1-yl)-N-(2,6-dioxopiperidin-3-yl)-2-fluorobenzamide as a white solid (200 mg, yield 80%). ESI-MS(M+H)⁺=406.2.

### Synthesis step 4: (S)-N-(2,6-dioxopiperidin-3-yl)-2-fluoro-4-(4-formylpiperidin-1-yl) benzamide

(S)-4-(4-(1,3-dioxolan-2-yl)piperidin-1-yl)-N-(2,6-dioxopiperidin-3-yl)-2-fluorobenzami de (200 mg, 0.49 mmol) was dissolved in 2 mL of TFA and the mixture was reacted at 80°C for 1 h. Then 1 mL of concentrated hydrochloric acid was added to the system and the mixture was reacted at 80°C for 3 h. After the reaction was completed, the system was adjusted to be alkaline by adding saturated aqueous sodium carbonate solution, extracted with DCM five times, and the organic phases were combined, dried over anhydrous sodium sulfate, concentrate under reduced pressure, and subjected to column chromatography to obtain (S)-N-(2,6-dioxopiperidin-3-yl) -2-fluoro-4-(4-formylpiperidin-1-yl)benzamide as a white solid (146 mg, yield 83%). ESI-MS(M+H)⁺ =362.1.

### Intermediate 210: N-(2,6-dioxopiperidin-3-yl)-5-(4-formylpiperidin-1-yl)pyridine carboxamide

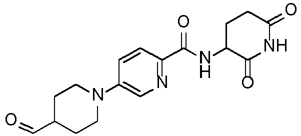

### Synthesis step 1: Methyl 5-(4-(1, 3-dioxolan-2-yl)piperidin-1-yl)picolinate

Methyl 5-fluoropicolinate (2.2 g, 14.18 mmol) was dissolved in 20 mL of DMF, and 4-(1,3-dioxolan-2-yl)piperidine (2.7 g, 17.02 mmol) and potassium carbonate (5.8 g, 42.54 mmol) was added to the system and the mixture was reacted at 90°C for 2 h. After the reaction was completed, the reaction liquid was quenched by adding water, and extracted with DCM three times. The organic phases were combined, dried over anhydrous sodium sulfate, concentrated under reduced pressure, and subjected to column chromatography to obtain methyl 5-(4-(1,3-dioxolan-2-yl)piperidin-1-yl)picolinate as a yellow solid (3.2 g, yield 78%). ESI-MS(M+H)⁺=293.1.

### Synthesis step 2: 5-(4-(1, 3-dioxolan-2-yl)piperidin-1-yl)picolinic acid

methyl 5-(4-(1,3-dioxolan-2-yl)piperidin-1-yl)picolinate (1.5 g, 5.13 mmol) was dissolved in 20 mL of tetrahydrofuran, and lithium hydroxide (615 mg, 25.67 mmol) and water (7.5 mL) were added to the system. The mixture was reacted at room temperature for 2 h. After the reaction was completed, the reaction liquid was concentrated to no solvent, slurried with DCM, and filtered, and the filtrate was concentrated to obtain 5-(4-(1,3-dioxolan-2-yl) piperidin-1-yl)picolinic acid as a white solid (1.3 g, yield 93%). ESI-MS(M+H)⁺=279.1.

### Synthesis step 3: 5-(4-(1,3-dioxolan-2-yl)piperidin-1-yl)-N-(2,6-dioxopiperidin -3-yl)pyridine carboxamide

5-(4-(1,3-dioxolan-2-yl)piperidin-1-yl)picolinic acid (500 mg, 1.08 mmol) was dissolved in 10 mL of DMF, and 3-aminopiperidin-2,6-dione (255 mg, 1.97 mmol), HATU (752 mg, 1.97 mmol) and triethylamine (0.75 mL, 5.39 mmol) were added to the system in sequence. The mixture was reacted at room temperature for 1 h. After the reaction was completed, the reaction liquid was extracted with DCM three times, and the organic phases were combined, dried over anhydrous sodium sulfate, concentrated under reduced pressure, and subjected to column chromatography (eluted with DCM/MeOH) to obtain 5-(4-(1,3-dioxolan-2-yl)piperidin-1-yl)-N-(2,6-dioxopiperidin-3-yl)pyridinecarboxamide as a gray solid (350 g, yield 51%). ESI-MS(M+H)⁺=389.2.

### Synthesis step 4: N-(2,6-dioxopiperidin-3-yl)-5-(4-formylpiperidin-1-yl)pyridine carboxamide

5-(4-(1,3-dioxolan-2-yl)piperidin-1-yl)-N-(2,6-dioxopiperidin-3-yl)pyridinecarboxamide (100 mg, 0.26 mmol) was dissolved in 2 mL of TFA and the mixture was reacted at 80°C for 1 h. Then 1 mL of concentrated hydrochloric acid was added to the system and the mixture was reacted at 80°C for 3 h. After the reaction was completed, the system was adjusted to be alkaline by adding saturated aqueous sodium carbonate solution, extracted with DCM five times, and the organic phases were combined, dried over anhydrous sodium sulfate, and concentrate under reduced pressure to obtain N-(2,6-dioxopiperidin-3-yl)-5-(4-formylpiperidin-1-yl) pyridinecarboxamide as a yellow solid (75 mg, yield 84%). ESI-MS(M+H)⁺=345.2.

### Referring to the synthetic route and method for Intermediates 209 and 210, the following intermediates were synthesized:

| Intermediate No. | Intermediate structure | LC-MS (ESI-MS) *m*/*z* [M+H]⁺ |
|---|---|---|
| 211 | 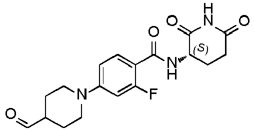 | 362.1 |
| 212 | 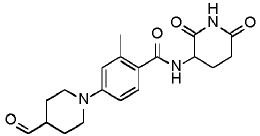 | 358.2 |
| 213 | 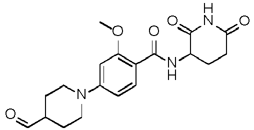 | 374.2 |
| 214 | 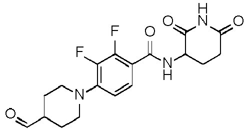 | 380.1 |
| 215 | 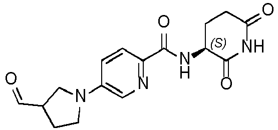 | 331.1 |
| 216 | 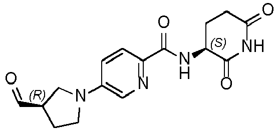 | 331.1 |
| 217 | 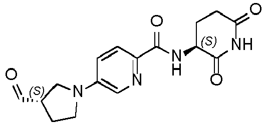 | 331.1 |
| 218 | 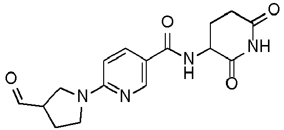 | 331.1 |
| 219 | 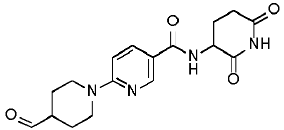 | 345.2 |
| 220 | 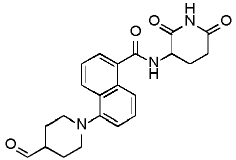 | 394.2 |
| 221 | 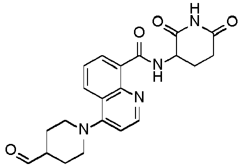 | 395.2 |

### Intermediate 222: 1-(6-((2,6-dioxopiperidin-3-yl)amino)pyridin-3-yl) pyrrolidin-3-carbaldehyde

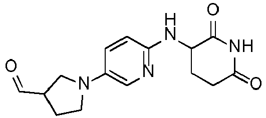

### Synthesis step 1: Synthesis of 5-(3-(1,3-dioxolan-2-yl)pyrrolidin-1-yl)-2-nitropyridine

5-fluoro-2-nitropyridine (1.5 g, 10.56 mmol) was dissolved in 20 mL of DMF, and 3-(1,3-dioxolan-2-yl)pyrrolidine (1.8 g, 12.67 mmol) and cesium carbonate (10 g, 31.68 mmol) were added to the system and the mixture was reacted at 80°C for 2 h. After the reaction was completed, the reaction liquid was quenched by adding water, and extracted with DCM three times. The organic phases were combined, dried over anhydrous sodium sulfate, concentrated under reduced pressure, and subjected to column chromatography to obtain 5-(3-(1,3-dioxolan-2-yl)pyrrolidin-1-yl)-2-nitropyridine as a yellow liquid (2.2 g, yield 75%). ESI-MS(M+H)⁺ =266.1.

### Synthesis step 2: 5-(3-(1,3-dioxolan-2-yl)pyrrolidin-1-yl)pyridin-2-amine

5-(3-(1,3-dioxolan-2-yl)pyrrolidin-1-yl)-2-nitropyridine (2.1 g, 7.92 mmol) was dissolved in 25 mL of tetrahydrofuran, and ammonium chloride (4.6 g, 79.24 mmol), water (5 mL), and iron powder (4.6 g, 79.24 mmol) were added to the system in sequence. The mixture was reacted at 70°C for 12 h. After the reaction was completed, a large amount of iron powder was filtered out while hot, and the resulting reaction liquid was extracted with DCM three times. The organic phases were combined, dried over anhydrous sodium sulfate, concentrated under reduced pressure, and subjected to column chromatography to obtain 5-(3-(1,3-dioxolan-2-yl)pyrrolidin-1-yl)pyridine-2-amine as a yellow solid (1.7 g, yield 93%). ESI-MS(M+H)⁺ =236.1.

### Synthesis step 3: 3-(5-(3-(1,3-dioxolan-2-yl)pyrrolidin-1-yl)pyridin-2-yl)amino) piperidin-2,6-dione

5-(3-(1,3-dioxolan-2-yl)pyrrolidin-1-yl)pyridin-2-amine (1.7 g, 7.23 mmol) was dissolved in 20 mL of DMF, and 3-bromopiperidin-2,6-dione (2 g, 10.84 mmol), Pd(dppf)Cl₂(526 mg, 0.72 mmol) and Na₂CO₃(2.6 g, 21.69 mmol) were added to the system in sequence. The mixture was reacted at 100°C for 3 h. After the reaction was completed, the reaction liquid was quenched by adding water, and extracted with DCM three times. The organic phases were combined, dried over anhydrous sodium sulfate, concentrated under reduced pressure, and subjected to column chromatography to obtain 3-(5-(3-(1,3-dioxolan-2-yl)pyrrolidin-1-yl)pyridin-2-yl)amino)piperidin-2,6-dione (2 g, yield 80%). ESI-MS(M+H)⁺=347.2.

### Synthesis step 4: 1-(6-((2,6-dioxopiperidin-3-yl)amino)pyridin-3-yl)pyrrolidin-3-carbaldehyde

3-(5-(3-(1,3-dioxolan-2-yl)pyrrolidin-1-yl)pyridin-2-yl)amino)piperidin-2,6-dione (2 g , 5.78 mmol) was dissolved in 10 mL of TFA and the mixture was reacted at 80°C for 1 h. Then 2 mL of concentrated hydrochloric acid was added to the system and the mixture was reacted at 80°C for 3 h. After the reaction was completed, the reaction liquid was adjusted to alkalinity by adding saturated aqueous sodium carbonate solution, extracted with DCM five times, and the organic phases were combined, dried over anhydrous sodium sulfate, and concentrate under reduced pressure to obtain 1-(6-((2,6-dioxopiperidin-3-yl)amino)pyridin-3-yl)pyrrolidin-3-carbaldehyde as a yellow solid (1.5 g, yield 85%). ESI-MS(M+H)⁺=303.1.

### Referring to the synthetic route and method for Intermediate 222, the following intermediates were synthesized:

| Intermediate No. | Intermediate structure | LC-MS (ESI-MS) *m*/*z* [M+H]⁺ |
|---|---|---|
| 223 | 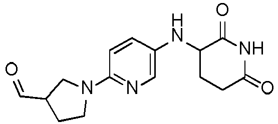 | 303.1 |
| 224 | 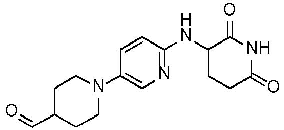 | 317.2 |
| 225 | 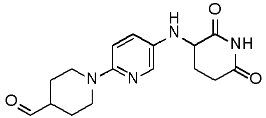 | 317.2 |
| 226 | 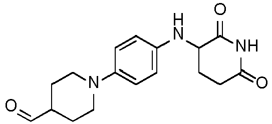 | 316.2 |
| 227 | 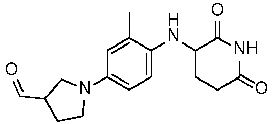 | 316.2 |
| 228 | 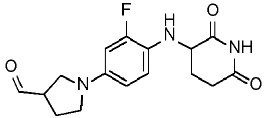 | 320.1 |
| 229 | 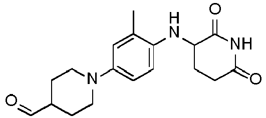 | 330.2 |
| 230 | 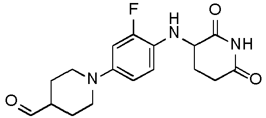 | 334.2 |
| 231 | 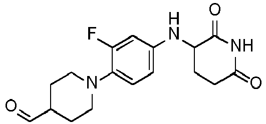 | 334.2 |
| 232 | 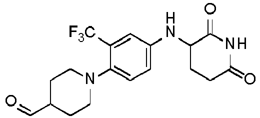 | 384.2 |

### Intermediate 233: methyl (1-(7-((2,6-dioxopiperidin-3-yl)carbamoyl)benzo[d][1,3] dioxolan-4-yl)piperidin-4-yl)4-methylbenzenesulfonate

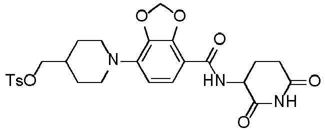

### Synthesis step 1: 7-aminobenzo[d][1,3]dioxolan-4-carboxylic acid

7-nitrobenzo[d][1,3]dioxolan-4-carboxylic acid (1 g, 5.52 mmol) was dissolved in 10 mL of THF, and ammonium chloride (2.9 g, 55.20 mmol), water (3 mL) and iron powder (3 g, 55.20 mmol) were added to the system and the mixture was reacted at 70°C for 12 h. After the reaction was completed, the reaction liquid was quenched by adding water, and extracted with DCM three times. The organic phases were combined, dried over anhydrous sodium sulfate, concentrated under reduced pressure, and subjected to column chromatography (eluted with EA/PE) to obtain 7-aminobenzo[d][1,3]dioxolan-4-carboxylic acid (729 mg, yield 85%). ESI-MS(M+H)⁺=182.0.

### Synthesis step 2: Synthesis of 7-fluorobenzo[d][1,3]dioxolan-4-carboxylic acid

7-aminobenzo[d][1,3]dioxolan-4-carboxylic acid (729 mg, 4.02 mmol) was dissolved in 10 mL of concentrated hydrochloric acid, and the mixture was cooled to 0°C. HF (70%, 20 mL) was added to the system at 0°C, and the resulting mixture was reacted at room temperature until the reactants were completely dissolved. NaNO₂ (305 mg, 4.42 mmol) was added to the system at -78°C, and the mixture was reacted at 0°C for 30 min. Then the temperature was raised to 60°C for further reaction for 15-30 min. After the reaction was completed, the reaction liquid was quenched by adding water, and extracted with DCM three times. The organic phases were combined, dried over anhydrous sodium sulfate, concentrated under reduced pressure, and subjected to column chromatography to obtain 7-fluorobenzo[d][1,3]dioxolan-4-carboxylic acid (628 mg, yield 85%). ESI-MS(M+H)⁺=185.0.

### Synthesis step 3: 7-(4-(hydroxymethyl)piperidin-1-yl)benzo[d][1,3] dioxolan-4-carboxylic acid

7-fluorobenzo[d][1,3]dioxolan-4-carboxylic acid (628 mg, 3.41 mmol) was dissolved in 10 mL of DMF, and piperidin-4-ylmethanol ( 588 mg, 5.11 mmol) and cesium carbonate (3.3 g, 10.23 mmol) were added to the system in sequence. The mixture was reacted at 80°C for 12 h. After the reaction was completed, the reaction liquid was quenched by adding water, and extracted with DCM three times. The organic phases were combined, dried over anhydrous sodium sulfate, concentrated under reduced pressure, and subjected to column chromatography to obtain 7-(4-(hydroxymethyl)piperidin-1-yl)benzo[d][1,3]dioxolan-4-carboxylic acid (761 mg, yield 80%). ESI-MS(M+H)⁺=280.1.

### Synthesis step 4: N-(2,6-dioxopiperidin-3-yl)-7-(4-(hydroxymethyl)piperidin-1-yl) benzo[d][1,3]dioxolan-4-carboxamide

7-(4-(hydroxymethyl)piperidin-1-yl)benzo[d][1,3]dioxolan-4-carboxylic acid (761 mg, 2.72 mmol) was dissolved in 10 mL of DMF, and 3-aminopiperidin-2,6-dione (452 mg, 3.53 mmol), DIPEA (1.0 g, 8.16 mmol) and HATU (1.3 g, 3.53 mmol) were added to the system in sequence. The mixture was reacted at room temperature for 2 h. After the reaction was completed, the reaction liquid was quenched by adding water, and extracted with DCM three times. The organic phases were combined, dried over anhydrous sodium sulfate, concentrated under reduced pressure, and subjected to column chromatography (eluted with MeOH/DCM) to obtain N-(2,6-dioxopiperidin-3-yl)-7-(4-(hydroxymethyl)piperidin-1-yl)benzo[d][1,3]dioxolan -4-carboxamide (793 mg, yield 75%), ESI -MS(M+H)⁺= 390.2.

### Synthesis step 5: methyl (1-(7-((2,6-dioxopiperidin-3-yl)carbamoyl)benzo[d][1,3] dioxolan-4-yl)piperidin-4-yl)4-methylbenzenesulfonate

N-(2,6-dioxopiperidin-3-yl)-7-(4-(hydroxymethyl)piperidin-1-yl)benzo[d][1,3]dioxolan-4 -carboxamide (793 mg, 2.03 mmol) was dissolved in 10 mL of DCM, and TsCl (580 mg, 3.05 mmol) and TEA (615 mg, 6.09 mmol) were added to the system in sequence. The mixture was reacted at room temperature for 2 h. After the reaction was completed, the reaction liquid was quenched by adding water, and extracted with DCM three times. The organic phases were combined, dried over anhydrous sodium sulfate, and concentrate under reduced pressure to obtain methyl (1-(7-((2,6-dioxopiperidin-3-yl)carbamoyl)benzo[d][1,3]dioxolan-4-yl)piperidin -4-yl)4-methylbenzenesulfonate (992 mg, yield 90%). ESI-MS(M+H)⁺=544.2.

### Intermediate 234: methyl (1-(7-((2,6-dioxopiperidin-3-yl)carbamoyl)benzo[d][1,3] dioxolan-4-yl)pyrrolidin-3-yl)4-methylbenzenesulfonate

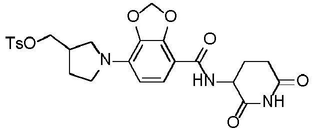

Referring to the synthetic route and method for Intermediate 233, and piperidin-4-ylmethanol in synthesis step 3 was replaced with pyrrolidin-3-ylmethanol to obtain methyl (1-(7-((2,6-dioxopipperidin-3-yl)carbamoyl)benzo[d][1,3]dioxolan-4-yl)pyrrolidin-3-yl) 4-methylbenzenesulfonate, ESI-MS (M+H)⁺=530.2.

### Intermediate 235: methyl (1-(7-((2,6-dioxopiperidin-3-yl)carbamoyl)-1H-benzo[d] imidazol-4-yl)piperidin-4-yl)4-methylbenzenesulfonate

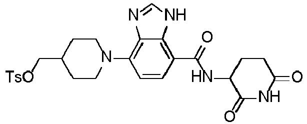

Referring to the synthetic route and method in synthesis steps 3, 4, and 5 of Intermediate 233, and 7-fluorobenzo[d][1,3]dioxolan-4-carboxylic acid in synthesis step 3 was replaced with 4-fluoro -1H-benzo[d]imidazol-7-carboxylic acid to obtain methyl (1-(7-((2,6-dioxopiperidin-3-yl)carbamoyl)-1H-benzo[d]imidazol-4-yl)piperidin-4-yl)4-methylbe nzenesulfonate, ESI-MS (M+H)⁺=540.2.

### Intermediate 236: methyl (1-(7-((2,6-dioxopiperidin-3-yl)carbamoyl)-1H-benzo[d] imidazol-4-yl)pyrrolidin-3-yl)4-methylbenzenesulfonate

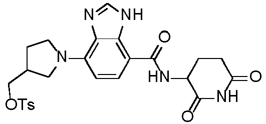

Referring to the synthetic route and method for Intermediate 235, and pyrrolidin-3-ylmethanol was replaced with piperidin-4-ylmethanol to obtain methyl (1-(7-((2,6-dioxopiperidin-3-yl )carbamoyl)-1H-benzo[d]imidazol-4-yl)pyrrolidin-3-yl)4-methyl benzenesulfonate, ESI-MS (M+H)⁺=526.5.

### Intermediate 237: methyl (1-(8-((2,6-dioxopiperidin-3-yl)carbamoyl)-2,3-dihydrobenzo [b] [1,4] dioxin-5-yl)piperidin-4-yl)4-methylbenzenesulfonate

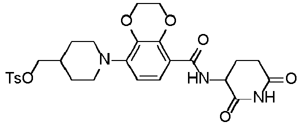

Referring to the synthetic route and method for Intermediate 233, and 7-nitrobenzo[d][1,3]dioxolan-4-carboxylic acid in synthesis step 1 was replaced with 8-nitro-2,3-dihydrobenzo[b][1,4]dioxin-5-carboxylic acid to obtain methyl (1-(8-((2,6-dioxopiperidin-3-yl)carbamoyl)-2,3-dihydrobenzo[b][1,4]dioxin-5-yl)piperidin-4-yl) 4-methylbenzenesulfonate, ESI-MS(M+H)⁺=558.2.

### Intermediate 238: 1-(7-(2,4-dioxotetrahydropyrimidin-1(2H)-yl)benzo[d]oxazol-4-yl) piperidin-4-carbaldehyde

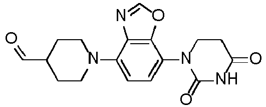

### Synthesis step 1: 4-fluoro-7-nitrobenzoxazole

5-fluoro-2-nitrobenzamide (1 g, 5.43 mmol) was dissolved in 15 mL of water, and cesium carbonate (5.3 g, 16.29 mmol) was added to the system, and the mixture was reacted for 10 min under microwave at 120W. After the reaction was completed, the reaction liquid was extracted with DCM three times, and the organic phases were combined, dried over anhydrous sodium sulfate, and concentrated under reduced pressure to obtain 4-fluoro-7-nitrobenzoxazole (750 mg, yield 75%). ESI-MS(M+H)⁺=183.1.

### Synthesis step 2: 4-(4-(1,3-dioxolan-2-yl)piperidin-1-yl)-7-nitrobenzoxazole

4-fluoro-7-nitrobenzoxazole (750 mg, 4.12 mmol) was dissolved in 10 mL of DMF, and 4-(1,3-dioxolan-2-yl)piperidine (776 mg, 4.94 mmol) and cesium carbonate (4 g, 12.36 mmol) were added to the system in sequence. The mixture was reacted at 80°C for 3 h. After the reaction was completed, the reaction liquid was quenched by adding water, and extracted with DCM three times. The organic phases were combined, dried over anhydrous sodium sulfate, concentrated under reduced pressure, and subjected to column chromatography to obtain 4-(4-(1,3-dioxolan-2-yl)piperdin-1-yl)-7-nitrobenzoxazole (900 mg, yield 68%), ESI-MS (M+H)⁺=320.1.

### Synthesis step 3: 4-(4-(1,3-dioxolan-2-yl)piperidin-1-yl)benzo[d]oxazol-7-amine

4-(4-(1,3-dioxolan-2-yl)piperdin-1-yl)-7-nitrobenzoxazole (900 mg, 2.82 mmol) was dissolved in 10 mL of tetrahydrofuran, and ammonium chloride (1.6 g, 28.21 mmol), water (10 mL), and iron powder (1.6 g, 28.21 mmol) were added to the system in sequence. The mixture was reacted at 70°C for 12 h. After the reaction was completed, the reaction liquid was extracted with DCM three times. The organic phases were combined, dried over anhydrous sodium sulfate, concentrated under reduced pressure, and subjected to column chromatography to obtain 4-(4-(1,3-dioxolan-2-yl)piperidin-1-yl)benzo[d]oxazol-7-amine (500 mg, yield 61%). ESI-MS(M+H)⁺ =290.1.

### Synthesis step 4: 3-((4-(4-(1,3-dioxolan-2-yl)piperidin-1-yl)benzo[d]oxazol-7-yl) amino)propionic acid

4-(4-(1,3-dioxolan-2-yl)piperidin-1-yl)benzo[d]oxazol-7-amine (500 mg, 1.73 mmol) was dissolved in 10 mL of toluene, and acrylic acid (128 mg, 2.07 mmol) was added to the system, and the mixture was reacted at 100°C for 12 h. After the reaction was completed, the reaction liquid was extracted with DCM three times. The organic phases were combined, dried over anhydrous sodium sulfate, and concentrated under reduced pressure to obtain 3-((4-(4-(1,3-dioxolan-2-yl)piperidin-1-yl)benzo[d]oxazol-7-yl)amino)propionic acid as a yellow liquid (580 mg, yield 94%). ESI-MS(M+H)⁺=362.2.

### Synthesis step 5: 1-(4-(4-(1,3-dioxolan-2-yl)piperidin-1-yl)benzo[d]oxazol-7-yl) dihydropyrimidin-2,4(1H, 3H)-dione

3-((4-(4-(1,3-dioxolan-2-yl)piperidin-1-yl)benzo[d]oxazol-7-yl)amino)propionic acid (580 mg, 1.60 mmol) was dissolved in 10 mL of acetic acid, and urea (200 mg, 3.20 mmol) was added to the system, and the mixture was reacted at 120°C for 12 h. After the reaction was completed, the reaction liquid was extracted with DCM three times. The organic phases were combined, dried over anhydrous sodium sulfate, concentrated under reduced pressure, and subjected to column chromatography (eluted with DCM/MeOH) to obtain 1-(4-(4-(1,3-dioxolan-2-yl)piperidin-1-yl)benzo[d]oxazol-7-yl)dihydropyrimidin-2,4(1H,3H)-dio ne as a brown solid (380 mg, yield 58%). ESI-MS(M+H)⁺=387.2.

### Synthesis step 6: 1-(7-(2,4-dioxotetrahydropyrimidin-1(2H)-yl)benzo[d]oxazol-4-yl) piperidin-4-carbaldehyde

1-(4-(4-(1,3-dioxolan-2-yl)piperidin-1-yl)benzo[d]oxazol-7-yl)dihydropyrimidin-2,4(1H, 3H)-dione (380 mg, 0.98 mmol) was dissolved in 3 mL of TFA and the mixture was reacted at 80°C for 1 h, then 0.2 mL of concentrated hydrochloric acid was added to the system, and the mixture was reacted at 80°C for 3 h. After the reaction was completed, the reaction liquid was adjusted to alkalinity by adding saturated aqueous sodium carbonate solution, extracted with MeOH/DCM five times, and the organic phases were combined, dried over anhydrous sodium sulfate, and concentrate under reduced pressure to obtain 1-(7-(2,4-dioxotetrahydropyrimidin-1(2H)-yl)benzo[d]oxazol-4-yl)piperidin-4-carbaldehyde as a yellow solid (310 mg, yield 92%). ESI-MS(M+H)⁺=343.1.

### Referring to the synthetic route and method for Intermediate 238, the following intermediates were synthesized:

| Intermediate No. | Intermediate structure | LC-MS (ESI-MS) *m*/*z* [M+H]⁺ |
|---|---|---|
| 239 | 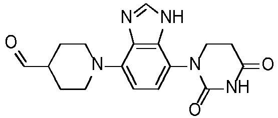 | 342.2 |
| 240 | 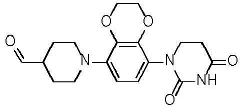 | 360.2 |

### Intermediate 241: methyl (1-(7-((2,6-dioxopiperidin-3-yl)amino)benzo[d][1,3] dioxolan-4-yl)pyrrolidin-3-yl)4-methylbenzenesulfonate

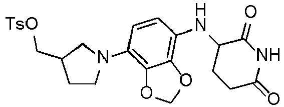

### Synthesis step 1: (1-(7-nitrobenzo[d][1,3]dioxolan-4-yl)pyrrolidin-3-yl)methanol

4-fluoro-7-nitrobenzo[d][1,3]dioxolane (3 g, 16.22 mmol) was dissolved in 20 mL of DMF, and pyrrolidin-3-ylmethanol ( 2 g, 19.46 mmol) and cesium carbonate (15.81 g, 48.64 mmol) were added to the system in sequence. The mixture was reacted at 90°C for 2 h. After the reaction was completed, the reaction liquid was quenched by adding water, and extracted with DCM three times. The organic phases were combined, dried over anhydrous sodium sulfate, concentrated under reduced pressure, and subjected to column chromatography to obtain (1-(7-nitrobenzo[d][1,3]dioxolan-4-yl)pyrrolidin-3-yl)methanol as a yellow solid (3.6 g, yield 85%). ESI-MS(M+H)⁺=267.1.

### Synthesis step 2: (1-(7-aminobenzo[d][1,3]dioxolan-4-yl)pyrrolidin-3-yl)methanol

(1-(7-nitrobenzo[d][1,3]dioxolan-4-yl)pyrrolidin-3-yl)methanol (2 g, 8.47 mmol) was dissolved in 30 mL of tetrahydrofuran, and ammonium chloride (7.5 g, 147.26 mmol), water (7.5 mL), and iron powder (8 g, 147.26 mmol) were added to the system in sequence. The mixture was reacted at 70°C for 12 h. After the reaction was completed, a large amount of iron powder was filtered out while hot, and the resulting reaction liquid was extracted with DCM three times. The organic phases were combined, dried over anhydrous sodium sulfate, concentrated under reduced pressure, and subjected to column chromatography to obtain (1-(7-aminobenzo[d][1,3]dioxolan-4-yl)pyrrolidin-3-yl)methanol as a yellow solid (1.2 g, yield 60%). ESI-MS(M+H)⁺=237.1.

### Synthesis step 3: 3-((7-(3-(hydroxymethyl)pyrrolidin-1-yl)benzo[d][1,3]dioxolan-4-yl) amino)piperidin-2,6-dione

(1-(7-aminobenzo[d][1,3]dioxolan-4-yl)pyrrolidin-3-yl)methanol (1.2 g, 5.08 mmol) was dissolved in 10 mL of DMF, and 3-bromopiperidin-2,6-dione (1.3 g, 6.61 mmol) and sodium bicarbonate (1.28 g, 15.27 mmol) were added to the system and the mixture was reacted at 70°C for 1 h, and the reaction was completed. the reaction liquid was extracted with DCM three times. The organic phases were combined, dried over anhydrous sodium sulfate, concentrated under reduced pressure, and subjected to column chromatography to obtain 3-((7-(3-(hydroxymethyl)pyrrolidin-1-yl)benzo[d][1,3]dioxolan-4-yl)amino)piperidin-2,6-dione as a yellow solid (1.3 g, yield 75%). ESI-MS(M+H)⁺=348.2.

### Synthesis step 4: methyl (1-(7-((2,6-dioxopiperidin-3-yl)amino)benzo[d][1,3]dioxolan -4-yl)pyrrolidin-3-yl)4-methylbenzenesulfonate

3-((7-(3-(hydroxymethyl)pyrrolidin-1-yl)benzo[d][1,3]dioxolan-4-yl)amino)piperidin-2,6 -dione (1 g, 2.88 mmol) was dissolved in 10 mL of DCM, and p-toluenesulfonyl chloride (820 mg, 4.32 mmol) and 3.5 mL of DIPEA were added to the system in sequence. The mixture was reacted at room temperature for 2 h. After the reaction was completed, the reaction liquid was extracted with DCM three times. The organic phases were combined, dried over anhydrous sodium sulfate, concentrate under reduced pressure, and subjected to column chromatography to obtain methyl (1-(7-((2,6-dioxopiperidin-3-yl)amino)benzo[d][1,3]dioxolan-4-yl)pyrrolidin -3-yl)4-methylbenzenesulfonate as a yellow solid (1.2 g, yield 85%). ESI-MS(M+H)⁺=502.2.

### Referring to the synthetic route and method for Intermediate 241, the following intermediates were synthesized:

| Intermediate No. | Intermediate structure | LC-MS (ESI-MS) *m*/*z* [M+H]⁺ |
|---|---|---|
| 242 | 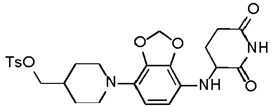 | 516.2 |
| 243 | 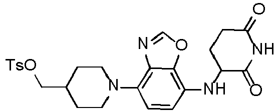 | 513.2 |
| 244 | 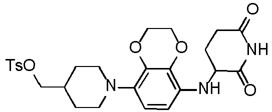 | 530.2 |

### Intermediate 245: 1-(3-(2,6-dioxopiperidin-3-yl)-1-methyl-1H-indazol-6-yl) pyrrolidin-3-carbaldehyde

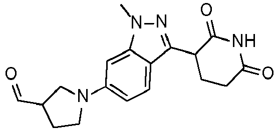

### Synthesis step 1: 3-(2,6-bis(benzyloxy)pyridin-3-yl)-6-bromo-1-methyl-1H-indazole

6-bromo-3-iodo-1-methyl-1H-indazole (2 g, 5.97 mmol) was dissolved in 20 mL of THF, and 2,6-bis(benzyloxy)-3-(4,4,5,5-tetramethyl-1,3,2-dioxobenzaldehyde-2-yl)pyridine (3.7 g, 8.96 mmol), DPPF palladium dichloride (432 mg, 0.60 mmol), and cesium carbonate (5.8 g, 17.91 mmol) were added to the system in sequence. The mixture was reacted at room temperature for 12 h under introduced nitrogen protection. After the reaction was completed, the reaction liquid was quenched by adding water, and extracted with DCM three times. The organic phases were combined, dried over anhydrous sodium sulfate, concentrated under reduced pressure, and subjected to column chromatography to obtain 3-(2,6-bis(benzyloxy)pyridin-3-yl)-6-bromo-1-methyl-1H-indazole as a yellow solid (1.4 g, yield 49%). ESI-MS(M+H)⁺=500.1.

### Synthesis step 2: 6-(3-(1,3-dioxolan-2-yl)pyrrolidin-1-yl)-3-(2,6-bis(benzyloxy) pyridin-3-yl)-1-methyl-1H-indazole

3-(2,6-bis(benzyloxy)pyridin-3-yl)-6-bromo-1-methyl-1H-indazole (1.4 g, 2.81 mmol) was dissolved in 20 mL of 1,4-dioxane, and 3-(1,3-dioxolan-2-yl)pyrrolidine (485 mg, 3.37 mmol), Pd₂(dba)₃(257 mg, 0.28 mmol), XPhos (267 mg, 0.56 mmol), and cesium carbonate (1.8 g, 5.62 mmol) were added to the system in sequence. The mixture was reacted at 160°C for 12 h under introduced nitrogen protection. After the reaction was completed, the reaction liquid was quenched by adding water, and extracted with DCM three times. The organic phases were combined, dried over anhydrous sodium sulfate, concentrated under reduced pressure, and subjected to column chromatography to obtain 6-(3-(1,3-dioxolan-2-yl)pyrrolidin-1 -yl)-3-(2,6-bis(benzyloxy)pyridin-3-yl)-1-methyl-1H-indazole as a yellow solid (860 mg, yield 50%). ESI-MS(M+H)⁺=563.3.

### Synthesis step 3: 3-(6-(3-(1,3-dioxolan-2-yl)pyrrolidin-1-yl)-1-methyl-1H-indazol -3-yl)piperidin-2,6-dione

6-(3-(1,3-dioxolan-2-yl)pyrrolidin-1-yl)-3-(2,6-bis(benzyloxy)pyridin-3-yl)-1-methyl-1H -indazole (860 mg, 1.53 mmol) was dissolved in 10 mL of 1,4-dioxane, and palladium hydroxide (21 mg, 0.15 mmol) was added to the system, and the mixture was reacted at room temperature for 12 h under introduced hydrogen. After the reaction was completed, the reaction liquid was filtered and concentrated under reduced pressure to obtain 3-(6-(3-(1,3-dioxolan-2-yl) pyrrolidin-1-yl)-1-methyl-1H-indazol-3-yl)piperidin-2,6-dione as a white solid (500 mg, yield 85%). ESI-MS(M+H)⁺=385.2

### Synthesis step 4: 1-(3-(2,6-dioxopiperidin-3-yl)-1-methyl-1H-indazol-6-yl) pyrrolidin-3-carbaldehyde

3-(6-(3-(1,3-dioxolan-2-yl)pyrrolidin-1-yl)-1-methyl-1H-indazol-3-yl)piperidin-2,6-dione (500 mg, 1.30 mmol) was dissolved in 5 mL of TFA and the mixture was reacted at 80°C for 1 h. 1 mL of concentrated hydrochloric acid was added dropwise and the mixture was reacted at 80°C for 2 h. The reaction liquid was quenched with aqueous sodium bicarbonate solution, extracted with DCM 5 times, and concentrated under reduced pressure to obtain 1-(3-(2,6-dioxopiperidin-3-yl)-1-methyl-1H-indazol-6-yl)pyrrolidin-3-carbaldehyde as a white solid (400 mg, yield 90%). ESI-MS(M+H)⁺=341.2.

### Referring to the synthetic route and method for Intermediate 246, the following intermediates were synthesized:

| Intermediate No. | Intermediate structure | LC-MS (ESI-MS) *m*/*z* [M+H]⁺ |
|---|---|---|
| 247 | 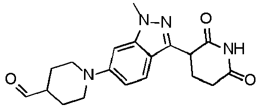 | 355.2 |
| 248 | 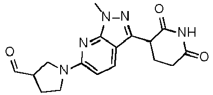 | 342.2 |
| 249 | 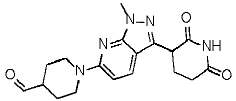 | 356.2 |
| 250 | 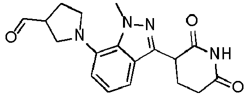 | 341.2 |
| 251 | 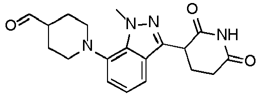 | 355.2 |
| 252 | 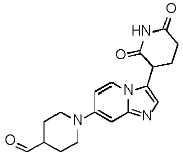 | 341.2 |
| 253 | 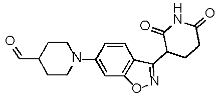 | 342.1 |
| 254 | 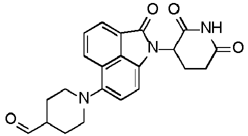 | 392.2 |

### Intermediate 255: 1-(1-(2,6-dioxopiperidin-3-yl)-3-methyl-2-oxo-2,3-dihydro-1H-benzo[d]imidazol-5-yl)pyrrolidin-3-carbaldehyde

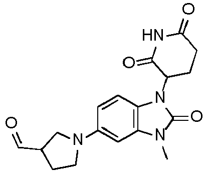

### Synthesis step 1: 3-(5-(3-(1,3-dioxolan-2-yl)pyrrolidin-1-yl)-3-methyl-2-oxo-2,3-dihydro-1H-benzo[d]imidazol-1-yl)piperidin-2,6-dione

3-(5-bromo-3-methyl-2-oxo-2,3-dihydro-1H-benzo[d]imidazol-1-yl)piperidin-2,6-dione (1 g , 2.97 mmol) was dissolved in 20 mL of DMF, and 3-(1,3-dioxolan-2-yl)pyrrolidine (3.6 g, 3.56 mmol), Pd₂(dba)₃ (280 mg, 0.30 mmol), XPhos (285 mg, 0.59 mmol), and cesium carbonate (1.8 g, 5.62 mmol) were added to the system in sequence. The mixture was reacted at 160°C for 12 h under introduced nitrogen protection. After the reaction was completed, the reaction liquid was quenched by adding water, and extracted with DCM three times. The organic phases were combined, dried over anhydrous sodium sulfate, concentrated under reduced pressure, and subjected to column chromatography to obtain 3-(5-(3-(1,3-dioxolan-2-yl)pyrrolidin-1-yl) -3-methyl-2-oxo-2,3-dihydro-1H-benzo[d]imidazol-1-yl)piperidin-2,6-dione as a yellow solid (530 mg, yield 45%). ESI-MS(M+H)⁺=401.2.

### Synthesis step 2: 1-(1-(2,6-dioxopiperidin-3-yl)-3-methyl-2-oxo-2,3-dihydro-1H-benzo [d] imidazol-5-yl)pyrrolidin-3-carbaldehyde

3-(5-(3-(1,3-dioxolan-2-yl)pyrrolidin-1-yl)-3-methyl-2-oxo-2,3-dihydro-1H-benzo[d]imi dazol-1-yl)piperidin-2,6-dione (530 mg, 1.33 mmol) was dissolved in 5 mL of TFA and the mixture was reacted at 80°C for 1 h. 1 mL of concentrated hydrochloric acid was added dropwise and the mixture was reacted at 80°C for 2 h. After the reaction was completed, the system was adjusted to be alkaline by adding saturated aqueous sodium bicarbonate solution, extracted with DCM 5 times, concentrated under reduced pressure, and subjected to column chromatography to obtain 1-(1-(2,6-dioxopiperidin-3-yl)-3-methyl-2-oxo-2,3-dihydro-1H-benzo[d]imidazol-5-yl) pyrrolidin-3-carbaldehyde as a white solid (450 mg, yield 95%). ESI-MS(M+H)⁺=357.2.

### Referring to the synthetic route and method for Intermediate 256, the following intermediates were synthesized:

| Intermediate No. | Intermediate structure | LC-MS (ESI-MS) *m*/*z* [M+H]⁺ |
|---|---|---|
| 257 | 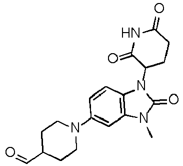 | 371.2 |
| 258 | 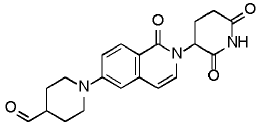 | 368.2 |
| 259 | 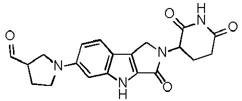 | 381.2 |
| 260 | 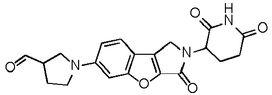 | 382.1 |
| 261 | 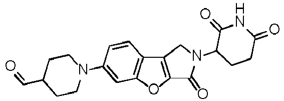 | 396.2 |
| 262 | 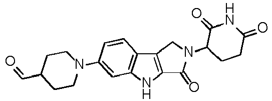 | 395.2 |

### Intermediate 263: 3-(6-((2,6-dioxopiperidin-3-yl)carbamoyl)pyridin-3-yl)propan-2-yn-1-ylmesylate

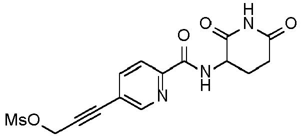

### Synthesis step 1: 5-(3-hydroxylpropan-1-yne-1-yl)picolinic acid

5-bromopicolinic acid (2 g, 10.00 mmol) was dissolved in 20 mL of glycol dimethyl ether, and propargyl alcohol (675 mg, 12.00 mmol), tetrakis(triphenylphosphine)palladium (570 mg, 0.50 mmol), copper iodide (380 mg, 2.00 mmol), and DIPEA (4 mL, 30.00 mmol) were added to the system in sequence. The mixture was reacted at 120°C for 20 min. After the reaction was completed, the reaction liquid was filtered, concentrated under reduced pressure, dissolved in DCM, and subjected to column chromatography to obtain 5-(3-hydroxyprop-1-yn-1-yl)picolinic acid as a yellow liquid (750 mg, yield 40%). ESI-MS(M+H)⁺=178.0.

### Synthesis step 2: 5-(3-((methylsulfonyl)oxy)propan-1-yne-1-yl)picolinic acid

5-(3-hydroxyprop-1-yn-1-yl)picolinic acid (750 mg, 4.23 mmol) was dissolved in 15 mL of DCM, and methanesulfonyl chloride (724 mg, 6.35 mmol) and TEA ( 855 mg, 8.47 mmol) were added in sequence. The mixture was reacted at room temperature for 3 h. After the reaction was completed, water was added and a solid was precipitated. The resulting system was extracted by adding DCM, dried over anhydrous sodium sulfate, concentrated under reduced pressure, and subjected to column chromatography to obtain 5-(3-((methylsulfonyl)oxy)prop-1-yn-1-yl)picolinic acid as a white solid (700 mg, yield 70%). ESI-MS(M+H)⁺=256.0.

### Synthesis step 3: 3-(6-((2,6-dioxopiperidin-3-yl)carbamoyl)pyridin-3-yl)propan-2-yn -1-ylmesylate

5-(3-((methylsulfonyl)oxy)prop-1-yn-1-yl)picolinic acid (700 mg, 2.73 mmol) was dissolved in 15 mL of DMF, and 3-aminopiperdine-2,6-dione (418 mg, 3.27 mmol), HATU (1.2 g, 3.27 mmol), and DIPEA (827 mg, 8.19 mmol) were added to the system in sequence. The mixture was reacted at room temperature for 2 h. After the reaction was completed, water was added and a solid was precipitated. The resulting system was extracted by adding DCM, dried over anhydrous sodium sulfate, concentrated under reduced pressure, and subjected to column chromatography to obtain 3-(6-((2,6-dioxopiperidin-3-yl)carbamoyl)pyridin-3-yl)prop-2-yn-1-yl methanesulfonate as a white solid (740 mg, yield 75%). ESI-MS(M+H)⁺=366.1.

**Referring to the synthetic route and method for Intermediate 263, the following intermediates were synthesized:**

| Intermediate No. | Intermediate structure | LC-MS (ESI-MS) *m*/*z* [M+H]⁺ |
|---|---|---|
| 264 | 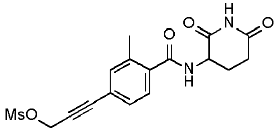 | 379.1 |
| 265 | 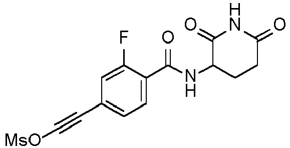 | 369.1 |
| 266 | 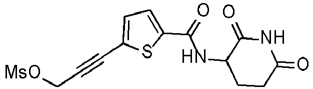 | 371.0 |
| 267 | 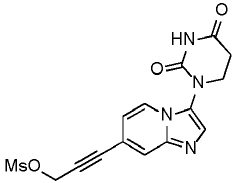 | 363.1 |
| 268 | 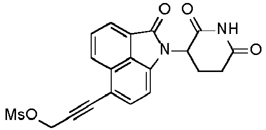 | 413.1 |
| 269 | 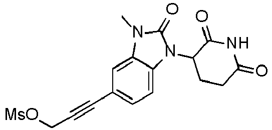 | 392.1 |
| 270 | 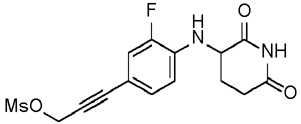 | 355.1 |
| 271 | 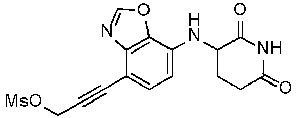 | 378.1 |

### Example 1. 5-(3-(3-cyclopentyl-2-oxoimidazolin-1-yl)piperidin-1-yl)-3-((4-(1-(1-(2-(2,6-dioxopiperidin-3-yl)-1,3-dioxoisoindol-5-yl)azetidin-3-yl)-4-methylpiperidin-4-yl)ph enyl)amino)pyrazin-2-carboxamide

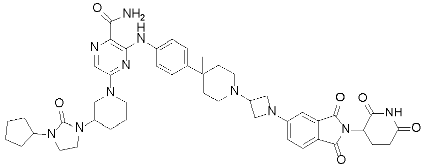

Intermediate 63 (5.47 g, 10 mmol) and Intermediate 133 (3.28 g, 10 mmol) were dissolved in a solution of triethylamine (101 mg, 1 mmol) in dichloroethane (40 mL), and the mixture was reacted at room temperature for 5 min under stirring. Sodium triacetoxyborohydride (10.60 g, 50 mmol) was added, and the mixture was reacted at room temperature for 5 h under stirring. After the reaction was completed, the reaction liquid was extracted by adding ethyl acetate and water solution. The organic layers were combined, washed with saturated sodium chloride, dried over anhydrous sodium sulfate, and filtered with suction. The solvent was removed under pressure and purification was performed by column chromatography to obtain Compound 001. ESI-MS: *m*/*z* = 858 [M+H]⁺.

### Example 2. 3-((4-(1-(1-(2-(2,6-dioxopiperidin-3-yl)-1,3-dioxoisoindol-5-yl)azetidine -3-yl)-4-methylpiperidin-4-yl)phenyl)amino)-5-(3-(3-(oxetan-3-yl)-2-oxoimidazolin-1-yl)pip eridin-1-yl)pyrazin-2-carboxamide

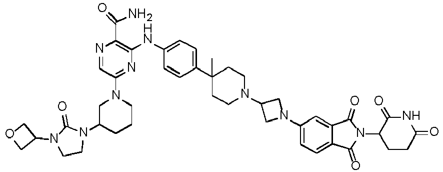

The synthesis method was the same as that for Example 1, except that Intermediate 63 was replaced with Intermediate 64 to obtain Compound 002. ESI-MS: *m*/*z* = 846 [M+H]⁺.

### Example 3. 5-(3-(3-cyclohexyl-2-oxoimidazolin-1-yl)piperidin-1-yl)-3-((4-(1-(1-(2-(2,6-dioxopiperidin-3-yl)-1,3-dioxoisoindol-5-yl)azetidin-3-yl)-4-methylpiperidin-4-yl)pheny l)amino)pyrazin-2-carboxamide

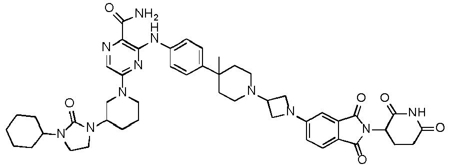

The synthesis method was the same as that for Example 1, except that Intermediate 63 was replaced with Intermediate 65 to obtain Compound 003, ESI-MS: m/z = 872 [M+H]⁺.

### Example 4. 3-((4-(1-(1-(2-(2,6-dioxopiperidin-3-yl)-1,3-dioxoisoindol-5-yl)azetidine -3-yl)-4-methylpiperidin-4-yl)phenyl)amino)-5-(3-(2-oxo-3-(tetrahydro-2H-pyran-4-yl)imid azolin-1-yl)piperidin-1-yl)pyrazin-2-carboxamide

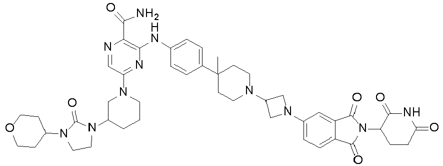

The synthesis method was the same as that for Example 1, except that Intermediate 63 was replaced with Intermediate 66 to obtain Compound 004, ESI-MS: m/z = 874 [M+H]⁺.

### Example 5. 3-((4-(1-(1-(2-(2,6-dioxopiperidin-3-yl)-1,3-dioxoisoindol-5-yl)azetidin-3 -yl)-4-methylpiperidin-4-yl)phenyl)amino)-5-(3-(3-oxotetrahydro-1H-pyrrolo [1,2-c] imidazo l-2(3H)-yl)piperidin-1-yl)pyrazin-2-carboxamide

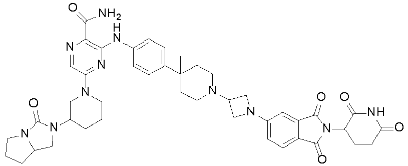

The synthesis method was the same as that for Example 1, except that Intermediate 63 was replaced with Intermediate 67 to obtain Compound 005, ESI-MS: m/z = 830 [M+H]⁺.

### Example 6. 3-((4-(1-(1-(2-(2,6-dioxopiperidin-3-yl)-1,3-dioxoisoindol-5-yl)azetidin-3 -yl)-4-methylpiperidin-4-yl)phenyl)amino)-5-(3-(3-oxohexahydroimidazo[1,5-a]pyridin-2(3 H)-yl)piperidin-1-yl)pyrazin-2-carboxamide

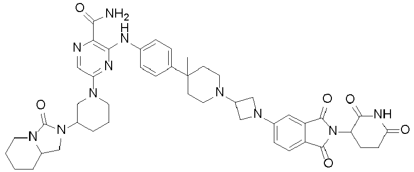

The synthesis method was the same as that for Example 1, except that Intermediate 63 was replaced with Intermediate 68 to obtain Compound 006, ESI-MS: m/z = 844 [M+H]⁺.

### Example 7. 3-((4-(1-(1-(2-(2,6-dioxopiperidin-3-yl)-1,3-dioxoisoindol-5-yl)azetidin-3 -yl)-4-methylpiperidin-4-yl)phenyl)amino)-6-ethyl-5-(3-(3-(3-methyl-2-oxyimidazolin-1-yl)p iperidin-1-yl)pyrazin-2-carboxamide

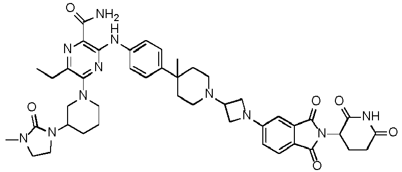

The synthesis method was the same as that for Example 1, except that Intermediate 63 was replaced with Intermediate 69 to obtain Compound 007, ESI-MS: m/z = 832 [M+H]⁺.

### Example 8. 3-((4-(1-(1-(2-(2,6-dioxopiperidin-3-yl)-1,3-dioxoisoindol-5-yl)azetidin-3 -yl)-4-methylpiperidin-4-yl)phenyl)amino)-5-(3-(3-methyl-2-oxohexahydrocyclopenta[d]imi dazol-1(2H)-yl)piperidin-1-yl)pyrazin-2-carboxamide

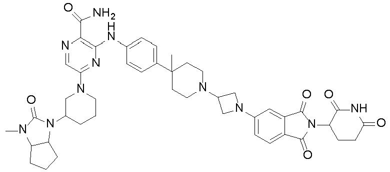

The synthesis method was the same as that for Example 1, except that Intermediate 63 was replaced with Intermediate 70 to obtain Compound 008, ESI-MS: m/z = 844 [M+H]⁺.

### Example 9. 3-((4-(1-(1-(2-(2,6-dioxopiperidin-3-yl)-1,3-dioxoisoindol-5-yl)azetidin-3 -yl)-4-methylpiperidin-4-yl)phenyl)amino)-5-(3-(3-methyl-2-oxooctahydro-1H-benzo[d]imi dazol-1-yl)piperidin-1-yl)pyrazin-2-carboxamide

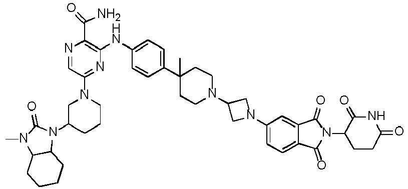

The synthesis method was the same as that for Example 1, except that Intermediate 63 was replaced with Intermediate 71 to obtain Compound 009. ESI-MS: *m*/*z* = 858 [M+H]⁺.

### Example 10. 3-((4-(1-(1-(2-(2,6-dioxopiperidin-3-yl)-1,3-dioxoisoindol-5-yl)azetidin-3 -yl)-4-methylpiperidin-4-yl)phenyl)amino)-5-(3-(4-methyl-5-oxo-4,6-diazaspirocyclo[2.4]he ptan-6-yl)piperidin-1-yl)pyrazin-2-carboxamide

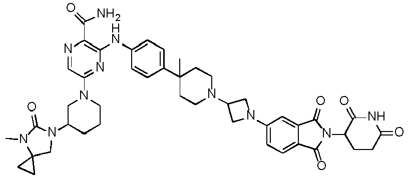

The synthesis method was the same as that for Example 1, except that Intermediate 63 was replaced with Intermediate 72 to obtain Compound 010. ESI-MS: *m*/*z* = 830 [M+H]⁺.

### Example 11. 3-((4-(1-(1-(2-(2,6-dioxopiperidin-3-yl)-1,3-dioxoisoindol-5-yl)azetidin-3 -yl)-4-methylpiperidin-4-yl)phenyl)amino)-5-(3-(5-methyl-6-oxo-5,7-diazaspirocyclo [3.4] oct an-7-yl)piperidin-1-yl)pyrazin-2-carboxamide

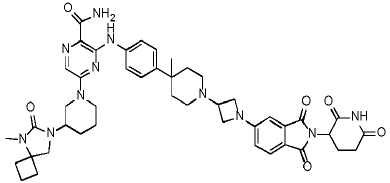

The synthesis method was the same as that for Example 1, except that Intermediate 63 was replaced with Intermediate 73 to obtain Compound 011. ESI-MS: *m*/*z* = 844 [M+H]⁺.

### Example 12. 3-((4-(1'-(2-(2,6-dioxopiperidin-3-yl)-1,3-dioxoisoindol-5-yl)-4-methyl-[1,4'-bipiperidin]-4-yl)phenyl)amino)-5-(3-(3-(oxetan-3-yl)-2-oxoimidazolin-1-yl)piperidin-1 -yl)pyrazin-2-carboxamide

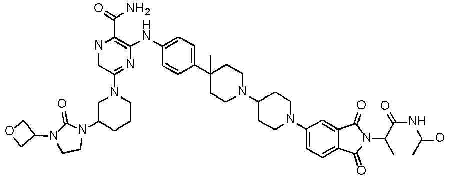

The synthesis method was the same as that for Example 2, except that Intermediate 133 was replaced with Intermediate 134 to obtain Compound 012. ESI-MS: *m*/*z* = 874 [M+H]⁺.

### Example 13. 3-((4-(1'-(2-(2,6-dioxopiperidin-3-yl)-1-oxoisoindol-5-yl)-4-methyl-[1,4'-bipiperidin]-4-yl)phenyl)amino)-5-(3-(3-(oxetan-3-yl)-2-oxoimidazolin-1-yl)piperidin-1 -yl)pyrazin-2-carboxamide

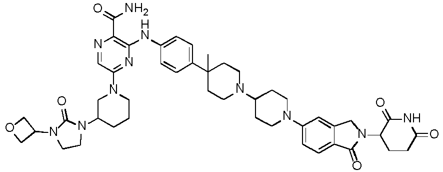

The synthesis method was the same as that for Example 2, except that Intermediate 133 was replaced with Intermediate 135 to obtain Compound 013. ESI-MS: *m*/*z* = 860 [M+H]⁺.

### Example 14. 3-((4-(1-(1-(2-(2,6-dioxopiperidin-3-yl)-1-oxoisoindol-5-yl)azetidin-3-yl) -4-methylpiperidin-4-yl)phenyl)amino)-5-(3-(3-(oxetan-3-yl)-2-oxoimidazolin-1-yl)piperidin -1-yl)pyrazin-2-carboxamide

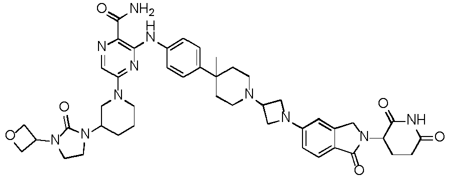

The synthesis method was the same as that for Example 2, except that Intermediate 133 was replaced with Intermediate 136 to obtain Compound 014. ESI-MS: *m*/*z* = 832 [M+H]⁺.

### Example 15. 3-((4-(4-(1-(2-(2,6-dioxopiperidin-3-yl)-1,3-dioxoisoindol-5-yl)azetidin-3 -yl)piperazin-1-yl)phenyl)amino)-5-(3-(3-(oxetan-3-yl)-2-oxoimidazolin-1-yl)piperidin-1-yl) pyrazin-2-carboxamide

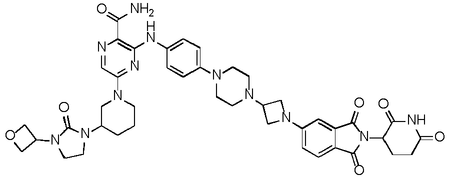

The synthesis method was the same as that for Example 1, except that Intermediate 63 was replaced with Intermediate 74 to obtain Compound 015. ESI-MS: *m*/*z* = 833 [M+H]⁺.

### Example 16. 3-((4-(4-((1-(2-(2,6-dioxopiperidin-3-yl)-1,3-dioxoisoindol-5-yl)azetidine -3-yl)methyl)piperazin-1-yl)phenyl)amino)-5-(3-(3-(oxetan-3-yl)-2-oxoimidazolin-1-yl)piper idin-1-yl)pyrazin-2-carboxamide

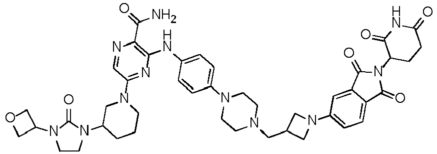

The synthesis method was the same as that for Example 15, except that Intermediate 133 was replaced with Intermediate 137 to obtain Compound 016. ESI-MS: *m*/*z* = 847 [M+H]⁺.

### Example 17. 3-((4-(9-(2-(2,6-dioxopiperidin-3-yl)-1,3-dioxoisoindol-5-yl)-3,9-diazaspirocyclo[5.5]undec-3-yl)phenyl)amino)-5-(3-(3-(oxetan-3-yl)-2-oxoimidazolin-1-yl)pi peridin-1-yl)pyrazin-2-carboxamide

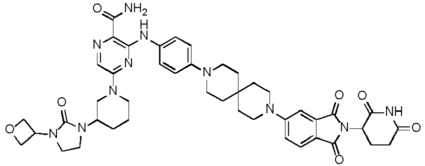

The synthesis method was the same as that for Example 1, except that Intermediate 63 was replaced with Intermediate 75, and Intermediate 1339 was replaced with Compound 133-1 to obtain Compound 017. ESI-MS: *m*/*z* = 846 [M+H]⁺.

### Example 18. 3-((4-(6-(2-(2,6-dioxopiperidin-3-yl)-1,3-dioxoisoindol-5-yl)-2,6-diazaspirocyclo[3.3]heptan-2-yl)phenyl)amino)-5-(3-(3-(oxetan-3-yl)-2-oxoimidazolin-1-yl)p iperidin-1-yl)pyrazin-2-carboxamide

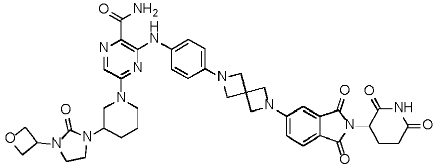

The synthesis method was the same as that for Example 17, except that Intermediate 75 was replaced with Intermediate 76 to obtain Compound 018. ESI-MS: *m*/*z* = 790 [M+H]⁺.

### Example 19. 3-((4-(2-(4-(2,6-dioxopiperidin-3-yl)-1,3-dioxoisoindol-5-yl)piperazin -1-yl)-7-azaspiro [3.5] non-7-yl)phenyl)amino)-5-(3-(3-(oxetan-3-yl)-2-oxoimidazolin-1-yl)pip eridin-1-yl)pyrazin-2-carboxamide

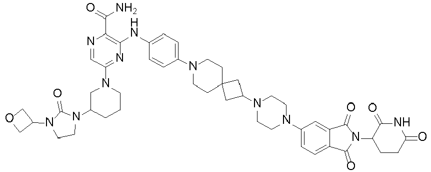

The synthesis method was the same as that for Example 1, except that Intermediate 63 was replaced with Intermediate 77, and Intermediate 133 was replaced with Intermediate 138 to obtain Compound 019. ESI-MS: *m*/*z* = 901 [M+H]⁺.

### Example 20. 5-(3-(3-cyclopentyl-2-oxoimidazolin-1-yl)piperidin-1-yl)-3-((4-(1-(1-(2-(2,6-dioxopiperidin-3-yl)-1,3-dioxoisoindol-4-yl)azetidin-3-yl)-4-methylpiperidin-4-yl)pheny l)amino)pyrazin-2-carboxamide

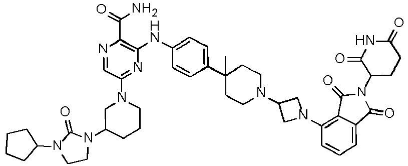

The synthesis method was the same as that for Example 1, except that Intermediate 133 was replaced with Intermediate 139 to obtain Compound 020. ESI-MS: *m*/*z* = 858 [M+H]⁺.

### Example 21. 5-(3-(3-cyclopentyl-2-oxoimidazolin-1-yl)piperidin-1-yl)-3-((4-(1-(5-((2,6-dioxopiperidin-3-yl)amino)pyridin-2-yl)azetidin-3-yl)-4-methylpiperidin-4-yl)phenyl)a mino)pyrazin-2-carboxamide

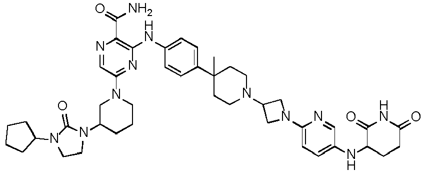

The synthesis method was the same as that for Example 1, except that Intermediate 133 was replaced with 3-((6-(3-oxoazetidin-1-yl)pyridin-3-yl)amino)piperidin-2,6-dione to obtain Compound 021. ESI-MS: *m*/*z* = 805 [M+H]⁺.

### Example 22. 5-(3-(3-cyclopentyl-2-oxoimidazolin-1-yl)piperidin-1-yl)-3-((4-(1-(1-(2,6-dioxopiperidin-3-yl)-3-methyl-2-oxo-2,3-dihydro-1H-benzo[d]imidazol-4-yl)azetidin-3-yl)-4-methylpiperidin-4-yl)phenyl)amino)pyrazin-2-carboxamide

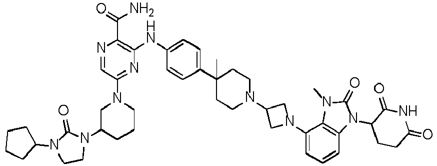

The synthesis method was the same as that for Example 1, except that Intermediate 133 was replaced with 3-(3-methyl-2-oxo-4-(3-oxoazetidin-1-yl)-2,3-dihydro-1H-benzo[d] imidazol-1-yl)piperidin-2,6-dione to obtain Compound 022. ESI-MS: *m*/*z* = 859 [M+H]⁺.

### Example 23. 5-(3-(3-cyclopentyl-2-oxoimidazolin-1-yl)piperidin-1-yl)-3-((4-(1-(1-(2-(2,6-dioxopiperidin-3-yl)-1-oxo-1,2-dihydroisoquinolin-6-yl)azetidin-3-yl)-4-methylpiperidi n-4-yl)phenyl)amino)pyrazin-2-carboxamide

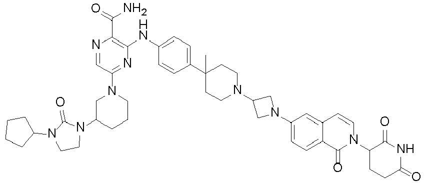

The synthesis method was the same as that for Example 1, except that Intermediate 133 was replaced with 3-(1-oxo-6-(3-oxoazetidin-1-yl)isoquinolin-2(1H)-yl)piperidin-2,6-dione to obtain Compound 023. ESI-MS: *m*/*z* = 856 [M+H]⁺.

### Example 24. 5-(3-(3-cyclopentyl-2-oxoimidazolin-1-yl)piperidin-1-yl)-3-((4-(1-(1-(2-(2,6-dioxopiperidin-3-yl)-1-oxo-1,2,3,4-tetrahydroisoquinolin-6-yl)azetidin-3-yl)-4-methylpi peridin-4-yl)phenyl)amino)pyrazin-2-carboxamide

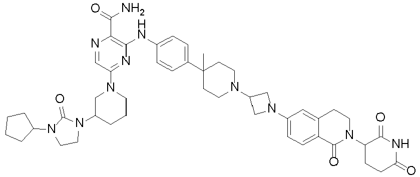

The synthesis method was the same as that for Example 1, except that Intermediate 133 was replaced with 3-(1-oxo-6-(3-oxoazetidin-1-yl)-3,4-dihydroisoquinolin-2 (1H)-yl)piperidin -2,6-dione to obtain Compound 023. ESI-MS: *m*/*z* = 858 [M+H]⁺.

### Example 25. 3-((4-(1-(1-(2-(2,6-dioxopiperidin-3-yl)-1,3-dioxoisoindol-5-yl) azetidin-3-yl)-4-methylpiperidin-4-yl)phenyl)amino)-5-(3-(3-methyl-2-oxo-2,3-dihydro-1H-benzo [d] imidazol-1-yl)piperidin-1-yl)pyrazin-2-carboxamide

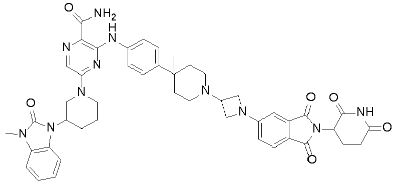

The synthesis method was the same as that for Example 1, except that Intermediate 63 was replaced with Intermediate 78 to obtain Compound 025. ESI-MS: *m*/*z* = 852 [M+H]⁺.

### Example 26. 3-((4-(1-(1-(2-(2,6-dioxopiperidin-3-yl)-1,3-dioxoisoindolin-5-yl) azetidin-3-yl)-4-methylpiperidin-4-yl)phenyl)amino)-5-(3-(3-oxoimidazo[1,5-a]pyridin-2(3H )-yl)piperidin-1-yl)pyrazin-2-carboxamide

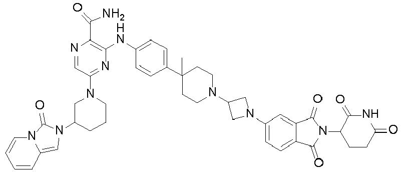

The synthesis method was the same as that for Example 1, except that Intermediate 63 was replaced with Intermediate 79 to obtain Compound 026. ESI-MS: *m*/*z* = 838 [M+H]⁺.

### Example 27. 5-(3-(3-cyclopentyl-2-oxyimidazolin-1-yl)piperidin-1-yl)-3-((4-(1-(1-(2-(2,6-dioxopiperidin-3-yl)-1,3-dioxoisoindol-5-yl)azetidin-3-yl)piperidin-4-yl)phenyl)amino) pyrazin-2-carboxamide

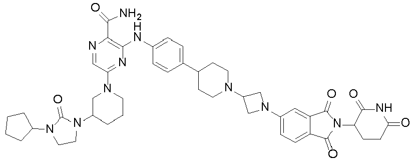

The synthesis method was the same as that for Example 1, except that Intermediate 63 was replaced with Intermediate 80 to obtain Compound 027. ESI-MS: *m*/*z* = 844 [M+H]⁺.

### Example 28. 5-(3-(3-cyclopentyl-2-oxoimidazolin-1-yl)piperidin-1-yl)-3-((4-(1-((1-(2-(2,6-dioxopiperidin-3-yl)-1,3-dioxoisoindolin-5-yl)piperidin-4-yl)methyl)piperidin-4-formyl )phenyl)amino)pyrazin-2-carboxamide

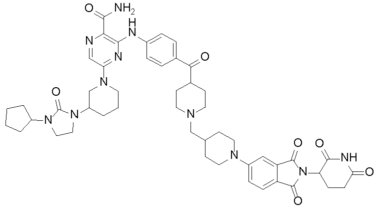

The synthesis method was the same as that for Example 1, except that Intermediate 63 was replaced with Intermediate 81, and Intermediate 133 was replaced with Intermediate 140 to obtain Compound 028. ESI-MS: *m*/*z* = 914 [M+H]⁺.

### Example 29. 5-(3-(3-cyclopentyl-2-oxoimidazolin-1-yl)piperidin-1-yl)-3-((4-(4-(3-(4-(2-(2,6-dioxopiperidin-3-yl)-1,3-dioxoisoindolin-5-yl)piperazin-1-yl)propan-1-yne-1-yl)pi peridin-1-yl)phenyl)amino)pyrazin-2-carboxamide

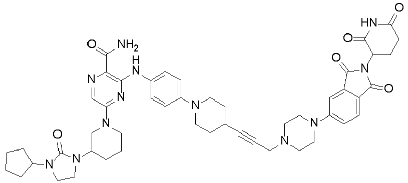

The synthesis step was the same as synthesis step 1 of Intermediate 63, except that compound Intermediate 9 was replaced with Intermediate 82 and Intermediate 52 was replaced with Intermediate 141 to obtain Compound 029. ESI-MS: *m*/*z* = 911 [M+H]⁺.

### Example 30. 5-(3-(3-cyclopentyl-2-oxoimidazolin-1-yl)piperidin-1-yl)-3-((2-((1-(2-(2,6-dioxopiperidin-3-yl)-1,3-dioxoisoindol-5-yl)piperidin-4-yl)methyl)-1,2,3,4-tetrahydrois oquinolin-6-yl)amino)pyrazin-2-carboxamide

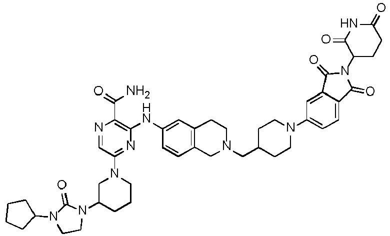

The synthesis method was the same as that for Example 1, except that Intermediate 63 was replaced with Intermediate 83, and Intermediate 133 was replaced with Intermediate 140 to obtain Compound 030. ESI-MS: *m*/*z* = 858 [M+H]⁺.

### Example 31. 3-((2-((1-(2-(2,6-dioxopiperidin-3-yl)-1,3-dioxoisoindol-5-yl)piperidin-4-yl)methyl)-1,2,3,4-tetrahydroisoquinolin-6-yl)amino)-5-(3-(3-(oxetan-3-yl)-2-oxoimidazoli n-1-yl)piperidin-1-yl)pyrazin-2-carboxamide

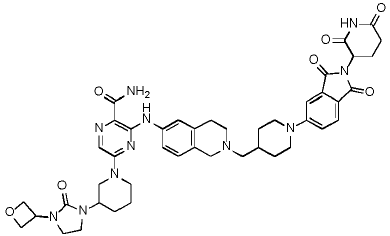

The synthesis method was the same as that for Example 1, except that Intermediate 63 was replaced with Intermediate 84, and Intermediate 133 was replaced with Intermediate 140 to obtain Compound 031. ESI-MS: *m*/*z* = 846 [M+H]⁺.

### Example 32. 3-((2-((1-(2-(2,6-dioxopiperidin-3-yl)-1,3-dioxoisoindol-5-yl)azetidine -3-yl)methyl)-1,2,3,4-tetrahydroisoquinolin-6-yl)amino)-5-(3-(3-(oxetan-3-yl)-2-oxyimidazol in-1-yl)piperidin-1-yl)pyrazin-2-carboxamide

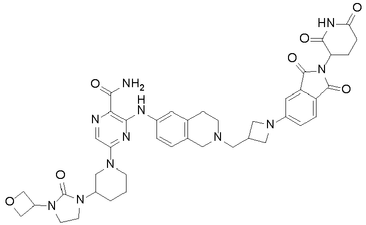

The synthesis method was the same as that for Example 31, except that Intermediate 140 was replaced with Intermediate 137 to obtain Compound 032. ESI-MS: *m*/*z* = 818 [M+H]⁺.

### Example 33. 5-(3-(3-cyclopentyl-2-oxoimidazolin-1-yl)piperidin-1-yl)-3-((2-((1-(2-(2,6-dioxopiperidin-3-yl)-1,3-dioxoisoindol-5-yl)azetidin-3-yl)methyl)-1,2,3,4-tetrahydroiso quinolin-6-yl)amino)pyrazin-2-carboxamide

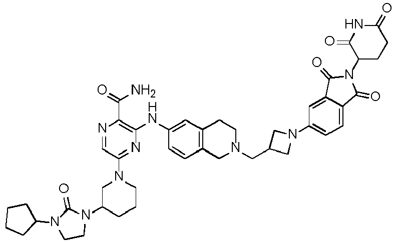

The synthesis method was the same as that for Example 1, except that Intermediate 63 was replaced with Intermediate 83, and Intermediate 133 was replaced with Intermediate 137 to obtain Compound 033. ESI-MS: *m*/*z* = 830 [M+H]⁺.

### Example 34. 5-(3-(3-cyclopentyl-2-oxoimidazolin-1-yl)piperidin-1-yl)-3-((4-(1-((1-(2-(2,6-dioxopiperidin-3-yl)-1,3-dioxoisoindolin-5-yl)pyrrolidin-3-yl)methyl)-4-methylpiperi din-4-yl)phenyl)amino)pyrazin-2-carboxamide

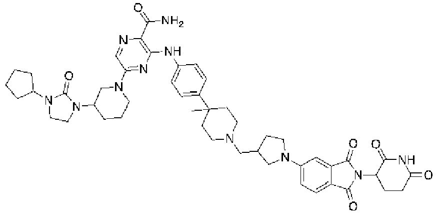

The synthesis method was the same as that for Example 1, except that Intermediate 133 was replaced with Intermediate 142 to obtain Compound 034. ESI-MS: *m*/*z* = 886 [M+H]⁺.

### Example 35. 5-((4-(1-((1-(2-(2,6-dioxopiperidin-3-yl)-1,3-dioxoisoindolin-5-yl) pyrrolidin-3-yl)methyl)-4-methylpiperidin-4-yl)phenyl)amino)-3-(3-(3-methyl-2-oxoimidaz olin-1-yl)piperidin-1-yl)-1,2,4-triazin-6-carboxamide

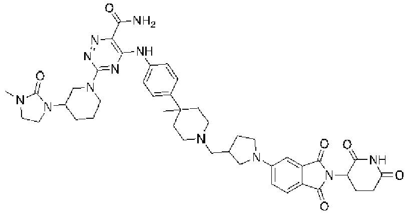

The synthesis method was the same as that for Example 34, except that Intermediate 63 was replaced with Intermediate 85 to obtain Compound 035. ESI-MS: *m*/*z* = 833 [M+H]⁺.

### Example 36. 4-((4-(1-((1-(2-(2,6-dioxopiperidin-3-yl)-1,3-dioxoisoindolin-5-yl) pyrrolidin-3-yl)methyl)-4-methylpiperidin-4-yl)phenyl)amino)-2-(3-(3-methyl-2-oxoimidaz olin-1-yl)piperidin-1-yl)pyrimidin-5-carboxamide

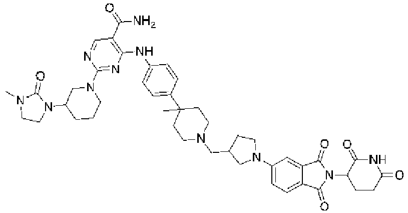

The synthesis method was the same as that for Example 34, except that Intermediate 63 was replaced with Intermediate 86 to obtain the compound. ESI-MS: *m*/*z* = 832 [M+H]⁺.

### Example 37. 2-((4-(1-((1-(2-(2,6-dioxopiperidin-3-yl)-1,3-dioxoisoindolin-5-yl) pyrrolidin-3-yl)methyl)-4-methylpiperidin-4-yl)phenyl)amino)-5-fluoro-6-(3-(3-methyl-2-ox oimidazolin-1-yl)piperidin-1-yl)nicotinamide

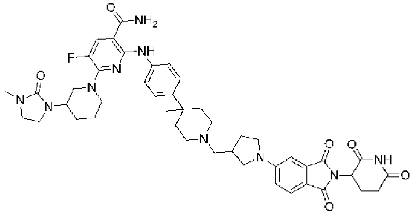

The synthesis method was the same as that for Example 34, except that Intermediate 63 was replaced with Intermediate 87 to obtain Compound 037. ESI-MS: *m*/*z* = 849 [M+H]⁺.

### Example 38. 2-((4-(1-((1-(2-(2,6-dioxopiperidin-3-yl)-1,3-dioxoisoindolin-5-yl) pyrrolidin-3-yl)methyl)-4-methylpiperidin-4-yl)phenyl)amino)-5-methoxy-6-(3-(3-methyl-2-oxoimidazolin-1-yl)piperidin-1-yl)nicotinamide

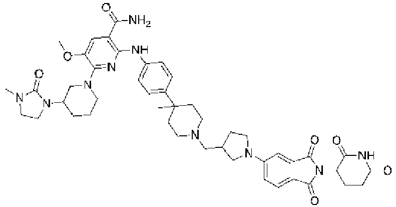

The synthesis method was the same as that for Example 34, except that Intermediate 63 was replaced with Intermediate 88 to obtain Compound 038. ESI-MS: *m*/*z* = 861 [M+H]⁺.

### Example 39. 5-(3-(3-cyclopentyl-2-oxoimidazolin-1-yl)piperidin-1-yl)-3-((4-(1-((1-(2-(2,6-dioxopiperidin-3-yl)-1,3-dioxoisoindolin-5-yl)pyrrolidin-3-yl)methyl)piperidin-4-yl)phe nyl)amino)pyrazin-2-carboxamide

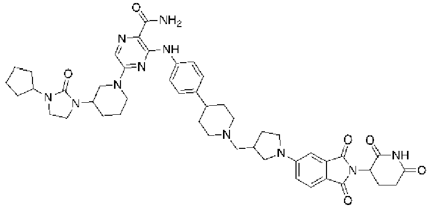

The synthesis method was the same as that for Example 34, except that Intermediate 63 was replaced with Intermediate 80 to obtain Compound 039. ESI-MS: *m*/*z* = 872 [M+H]⁺.

### Example 40. 5-(3-(3-cyclopentyl-2-oxoimidazolin-1-yl)piperidin-1-yl)-3-((4-(1-((1-(2-(2,6-dioxopiperidin-3-yl)-1,3-dioxoisoindolin-5-yl)pyrrolidin-3-yl)methyl)-4-fluoropiperidin -4-yl)phenyl)amino)pyrazin-2-carboxamide

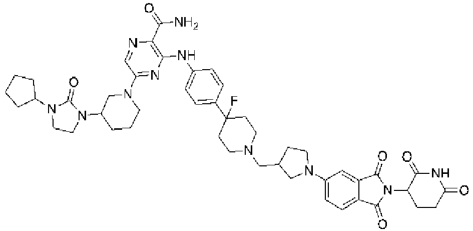

The synthesis method was the same as that for Example 34, except that Intermediate 63 was replaced with Intermediate 89 to obtain Compound 040. ESI-MS: *m*/*z* = 890 [M+H]⁺.

### Example 41. 5-(3-(3-cyclopentyl-2-oxoimidazolin-1-yl)piperidin-1-yl)-3-((4-(1-((1-(2-(2,6-dioxopiperidin-3-yl)-1,3-dioxoisoindolin-5-yl)pyrrolidin-3-yl)methyl)piperidin-4-yl)-2-f luorophenyl)amino)pyrazin-2-carboxamide

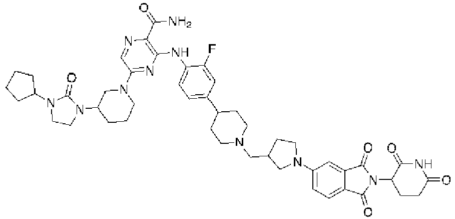

The synthesis method was the same as that for Example 34, except that Intermediate 63 was replaced with Intermediate 90 to obtain Compound 041. ESI-MS: *m*/*z* = 890 [M+H]⁺.

### Example 42. 3-((3-cyano-4-(1-((1-(2-(2,6-dioxopiperidin-3-yl)-1,3-dioxoisoindolin-5 -yl)pyrrolidin-3-yl)methyl)piperidin-4-yl)phenyl)amino)-5-(3-(3-cyclopentyl-2-oxoimidazoli n-1-yl)piperidin-1-yl)pyrazin-2-carboxamide

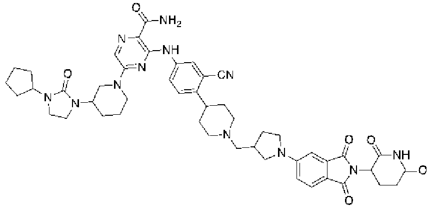

The synthesis method was the same as that for Example 34, except that Intermediate 63 was replaced with Intermediate 91 to obtain Compound 042. ESI-MS: *m*/*z* = 897 [M+H]⁺.

### Example 43. 5-(3-(3-cyclopentyl-2-oxoimidazolin-1-yl)piperidin-1-yl)-3-((4-(1-((1-(2-(2,6-dioxopiperidin-3-yl)-1,3-dioxoisoindolin-5-yl)pyrrolidin-3-yl)methyl)-3-fluoropiperidin -4-yl)phenyl)amino)pyrazin-2-carboxamide

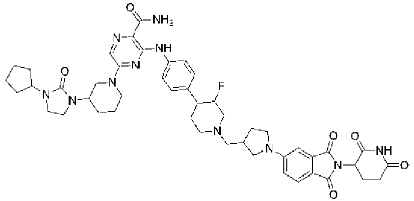

The synthesis method was the same as that for Example 34, except that Intermediate 63 was replaced with Intermediate 92 to obtain Compound 043. ESI-MS: *m*/*z* = 890 [M+H]⁺.

### Example 44. 3-((4-(1-((1-(2-(2,6-dioxopiperidin-3-yl)-1,3-dioxoisoindolin-5-yl) pyrrolidin-3-yl)methyl)piperidin-4-yl)phenyl)amino)-5-(3-(4-methyl-5-oxo-4,6-diazaspiro[2. 4]cycloheptan-6-yl)piperidin-1-yl)pyrazin-2-carboxamide

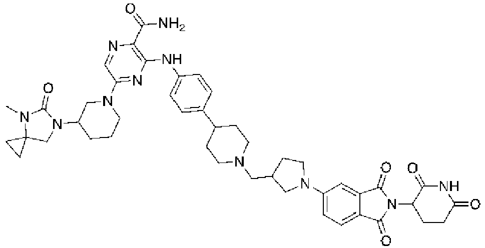

The synthesis method was the same as that for Example 34, except that Intermediate 63 was replaced with Intermediate 93 to obtain Compound 044. ESI-MS: *m*/*z* = 844 [M+H]⁺.

### Example 45. 3-((4-(1-((1-(2-(2,6-dioxopiperidin-3-yl)-1,3-dioxoisoindolin-5-yl) pyrrolidin-3-yl)methyl)piperidin-4-yl)phenyl)amino)-5-(3-(3-oxohexahydroimidazo[1,5-a]p yridin-2(3H)-yl)piperidin-1-yl)pyrazin-2-carboxamide

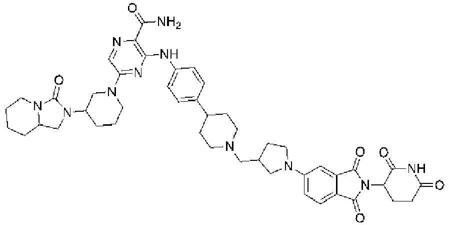

The synthesis method was the same as that for Example 34, except that Intermediate 63 was replaced with Intermediate 94 to obtain Compound 045, ESI-MS: *m*/*z* = 858 [M+H]⁺.

### Example 46. 3-((4-(1-(1-(2-(2,6-dioxopiperidin-3-yl)-1,3-dioxoisoindolin-5-yl) azetidin-3-yl)piperidin-4-yl)phenyl)amino)-5-(-6-(3-methyl-2-oxoimidazolin-1-yl)-2-azabicy clo[2.2.1]heptan-2-yl)pyrazin-2-carboxamide

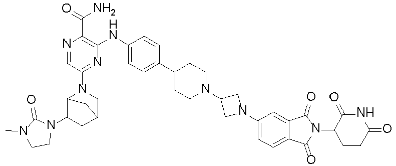

The synthesis method was the same as that for Example 1, except that Intermediate 63 was replaced with Intermediate 95 to obtain Compound 046, ESI-MS: *m*/*z* = 802 [M+H]⁺.

### Example 47. 3-((4-(1-(1-(2-(2,6-dioxopiperidin-3-yl)-1,3-dioxoisoindolin-5-yl) azetidin-3-yl)piperidin-4-yl)phenyl)amino)-5-(-6-(3-isopropyl-2-oxoimidazolin-1-yl)-2-azabi cyclo[2.2.1]heptan-2-yl)pyrazin-2-carboxamide

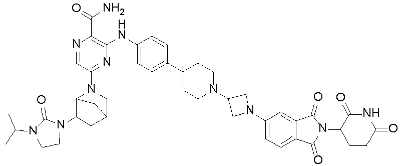

The synthesis method was the same as that for Example 1, except that Intermediate 63 was replaced with Intermediate 96 to obtain Compound 047, ESI-MS: *m*/*z* = 830 [M+H]⁺.

### Example 48. 2-((4-(1-(1-(2-(2,6-dioxopiperidin-3-yl)-1,3-dioxoisoindolin-5-yl) azetidin-3-yl)piperidin-4-yl)phenyl)amino)-6-(-6-(3-methyl-2-oxoimidazolin-1-yl)-2-azabicy clo[2.2.1]heptan-2-yl)nicotinamide

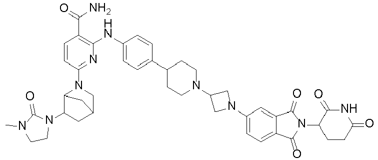

The synthesis method was the same as that for Example 1, except that Intermediate 63 was replaced with Intermediate 97 to obtain Compound 048, ESI-MS: *m*/*z* = 801 [M+H]⁺.

### Example 49. 5-((4-(1-(1-(2-(2,6-dioxopiperidin-3-yl)-1,3-dioxoisoindolin-5-yl) azetidin-3-yl)piperidin-4-yl)phenyl)amino)-3-(-6-(3-methyl-2-oxoimidazolin-1-yl)-2-azabicy clo[2.2.1]heptan-2-yl)-1,2,4-triazin-6-carboxamide

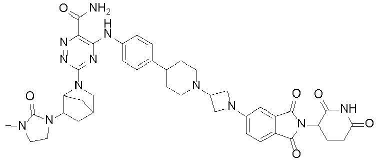

The synthesis method was the same as that for Example 1, except that Intermediate 63 was replaced with Intermediate 98 to obtain Compound 049, ESI-MS: *m*/*z* = 803 [M+H]⁺.

### Example 50. 3-((4-(1-(1-(2-(2,6-dioxopiperidin-3-yl)-1,3-dioxoisoindolin-5-yl) azetidin-3-yl)piperidin-4-yl)phenyl)amino)-5-(-6-(3-methyl-2-oxoimidazolin-1-yl)-2-azabicy clo[2.2.2]octan-2-yl)pyrazin-2-carboxamide

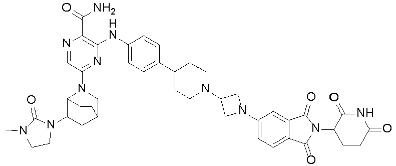

The synthesis method was the same as that for Example 1, except that Intermediate 63 was replaced with Intermediate 99 to obtain Compound 050, ESI-MS: *m*/*z* = 816 [M+H]⁺.

### Example 51. 5-((4-(1-(1-(2-(2,6-dioxopiperidin-3-yl)-6-fluoro-1,3-dioxoisoindolin-5-yl) azetidin-3-yl)piperidin-4-yl)phenyl)amino)-3-(-6-(3-methyl-2-oxoimidazolin-1-yl)-2-azabicy clo[2.2.1]heptan-2-yl)-1,2,4-triazin-6-carboxamide

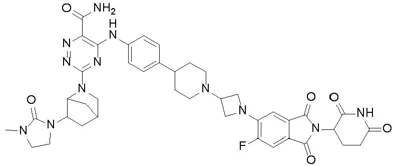

The synthesis method was the same as that for Example 49, except that Intermediate 133 was replaced with Intermediate 143 to obtain Compound 051, ESI-MS: *m*/*z* = 821 [M+H]⁺.

### Example 52. 5-(3-(3-cyclopentyl-2-oxoimidazolin-1-yl)piperidin-1-yl)-3-((4-(1-((1-(2-(2,6-dioxopiperidin-3-yl)-6-fluoro-1,3-dioxoisoindolin-5-yl)pyrrolidin-3-yl)methyl)piperidin -4-yl)phenyl)amino)pyrazin-2-carboxamide

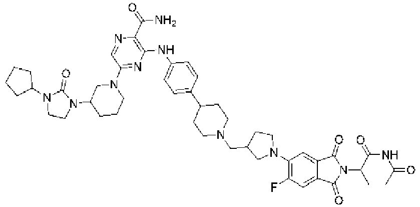

The synthesis method was the same as that for Example 1, except that Intermediate 63 was replaced with Intermediate 80, and Intermediate 133 was replaced with Intermediate 144 to obtain Compound 052, ESI-MS: *m*/*z* =890 [M+H]⁺.

### Example 53. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(3-methyl-2-oxoimidazolin-1-yl)pi peridin-1-yl)-1,2,4-triazin-6-carboxamide

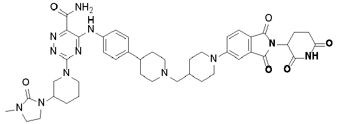

The synthesis method was the same as that for Example 1, except that Intermediate 63 was replaced with Intermediate 100, and Intermediate 133 was replaced with Intermediate 140 to obtain Compound 053, ESI-MS: *m*/*z* =833 [M+H]⁺.

### Example 54. 5-((4-(1-((1-(2-(2,6-dioxopiperidin-3-yl)-1,3-dioxoisooctyl-5-yl) piperidin-4-yl)methyl)piperidin-4-yl)phenyl)amino)-3-(3-(3-ethyl-2-oxyimidazolin-1-yl)pipe ridin-1-yl)-1,2,4-triazin-6-carboxamide

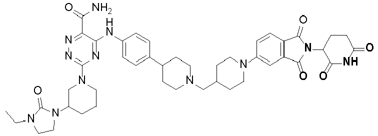

The synthesis method was the same as that for Example 1, except that Intermediate 63 was replaced with Intermediate 101, and Intermediate 133 was replaced with Intermediate 140 to obtain Compound 054, ESI-MS: *m*/*z* =847 [M+H]⁺.

### Example 55. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(3-isopropyl-2-oxoimidazolin-1-yl) piperidin-1-yl)-1,2,4-triazin-6-carboxamide

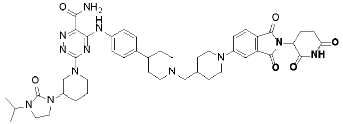

The synthesis method was the same as that for Example 1, except that Intermediate 63 was replaced with Intermediate 102, and Intermediate 133 was replaced with Intermediate 140 to obtain Compound 055, ESI-MS: *m*/*z* =861 [M+H]⁺.

### Example 56. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(2-oxo-3-(trifluoromethyl)imidazo lin-1-yl)piperidin-1-yl)-1,2,4-triazin-6-carboxamide

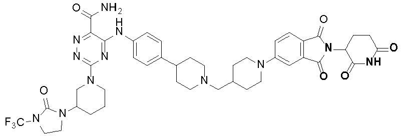

The synthesis method was the same as that for Example 1, except that Intermediate 63 was replaced with Intermediate 103, and Intermediate 133 was replaced with Intermediate 140 to obtain Compound 056, ESI-MS: *m*/*z* =887 [M+H]⁺.

### Example 57. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(2-oxo-3-(2,2,2-trifluoroethyl)imid azolin-1-yl)piperidin-1-yl)-1,2,4-triazin-6-carboxamide

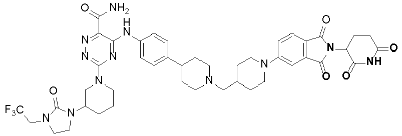

The synthesis method was the same as that for Example 1, except that Intermediate 63 was replaced with Intermediate 104, and Intermediate 133 was replaced with Intermediate 140 to obtain Compound 057, ESI-MS: *m*/*z* =901 [M+H]⁺.

### Example 58. 3-(3-(3-cyclopropyl-2-oxoimidazolin-1-yl)piperidin-1-yl)-5-((4-(1-((1-(2-(2,6-dioxopiperidin-3-yl)-1,3-dioxoisoindol-5-yl)piperidin-4-yl)methyl)piperidin-4-yl)phenyl )amino)-1,2,4-triazin-6-carboxamide

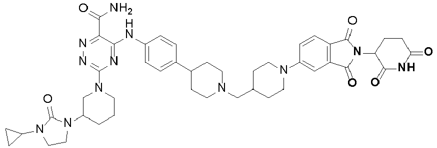

The synthesis method was the same as that for Example 1, except that Intermediate 63 was replaced with Intermediate 105, and Intermediate 133 was replaced with Intermediate 140 to obtain Compound 058, ESI-MS: *m*/*z* =859 [M+H]⁺.

### Example 59. 3-(3-(3-cyclobutyl-2-oxoimidazolin-1-yl)piperidin-1-yl)-5-((4-(1-((1-(2-(2,6-dioxopiperidin-3-yl)-1,3-dioxoisoindol-5-yl)piperidin-4-yl)methyl)piperidin-4-yl)phenyl )amino)-1,2,4-triazin-6-carboxamide

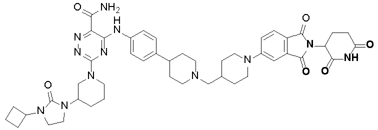

The synthesis method was the same as that for Example 1, except that Intermediate 63 was replaced with Intermediate 106, and Intermediate 133 was replaced with Intermediate 140 to obtain Compound 059, ESI-MS: *m*/*z* =873 [M+H]⁺.

### Example 60. 3-(3-(3-cyclopentyl-2-oxoimidazolin-1-yl)piperidin-1-yl)-5-((4-(1-((1-(2-(2,6-dioxopiperidin-3-yl)-1,3-dioxoisoindol-5-yl)piperidin-4-yl)methyl)piperidin-4-yl)phenyl )amino)-1,2,4-triazin-6-carboxamide

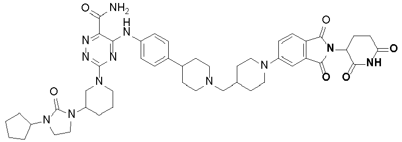

The synthesis method was the same as that for Example 1, except that Intermediate 63 was replaced with Intermediate 107, and Intermediate 133 was replaced with Intermediate 140 to obtain Compound 060, ESI-MS: *m*/*z* =887 [M+H]⁺.

### Example 61. 3-(3-(3-cyclohexyl-2-oxoimidazolin-1-yl)piperidin-1-yl)-5-((4-(1-((1-(2-(2,6-dioxopiperidin-3-yl)-1,3-dioxoisoindol-5-yl)piperidin-4-yl)methyl)piperidin-4-yl)phenyl )amino)-1,2,4-triazin-6-carboxamide

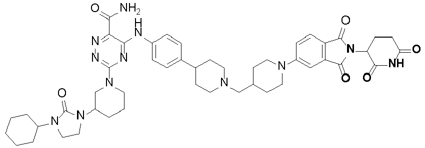

The synthesis method was the same as that for Example 1, except that Intermediate 63 was replaced with Intermediate 108, and Intermediate 133 was replaced with Intermediate 140 to obtain Compound 061, ESI-MS: *m*/*z* =901 [M+H]⁺.

### Example 62. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(3-(oxetan-3-yl)-2-oxoimidazolin-1 -yl)piperidin-1-yl)-1,2,4-triazin-6-carboxamide

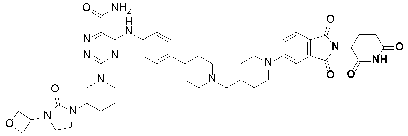

The synthesis method was the same as that for Example 1, except that Intermediate 63 was replaced with Intermediate 109, and Intermediate 133 was replaced with Intermediate 140 to obtain Compound 062, ESI-MS: *m*/*z* =875 [M+H]⁺.

### Example 63. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(2-oxo-3-(tetrahydro-2H-pyran-4-yl)imidazolin-1-yl)piperidin-1-yl)-1,2,4-triazin-6-carboxamide

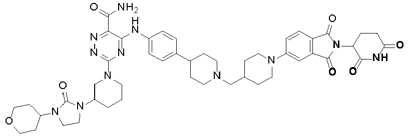

The synthesis method was the same as that for Example 1, except that Intermediate 63 was replaced with Intermediate 110, and Intermediate 133 was replaced with Intermediate 140 to obtain Compound 063, ESI-MS: *m*/*z* =903 [M+H]⁺.

### Example 64. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(2-oxo-3-phenylimidazolin-1-yl)pi peridin-1-yl)-1,2,4-triazin-6-carboxamide

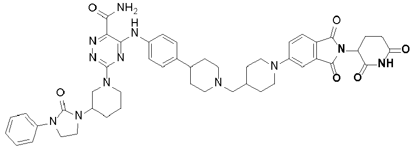

The synthesis method was the same as that for Example 1, except that Intermediate 63 was replaced with Intermediate 111, and Intermediate 133 was replaced with Intermediate 140 to obtain Compound 064, ESI-MS: *m*/*z* =895 [M+H]⁺.

### Example 65. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(3-(2-fluorophenyl)-2-oxoimidazol in-1-yl)piperidin-1-yl)-1,2,4-triazin-6-carboxamide

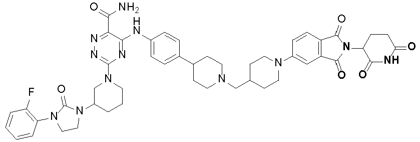

The synthesis method was the same as that for Example 1, except that Intermediate 63 was replaced with Intermediate 112, and Intermediate 133 was replaced with Intermediate 140 to obtain Compound 065, ESI-MS: *m*/*z* =913 [M+H]⁺.

### Example 66. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(3-(3-fluorophenyl)-2-oxoimidazol in-1-yl)piperidin-1-yl)-1,2,4-triazin-6-carboxamide

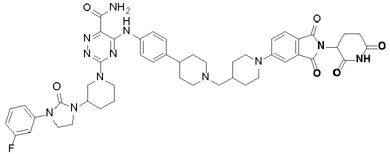

The synthesis method was the same as that for Example 1, except that Intermediate 63 was replaced with Intermediate 113, and Intermediate 133 was replaced with Intermediate 140 to obtain Compound 066, ESI-MS: *m*/*z* =913 [M+H]⁺.

### Example 67. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(2-oxo-3-(pyridin-3-yl)imidazolin-1-yl)piperidin-1-yl)-1,2,4-triazin-6-carboxamide

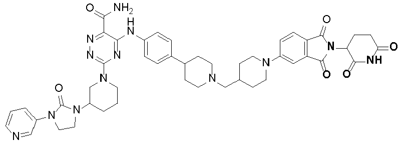

The synthesis method was the same as that for Example 1, except that Intermediate 63 was replaced with Intermediate 114, and Intermediate 133 was replaced with Intermediate 140 to obtain Compound 067, ESI-MS: *m*/*z* =896 [M+H]⁺.

### Example 68. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(4-methyl-5-oxo-4,6-diazaspirocyc lo[2.4]heptan-6-yl)piperidin-1-yl)-1,2,4-triazin-6-carboxamide

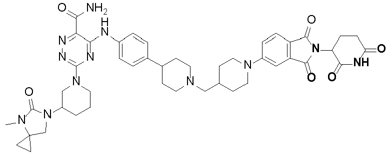

The synthesis method was the same as that for Example 1, except that Intermediate 63 was replaced with Intermediate 115, and Intermediate 133 was replaced with Intermediate 140 to obtain Compound 068, ESI-MS: *m*/*z* =859 [M+H]⁺.

### Example 69. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(5-methyl-6-oxo-5,7-diazaspirocyc lo[3.4]octan-7-yl)piperidin-1-yl)-1,2,4-triazin-6-carboxamide

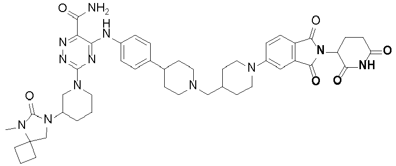

The synthesis method was the same as that for Example 1, except that Intermediate 63 was replaced with Intermediate 116, and Intermediate 133 was replaced with Intermediate 140 to obtain Compound 069, ESI-MS: *m*/*z* =873 [M+H]⁺.

### Example 70. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(3-(3-oxytrihydro-1H-pyrrolo[1,2-c]i midazol-2(3H)-yl)piperidin-1-yl)-1,2,4-triazin-6-carboxamide

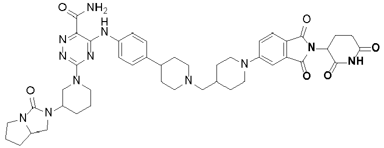

The synthesis method was the same as that for Example 1, except that Intermediate 63 was replaced with Intermediate 117, and Intermediate 133 was replaced with Intermediate 140 to obtain Compound 070, ESI-MS: *m*/*z* =859 [M+H]⁺.

### Example 71. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(6-(3-methyl-2-oxoimidazolin-1-yl)-2 -azabicyclo[2.2.1]heptan-2-yl)-1,2,4-triazin-6-carboxamide

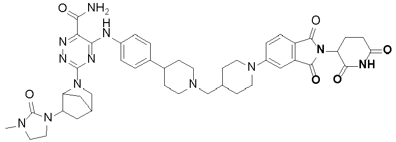

The synthesis method was the same as that for Example 1, except that Intermediate 63 was replaced with Intermediate 98, and Intermediate 133 was replaced with Intermediate 140 to obtain Compound 071, ESI-MS: *m*/*z* =845 [M+H]⁺.

### Example 72. 3-(6-(3-cyclopentyl-2-oxoimidazolin-1-yl)-2-azabicyclo[2.2.1]heptan-2 -yl)-5-((4-(1-((1-(2-(2,6-dioxopiperidin-3-yl)-1,3-dioxoisoindol-5-yl)piperidin-4-yl)methyl)pi peridin-4-yl)phenyl)amino)-1,2,4-triazin-6-carboxamide

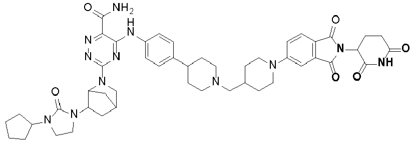

The synthesis method was the same as that for Example 1, except that Intermediate 63 was replaced with Intermediate 118, and Intermediate 133 was replaced with Intermediate 140 to obtain Compound 072, ESI-MS: *m*/*z* =899 [M+H]⁺.

### Example 73. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(6-(2-oxo-3-phenylimidazolin-1-yl)-2-azabicyclo[2.2.1]heptan-2-yl)-1,2,4-triazin-6-carboxamide

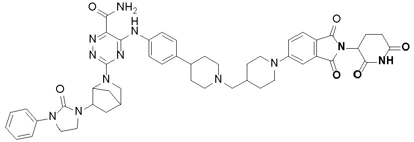

The synthesis method was the same as that for Example 1, except that Intermediate 63 was replaced with Intermediate 119, and Intermediate 133 was replaced with Intermediate 140 to obtain Compound 073, ESI-MS: *m*/*z* =907 [M+H]⁺.

### Example 74. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(6-(3-oxotetrahydro-1H-pyrrolo[1,2-c] imidazol-2(3H)-yl)-2-azabicyclo [2.2.1] heptan-2-yl)-1,2,4-triazin-6-carboxamide

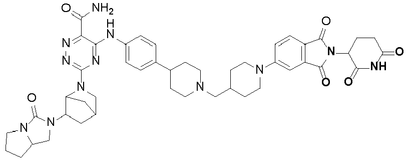

The synthesis method was the same as that for Example 1, except that Intermediate 63 was replaced with Intermediate 120, and Intermediate 133 was replaced with Intermediate 140 to obtain Compound 074, ESI-MS: *m*/*z* =871 [M+H]⁺.

### Example 75. 5-((4-(1-((1-(2-(2,6-dioxopiperidin-3-yl)-1,3-dioxoisoindol-5-yl) piperidin-4-yl)methyl)piperidin-4-yl)phenyl)amino)-3-(6-(3-methyl-2-oxoimidazolin-1-yl)-2 -azabicyclo[2.2.2]octan-2-yl)-1,2,4-triazin-6-carboxamide

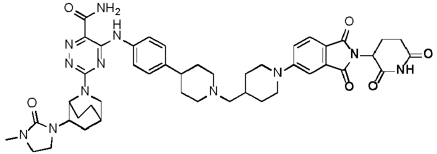

The synthesis method was the same as that for Example 1, except that Intermediate 63 was replaced with Intermediate 121, and Intermediate 133 was replaced with Intermediate 140 to obtain Compound 075, ESI-MS: *m*/*z* =859 [M+H]⁺.

### Example 76. 5-((4-(1-((1-(2-(2,6-dioxopiperidin-3-yl)-1,3-dioxoisooctyl-5-yl) pyrrolidin-3-yl)methyl)piperidin-4-yl)phenyl)amino)-3-(3-(3-methyl-2-oxoimidazolin-1-yl)p iperidin-1-yl)-1,2,4-triazin-6-carboxamide

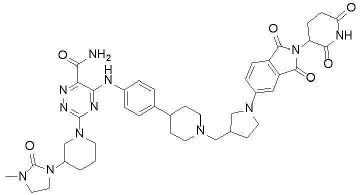

The synthesis method was the same as that for Example 1, except that Intermediate 63 was replaced with Intermediate 100, and Intermediate 133 was replaced with Intermediate 142 to obtain Compound 076, ESI-MS: *m*/*z* =819 [M+H]⁺.

### Example 77. 5-((4-(1-(1-(2-(2,6-dioxopiperidin-3-yl)-1,3-dioxoisoindol-5-yl) azetidin-3-yl)piperidin-4-yl)phenyl)amino)-3-(3-(3-methyl-2-oxoimidazolin-1-yl)piperidin-1 -yl)-1,2,4-triazin-6-carboxamide

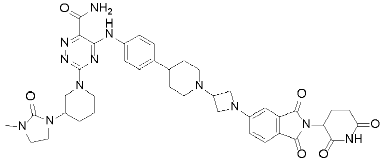

The synthesis method was the same as that for Example 1, except that Intermediate 63 was replaced with Intermediate 100 to obtain Compound 077, ESI-MS: *m*/*z* = 791 [M+H]⁺.

### Example 78. 5-((4-(1'-(2-(2,6-dioxopiperidin-3-yl)-1,3-dioxoisoindol-5-yl)-4-methyl-[1,4'-bipiperidin]-4-yl)phenyl)amino)-3-(3-(3-methyl-2-oxoimidazolidin-1-yl)piperidin-1-yl) -1,2,4-triazin-6-carboxamide

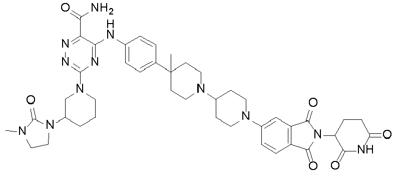

The synthesis method was the same as that for Example 1, except that Intermediate 63 was replaced with Intermediate 85, and Intermediate 133 was replaced with Intermediate 134 to obtain Compound 078, ESI-MS: *m*/*z* =833 [M+H]⁺.

### Example 79. 5-((4-(1-(1-(2-(2,6-dioxopiperidin-3-yl)-1,3-dioxoisoindol-5-yl)azetidin-3 -yl)-4-methylpiperidin-4-yl)phenyl)amino)-3-(3-(3-methyl-2-oxoimidazolin-1-yl)piperidin-1 -yl)-1,2,4-triazin-6-carboxamide

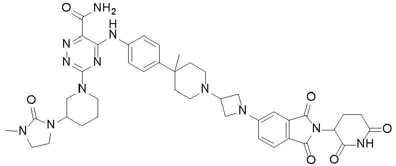

The synthesis method was the same as that for Example 1, except that Intermediate 63 was replaced with Intermediate 85 to obtain Compound 079, ESI-MS: *m*/*z* = 805 [M+H]⁺.

### Example 80. 5-((2-(1-(2,6-dioxopiperidin-3-yl)-1,3-dioxoisoindol-5-yl)azetidin-3-yl) -1,2,3,4-tetrahydroisoquinolin-6-yl)amino)-3-(3-(3-methyl-2-oxoimidazolin-1-yl)piperidin-1 -yl)-1,2,4-triazin-6-carboxamide

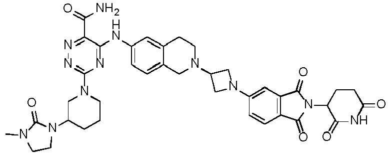

The synthesis method was the same as that for Example 1, except that Intermediate 63 was replaced with Intermediate 122 to obtain Compound 080, ESI-MS: *m*/*z* = 763 [M+H]⁺.

### Example 81. 5-((2-((1-(2-(2,6-dioxopiperidin-3-yl)-1,3-dioxoisoindol-5-yl)piperidin-4 -yl)methyl)-1,2,3,4-tetrahydroisoquinolin-6-yl)amino)-3-(3-(3-methyl-2-oxoimidazolin-1-yl) piperidin-1-yl)-1,2,4-triazin-6-carboxamide

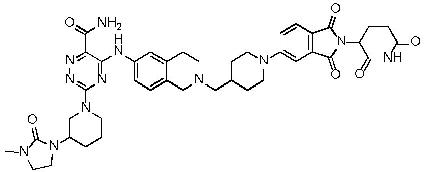

The synthesis method was the same as that for Example 1, except that Intermediate 63 was replaced with Intermediate 122, and Intermediate 133 was replaced with Intermediate 140 to obtain Compound 081, ESI-MS: *m*/*z* =805 [M+H]⁺.

### Example 82. 5-((4-(4-((1-(2-(2,6-dioxopiperidin-3-yl)-1,3-dioxoisoindol-5-yl) piperidin-4-yl)methyl)piperazin-1-yl)phenyl)amino)-3-(3-(3-methyl-2-oxoimidazolin-1-yl)pi peridin-1-yl)-1,2,4-triazin-6-carboxamide

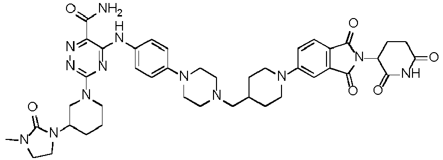

The synthesis method was the same as that for Example 1, except that Intermediate 63 was replaced with Intermediate 123, and Intermediate 133 was replaced with Intermediate 140 to obtain Compound 082, ESI-MS: *m*/*z* =834 [M+H]⁺.

### Example 83. 5-((4-(1'-(2-(2,6-dioxopiperidin-3-yl)-1,3-dioxoisoindol-5-yl)-[1,4'-bipiperidin]-4-yl)phenyl)amino)-3-(3-(3-methyl-2-oxoimidazolin-1-yl)piperidin-1-yl)-1 ,2,4-triazin-6-carboxamide

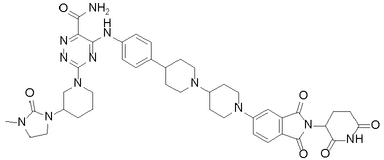

The synthesis method was the same as that for Example 1, except that Intermediate 63 was replaced with Intermediate 100, and Intermediate 133 was replaced with Intermediate 134 to obtain Compound 083, ESI-MS: *m*/*z* =819 [M+H]⁺.

### Example 84. 5-((4-(4-(3-(2-(2,6-dioxopiperidin-3-yl)-1-oxoisoindol-5-yl)propan-2-yn -1-yl)piperazin-1-yl)piperidin-1-yl)phenyl)amino)-3-(3-(3-methyl-2-oxoimidazolin-1-yl)pipe ridin-1-yl)-1,2,4-triazin-6-carboxamide

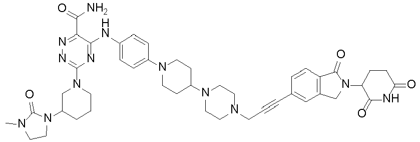

The synthesis method was the same as that for Example 1, except that Intermediate 63 was replaced with Intermediate 124, and Intermediate 133 was replaced with Intermediate 145 to obtain Compound 084, ESI-MS: *m*/*z* =844 [M+H]⁺.

### Example 85. 5-((4-(1-((1-(2-(2,6-dioxopiperidin-3-yl)-6-fluoro-1,3-dioxoisoindol-5-yl) piperidin-4-yl)methyl)piperidin-4-yl)phenyl)amino)-3-(3-(3-methyl-2-oxoimidazolin-1-yl)pi peridin-1-yl)-1,2,4-triazin-6-carboxamide

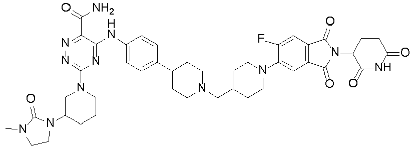

The synthesis method was the same as that for Example 1, except that Intermediate 63 was replaced with Intermediate 100, and Intermediate 133 was replaced with Intermediate 147 to obtain Compound 085, ESI-MS: *m*/*z* =851 [M+H]⁺.

### Example 86. 5-((4-(1-((1-(2-(2,6-dioxopiperidin-3-yl)-3-oxoisoindol-5-yl)piperidin-4 -yl)methyl)piperidin-4-yl)phenyl)amino)-3-(3-(3-methyl-2-oxoimidazolin-1-yl)piperidin-1-yl )-1,2,4-triazin-6-carboxamide

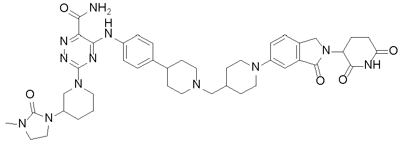

The synthesis method was the same as that for Example 1, except that Intermediate 63 was replaced with Intermediate 100, and Intermediate 133 was replaced with Intermediate 146 to obtain Compound 086, ESI-MS: *m*/*z* =819 [M+H]⁺.

### Example 87. 5-((4-(1-((1-(2-(2,6-dioxopiperidin-3-yl)-1-oxoisooctyl-5-yl)piperidin-4 -yl)methyl)piperidin-4-yl)phenyl)amino)-3-(3-(3-methyl-2-oxyimidazolin-1-yl)piperidin-1-yl )-1,2,4-triazin-6-carboxamide

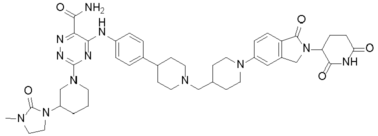

The synthesis method was the same as that for Example 1, except that Intermediate 63 was replaced with Intermediate 100, and Intermediate 133 was replaced with Intermediate 148 to obtain Compound 087, ESI-MS: *m*/*z* =819 [M+H]⁺.

### Example 88. 5-((4-(1-((1-(1-(2,6-dioxopiperidin-3-yl)-3-methyl-2-oxo-2,3-dihydro -1H-benzo[d]imidazol-5-yl)piperidin-4-yl)methyl)piperidin-4-yl)phenyl)amino)-3-(3-(3-met hyl-2-oxoimidazolin-1-yl)piperidin-1-yl)-1,2,4-triazin-6-carboxamide

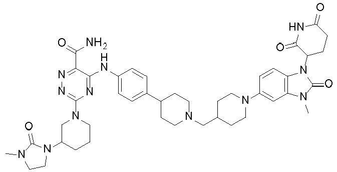

The synthesis method was the same as that for Example 1, except that Intermediate 63 was replaced with Intermediate 100, and Intermediate 133 was replaced with 1-(1-(2,6-dioxopiperidin-3-yl)-3-methyl-2 -oxo-2,3-dihydro-1H-benzo[d]imidazol-5-yl)piperidin -4-aminocarbaldehyde to obtain Compound 088, ESI-MS: *m*/*z* =834 [M+H]⁺.

### Example 89. 5-((4-(1-((1-(2-(2,6-dioxopiperidin-3-yl)-1,3-dioxoisoindol-5-yl) pyrrolidin-3-yl)methyl)piperidin-4-yl)phenyl)amino)-3-(3-(2-oxo-3-phenylimidazolin-1-yl)p iperidin-1-yl)-1,2,4-triazin-6-carboxamide

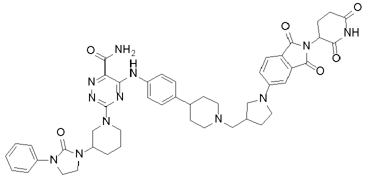

The synthesis method was the same as that for Example 1, except that Intermediate 63 was replaced with Intermediate 111, and Intermediate 133 was replaced with Intermediate 142 to obtain Compound 089, ESI-MS: *m*/*z* =881 [M+H]⁺.

### Example 90. 5-((4-(1-(1-(2-(2,6-dioxopiperidin-3-yl)-1,3-dioxoisoindol-5-yl)azetidin-3 -yl)piperidin-4-yl)phenyl)amino)-3-(3-(2-oxo-3-phenylimidazolin-1-yl)piperidin-1-yl)-1,2,4-t riazin-6-carboxamide

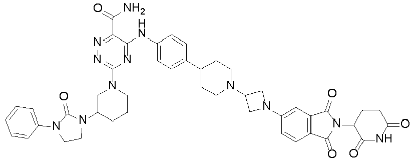

The synthesis method was the same as that for Example 1, except that Intermediate 63 was replaced with Intermediate 111 to obtain Compound 090, ESI-MS: *m*/*z* = 853 [M+H]⁺.

### Example 91. 5-((4-(1'-(2-(2,6-dioxopiperidin-3-yl)-1,3-dioxoisoindol-5-yl)-4-methyl-[1,4'-bipiperidin]-4-yl)phenyl)amino)-3-(3-(2-oxo-3-phenylimidazolin-1-yl)piperidin-1-yl)-1, 2,4-triazin-6-carboxamide

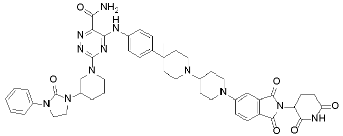

The synthesis method was the same as that for Example 1, except that Intermediate 63 was replaced with Intermediate 125, and Intermediate 133 was replaced with Intermediate 134 to obtain Compound 091, ESI-MS: *m*/*z* =895 [M+H]⁺.

### Example 92. 5-((4-(1-(1-(2-(2,6-dioxopiperidin-3-yl)-1,3-dioxoisoindol-5-yl)azetidine -3-yl)-4-methylpiperidin-4-yl)phenyl)amino)-3-(3-(2-oxo-3-phenylimidazolin-1-yl)piperidin-1-yl)-1,2,4-triazin-6-carboxamide

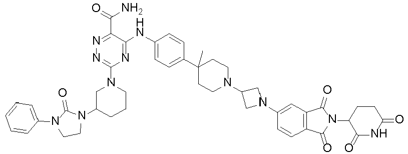

The synthesis method was the same as that for Example 1, except that Intermediate 63 was replaced with Intermediate 125 to obtain Compound 092, ESI-MS: *m*/*z* = 867 [M+H]⁺.

### Example 93. 5-((2-(1-(2,6-dioxopiperidin-3-yl)-1,3-dioxoisoindol-5-yl)azetidin-3-yl) -1,2,3,4-tetrahydroisoquinolin-6-yl)amino)-3-(3-(2-oxo-3-phenylimidazolin-1-yl)piperidin-1-yl)-1,2,4-triazin-6-carboxamide

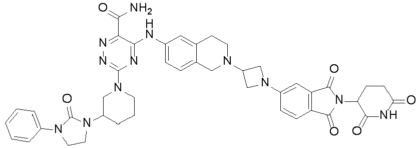

The synthesis method was the same as that for Example 1, except that Intermediate 63 was replaced with Intermediate 126 to obtain Compound 093, ESI-MS: *m*/*z* = 825 [M+H]⁺.

### Example 94. 5-((2-((1-(2-(2,6-dioxopiperidin-3-yl)-1,3-dioxoisoindol-5-yl)piperidin-4 -yl)methyl)-1,2,3,4-tetrahydroisoquinolin-6-yl)amino)-3-(3-(2-oxo-3-phenylimidazolin-1-yl) piperidin-1-yl)-1,2,4-triazin-6-carboxamide

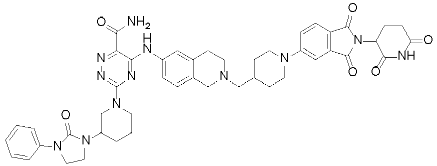

The synthesis method was the same as that for Example 1, except that Intermediate 63 was replaced with Intermediate 126, and Intermediate 133 was replaced with Intermediate 140 to obtain Compound 094, ESI-MS: *m*/*z* =867 [M+H]⁺.

### Example 95. 5-((4-(4-((1-(2-(2,6-dioxopiperidin-3-yl)-1,3-dioxoisoindol-5-yl) piperidin-4-yl)methyl)piperazin-1-yl)phenyl)amino)-3-(3-(2-oxo-3-phenylimidazolin-1-yl)pi peridin-1-yl)-1,2,4-triazin-6-carboxamide

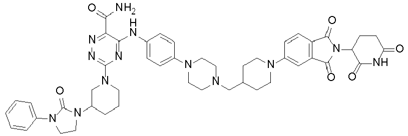

The synthesis method was the same as that for Example 1, except that Intermediate 63 was replaced with Intermediate 127, and Intermediate 133 was replaced with Intermediate 140 to obtain Compound 095, ESI-MS: *m*/*z* =896 [M+H]⁺.

### Example 96. 5-((4-(1'-(2-(2,6-dioxopiperidin-3-yl)-1,3-dioxoisoindol-5-yl)-[1,4'-bipiperidin]-4-yl)phenyl)amino)-3-(3-(2-oxo-3-phenylimidazolin-1-yl)piperidin-1-yl)-1, 2,4-triazin-6-carboxamide

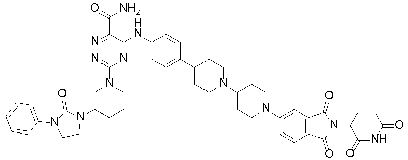

The synthesis method was the same as that for Example 1, except that Intermediate 63 was replaced with Intermediate 111, and Intermediate 133 was replaced with Intermediate 134 to obtain Compound 096, ESI-MS: *m*/*z* =881 [M+H]⁺.

### Example 97. 5-((4-(4-(3-(2-(2,6-dioxopiperidin-3-yl)-1-oxoisoquinolin-5-yl) propan-2-yn-1-yl)piperazin-1-yl)piperidin-1-yl)phenyl)amino)-3-(3-(2-oxo-3-phenylimidazo lin-1-yl)piperidin-1-yl)-1,2,4-triazin-6-carboxamide

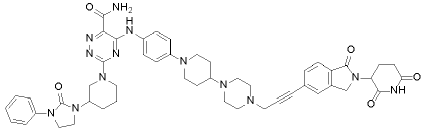

The synthesis method was the same as that for Example 1, except that Intermediate 63 was replaced with Intermediate 128, and Intermediate 133 was replaced with Intermediate 145 to obtain Compound 097, ESI-MS: *m*/*z* =906 [M+H]⁺.

### Example 98. 5-((4-(1-((1-(2-(2,6-dioxopiperidin-3-yl)-6-fluoro-1,3-dioxoisoindol-5-yl) piperidin-4-yl)methyl)piperidin-4-yl)phenyl)amino)-3-(3-(2-oxo-3-phenylimidazolin-1-yl)pi peridin-1-yl)-1,2,4-triazin-6-carboxamide

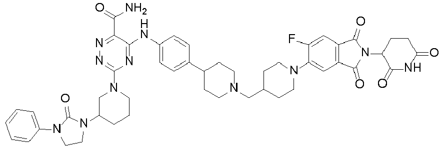

The synthesis method was the same as that for Example 1, except that Intermediate 63 was replaced with Intermediate 111, and Intermediate 133 was replaced with Intermediate 147 to obtain Compound 098, ESI-MS: *m*/*z* =913 [M+H]⁺.

### Example 99. 5-((4-(1-((1-(2-(2,6-dioxopiperidin-3-yl)-3-oxoisoindol-5-yl)piperidin-4-yl)methyl)piperidin-4-yl)phenyl)amino)-3-(3-(2-oxo-3-phenylimidazolin-1-yl)piperidin-1-yl) -1,2,4-triazin-6-carboxamide

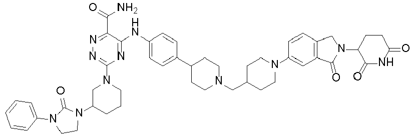

The synthesis method was the same as that for Example 1, except that Intermediate 63 was replaced with Intermediate 111, and Intermediate 133 was replaced with Intermediate 146 to obtain Compound 099, ESI-MS: *m*/*z* =881 [M+H]⁺.

### Example 100. 5-((4-(1-((1-(2-(2,6-dioxopiperidin-3-yl)-1-oxoisoindol-5-yl)piperidin-4-yl)methyl)piperidin-4-yl)phenyl)amino)-3-(3-(2-oxo-3-phenylimidazolin-1-yl)piperidin-1-yl)-1,2,4-triazin-6-carboxamide

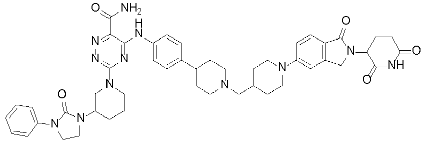

The synthesis method was the same as that for Example 1, except that Intermediate 63 was replaced with Intermediate 111, and Intermediate 133 was replaced with Intermediate 145 to obtain Compound 100, ESI-MS: *m*/*z* =881 [M+H]⁺.

### Example 101. 5-((4-(1-((1-(1-(2,6-dioxopiperidin-3-yl)-3-methyl-2-oxo-2,3-dihydro-1H-benzo[d]imidazol-5-yl)piperidin-4-yl)methyl)piperidin-4-yl)phenyl)amino)-3-(3-(2-oxo-3-phenylimidazol-1-yl)piperidin-1-yl)-1,2,4-triazin-6-carboxamide

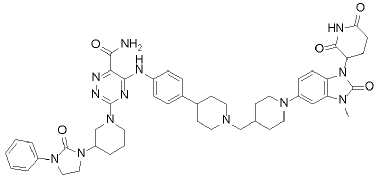

The synthesis method was the same as that for Example 1, except that Intermediate 63 was replaced with Intermediate 111, and Intermediate 133 was replaced with 1-(1-(2,6-dioxopiperidin-3-yl)-3-methyl-2 -oxo-2,3-dihydro-1H-benzo[d]imidazol-5-yl)piperidin -4-aminocarbaldehyde to obtain Compound 101, ESI-MS: *m*/*z* =896 [M+H]⁺.

### Example 102. 5-((4-(1-((1-(2-(2,6-dioxopiperidin-3-yl)-1,3-dioxoisoindol-5-yl) pyrrolidin-3-yl)methyl)piperidin-4-yl)phenyl)amino)-3-(6-(3-methyl-2-oxoimidazolin-1-yl)-2-azabicyclo[2.2.1]heptan-2-yl)-1,2,4-triazin-6-carboxamide

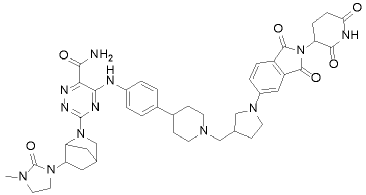

The synthesis method was the same as that for Example 1, except that Intermediate 63 was replaced with Intermediate 98, and Intermediate 133 was replaced with Intermediate 142 to obtain Compound 102, ESI-MS: *m*/*z* =831 [M+H]⁺.

### Example 103. 5-((4-(1-(1-(2-(2,6-dioxopiperidin-3-yl)-1,3-dioxoisoindol-5-yl)azetidin-3-yl)piperidin-4-yl)phenyl)amino)-3-(6-(3-methyl-2-oxoimidazolin-1-yl)-2-azabicyclo[2.2.1] heptan-2-yl)-1,2,4-triazin-6-carboxamide

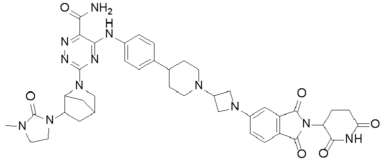

The synthesis method was the same as that for Example 1, except that Intermediate 63 was replaced with Intermediate 98 to obtain Compound 103, ESI-MS: *m*/*z* = 831 [M+H]⁺.

### Example 104. 5-((4-(1'-(2-(2,6-dioxopiperidin-3-yl)-1,3-dioxoisoindol-5-yl)-4-methyl-[1,4'-bipiperidin]-4-yl)phenyl)amino)-3-(6-(3-methyl-2-oxoimidazolin-1-yl)-2-azabicyclo[2.2 .1]heptan-2-yl)-1,2,4-triazin-6-carboxamide

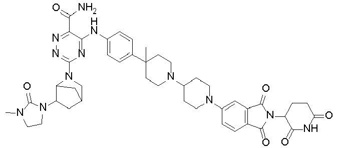

The synthesis method was the same as that for Example 1, except that Intermediate 63 was replaced with Intermediate 129, and Intermediate 133 was replaced with Intermediate 134 to obtain Compound 104, ESI-MS: *m*/*z* =845 [M+H]⁺.

### Example 105. 5-((4-(1-(1-(2-(2,6-dioxopiperidin-3-yl)-1,3-dioxoisoindol-5-yl)azetidin -3-yl)-4-methylpiperidin-4-yl)phenyl)amino)-3-(6-(3-methyl-2-oxoimidazolin-1-yl)-2-azabic yclo[2.2.1]heptan-2-yl)-1,2,4-triazin-6-carboxamide

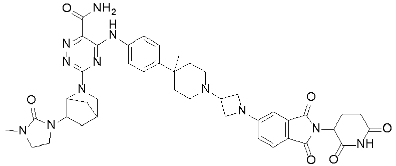

The synthesis method was the same as that for Example 1, except that Intermediate 63 was replaced with Intermediate 129 to obtain Compound 105, ESI-MS: *m*/*z* = 817 [M+H]⁺.

### Example 106. 5-((2-(1-(2-(2,6-dioxopiperidin-3-yl)-1,3-dioxoisoindol-5-yl)azetidine -3-yl)-1,2,3,4-tetrahydroisoquinolin-6-yl)amino)-3-(6-(3-methyl-2-oxoimidazolin-1-yl)-2-aza bicyclo[2.2.1]heptan-2-yl)-1,2,4-triazin-6-carboxamide

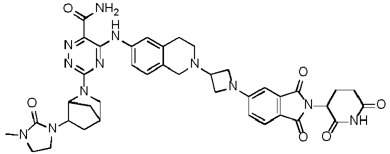

The synthesis method was the same as that for Example 1, except that Intermediate 63 was replaced with Intermediate 130 to obtain Compound 106, ESI-MS: *m*/*z* = 775 [M+H]⁺.

### Example 107. 5-((2-((1-(2-(2,6-dioxopiperidin-3-yl)-1,3-dioxoisoindol-5-yl)piperidin -4-yl)methyl)-1,2,3,4-tetrahydroisoquinolin-6-yl)amino)-3-(6-(3-methyl-2-oxoimidazolin-1-y l)-2-azabicyclo[2.2.1]heptan-2-yl)-1,2,4-triazin-6-carboxamide

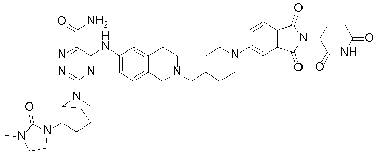

The synthesis method was the same as that for Example 1, except that Intermediate 63 was replaced with Intermediate 130, and Intermediate 133 was replaced with Intermediate 140 to obtain Compound 107, ESI-MS: *m*/*z* =817 [M+H]⁺.

### Example 108. 5-((4-(4-((1-(2-(2,6-dioxopiperidin-3-yl)-1,3-dioxoisoindol-5-yl) piperidin-4-yl)methyl)piperazin-1-yl)phenyl)amino)-3-(6-(3-methyl-2-oxoimidazolin-1-yl)-2 -azabicyclo[2.2.1]heptan-2-yl)-1,2,4-triazin-6-carboxamide

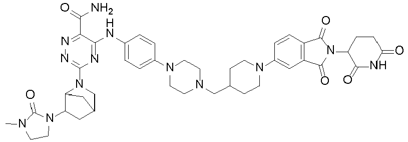

The synthesis method was the same as that for Example 1, except that Intermediate 63 was replaced with Intermediate 131, and Intermediate 133 was replaced with Intermediate 140 to obtain Compound 108, ESI-MS: *m*/*z* =846 [M+H]⁺.

### Example 109. 5-((4-(1'-(2-(2,6-dioxopiperidin-3-yl)-1,3-dioxoisoindol-5-yl)-[1,4'-bipiperidin]-4-yl)phenyl)amino)-3-(6-(3-methyl-2-oxoimidazolin-1-yl)-2-azabicyclo [2.2 .1]heptan-2-yl)-1,2,4-triazin-6-carboxamide

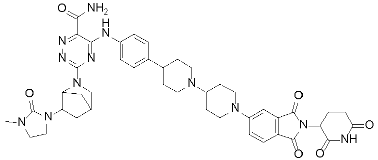

The synthesis method was the same as that for Example 1, except that Intermediate 63 was replaced with Intermediate 98, and Intermediate 133 was replaced with Intermediate 134 to obtain Compound 109, ESI-MS: *m*/*z* =831 [M+H]⁺.

### Example 110. 5-((4-(4-(3-(2-(2,6-dioxopiperidin-3-yl)-1-oxoisoquinolin-5-yl) propan-2-yn-1-yl)piperazin-1-yl)piperidin-1-yl)phenyl)amino)-3-(6-(3-methyl-2-oxoisoimid azol-1-yl)-2-azabicyclo[2.2.1]heptan-2-yl)-1,2,4-triazin-6-carboxamide

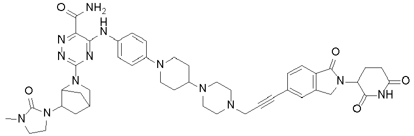

The synthesis method was the same as that for Example 1, except that Intermediate 63 was replaced with Intermediate 132, and Intermediate 133 was replaced with Intermediate 145 to obtain Compound 110, ESI-MS: *m*/*z* =856 [M+H]⁺.

### Example 111. 5-((4-(1-((1-(2-(2,6-dioxopiperidin-3-yl)-6-fluoro-1,3-dioxoisoindol-5-yl) piperidin-4-yl)methyl)piperidin-4-yl)phenyl)amino)-3-(6-(3-methyl-2-oxoimidazolin-1-yl)-2 -azabicyclo[2.2.1]heptan-2-yl)-1,2,4-triazin-6-carboxamide

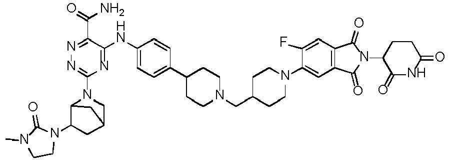

The synthesis method was the same as that for Example 1, except that Intermediate 63 was replaced with Intermediate 98, and Intermediate 133 was replaced with Intermediate 147 to obtain Compound 111, ESI-MS: *m*/*z* =863 [M+H]⁺.

### Example 112. 5-((4-(1-((1-(2-(2,6-dioxopiperidin-3-yl)-3-oxoisoindol-5-yl)piperidin-4-yl)methyl)piperidin-4-yl)phenyl)amino)-3-(6-(3-methyl-2-oxoimidazolin-1-yl)-2-azabicycl o[2.2.1]heptan-2-yl)-1,2,4-triazin-6-carboxamide

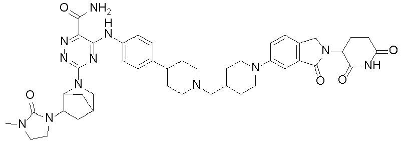

The synthesis method was the same as that for Example 1, except that Intermediate 63 was replaced with Intermediate 98, and Intermediate 133 was replaced with Intermediate 146 to obtain Compound 112, ESI-MS: *m*/*z* =831 [M+H]⁺.

### Example 113. 5-((4-(1-((1-(2-(2,6-dioxopiperidin-3-yl)-1-oxoisoindol-5-yl)piperidin-4 -yl)methyl)piperidin-4-yl)phenyl)amino)-3-(6-(3-methyl-2-oxoimidazolin-1-yl)-2-azabicyclo [2.2.1]heptan-2-yl)-1,2,4-triazin-6-carboxamide

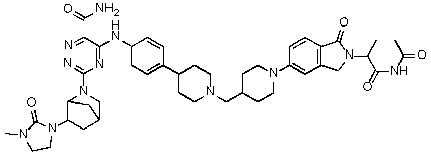

The synthesis method was the same as that for Example 1, except that Intermediate 63 was replaced with Intermediate 98, and Intermediate 133 was replaced with Intermediate 148 to obtain Compound 113, ESI-MS: *m*/*z* =831 [M+H]⁺.

### Example 114. 5-((4-(1-((1-(1-(2,6-dioxopiperidin-3-yl)-3-methyl-2-oxo-2,3-dihydro -1H-benzo[d]imidazol-5-yl)piperidin-4-yl)methyl)piperidin-4-yl)phenyl)amino)-3-(6-(3-met hyl-2-oxoimidazolin-1-yl)-2-azabicyclo[2.2.1]heptan-2-yl)-1,2,4-triazin-6-carboxamide

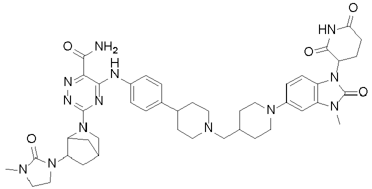

The synthesis method was the same as that for Example 1, except that Intermediate 63 was replaced with Intermediate 98, and Intermediate 133 was replaced with 1-(1-(2,6-dioxopiperidin-3-yl)-3-methyl-2 -oxo-2,3-dihydro-1H-benzo[d]imidazol-5-yl)piperidin -4-aminocarbaldehyde to obtain Compound 114, ESI-MS: *m*/*z* =846 [M+H]⁺.

### Example 115: 5-((4-(4-((1-(2-(2,6-dioxopiperidin-3-yl)-1,3-dioxoisoindolin-5-yl) pyrrolidin-3-yl)methyl)piperazin-1-yl)-3-fluorophenyl)amino)-3-((R)-3-(3-methyl-2-oxoimid azolin-1-yl)piperidin-1-yl)-1,2,4-triazin-6-carboxamide (130)

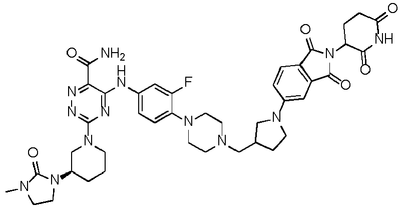

Intermediate 165 (150 mg, 0.25 mmol) was dissolved in 2 mL of DCM, and TFA (1.00 mL) was added to the system, and the mixture was stirred at room temperature for 1 h. The reaction was completed and concentrated under reduced pressure to obtain (R)-5-((3-fluoro-4-(piperazin-1-yl)phenyl)amino)-3-(3-(3-methyl-2-oxoimidazolidin-1-yl)piperid in-1-yl)-1,2,4-triazin-6-carboxamide as a yellow liquid (122 mg). The above product was dissolved in 3 mL of DMF, and DIPEA (97 mg, 0.75 mmol) and Intermediate 142 (107 mg, 0.30 mmol) were added to the system in sequence. Tthe mixture was stirred at room temperature for 10 min, then acetic acid (0.1 mL) and sodium cyanoborohydride (47 mg, 0.75 mmol) were added to the system and the mixture was reacted at room temperature for 1 h. After the reaction was completed, the reaction liquid was adjusted to be alkaline by adding saturated aqueous sodium bicarbonate solution, and a solid was precipitated. Filtration was performed to obtain a yellow solid which was dissolved by adding DCM, and the resulting mixture was dried over anhydrous sodium sulfate, and concentrated under reduced pressure. Column chromatography (eluted with DCM/MeOH) was performed to obtain 5-((4-(4-((1-(2-(2,6-dioxopiperidin-3-yl)-1,3-dioxoisoindolin-5-yl)pyrrolidin-3-yl)methyl)pipera zin-1-yl)-3-fluorophenyl)amino)-3-((*R*)-3-(3-methyl-2-oxoimidazolin-1-yl)piperidin-1-yl)-1,2,4-t riazin-6-carboxamide as a yellow solid (84 mg, yield 40%). ESI-MS: *m*/*z* = 838.4 [M+H]⁺. ¹H-NMR (400 MHz, CDCl₃) δ 10.87 (brs, 1H), 8.23 (brs, 1H), 7.62 - 7.71 (m, 2H), 7.50 - 7.52 (m, 1H), 7.20 - 7.30 (m, 1H), 6.92 - 6.97 (m, 2H), 6.65 - 6.69 (m, 1H), 5.54 (brs, 1H), 4.91 - 4.94 (m, 1H), 3.73 - 3.79 (m, 1H), 2.98 - 3.61 (m, 13H), 2.43 - 2.89 (m, 13H), 2.09 - 2.25 (m, 2H), 1.85 - 2.01 (m, 8H).

### Example 116: 5-((4-(4-((1-(2-(2,6-dioxopiperidin-3-yl)-1,3-dioxoisoindolin-5-yl) piperidin-4-yl)methyl)piperazin-1-yl)-3-fluorophenyl)amino)-3-((R)-3-(3-methyl-2-oxoimid azolin-1-yl)piperidin-1-yl)-1,2,4-triazin-6-carboxamide (134)

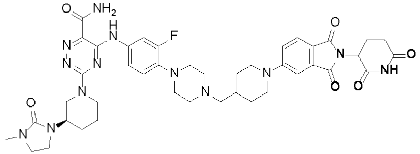

Intermediate 165 (150 mg, 0.25 mmol) was dissolved in 3 mL of DCM, and TFA (1.00 mL) was added to the system, and the mixture was stirred at room temperature for 1 h. The reaction was completed and concentrated under reduced pressure to obtain (R)-5-((3-fluoro-4-(piperazin-1-yl)phenyl)amino)-3-(3-(3-methyl-2-oxoimidazolidin-1-yl)piperid in-1-yl)-1,2,4-triazin-6-carboxamide as a yellow liquid (124 mg). The above product was dissolved in 3 mL of DMF, and DIPEA (97 mg, 0.75 mmol) and Intermediate 140 (111 mg, 0.30 mmol) were added to the system in sequence. The mixture was stirred at room temperature for 10 min, then acetic acid (0.1 mL) and sodium cyanoborohydride (47 mg, 0.75 mmol) were added to the system and the mixture was reacted at room temperature for 1 h. After the reaction was completed, the reaction liquid was adjusted to be alkaline by adding saturated aqueous sodium bicarbonate solution, and a solid was precipitated. Filtration was performed to obtain a yellow solid which was dissolved by adding DCM, and the resulting mixture was dried over anhydrous sodium sulfate, and concentrated under reduced pressure. Column chromatography (eluted with DCM/MeOH) was performed to obtain 5-((4-(4-((1-(2-(2,6-dioxopiperidin-3-yl) -1,3-dioxoisoindolin-5-yl)piperidin-4-yl)methyl)piperazin-1-yl)-3-fluorophenyl)amino)-3-((R)-3-(3-methyl-2-oxoimidazolin-1-yl)piperidin-1-yl)-1,2,4-triazin-6-carboxamide as a yellow solid (89 mg, yield 42%). ESI-MS: *m*/*z* = 852.4 [M+H]⁺. ¹H-NMR (400 MHz, CDCl₃) δ 10.88 (brs, 1H), 8.22 - 8.23 (m, 1H), 7.76 - 7.71 (m, 2H), 7.51 - 7.55 (m, 1H), 7.19 - 7.34 (m, 2H), 7.03 - 7.06 (m, 1H), 6.93 - 6.97 (m, 1H), 5.50 (brs, 1H), 4.91 - 4.96 (m, 1H), 3.95 - 3.98 (m, 2H), 3.75 - 3.80 (m, 1H), 3.29 - 3.42 (m, 4H), 2.62 - 3.11 (m, 18H), 2.29 - 2.31 (m, 2H), 2.10 - 2.15 (m, 1H), 1.79 - 2.00 (m, 7H), 1.25 - 1.35 (m, 4H).

### Example 117: 5-((4-(4-((1-(6-(((S)-2,6-dioxopiperidin-3-yl)carbamoyl)pyridin-3-yl) pyrrolidin-3-yl)methyl)piperazin-1-yl)-3-fluorophenyl)amino)-3-((R)-3-(3-methyl-2-oxoimid azolin-1-yl)piperidin-1-yl)-1,2,4-triazin-6-carboxamide (145)

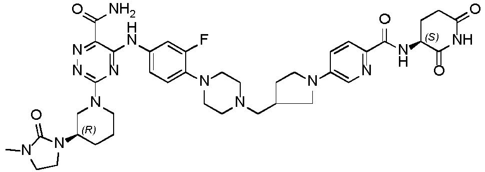

Intermediate 165 (300 mg, 0.50 mmol) was dissolved in 5 mL of DCM, and TFA (1.5 mL) was added to the system, and the mixture was stirred at room temperature for 1 h. After the reaction was completed, the reaction liquid was concentrated under reduced pressure to obtain a yellow liquid (247 mg). The above product was dissolved in 10 mL of DMF, and DIPEA (193 mg, 1.50 mmol) and Intermediate 215 (198 mg, 0.60 mmol) were added in sequence. The mixture was stirred at room temperature for 10 min, then acetic acid (0.2 mL) and sodium cyanoborohydride (94 mg, 1.50 mmol) were added to the system and the mixture was reacted at room temperature for 1 h. After the reaction was completed, the reaction liquid was adjusted to be alkaline by adding saturated aqueous sodium bicarbonate solution, and a solid was precipitated. Filtration was performed to obtain a yellow solid which was dissolved by adding DCM, and the resulting mixture was dried over anhydrous sodium sulfate, and concentrated under reduced pressure. Column chromatography (eluted with DCM/MeOH) was performed to obtain 5-((4-(4-((1-(6-(((*S*)-2,6-dioxopiperidin-3-yl)carbamoyl)pyridin-3-yl)pyrrolidin-3-yl) methyl)piperazin-1-yl)-3-fluorophenyl)amino)-3-((R)-3-(3-methyl-2-oxoimidazolin-1-yl)piperidi n-1-yl)-1,2,4-triazin-6-carboxamide as a yellow solid (240 mg, yield 59%). ESI-MS: *m*/*z* = 813.4 [M+H]⁺. ¹H-NMR (400 MHz, CDCl₃) δ 10.92 (brs, 1H), 8.69 - 8.83 (m, 1H), 8.42 - 8.44 (m, 1H), 7.87 - 8.01 (m, 2H), 7.75 - 7.78 (m, 1H), 7.51 - 7.56 (m, 1H), 7.22 - 7.30 (m, 1H), 6.83 - 6.94 (m, 2H), 5.76 (brs, 1H), 4.78 - 4.83 (m, 1H), 3.76 - 3.82 (m, 1H), 3.30 - 3.54 (m, 8H), 3.11 - 3.18 (m, 6H), 2.79 - 2.80 (m, 5H), 2.61 - 2.69 (m, 4H), 2.47 - 2.49 (m, 2H), 2.17 - 2.24 (m, 1H), 1.81 - 1.95 (m, 10H).

### Example 118: 5-((4-(4-((1-(6-((2,6-dioxopiperidin-3-yl)carbamoyl)pyridin-3-yl) piperidin-4-yl)methyl)piperazin-1-yl)-3-fluorophenyl)amino)-3-((R)-3-(3-methyl-2-oxoimid azolin-1-yl)piperidin-1-yl)-1,2,4-triazin-6-carboxamide (146)

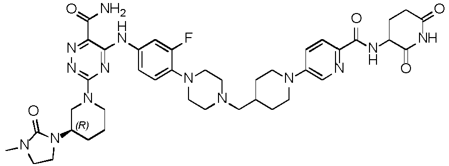

Intermediate 165 (300 mg, 0.50 mmol) was dissolved in 5 mL of DCM, and TFA (1.5 mL) was added to the system, and the mixture was stirred at room temperature for 1 h. After the reaction was completed, the reaction liquid was concentrated under reduced pressure to obtain a yellow liquid (245 mg). The above product was dissolved in 8 mL of DMF, and DIPEA (193 mg, 1.50 mmol) and Intermediate 210 (207 mg, 0.60 mmol) were added in sequence. The mixture was stirred at room temperature for 10 min, then acetic acid (0.2 mL) and sodium cyanoborohydride (94 mg, 1.50 mmol) were added to the system and the mixture was reacted at room temperature for 1 h. After the reaction was completed, the reaction liquid was adjusted to be alkaline by adding saturated aqueous sodium bicarbonate solution, and a solid was precipitated. Filtration was performed to obtain a yellow solid which was dissolved by adding DCM, and the resulting mixture was dried over anhydrous sodium sulfate, and concentrated under reduced pressure. Column chromatography (eluted with DCM/MeOH) was performed to obtain 5-((4-(4-((1-(6-((2,6-dioxopiperidin-3-yl)carbamoyl)pyridin-3-yl)piperidin-4-yl)methyl) piperazin-1-yl)-3-fluorophenyl)amino)-3-((R)-3-(3-methyl-2-oxoimidazolin-1-yl)piperidin-1-yl)-1,2,4-triazin-6-carboxamide as a yellow solid (199 mg, yield 48%). ESI-MS: *m*/*z* = 827.4 [M+H]⁺. ¹H-NMR (400 MHz, DMSO-d₆) δ 11.23 (brs, 1H), 10.81 (brs, 1H), 8.64 - 8.68 (m, 1H), 8.25 - 8.32 (m, 2H), 8.14 (brs, 1H), 7.78 - 7.82 (m, 1H), 7.70 (s, 1H), 7.34 - 7.53 (m, 2H), 6.96 - 7.00 (m, 1H), 4.67 - 4.72 (m, 1H), 3.89 - 3.91 (m, 2H), 3.20 - 3.23 (m, 5H), 2.71 - 2.95 (m, 15H), 2.63 - 2.65 (m, 4H), 2.11 - 2.19 (m, 4H), 1.93 - 1.98 (m, 2H), 1.73 - 1.81 (m, 7H).

### Example 119: 5-((4-(4-((1-(4-((2,6-dioxopiperidin-3-yl)carbamoyl)-3-fluorophenyl) piperidin-4-yl)methyl)piperazin-1-yl)-3-fluorophenyl)amino)-3-((R)-3-(3-methyl-2-oxoimid azolidin-1-yl)piperidin-1-yl)-1,2,4-triazin-6-carboxamide (254)

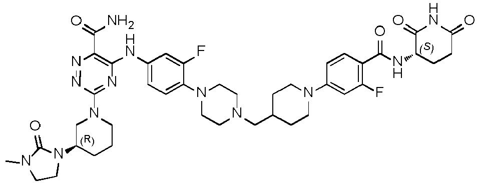

(R)-5-((3-fluoro-4-(piperazin-1-yl)phenyl)amino)-3-(3-(3-methyl-2-oxoimidazolidin-1-yl )piperidin-1-yl)-1,2,4-triazin-6-carboxamide (60 mg, 0.12 mmol) was dissolved in 3 mL of DMF, and DIPEA (50 mg, 0.36 mmol) and Intermediate 209 (60 mg, 0.18 mmol) were added to the system in sequence. The mixture was stirred at room temperature for 10 min, then acetic acid (0.2 mL) and sodium cyanoborohydride (22 mg, 0.36 mmol) were added to the system and the mixture was reacted at room temperature for 1 h. After the reaction was completed, a solid was precipitated by adding water. Filtration was performed to obtain a yellow solid which was dissolved by adding DCM, and the resulting mixture was dried over anhydrous sodium sulfate, and concentrated under reduced pressure. Column chromatography (eluted with DCM/MeOH) was performed to obtain 5-(4-(4-(1-(4-((S)-2,6-dioxopiperidin-3-yl)carbamoyl)-3-fluorophenyl)piperidin-4-yl)methyl)pip erazin-1-yl)-3-fluorophenyl)amino)-3-(R)-3-(3-methyl-2-oxoimidazolin-1-yl)piperidin-1-yl)-1,2, 4-triazin-6-carboxamide as a yellow solid (40 mg, yield 40%). ESI-MS: *m*/*z* = 844.4 [M+H]⁺. ¹H-NMR (400 MHz, DMSO-d₆) δ 11.31 (brs, 1H), 10.84 (brs, 1H), 8.36 (s, 1H), 7.98 - 8.01 (m, 1H), 7.59 - 7.74 (m, 3H), 6.73 - 6.82 (m, 2H), 4.67 - 4.72 (m, 1H), 3.89 - 3.91 (m, 2H), 3.56 - 3.63 (m, 4H), 2.96 - 3.24 (m, 12H), 2.51 - 2.52 (m, 1H), 2.66 - 2.84 (m, 7H), 2.08 - 2.19 (m, 3H), 1.93 - 2.01 (m, 3H), 1.76 - 1.81 (m, 7H).

### Example 120: 5-((4-(4-((1-(3-(2,4-dioxotetrahydropyrimidin-1(2H)-yl)-4-fluorophenyl) pyrrolidin-3-yl)methyl)piperazin-1-yl)-3-fluorophenyl)amino)-3-((R)-3-(3-methyl-2-oxoimid azolidin-1-yl)piperidin-1-yl)-1,2,4-triazin-6-carboxamide (256)

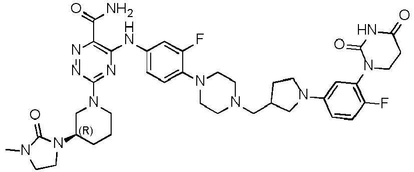

(R)-5-((3-fluoro-4-(piperazin-1-yl)phenyl)amino)-3-(3-(3-methyl-2-oxoimidazolin-1-yl)p iperidin-1-yl)-1,2,4-triazin-6-carboxamide (100 mg, 0.20 mmol) was dissolved in 5 mL of DMF, and Intermediate 185 (80 mg, 0.26 mmol) was added to the system in sequence. The mixture was stirred at room temperature for 10 min, then acetic acid (0.2 mL) and sodium cyanoborohydride (40 mg, 0.60 mmol) were added to the system and the mixture was reacted at room temperature for 1 h. After the reaction was completed, a solid was precipitated by adding water. Filtration was performed to obtain a yellow solid which was dissolved by adding DCM, and the resulting mixture was dried over anhydrous sodium sulfate, concentrated under reduced pressure, and subjected to column chromatography (eluted with DCM/MeOH) to obtain 5-(4-(4-(1-(3-(2,4-dioxotrihydropyrimidin-1(2H)-yl)-4-fluorophenyl) pyrrolidin-3-yl)methyl)piperazin-1-yl)-3-fluorophenyl)amino)-3-(R)-3-(3-methyl-2-oxoimidazol -1-yl)piperidin-1-yl)-1,2,4-triazin-6-carboxamide as a yellow solid (60 mg, yield 38%). ESI-MS(M+H)⁺=788.4. ¹H-NMR (400 MHz, DMSO-d₆) δ 11.24 (brs, 1H), 10.38 (brs, 1H), 8.32 (s, 1H), 7.72 (s, 1H), 7.35 - 7.49 (m, 2H), 6.98 - 7.06 (m, 2H), 6.39 - 6.47 (m, 2H), 3.51 - 3.66 (m, 4H), 3.14 - 3.25 (m, 5H), 2.89 - 2.96 (m, 8H), 2.55 - 2.68 (m, 11H), 2.32 - 2.34 (m, 2H), 2.03 - 2.05 (m, 1H), 1.66 - 1.81 (m, 4H), 1.22 - 1.27 (m, 2H).

### Example 121: (R)-5-((4-(4-((1-(4-(2,4-dioxotetrahydropyrimidin-1(2H)-yl)-3-methylphenyl)piperidin-4-yl)methyl)piperazin-1-yl)-3-fluorophenyl)amino)-3-(3-(3-methyl-2-oxoimidazolidin-1-yl)piperidin-1-yl)-1,2,4-triazin-6-carboxamide (257)

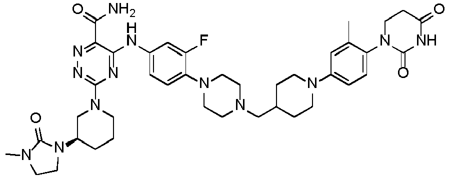

Intermediate 192 (45 mg, 0.15 mmol) was dissolved in 5 mL of DMF, and (R)-5-((3-fluoro-4-(piperazin-1-yl)phenyl)amino)-3-(3-(3-methyl-2-oxoimidazolin-1-yl)piperidin -1-yl)-1,2,4-triazin-6-carboxamide (90 mg, 0.18 mmol) was added to the system, and the mixture was stirred at room temperature for 10 min, then acetic acid (0.1 mL) and sodium cyanoborohydride (29 mg, 0.46 mmol) were added to the system and the mixture was reacted at room temperature for 1 h. After the reaction was completed, a solid was precipitated by adding water. Filtration was performed to obtain a yellow solid which was dissolved by adding DCM, and the resulting mixture was dried over anhydrous sodium sulfate, concentrated under reduced pressure, and subjected to column chromatography (eluted with DCM/MeOH) to obtain (R)-5-((4-(4-((1-(4-(2,4-dioxotetrahydropyrimidin-1(2H)-yl)-3-methylphenyl)piperidin-4-yl)methyl)piperazin-1-yl)-3-fluorophenyl)amino)-3-(3-(3-methyl-2-ox oimidazolidin-1-yl)piperidin-1-yl)-1,2,4-triazin-6-carboxamide as a yellow solid (49 mg, yield 41%), ESI-MS(M+H)⁺=798.4. ¹H-NMR (400 MHz, CDCl₃) δ 10.89 (brs, 1H), 7.88 (brs, 1H), 7.70 (s, 1H), 7.50 - 7.53 (m, 1H), 7.19 - 7.26 (m, 1H), 7.00 - 7.02 (m, 1H), 6.90 - 6.95 (m, 1H), 6.75 - 6.79 (m, 2H), 5.72 (brs, 1H), 3.55 - 3.77 (m, 6H), 3.26 - 3.39 (m, 5H), 3.07 - 3.10 (m, 5H), 2.59 - 2.81 (m, 12H), 2.27 - 2.29 (m, 2H), 2.20 (s, 3H), 1.79 - 1.98 (m, 9H)

### Example 122: (R)-5-((4-(4-((1-(4-(2,4-dioxotetrahydropyrimidin-1(2H)-yl)-3-methoxyphenyl)piperidin-4-yl)methyl)piperazin-1-yl)-3-fluorophenyl)amino)-3-(3-(3-methy l-2-oxoimidazolidin-1-yl)piperidin-1-yl)-1,2,4-triazin-6-carboxamide (259)

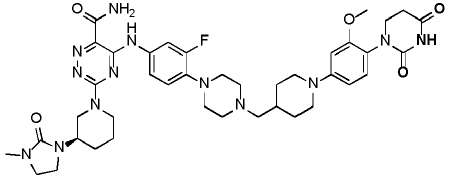

Intermediate 272 (50 mg, 0.15 mmol) was dissolved in 5 mL of DMF, and (R)-5-((3-fluoro-4-(piperazin-1-yl)phenyl)amino)-3-(3-(3-methyl-2-oxoimidazolin-1-yl)piperidin -1-yl)-1,2,4-triazin-6-carboxamide (90 mg, 0.18 mmol) was added to the system, and the mixture was stirred at room temperature for 10min, then acetic acid (0.1 mL) and sodium cyanoborohydride (29 mg, 0.46 mmol) were added to the system and the mixture was reacted at room temperature for 1 h. After the reaction was completed, a solid was precipitated by adding water. Filtration was performed to obtain a yellow solid which was dissolved by adding DCM, and the resulting mixture was dried over anhydrous sodium sulfate, concentrated under reduced pressure, and subjected to column chromatography (eluted with DCM/MeOH) to obtain (R)-5-((4-(4-(-1-(4-(2,4-dioxotetrahydropyrimidin-1(2H)-yl)-3-methoxyphenyl)piperidin-4-yl)methyl)piperazin-1-yl)-3-fluorophenyl)amino)-3-(3-(3-methyl-2-oxoimidazolidin-1-yl)piperidin-1-yl)-1,2,4-triazin-6-carboxamide as a yellow solid (54 mg, yield 44%), ESI-MS(M+H)⁺=814.4. ¹H-NMR (400 MHz, CDCl₃) δ 10.89 (brs, 1H), 7.52 - 7.71 (m, 3H), 7.20 - 7.30 (m, 1H), 7.08 - 7.10 (m, 1H), 6.92 - 6.97 (m, 1H), 6.49 - 6.53 (m, 2H), 5.58 (brs, 1H), 3.65 - 3.83 (m, 9H), 3.29 - 3.42 (m, 5H), 3.10 - 3.12 (m, 5H), 2.72 - 2.81 (m, 8H), 2.61 - 2.63 (m, 4H), 2.29 - 2.31 (m, 2H), 1.78 - 2.02 (m, 9H).

### Example 123: (R)-5-((4-(4-((1-(4-(2,4-dioxotetrahydropyrimidin-1(2H)-yl)phenyl) piperidin-4-yl)methyl)piperazin-1-yl)-3-fluorophenyl)amino)-3-(3-(3-methyl-2-oxoimidazoli din-1-yl)piperidin-1-yl)-1,2,4-triazin-6-carboxamide (255)

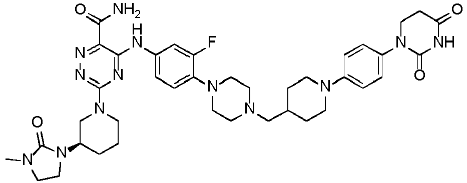

Intermediate 191 (45 mg, 0.15 mmol) was dissolved in 5 mL of DMF, and (R)-5-((3-fluoro-4-(piperazin-1-yl)phenyl)amino)-3-(3-(3-methyl-2-oxoimidazolin-1-yl)piperidin -1-yl)-1,2,4-triazin-6-carboxamide (90 mg, 0.18 mmol) was added to the system, and the mixture was stirred at room temperature for 10min, then acetic acid (0.1 mL) and sodium cyanoborohydride (29 mg, 0.46 mmol) were added to the system and the mixture was reacted at room temperature for 1 h. After the reaction was completed, a solid was precipitated by adding water. Filtration was performed to obtain a yellow solid which was dissolved by adding DCM, and the resulting mixture was dried over anhydrous sodium sulfate, concentrated under reduced pressure, and subjected to column chromatography (eluted with DCM/MeOH) to obtain (R)-5-((4-(4-((1-(4-(2,4-dioxotetrahydropyrimidin-1(2H)-yl)phenyl) piperidin-4-yl)methyl)piperazin-1-yl)-3-fluorophenyl)amino)-3-(3-(3-methyl-2-oxoimidazolidin-1-yl)piperidin-1-yl)-1,2,4-triazin-6-carboxamide as a yellow solid (46 mg, yield 39%), ESI-MS(M+H)⁺=784.4. ¹H-NMR (400 MHz, DMSO-d₆) δ 11.28 (brs, 1H), 10.27 (brs, 1H), 8.35 (s, 1H), 7.75 (s, 1H), 7.36 - 7.54 (m, 2H), 7.11 - 7.13 (m, 2H), 7.01 - 7.05 (m, 1H), 6.90 - 6.93 (m, 2H), 3.57 - 3.70 (m, 6H), 3.36 - 3.42 (m, 1H), 3.21 - 3.26 (m, 3H), 2.92 - 3.09 (m, 7H), 2.63 - 2.68 (m, 9H), 2.53 - 2.54 (m, 2H), 2.19 - 2.21 (m, 2H), 1.78 - 1.81 (m, 6H), 1.22 - 1.26 (m, 3H).

### Example 124: 5-((4-(4-((1-(3-(2,4-dioxotetrahydropyrimidin-1(2H)-yl)-4-methoxyphenyl)pyrrolidin-3-yl)methyl)piperazin-1-yl)-3-fluorophenyl)amino)-3-((R)-3-(3-methyl-2-oxoimidazolidin-1-yl)piperidin-1-yl)-1,2,4-triazin-6-carboxamide (258)

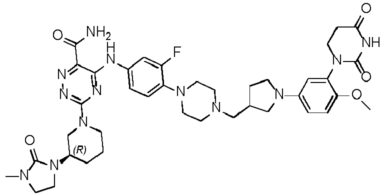

(R)-5-((3-fluoro-4-(piperazin-1-yl)phenyl)amino)-3-(3-(3-methyl-2-oxoimidazolin-1-yl)p iperidin-1-yl)-1,2,4-triazin-6-carboxamide (100 mg, 0.20 mmol) was dissolved in 5 mL of DMF, and Intermediate 186 (76 mg, 0.24 mmol) was added to the system in sequence. The mixture was stirred at room temperature for 10 min, then acetic acid (0.2 mL) and sodium cyanoborohydride (40 mg, 0.60 mmol) were added to the system and the mixture was reacted at room temperature for 1 h. After the reaction was completed, a solid was precipitated by adding water. Filtration was performed to obtain a yellow solid which was dissolved by adding DCM, and the resulting mixture was dried over anhydrous sodium sulfate, concentrated under reduced pressure, and subjected to column chromatography (eluted with DCM/MeOH) to obtain 5-((4-(4-((1-(3-(2,4-dioxotetrahydropyrimidin-1(2H)-yl)-4-methoxyphenyl)pyrrolidin-3-yl)methyl)piperazin-1-yl)-3-fluorophenyl)amino)-3-((R)-3-(3-meth yl-2-oxoimidazolidin-1-yl)piperidin-1-yl)-1,2,4-triazin-6-carboxamide as a yellow solid (66 mg, yield 41%). ESI-MS(M+H)⁺=800.4. ¹H-NMR (400 MHz, CDCl₃) δ 10.89 (brs, 1H), 7.51 - 7.73 (m, 3H), 7.31 - 7.36 (m, 1H), 6.89 - 7.05 (m, 3H), 6.49 - 6.53 (m, 1H), 5.56 (brs, 1H), 3.70 - 3.90 (m, 9H), 3.17 - 3.41 (m, 10H), 2.61 - 2.86 (m, 12H), 2.10 - 2.25 (m, 9H).

### Example 125: 5-((4-(4-((1-(4-(2,4-dioxotetrahydropyrimidin-1(2H)-yl)phenyl) pyrrolidin-3-yl)methyl)piperazin-1-yl)-3-fluorophenyl)amino)-3-((R)-3-(3-methyl-2-oxoimi dazolin-1-yl)piperidin-1-yl)-1,2,4-triazin-6-carboxamide (147)

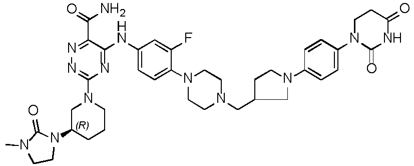

Intermediate 193 (43 mg, 0.15 mmol) was dissolved in 5 mL of DMF, and (R)-5-((3-fluoro-4-(piperazin-1-yl)phenyl)amino)-3-(3-(3-methyl-2-oxoimidazolin-1-yl)piperidin -1-yl)-1,2,4-triazin-6-carboxamide (90 mg, 0.18 mmol) was added to the system, and the mixture was stirred at room temperature for 10 min, then acetic acid (0.1 mL) and sodium cyanoborohydride (29 mg, 0.46 mmol) were added to the system and the mixture was reacted at room temperature for 1 h. After the reaction was completed, a solid was precipitated by adding water. Filtration was performed to obtain a yellow solid which was dissolved by adding DCM, and the resulting mixture was dried over anhydrous sodium sulfate, concentrated under reduced pressure, and subjected to column chromatography (eluted with DCM/MeOH) to obtain 5-((4-(4-((1-(4-(2,4-dioxotetrahydropyrimidin-1(2H)-yl)phenyl) pyrrolidin-3-yl)methyl)piperazin-1-yl)-3-fluorophenyl)amino)-3-((R)-3-(3-methyl-2-oxoimidazo lin-1-yl)piperidin-1-yl)-1,2,4-triazin-6 -carboxamide as a yellow solid (39 mg, yield 34%), ESI-MS: *m*/*z* = 770.4 [M+H]⁺. ¹H-NMR (400 MHz, DMSO-d₆) δ 11.23 (brs, 1H), 10.18 (brs, 1H), 8.31 (s, 1H),7.71 (s, 1H), 7.32 - 7.50 (m, 2H), 6.97 - 7.05 (m, 3H), 6.45 - 6.47 (m, 2H), 3.52 - 3.63 (m, 4H), 3.35 - 3.39 (m, 1H), 3.15 - 3.32 (m, 4H), 2.89 - 3.06 (m, 8H), 2.51 - 2.64 (m, 11H), 2.34 - 2.36 (m, 2H), 2.03 - 2.08 (m, 1H), 1.65 - 1.81 (m, 4H), 1.47 - 1.51 (m, 1H), 1.21 - 1.23 (m, 1H).

### Referring to the synthetic methods and routes for the above-mentioned Examples 115-125, the following compounds were synthesized:

| Example | Compound No. | Structural formula | Characterization data |
|---|---|---|---|
| 126 | 115 | 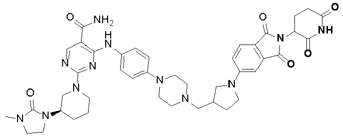 | ESI-MS: *m*/*z* = 819 [M+H]⁺ |
| 127 | 116 | 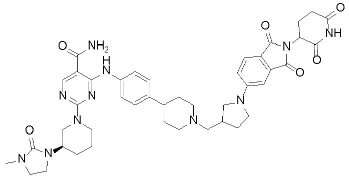 | ESI-MS: *m*/*z* = 818 [M+H]⁺ |
| | | | ¹H-NMR (400 MHz, CDCl₃) δ 10.88 (s, 1H), 8.29 (s, 1H), 8.24 (brs, 1H), 7.66 (d, *J* = 8.5 Hz, 1H), 7.58 (d, *J* = 8.6 Hz, 2H), 7.23 - 7.15 (m, 2H), 7.09 - 6.88 (m, 1H), 6.69 (dd, *J* = 8.5, 2.2 Hz, 1H), 5.74 (s, 1H), 4.94 (dd, *J* = 12.3, 5.3 Hz, 1H), 4.62 (s, 1H), 3.78 - 3.82 (m, 1H), 3.17 - 3.60 (m, 8H), 2.62 - 3.10 (m, 11H), 2.41 - 2.51 (m, 3H), 1.92 - 2.24 (m, 5H), 1.73 - 1.88 (m, 10H). |
| 128 | 117 | 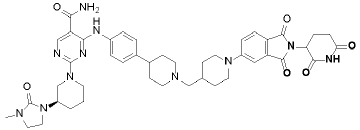 | ESI-MS: *m*/*z* = 832 [M+H]⁺ |
| | | | ¹H-NMR (400 MHz, CDCl₃) δ 10.88 (s, 1H), 8.34 (brs, 1H), 8.22 (s, 1H), 7.65 - 7.67 (m, 1H), 7.55 - 7.58 (m, 2H), 7.27 - 7.28 (m, 1H), 7.18 - 7.20 (m, 2H), 7.03 - 7.05 (m, 1H), 5.70 (brs, 1H), 4.91 - 4.96 (m, 1H), 4.60 - 4.63 (m, 1H), 3.94 - 3.97 (m, 2H), 3.77 - 3.79 (m, 1H), 3.27 - 3.39 (m, 4H), 2.70 - 3.02 (m, 12H), 1.62 - 2.48 (m, 21H). |
| 129 | 118 | 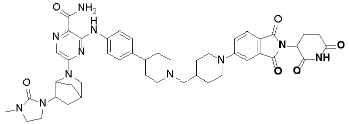 | ESI-MS: *m*/*z* = 844 [M+H]⁺ |
| | | | ¹H-NMR (400 MHz, CDCl₃) δ 10.91 (s, 1H), 8.16 (brs, 1H), 7.64 - 7.70 (m, 4H), 7.42 - 7.48 (m, 1H), 7.29 (d, *J* = 2.3 Hz, 1H), 7.14 (d, *J* = 7.8 Hz, 2H), 7.05 (dd, *J* = 8.6, 2.4 Hz, 1H), 5.15 - 5.23 (m, 1H), 4.94 (dd, *J* = 12.4, 5.3 Hz, 1H), 4.61 - 4.77 (brs, 1H), 3.96 (d, *J* = 12.9 Hz, 2H), 3.73 - 3.82 (m, 1H), 3.25 - 3.32 (m, 4H), 3.08 - 3.93 (m, 5H), 2.83 - 2.92 (m, 2H), 2.81 (s, 3H), 2.75 - 2.78 (m, 2H), 2.41 - 2.47 (m, 1H), 2.23 - 2.26 (m, 3H), 2.14 - 2.26 (m, 1H), 1.97 - 2.08 (m, 2H), 1.91 (d, *J* = 13.5 Hz, 4H), 1.74 - 1.85 (m, 9H). |
| 130 | 119 | 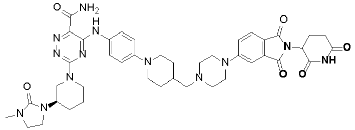 | ESI-MS: *m*/*z* = 834 [M+H]⁺ |
| | | | ¹H-NMR (400 MHz, CDCl₃) δ 10.68 (brs, 1H), 8.02 (brs, 1H), 7.68 - 7.72 (m, 2H), 7.51 - 7.54 (m, 2H), 7.29 - 7.29 (m, 1H), 7.04 - 7.07 (m, 1H), 6.95 - 6.97 (m, 2H), 5.34 - 5.38 (m, 1H), 4.92 - 4.96 (m, 1H), 3.62 - 3.78 (m, 4H), 3.28 - 3.45 (m, 8H), 2.67 - 2.92 (m, 8H), 2.57 - 2.60 (m, 4H), 2.10 - 2.29 (m, 4H), 1.82 - 2.03 (m, 6H), 1.66 - 1.81 (m, 5H). |
| 131 | 120 | 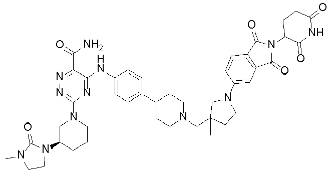 | ESI-MS: *m*/*z* = 833 [M+H]⁺ |
| | | | ¹H-NMR (400 MHz, CDCl₃) δ 10.86 (brs, 1H), 8.08 - 8.16 (m, 1H), 7.72 (brs, 1H), 7.64 - 7.66 (m, 1H), 7.58 - 7.60 (m, 2H), 7.21 - 7.24 (m, 2H), 6.94 (s, 1H), 6.66 - 6.69 (m, 1H), 5.52 (brs, 1H), 4.91 - 4.96 (m, 1H), 3.76 - 3.83 (m, 1H), 3.48 - 3.51 (m, 2H), 3.28 - 3.41 (m, 5H), 3.03 - 3.18 (m, 5H), 2.70 - 2.91 (m, 6H), 2.44 - 2.58 (m, 5H), 1.99 - 2.17 (m, 8H), 1.82 - 1.86 (m, 5H), 1.19 - 1.22 (m, 3H). |
| 132 | 121 | 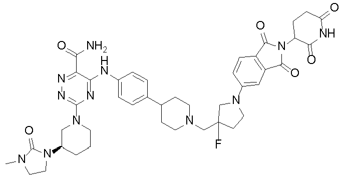 | ESI-MS: *m*/*z* = 837 [M+H]⁺ |
| | | | ¹H-NMR (400 MHz, CDCl₃) δ 10.89 (brs, 1H), 8.12 (brs, 1H), 7.68 - 7.73 (m, 2H), 7.59 - 7.61 (m, 2H), 7.24 - 7.25 (m, 2H), 6.98 (s, 1H), 6.71 - 6.73 (m, 1H), 5.46 (brs, 1H), 4.92 - 4.96 (m, 1H), 3.75 - 3.79 (m, 3H), 3.70 - 3.72 (m, 1H), 3.61 - 3.66 (m, 5H), 3.28 - 3.41 (m, 4H), 3.16 - 3.21 (m, 1H), 3.02 - 3.09 (m, 1H), 2.75 - 2.92 (m, 7H), 2.11 - 2.51 (m, 7H), 1.94 - 2.04 (m, 2H), 1.81 - 1.91 (m, 6H). |
| 133 | 122 | 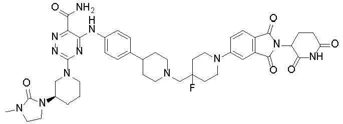 | ESI-MS: *m*/*z* = 851 [M+H]⁺ |
| | | | ¹H-NMR (400 MHz, CDCl₃) δ 10.89 (brs, 1H), 8.46 (brs, 1H), 7.67 - 7.73 (m, 2H), 7.57 - 7.59 (m, 2H), 7.30 - 7.31 (m, 1H), 7.21 - 7.23 (m, 2H), 7.06 - 7.09 (m, 1H), 5.62 (brs, 1H), 4.92 - 4.97 (m, 1H), 3.77 - 3.81 (m, 3H), 3.28 - 3.40 (m, 6H), 3.00 - 3.04 (m, 3H), 2.71 - 2.91 (m, 6H), 2.42 - 2.57 (m, 3H), 2.24 - 2.30 (m, 2H), 1.95 - 2.14 (m, 5H), 1.68 - 1.92 (m, 11H). |
| 134 | 123 | 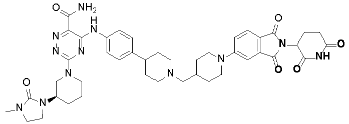 | ESI-MS: *m*/*z* = 833 [M+H]⁺ |
| | | | ¹H- NMR (400 MHz, CDCl₃) δ 10.91 (s, 1H), 8.55 (brs, 1H), 7.75 (brs, 1H), 7.67 (d, *J* = 8.6 Hz, 1H), 7.59 (d, *J* = 8.5 Hz, 2H), 7.25 (d, *J* = 2.3 Hz, 1H),7.23 (d, *J* = 8.6 Hz, 2H), 7.04 (dd, *J* = 8.7, 2.3 Hz, 1H), 5.64 (s, 1H), 4.95 (dd, *J* = 12.2, 5.3 Hz, 1H), 3.96 (d, *J* = 12.9 Hz, 2H), 3.79 - 3.83 (m, 1H), 3.29 - 3.42 (m, 5H), 3.07 - 3.11(m, 3H), 2.90 - 3.01 (m, 3H), 2.69 - 2.85 (m, 6H), 2.30 -2.36 (m, 2H), 2.10 -2.15 (m, 4H), 1.76 - 2.07 (m, 14H). |
| 135 | 124 | 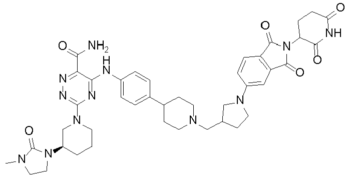 | ESI-MS: *m*/*z* = 819 [M+H]⁺ |
| | | | ¹H- NMR (400 MHz, CDCl₃) δ 10.90 (brs, 1H), 8.33 (brs, 1H), 7.72 (brs, 1H), 7.59 - 7.66 (m, 3H), 7.22 - 7.25 (m, 2H), 6.95 - 6.96 (m, 1H), 5.57 (brs, 1H), 4.91 - 4.95 (m, 1H), 3.77 - 3.83 (m, 1H), 3.60 - 3.72 (m, 2H), 3.07 - 3.51(m, 10H), 2.69 - 2.90 (m, 7H), 2.54 -2.57 (m, 2H), 2.10 -2.26 (m, 3H), 1.68 - 2.01 (m, 14H). |
| 136 | 125 | 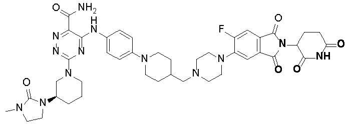 | ESI-MS: *m*/*z* = 852 [M+H]⁺ |
| | | | ¹H-NMR (400 MHz, CDCl₃) δ 10.69 (brs, 1H), 8.14 (brs, 1H), 7.71 (brs, 1H), 7.50 - 7.54 (m, 2H), 7.38 - 7.49 (m, 2H), 6.95 - 6.97 (m, 2H), 5.44 - 5.45 (m, 1H), 4.92 - 4.96 (m, 1H), 3.65 - 3.81 (m, 3H), 3.29 - 3.41 (m, 9H), 2.99 - 3.14 (m, 2H), 2.66 - 2.92 (m, 14H), 2.33 - 2.35 (m, 2H), 2.08 - 2.22 (m, 2H), 1.86 - 2.05 (m, 7H). |
| 137 | 126 | 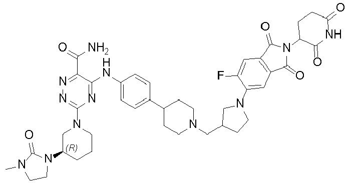 | ESI-MS: *m*/*z* = 837 [M+H]⁺ |
| | | | ¹H-NMR (400 MHz, CDCl₃) δ 10.82 (brs, 1H), 7.74 (brs, 1H), 7.59 - 7.61 (m, 2H), 7.36 - 7.40 (m, 1H), 7.22 - 7.26 (m, 3H), 7.03 - 7.04 (m, 1H), 5.63 (brs, 1H), 4.89 - 4.94 (m, 1H), 3.57 - 3.83 (m, 4H), 3.28 - 3.41 (m, 4H), 3.07 - 3.17 (m, 3H), 2.72 - 2.91 (m, 6H), 2.47 - 2.63 (m, 5H), 1.44 - 2.30 (m, 16H). |
| 138 | 128 | 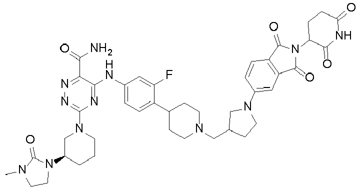 | ESI-MS: *m*/*z* = 837 [M+H]⁺ |
| | | | ¹H-NMR (400 MHz, CDCl₃) δ 11.00 (brs, 1H), 8.09 (brs, 1H), 7.70 - 7.72 (m, 1H), 7.64 - 7.67 (m, 1H), 7.51 - 7.56 (m, 1H), 7.22 - 7.32 (m, 2H), 6.96 - 6.97 (m, 1H), 6.68 - 6.71 (m, 1H), 5.52 (brs, 1H), 4.91 - 4.96 (m, 1H), 3.74 - 3.81 (m, 1H), 3.58 - 3.63 (m, 1H), 3.47 - 3.53 (m, 2H), 3.31 - 3.45 (m, 5H), 3.04 - 3.24 (m, 4H), 2.68 - 2.91 (m, 9H), 2.46 - 2.52 (m, 2H), 2.08 - 2.24 (m, 7H), 1.89 - 1.99 (m, 7H). |
| 139 | 129 | 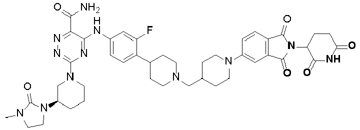 | ESI-MS: *m*/*z* = 851 [M+H]⁺ |
| | | | ¹H-NMR (400 MHz, CDCl₃) δ 11.00 (brs, 1H), 8.17 (brs, 1H), 7.72 (brs, 1H), 7.66 - 7.68 (m, 1H), 7.51 - 7.55 (m, 1H), 7.21 - 7.28 (m, 3H), 7.03 - 7.06 (m, 1H), 5.53 (brs, 1H), 4.91 - 4.96 (m, 1H), 3.94 - 3.96 (m, 2H), 3.75 - 3.80 (m, 1H), 3.30 - 3.42 (m, 4H), 3.08 - 3.15 (m, 3H), 2.94 - 3.00 (m, 3H), 2.71 - 2.91 (m, 8H), 2.32 - 2.37 (m, 2H), 2.11 - 2.23 (m, 4H) 1.86 - 2.03 (m, 13H). |
| 140 | 131 | 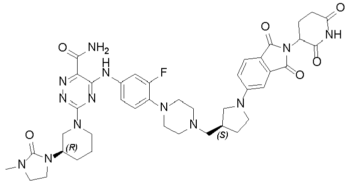 | ESI-MS: *m*/*z* = 838 [M+H]⁺ |
| | | | ¹H-NMR (400 MHz, CDCl₃) δ 10.88 (brs, 1H), 8.22 (s, 1H), 7.65 - 7.70 (m, 2H), 7.50 - 7.55 (m, 1H), 7.22 - 7.32 (m, 1H), 6.95 - 6.97 (m, 2H), 6.68 - 6.70 (m, 1H), 5.57 (brs, 1H), 4.91 - 4.96 (m, 1H), 3.75 - 3.81 (m, 1H), 3.10 - 3.61 (m, 13H), 2.65 - 2.61 (m, 11H), 2.45 - 2.52 (m, 2H), 1.82 - 2.35 (m, 10H) |
| 141 | 132 | 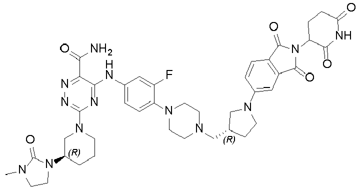 | ESI-MS: *m*/*z* = 838 [M+H]⁺ |
| | | | ¹H-NMR (400 MHz, CDCl₃) δ 10.89 (brs, 1H), 8.33 (s, 1H), 7.63 - 7.74 (m, 2H), 7.51 - 7.55 (m, 1H), 7.15 - 7.34 (m, 1H), 6.94 - 6.98 (m, 2H), 6.67 - 6.70 (m, 1H), 5.59 (brs, 1H), 4.91 - 4.96 (m, 1H), 3.75 - 3.80 (m, 1H), 3.09 - 3.60 (m, 13H), 2.43 - 2.89 (m, 13H), 2.09 - 2.25 (m, 2H), 1.65 - 2.01 (m, 8H) |
| 142 | 133 | 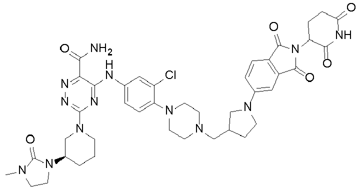 | ESI-MS: *m*/*z* = 854 [M+H]⁺ |
| 143 | 135 | 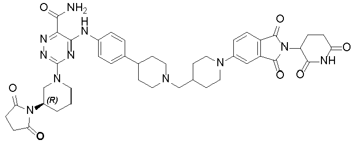 | ESI-MS: *m*/*z* = 832 [M+H]⁺ |
| | | | ¹H-NMR (400 MHz, CDCl₃) δ 10.90 (brs, 1H), 8.11 (brs, 1H), 7.66 - 7.71 (m, 2H), 7.53 - 7.57 (m, 2H), 7.20 - 7.28 (m, 3H), 7.03 - 7.05 (m, 1H), 5.47 (brs, 1H), 4.92 - 4.96 (m, 1H), 4.11 - 4.17 (m, 1H), 3.94 - 3.98 (m, 2H), 2.72 - 3.00 (m, 12H), 2.49 - 2.51 (m, 2H), 2.27 - 2.35 (m, 3H), 1.83 - 2.13 (m, 17H) |
| 144 | 136 | 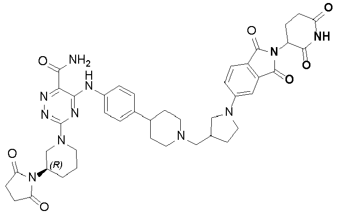 | ESI-MS: *m*/*z* = 818 [M+H]⁺ |
| | | | ¹H-NMR (400 MHz, CDCl₃) δ 10.91 (brs, 1H), 8.13 (brs, 1H), 7.65 - 7.72 (m, 2H), 7.54 - 7.57 (m, 2H), 7.21 - 7.22 (m, 2H), 6.96 - 6.97 (m, 1H), 6.68 - 6.71 (m, 1H), 5.45 (brs, 1H), 4.91 - 4.96 (m, 1H), 4.16 - 4.19 (m, 1H), 4.08 - 4.10 (m, 2H), 3.00 - 3.24 (m, 5H), 2.73 - 2.85 (m, 7H), 2.46 - 2.52 (m, 4H), 2.10 - 2.22 (m, 7H), 1.84 - 1.94 (m, 9H) |
| 145 | 137 | 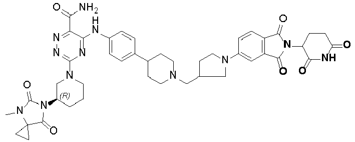 | ESI-MS: *m*/*z* = 859 [M+H]⁺ |
| | | | ¹H - NMR (400 MHz, CDCl₃) δ 10.91 (brs, 1H), 8.17 (brs, 1H), 7.72 (d, *J* = 3.8 Hz, 1H), 7.66 (d, *J* = 8.4 Hz, 1H), 7.56 - 7.62 (m, 2H), 7.18 - 7.24 (m, 2H), 6.97 (d, *J* = 2.0 Hz, 1H), 6.68 - 6.72 (m, 1H), 5.46 - 5.48 (m, 1H), 4.93 - 4.96 (m, 1H), 4.13 - 4.21 (m, 1H), 3.42 - 3.62 (m, 4H), 3.21 - 3.24 (m, 1H), 2.94 - 3.14(m, 4H), 2.68 - 2.91 (m, 8H), 2.41 - 2.55 (m, 4H), 2.13 -2.26 (m, 4H), 1.83 -1.98 (m, 8H), 1.36 - 1.43 (m, 4H). |
| 146 | 138 | 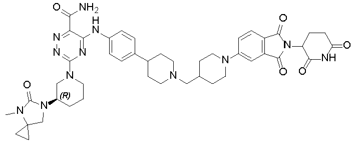 | ESI-MS: *m*/*z* = 859 [M+H]⁺ |
| | | | ¹H-NMR (400 MHz, CDCl₃) δ 10.91 (s, 1H), 8.10 (brs, 1H), 7.72 - 7.74 (m, 1H), 7.66 - 7.71 (m, 1H), 7.59 - 7.62 (m, 2H), 7.28 - 7.29 (m, 1H), 7.23 - 7.25 (m, 2H), 7.04 - 7.06 (m, 1H), 5.42 - 5.43 (m, 1H), 4.92 - 4.96 (m, 1H), 3.95 - 3.98 (m, 2H), 3.87 - 3.89 (m, 1H), 3.45 - 3.46 (m, 1H), 3.38 - 3.40 (m, 1H), 2.73 - 3.05 (m, 8H), 2.47 - 2.56 (m, 4H), 2.10 - 2.32 (m, 6H), 1.77 - 2.06 (m, 15H), 1.02 - 1.05 (m, 2H), 0.50 - 0.51 (m, 2H). |
| 147 | 140 | 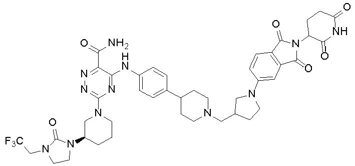 | ESI-MS: *m*/*z* = 887 [M+H]⁺ |
| | | | ¹H-NMR (400 MHz, CDCl₃) δ 10.89 (brs, 1H), 8.07 (brs, 1H), 7.70 - 7.73 (m, 1H), 7.58 - 7.66 (m, 3H), 7.22 - 7.25 (m, 2H), 6.95 - 6.97 (m, 1H), 6.68 - 6.71 (m, 1H), 5.46 (brs, 1H), 4.90 - 4.95 (m, 1H), 3.81 - 3.90 (m, 1H), 3.39 - 3.64 (m, 8H), 2.97 - 3.24 (m, 5H), 2.65 - 2.90 (m, 4H), 2.42 - 2.56 (m, 3H), 2.20 - 2.23 (m, 2H), 2.09 - 2.16 (m, 2H), 1.99 - 2.07 (m, 3H), 1.80 - 1.94 (m, 9H). |
| 148 | 141 | 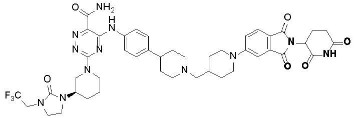 | ESI-MS: *m*/*z* = 901 [M+H]⁺ |
| | | | ¹H-NMR (400 MHz, CDCl₃) δ 10.90 (brs, 1H), 8.06 (brs, 1H), 7.66 - 7.71 (m, 2H), 7.58 - 7.61 (m, 2H), 7.27 - 7.28 (m, 1H), 7.22 - 7.24 (m, 2H), 7.04 - 7.06 (m, 1H), 5.43 (brs, 1H), 4.93 - 4.96 (m, 1H), 3.94 - 3.97 (m, 2H), 3.83 - 3.85 (m, 1H), 3.45 - 3.51 (m, 4H), 3.19 - 3.22 (m, 2H), 2.73 - 3.02 (m, 7H), 2.54 - 2.56 (m, 1H), 2.43 - 2.46 (m, 2H), 2.19 - 2.27 (m, 4H), 2.11 - 2.15 (m, 2H), 1.88 - 1.99 (m, 14H). |
| 149 | 142 | 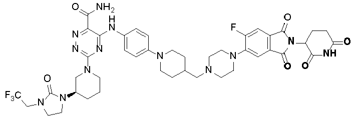 | ESI-MS: *m*/*z* = 920 [M+H]⁺ |
| 150 | 143 | 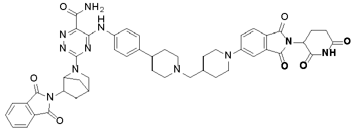 | ESI-MS: *m*/*z* = 892 [M+H]⁺ |
| | | | ¹H-NMR (400 MHz, CDCl₃) δ 10.84 - 11.00 (m, 1H), 8.27 (brs, 1H), 7.87 - 7.90 (m, 1H), 7.80 - 7.82 (m, 1H), 7.73 - 7.76 (m, 2H), 7.70 - 7.72 (m, 1H), 7.66 - 7.69 (m, 2H), 7.59 - 7.60 (m, 1H), 7.27 - 7.30 (m, 1H), 7.21 - 7.23 (m, 1H), 7.03 - 7.06 (m, 1H), 6.97 - 7.00 (m, 1H), 5.47 (brs, 1H), 4.92 - 4.96 (m, 2H), 4.40 - 4.45 (m, 1H), 3.95 - 3.98 (m, 2H), 3.56 - 3.70 (m, 1H), 3.29 - 3.49 (m, 1H), 2.71 - 3.02 (m, 10H), 2.47 - 2.60 (m, 2H), 2.08 - 2.34 (m, 7H), 1.81 - 1.94 (m, 8H). |
| 151 | 144 | 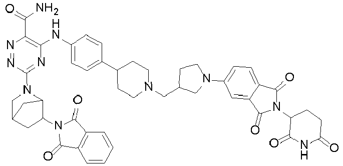 | ESI-MS: *m*/*z* = 878 [M+H]⁺ |
| 152 | 151 | 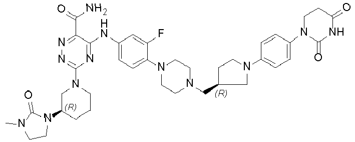 | ESI-MS: *m*/*z* = 813 [M+H]⁺ |
| 153 | 152 | 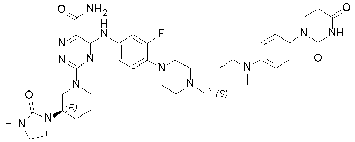 | ESI-MS: *m*/*z* = 813 [M+H]⁺ |
| 154 | 155 | 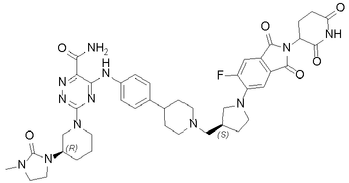 | ESI-MS: *m*/*z* = 837 [M+H]⁺ |
| 155 | 156 | 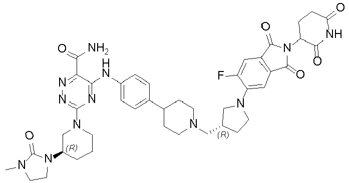 | ESI-MS: *m*/*z* = 837 [M+H]⁺ |
| 156 | 157 | 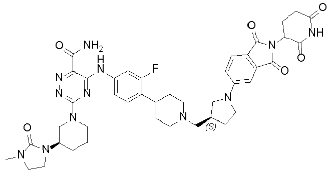 | ESI-MS: *m*/*z* = 837 [M+H]⁺ |
| 157 | 158 | 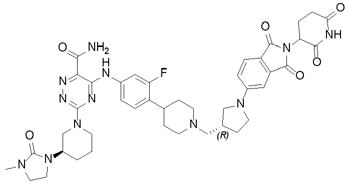 | ESI-MS: *m*/*z* = 837 [M+H]⁺ |
| 158 | 159 | 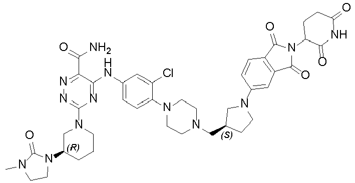 | ESI-MS: *m*/*z* = 854 [M+H]⁺ |
| 159 | 160 | 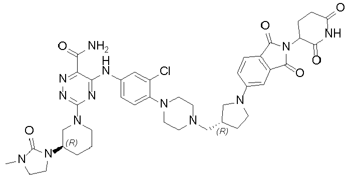 | ESI-MS: *m*/*z* = 854 [M+H]⁺ |
| 160 | 161 | 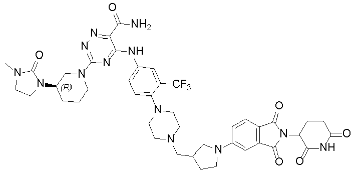 | ESI-MS: *m*/*z* = 888 [M+H]⁺ |
| 161 | 162 | 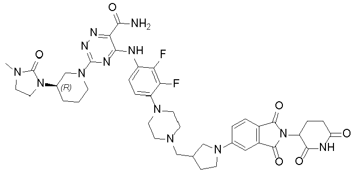 | ESI-MS: *m*/*z* = 856 [M+H]⁺ |
| 162 | 163 | 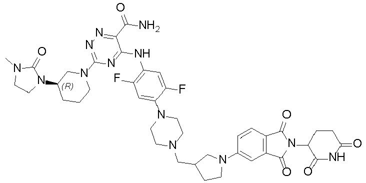 | ESI-MS: *m*/*z* = 856 [M+H]⁺ |
| 163 | 164 | 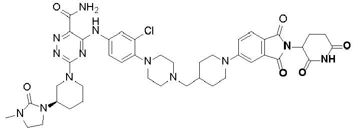 | ESI-MS: *m*/*z* = 868 [M+H]⁺ |
| 164 | 165 | 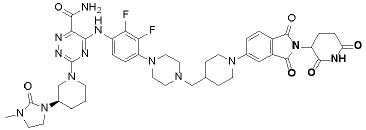 | ESI-MS: *m*/*z* = 870 [M+H]⁺ |
| 165 | 166 | 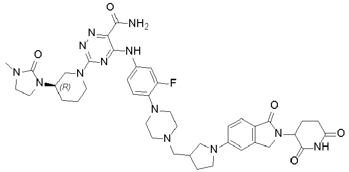 | ESI-MS: *m*/*z* = 824 [M+H]⁺ |
| 166 | 167 | 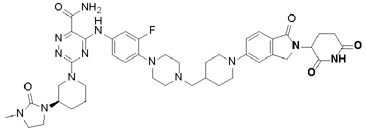 | ESI-MS: *m*/*z* = 838 [M+H]⁺ |
| 167 | 168 | 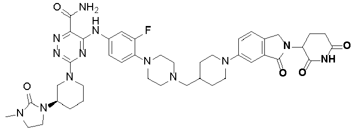 | ESI-MS: *m*/*z* = 838 [M+H]⁺ |
| 168 | 169 | 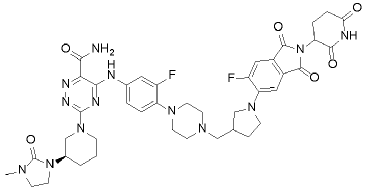 | ESI-MS: *m*/*z* = 856 [M+H]⁺ |
| 169 | 170 | 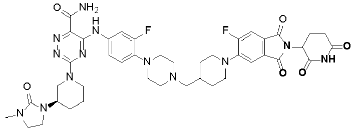 | ESI-MS: *m*/*z* = 870 [M+H]⁺ |
| 170 | 153 | 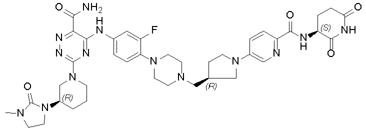 | ESI-MS: *m*/*z* = 813 [M+H]⁺. |
| 171 | 154 | 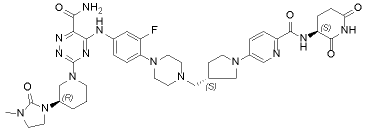 | ESI-MS: *m*/*z* = 813 [M+H]⁺. |
| 172 | 171 | 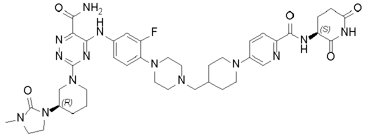 | ESI-MS: *m*/*z* = 827 [M+H]⁺ |
| 173 | 172 | 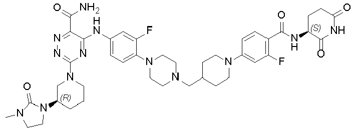 | ESI-MS: *m*/*z* = 844 [M+H]⁺ |
| 174 | 173 | 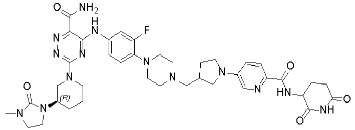 | ESI-MS: *m*/*z* = 813 [M+H]⁺ |
| 175 | 174 | 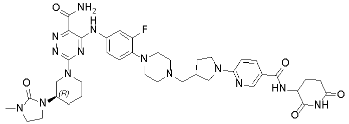 | ESI-MS: *m*/*z* = 813 [M+H]⁺ |
| 176 | 175 | 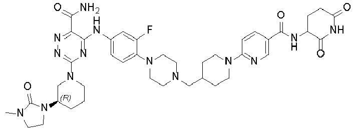 | ESI-MS: *m*/*z* = 827 [M+H]⁺ |
| 177 | 176 | 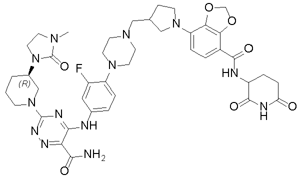 | ESI-MS: *m*/*z* = 856 [M+H]⁺ |
| 178 | 177 | 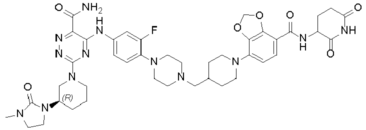 | ESI-MS: *m*/*z* = 870 [M+H]⁺ |
| 179 | 178 | 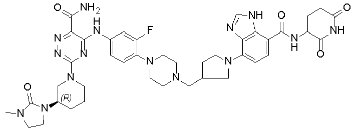 | ESI-MS: *m*/*z* = 852 [M+H]⁺ |
| 180 | 179 | 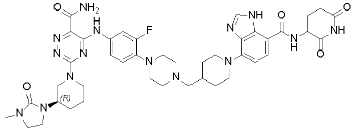 | ESI-MS: *m*/*z* = 866 [M+H]⁺ |
| 181 | 180 | 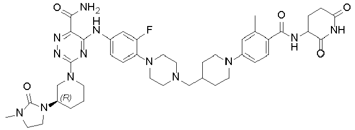 | ESI-MS: *m*/*z* = 840 [M+H]⁺ |
| 182 | 181 | 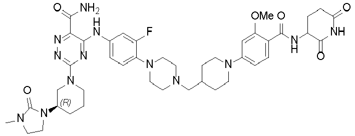 | ESI-MS: *m*/*z* = 856 [M+H]⁺ |
| 183 | 182 | 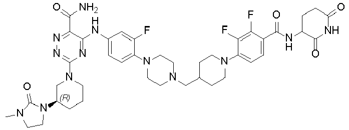 | ESI-MS: *m*/*z* = 862 [M+H]⁺ |
| 184 | 183 | 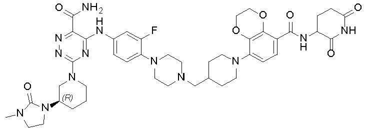 | ESI-MS: *m*/*z* = 884 [M+H]⁺ |
| 185 | 184 | 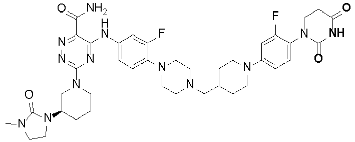 | ESI-MS: *m*/*z* = 802 [M+H]⁺ |
| 186 | 185 | 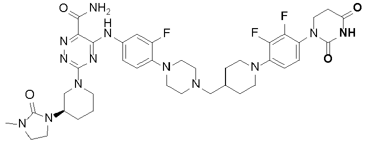 | ESI-MS: *m*/*z* = 820 [M+H]⁺ |
| 187 | 186 | 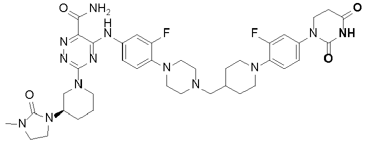 | ESI-MS: *m*/*z* = 802 [M+H]⁺ |
| 188 | 187 | 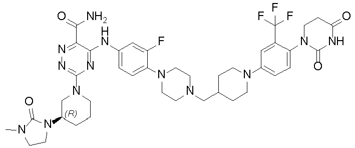 | ESI-MS: *m*/*z* = 852 [M+H]⁺ |
| 189 | 188 | 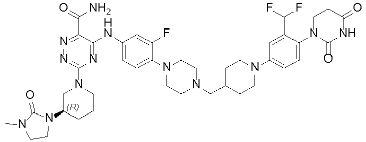 | ESI-MS: *m*/*z* = 834 [M+H]⁺ |
| 190 | 189 | 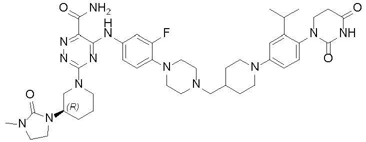 | ESI-MS: *m*/*z* = 826 [M+H]⁺ |
| 191 | 190 | 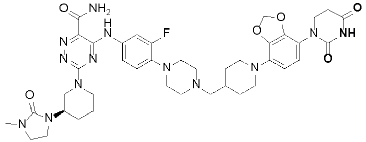 | ESI-MS: *m*/*z* = 828 [M+H]⁺ |
| 192 | 191 | 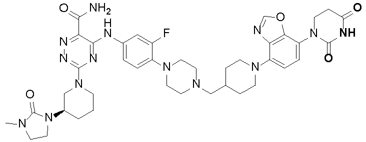 | ESI-MS: *m*/*z* = 825 [M+H]⁺ |
| 193 | 192 | 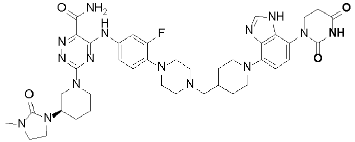 | ESI-MS: *m*/*z* = 824 [M+H]⁺ |
| 194 | 193 | 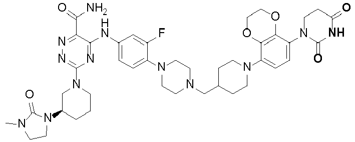 | ESI-MS: *m*/*z* = 842 [M+H]⁺ |
| 195 | 194 | 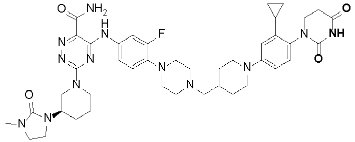 | ESI-MS: *m*/*z* = 824 [M+H]⁺ |
| 196 | 195 | 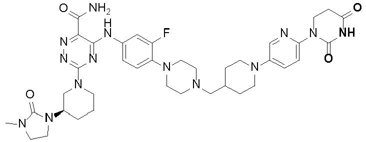 | ESI-MS: *m*/*z* = 785 [M+H]⁺ |
| 197 | 196 | 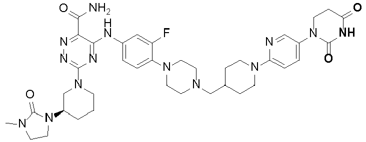 | ESI-MS: *m*/*z* = 785 [M+H]⁺ |
| 198 | 197 | 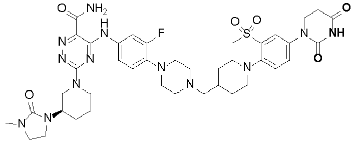 | ESI-MS: *m*/*z* = 862 [M+H]⁺ |
| 199 | 198 | 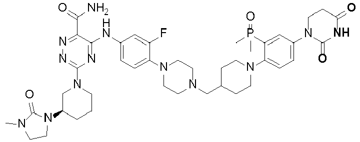 | ESI-MS: *m*/*z* = 860 [M+H]⁺ |
| 200 | 199 | 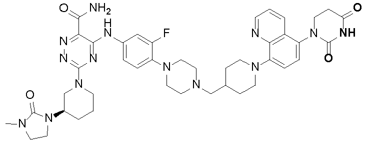 | ESI-MS: *m*/*z* = 835 [M+H]⁺ |
| 201 | 200 | 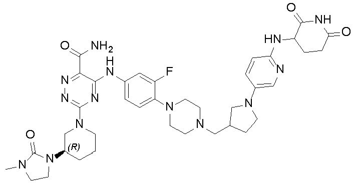 | ESI-MS: *m*/*z* = 785 [M+H]⁺ |
| 202 | 201 | 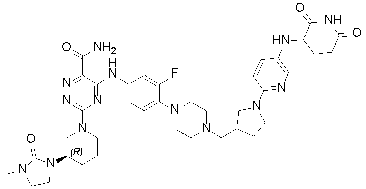 | ESI-MS: *m*/*z* = 785 [M+H]⁺ |
| 203 | 202 | 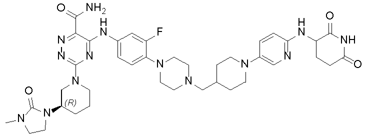 | ESI-MS: *m*/*z* = 799 [M+H]⁺ |
| 204 | 203 | 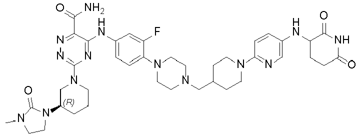 | ESI-MS: *m*/*z* = 799 [M+H]⁺ |
| 205 | 204 | 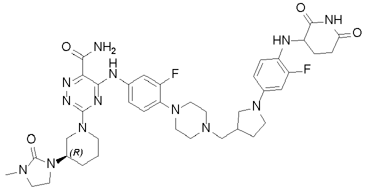 | ESI-MS: *m*/*z* = 802 [M+H]⁺ |
| 206 | 205 | 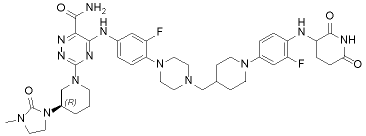 | ESI-MS: *m*/*z* = 816 [M+H]⁺ |
| 207 | 206 | 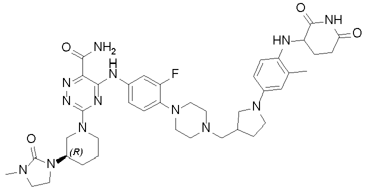 | ESI-MS: *m*/*z* = 798 [M+H]⁺ |
| 208 | 207 | 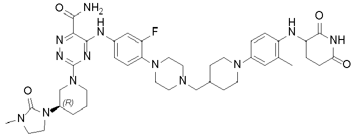 | ESI-MS: *m*/*z* = 812 [M+H]⁺ |
| 209 | 208 | 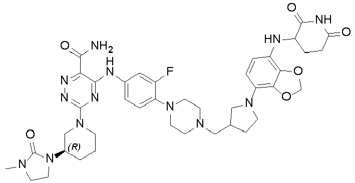 | ESI-MS: *m*/*z* = 828 [M+H]⁺ |
| 210 | 209 | 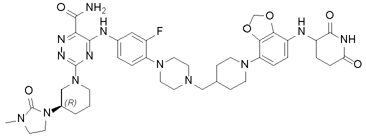 | ESI-MS: *m*/*z* = 842 [M+H]⁺ |
| 211 | 210 | 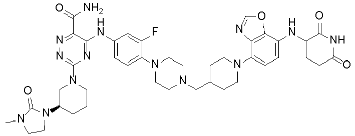 | ESI-MS: *m*/*z* = 839 [M+H]⁺ |
| 212 | 211 | 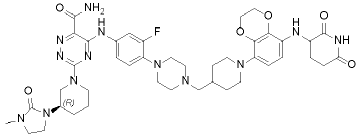 | ESI-MS: *m*/*z* = 856 [M+H]⁺ |
| 213 | 212 | 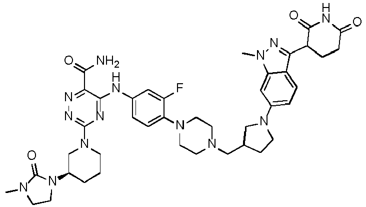 | ESI-MS: *m*/*z* = 823 [M+H]⁺ |
| 214 | 213 | 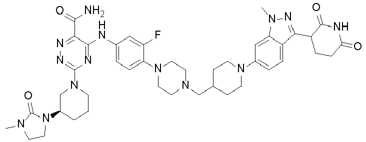 | ESI-MS: *m*/*z* = 837 [M+H]⁺ |
| 215 | 214 | 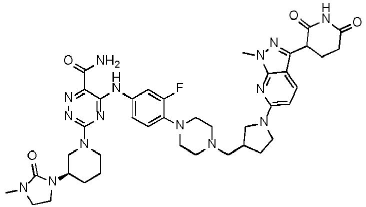 | ESI-MS: *m*/*z* = 824 [M+H]⁺ |
| 216 | 215 | 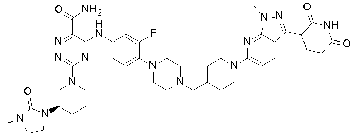 | ESI-MS: *m*/*z* = 838 [M+H]⁺ |
| 217 | 216 | 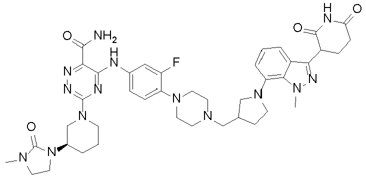 | ESI-MS: *m*/*z* = 823 [M+H]⁺ |
| 218 | 217 | 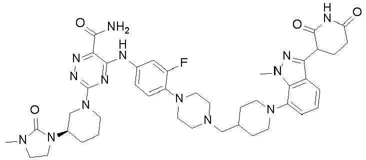 | ESI-MS: *m*/*z* = 837 [M+H]⁺ |
| 219 | 218 | 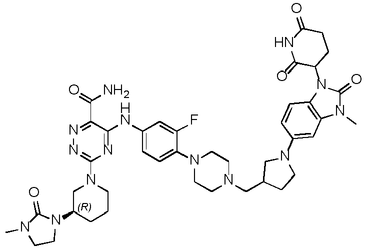 | ESI-MS: *m*/*z* = 839 [M+H]⁺ |
| 220 | 219 | 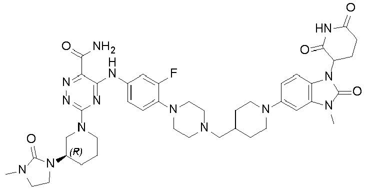 | ESI-MS: *m*/*z* = 853 [M+H]⁺ |
| 221 | 220 | 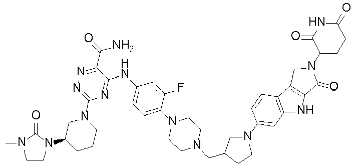 | ESI-MS: *m*/*z* = 863 [M+H]⁺ |
| 222 | 221 | 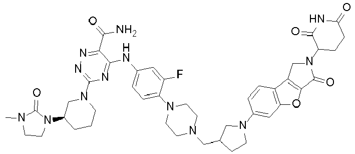 | ESI-MS: *m*/*z* = 864 [M+H]⁺ |
| 223 | 222 | 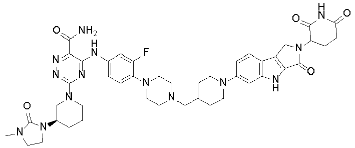 | ESI-MS: *m*/*z* = 877 [M+H]⁺ |
| 224 | 223 | 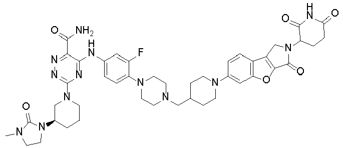 | ESI-MS: *m*/*z* = 878 [M+H]⁺ |
| 225 | 231 | 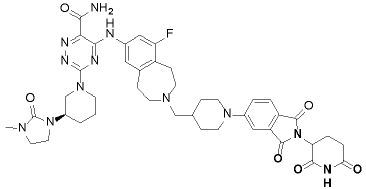 | ESI-MS: *m*/*z* = 837 [M+H]⁺ |
| 226 | 232 | 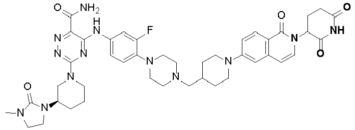 | ESI-MS: *m*/*z* = 850 [M+H]⁺ |
| 227 | 233 | 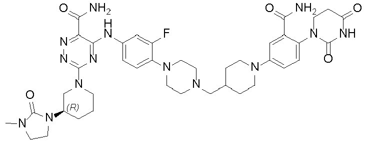 | ESI-MS: *m*/*z* = 827 [M+H]⁺ |
| 228 | 234 | 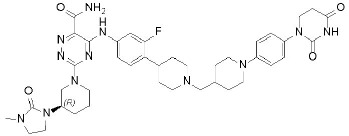 | ESI-MS: *m*/*z* = 783 [M+H]⁺ |
| 229 | 235 | 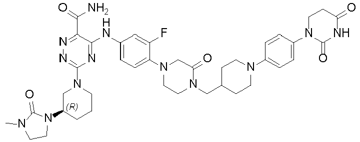 | ESI-MS: *m*/*z* = 798 [M+H]⁺ |
| 230 | 236 | 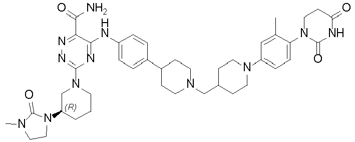 | ESI-MS: *m*/*z* = 779 [M+H]⁺ |
| 231 | 237 | 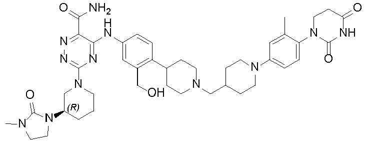 | ESI-MS: *m*/*z* = 809 [M+H]⁺ |
| 232 | 238 | 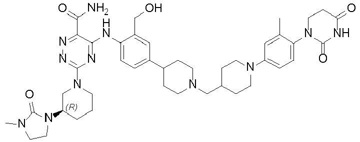 | ESI-MS: *m*/*z* = 809 [M+H]⁺ |
| 233 | 240 | 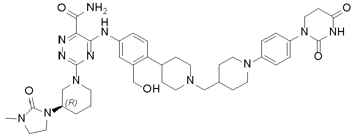 | ESI-MS: *m*/*z* = 795 [M+H]⁺ |
| 234 | 241 | 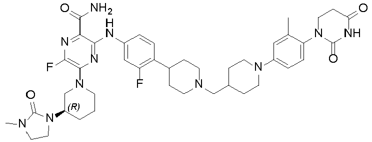 | ESI-MS: *m*/*z* = 814 [M+H]⁺ |
| 235 | 242 | 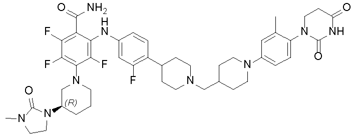 | ESI-MS: *m*/*z* = 848 [M+H]⁺ |
| 236 | 243 | 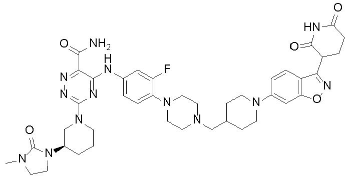 | ESI-MS: *m*/*z* = 824 [M+H]⁺ |
| 237 | 244 | 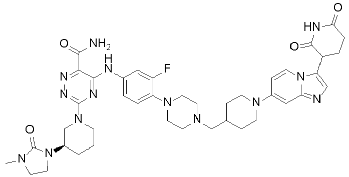 | ESI-MS: *m*/*z* = 823 [M+H]⁺ |
| 238 | 245 | 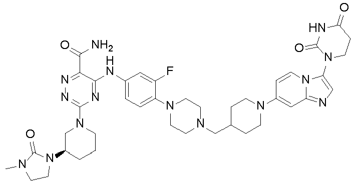 | ESI-MS: *m*/*z* = 824 [M+H]⁺ |
| 239 | 247 | 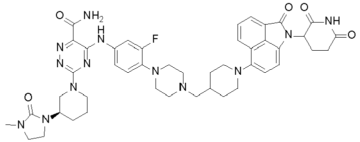 | ESI-MS: *m*/*z* = 874 [M+H]⁺ |
| 240 | 249 | 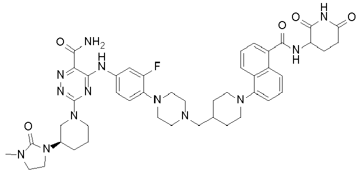 | ESI-MS: *m*/*z* = 876 [M+H]⁺ |
| 241 | 250 | 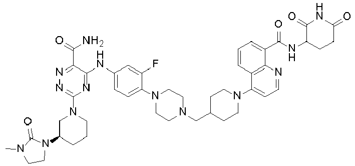 | ESI-MS: *m*/*z* = 877 [M+H]⁺ |
| 242 | 251 | 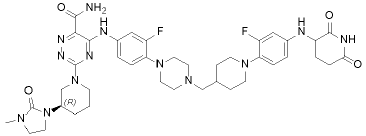 | ESI-MS: *m*/*z* = 816 [M+H]⁺ |
| 243 | 252 | 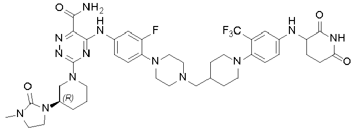 | ESI-MS: *m*/*z* = 866 [M+H]⁺ |
| 244 | 253 | 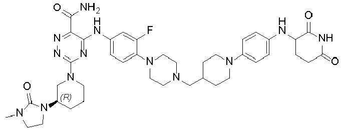 | ESI-MS: *m*/*z* = 798 [M+H]⁺ |

### Example 245: Methyl (3-(5-(3-((4-(4-((6-carbamoyl-3-((R)-3-(3-methyl-2-oxoimidazolin-1-yl)piperidin-1-yl)-1,2,4-triazin-5-yl)amino)phenyl)piperidin-1-yl)methyl)p yrrolidin-1-yl)-1,3-dioxoisoindolin-2-yl)-2,6-dioxopiperidin-1-yl)butyrate (139)

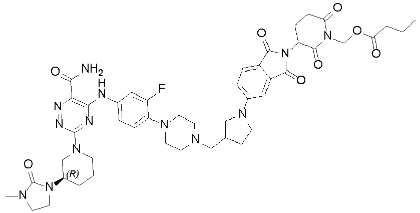

Compound 130 (100 mg, 0.12 mmol) was dissolved in 2 mL of DMF, and cesium carbonate (46 mg, 0.14 mmol) and chloromethyl butyrate (18 mg, 0.12 mmol) were added to the system in sequence. The mixture was reacted at room temperature for 12 h. After the reaction was completed, the reaction liquid was quenched by adding water, and extracted with ethyl acetate three times. The organic phases were combined, dried over anhydrous sodium sulfate, and concentrated under reduced pressure to obtain methyl (3-(5-(3-((4-(4-((6-carbamoyl-3-((R)-3-(3-methyl-2-oxoimidazolin-1-yl)piperidin-1-yl)-1,2,4-tria zin-5-yl)amino)phenyl)piperidin-1-yl)methyl)pyrrolidin-1-yl)-1,3-dioxoisoindolin-2-yl)-2,6-diox opiperidin-1-yl)butyrate as a yellow solid (30 mg, yield 26%). ESI-MS: *m*/*z* = 938 [M+H]⁺. ¹H-NMR (400 MHz, CDCl₃) δ 10.88 (brs, 1H), 7.69 (brs, 1H), 7.58 - 7.65 (m, 3H), 7.24 - 7.26 (m, 2H), 6.96 - 6.97 (m, 1H), 6.68 - 6.71 (m, 1H), 5.80 - 5.87 (m, 2H), 5.46 (brs, 1H), 4.96 - 5.01 (m, 1H), 3.77 - 3.80 (m, 1H), 3.19 - 3.61 (m, 9H), 2.95 - 3.10 (m, 4H), 2.79 - 2.86 (m, 4H), 2.62 - 2.89 (m, 1H), 2.42 - 2.50 (m, 3H), 2.26 - 2.31 (m, 2H), 1.81 - 2.24 (m, 13H), 1.60 - 1.66 (m, 2H), 0.91 - 0.95 (m, 3H).

### Example 246: 5-((4-(4-((1-(3-(2,4-dioxotetrahydropyrimidin-1(2H)-yl)-4-methylbenzoyl)pyrrolidin-3-yl)methyl)piperazin-1-yl)-3-fluorophenyl)amino)-3-((R)-3-(3-m ethyl-2-oxoimidazolin-1-yl)piperidin-1-yl)-1,2,4-triazin-6-carboxamide (150)

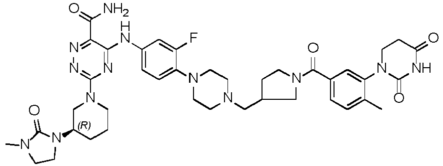

### Synthesis step 1: Tert-butyl 3-((4-(4-((6-carbamoyl-3-((R)-3-(3-methyl-2-oxoimidazolin -1-yl)piperidin-1-yl)-1,2,4-triazin-5-yl)amino)-2-fluorophenyl)piperazin-1-yl)methyl)pyrrolidin-1-carboxylate

Intermediate 165 (150 mg, 0.25 mmol) was dissolved in 2 mL of DCM, and TFA (1.5 mL) was added to the system. The mixture was stirred at room temperature for 1 h. The reaction was completed and concentrated under reduced pressure to obtain (R)-5-((3-fluoro-4-(piperazin-1-yl)phenyl)amino)-3-(3-(3-methyl-2-oxoimidazolin-1-yl)piperidin -1-yl)-1,2,4-triazin-6-carboxamide as a yellow liquid (120 mg). The above product was dissolved in 2 mL of DMF, and DIPEA (100 mg, 0.75 mmol) and tert-butyl 3-formylpyrrolidin-1-carboxylate (102 mg, 0.51 mmol) were added in sequence. The mixture was stirred at room temperature for 10 min, then acetic acid (0.1 mL) and sodium cyanoborohydride (50 mg, 0.75 mmol) were added to the system and the mixture was reacted at room temperature for 1 h. After the reaction was completed, the reaction liquid was adjusted to be alkaline by adding saturated aqueous sodium bicarbonate solution. A solid was precipitated. Filtration was performed to obtain a yellow solid which was dissolved by adding DCM, and the resulting mixture was dried over anhydrous sodium sulfate, concentrated under reduced pressure, and subjected to column chromatography (eluted with DCM/MeOH) to obtain tert-butyl 3-((4-(4-((6-carbamoyl-3-((R)-3-(3-methyl-2-oxoimidazolin-1-yl)piperidin-1-yl)-1,2,4-triazin-5-yl)amino)-2-fluorophenyl)piperazin-1-yl)methyl)pyrrolidin-1-carboxylate as a yellow solid (169 mg, yield 98%). ESI-MS: *m*/*z* = 682 [M+H]⁺.

### Synthesis step 2: 5-((4-(4-((1-(3-(2,4-dioxotetrahydropyrimidin-1(2H)-yl) -4-methylbenzoyl)pyrrolidin-3-yl)methyl)piperazin-1-yl)-3-fluorophenyl)amino)-3-((R)-3-(3 -methyl-2-oxoimidazolin-1-yl)piperidin-1-yl)-1,2,4-triazin-6-carboxamide

Tert-butyl 3-((4-(4-((6-carbamoyl-3-((R)-3-(3-methyl-2-oxoimidazolin-1-yl)piperidin-1-yl)-1,2,4-triazin-5-yl)amino)-2-fluorophenyl)piperazin-1-yl)methyl)pyrrolidin-1-carboxylate (123 mg, 0.18 mmol) was dissolved in 2 mL of DMF, and DIPEA (100 mg, 0.75 mmol), Intermediate 187 (44 mg, 0.18 mmol), N-HBTU (62 mg, 0.16 mmol) were added in sequence. The mixture was reacted at room temperature for 30 min. After the reaction was completed, a solid was precipitated by adding water. Filtration was performed to obtain a light yellow solid which was dissolved by adding DCM. The resulting mixture was dried over anhydrous sodium sulfate, and concentrated under reduced pressure. Column chromatography (eluted with DCM/MeOH) was performed to obtain 5-((4-(4-((1-(3-(2,4-dioxotetrahydropyrimidin-1(2*H*)-yl)-4- methylbenzoyl)pyrrolidin-3-yl) methyl)piperazin -1-yl)-3-fluorophenyl)amino)-3-((*R*)-3-(3-methyl-2-oxoimidazolin-1-yl)piperidin-1-yl)-1,2,4-tria zin-6-carboxamide as a yellow solid (12 mg, yield 10%). ESI-MS: *m*/*z* = 812 [M+H]⁺. ¹H-NMR (400 MHz, CDCl₃) δ 10.85 (brs, 1H), 8.09 (brs, 1H), 7.67 - 7.69 (m, 2H), 7.31 - 7.43 (m, 4H), 6.94 - 6.96 (m, 1H), 5.54 (brs, 1H), 3.58 - 3.87 (m, 6H), 3.29 - 3.41 (m, 5H), 3.12 - 3.17 (m, 5H), 2.79 - 2.86 (m, 7H), 2.56 - 2.62 (m, 5H), 2.30 (s, 3H), 1.63 - 2.13 (m, 9H).

### Example 247: 5-((4-(4-((1-(3-(2,4-dioxotetrahydropyrimidin-1(2H)-yl)-4-fluorobenzoyl) pyrrolidin-3-yl)methyl)piperazin-1-yl)-3-fluorophenyl)amino)-3-((R)-3-(3-methyl-2-oxoimid azolin-1-yl)piperidin-1-yl)-1,2,4-triazin-6-carboxamide (148)

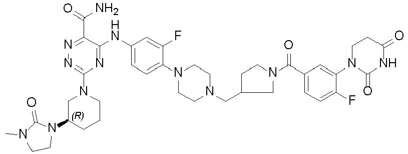

Referring to the synthetic route and method for Example 42, and Intermediate 187 was replaced with Intermediate 189 to synthesize and obtain target compound 148. ESI-MS:m/z = 816 [M+H]+. ¹H-NMR (400 MHz, DMSO-d6) δ 11.26 (brs, 1H), 10.52 (brs, 1H), 8.34 (s, 1H), 7.73 (s, 1H), 7.60 - 7.65 (m, 1H), 7.50 - 7.54 (m, 2H), 7.32 - 7.39 (m, 2H), 6.96 - 7.04 (m, 1H), 3.72 - 3.75 (m, 3H), 3.43 - 3.61 (m, 6H), 3.14 - 3.25 (m, 6H), 2.87 - 2.99 (m, 6H), 2.63 - 2.73 (m, 7H), 2.28 - 2.40 (m, 2H), 1.77 - 2.01 (m, 5H), 1.50 - 1.63 (m, 2H).

### Example 248: 5-((4-(4-((1-(3-(2,4-dioxotetrahydropyrimidin-1(2H)-yl)-4-methoxybenzoyl) pyrrolidin-3-yl)methyl)piperazin-1-yl)-3-fluorophenyl)amino)-3-((R)-3-(3-methyl-2-oxoimid azolin-1-yl)piperidin-1-yl)-1,2,4-triazin-6-carboxamide (149)

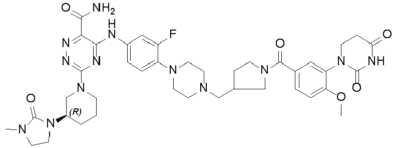

Referring to the synthetic route and method for Example 34, and Intermediate 187 was replaced with Intermediate 188 to synthesize and obtain target compound 149. ESI-MS:m/z = 828 [M+H]+. ¹H-NMR (400 MHz, DMSO-d6) δ 11.29 (brs, 1H), 10.33 (brs, 1H), 8.34 (s, 1H), 7.74 (s, 1H), 7.44 - 7.56 (m, 4H), 7.04 - 7.18 (m, 2H), 4.12 - 4.13 (m, 1H), 3.82 - 3.83 (m, 5H), 3.54 - 3.62 (m, 9H), 3.07 - 3.16 (m, 6H), 2.92 - 2.98 (m, 2H), 2.64 - 2.67 (m, 8H), 1.26 - 2.17 (m, 9H).

### Example 249: 5-((4-(4-(3-(6-((2,6-dioxopiperidin-3-yl)carbamoyl)pyridin-3-yl) propan-2-yn-1-yl)piperazin-1-yl)-3-fluorophenyl)amino)-3-((R)-3-(3-methyl-2-oxoimidazoli din-1-yl)piperidin-1-yl)-1,2,4-triazin-6-carboxamide (224)

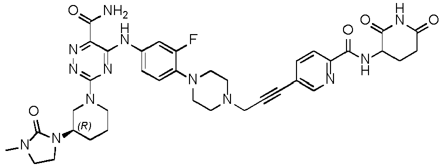

(R)-5-(3-fluoro-4-(piperazin-1-yl)phenyl)amino)-3-(3-(3-methyl-2-oxoimidazolin-1-yl)pi peridin-1-yl)-1,2,4-triazin-6-carboxamide (100 mg, 0.20 mmol) was dissolved in 5 mL of DCM, and Intermediate 263 (88 mg, 0.24 mmol) and DIPEA (50 mg, 0.36 mmol) were added to the system in sequence. The mixture was reacted at room temperature for 1 h. After the reaction was completed, a solid was precipitated by adding water and filtration was performed to obtain 5-((4-(4-(3-(6-((2,6-dioxopiperidin-3-yl)carbamoyl)pyridin-3-yl)propan-2-yn-1-yl)piperazin-1-yl )-3-fluorophenyl)amino)-3-((R)-3-(3-methyl-2-oxoimidazolidin-1-yl)piperidin-1-yl)-1,2,4-triazin -6-carboxamide as a yellow solid which was dissolved by adding DCM, and the resulting mixture was dried over anhydrous sodium sulfate, concentrate under reduced pressure, and subjected to column chromatography (eluted with DCM/MeOH) to obtain a yellow solid (70 mg, yield 46%). ESI-MS (M+H) ⁺=768.

### Referring to the synthetic methods and routes for the above-mentioned Example 249, the following compounds were synthesized:

| EXAMPLES | Compound No. | Structural formula | Characterization data |
|---|---|---|---|
| 250 | 225 | 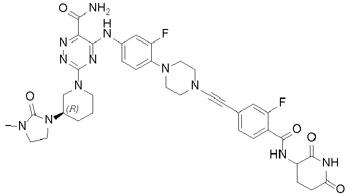 | ESI-MS: *m*/*z* = 771 [M+H]⁺ |
| 251 | 226 | 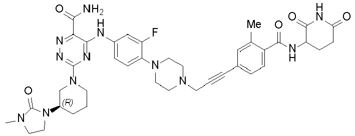 | ESI-MS: *m*/*z* = 781 [M+H]⁺ |
| 252 | 227 | 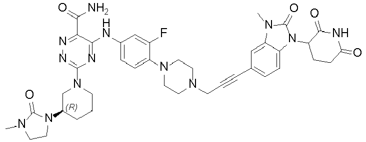 | ESI-MS: *m*/*z* = 794 [M+H]⁺ |
| 253 | 228 | 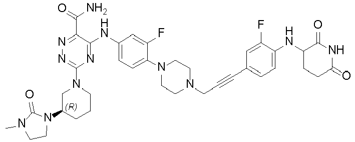 | ESI-MS: *m*/*z* = 757 [M+H]⁺ |
| 254 | 229 | 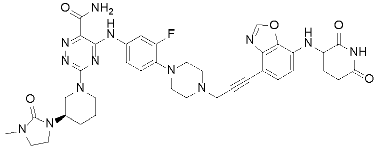 | ESI-MS: *m*/*z* = 780 [M+H]⁺ |
| 255 | 230 | 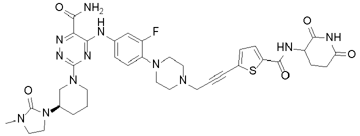 | ESI-MS: *m*/*z* = 773 [M+H]⁺ |
| 256 | 246 | 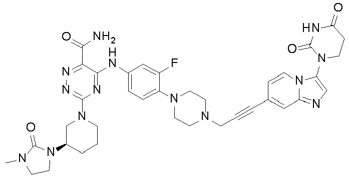 | ESI-MS: *m*/*z* = 765 [M+H]⁺ |
| 257 | 248 | 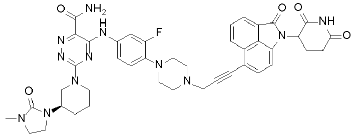 | ESI-MS: *m*/*z* = 815 [M+H]⁺ |

### Example 258: 5-((4-(1-(1-(2-(2,6-dioxopiperidin-3-yl)-1,3-dioxoisoindolin-5-yl) pyrrolidin-3-carbonyl)piperidin-4-yl)phenyl)amino)-3-((R)-3-(3-methyl-2-oxoimidazolin-1-yl)piperidin-1-yl)-1,2,4-triazin-6-carboxamide (127)

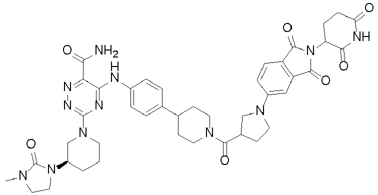

(R)-5-((3-fluoro-4-(piperazin-1-yl)phenyl)amino)-3-(3-(3-methyl-2-oxoimidazolidin-1-yl )piperidin-1-yl)-1,2,4-triazin-6-carboxamide (125 mg, 0.25 mmol) was dissolved in 3 mL of DMF, and DIPEA (97 mg, 0.75 mmol) and Intermediate 155 (112 mg, 0.30 mmol) were added to the system in sequence. The mixture was stirred at room temperature for 10 min, then acetic acid (0.1 mL) and sodium cyanoborohydride (47 mg, 0.75 mmol) were added to the system and the mixture was reacted at room temperature for 1 h. After the reaction was completed, the reaction liquid was adjusted to be alkaline by adding saturated aqueous sodium bicarbonate solution. A solid was precipitated. Filtration was performed to obtain a yellow solid which was dissolved by adding DCM, and the resulting mixture was dried over anhydrous sodium sulfate, concentrated under reduced pressure, and subjected to column chromatography (eluted with DCM/MeOH) to obtain 5-((4-(1-(1-(2-(2,6-dioxopiperidin-3-yl)-1,3-dioxoisoindolin-5-yl) pyrrolidin-3-carbonyl)piperidin-4-yl)phenyl)amino)-3-((R)-3-(3-methyl-2-oxoimidazolin-1-yl)pi peridin-1-yl)-1,2,4-triazin-6-carboxamide as a yellow solid (81 mg, yield 39%). ESI-MS: *m*/*z* = 833 [M+H]⁺. ¹H-NMR(400 MHz, CDCl₃) δ 10.97 (brs, 1H), 8.12 (s, 1H), 7.73 - 7.74 (m, 1H), 7.62 - 7.67 (m, 3H), 7.21 - 7.24 (m, 2H), 6.96 (s, 1H), 6.69 - 6.71 (m, 1H), 5.50 (brs, 1H), 4.91 - 4.96 (m, 1H), 4.79 - 4.82 (m, 1H), 4.08 - 4.11 (m, 1H), 3.62 - 3.82 (m, 4H), 3.24 - 3.52 (m, 7H), 2.68 - 2.90 (m, 8H), 2.29 - 2.43 (m, 2H), 2.09 - 2.15 (m, 1H), 1.68 - 1.99 (m, 12H)

### Example 259: (R)-5-((4-(1-(2-(1-(4-(2,4-dioxotetrahydropyrimidin-1(2H)-yl)-3-methylphenyl)azetidin-3-yl)acetyl)piperidin-4-yl)-2-(hydroxymethyl)phenyl)amino)-3-(3-(3-methyl-2-oxoimidazolidin-1-yl)piperidin-1-yl)-1,2,4-triazin-6-carboxamide (239)

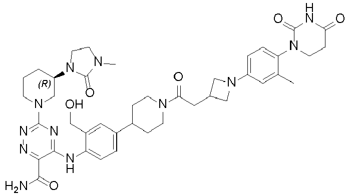

Referring to the synthetic route and method for Example 258, and Intermediate 155 was replaced with Intermediate 208 to synthesize and obtain target compound (R)-5-((4-(1-(2-(1-(4-(2,4-dioxotetrahydropyrimidin-1(2H)-yl)-3-methylphenyl)azetidin-3-yl)ace tyl)piperidin-4-yl)-2-(hydroxymethyl)phenyl)amino)-3-(3-(3-methyl-2-oxoimidazolidin-1-yl)pip eridin-1-yl)-1,2,4-triazin-6-carboxamide, ESI-MS: *m*/*z* = 809 [M+H]⁺.

### Example 260. BTK degradation activity test

Mino cell line cells were seeded at 500,000 to 1,000,000 cells/well in a 6-well plate. The compounds were diluted to different concentrations and added to the plate, and after 16 h of incubation, cells were harvested. After lysing the cells, the total protein concentration in each well was determined using the BCA method. The amount of BTK was determined using Western blot or Elisa method. The amount of undegraded BTK protein at each drug concentration was compared with the amount of BTK protein in the blank control group to calculate the degradation rate. The degradation curve was drawn based on the degradation rate at each concentration, and the half degradation concentration (DC50) was calculated by fitting. The results are shown in Table 1.

**Table 1 BTK degradation activity**

| **Compound No.** | **DC₅₀** | **Compound No.** | **DC₅₀** | **Compound No.** | **DC₅₀** |
|---|---|---|---|---|---|
| 001 | A | 002 | A | 003 | A |
| 004 | A | 005 | A | 006 | A |
| 007 | A | 008 | A | 009 | A |
| 010 | B | 011 | A | 012 | A |
| 013 | A | 014 | A | 015 | A |
| 016 | A | 017 | A | 018 | A |
| 019 | B | 020 | A | 021 | A |
| 022 | A | 023 | B | 024 | A |
| 025 | A | 026 | B | 027 | B |
| 028 | B | 029 | A | 030 | A |
| 031 | A | 032 | A | 033 | A |
| 034 | A | 035 | A | 036 | A |
| 037 | B | 038 | A | 039 | A |
| 040 | A | 041 | A | 042 | A |
| 043 | B | 044 | A | 045 | A |
| 046 | A | 047 | A | 048 | A |
| 049 | A | 050 | A | 051 | A |
| 052 | A | 053 | A | 054 | A |
| 055 | A | 056 | A | 057 | A |
| 058 | A | 059 | B | 060 | A |
| 061 | A | 062 | A | 063 | B |
| 064 | A | 065 | A | 066 | A |
| 067 | A | 068 | A | 069 | A |
| 070 | A | 071 | B | 072 | A |
| 072 | B | 074 | A | 075 | B |
| 076 | A | 077 | A | 078 | A |
| 079 | A | 080 | A | 081 | A |
| 082 | A | 083 | A | 084 | A |
| 085 | A | 086 | A | 087 | A |
| 088 | A | 089 | A | 090 | A |
| 091 | B | 092 | B | 093 | B |
| 094 | A | 095 | A | 096 | A |
| 097 | B | 098 | A | 099 | A |
| 100 | A | 101 | A | 102 | A |
| 103 | A | 104 | A | 105 | A |
| 106 | A | 107 | A | 108 | B |
| 109 | A | 110 | A | 111 | A |
| 112 | A | 113 | A | 114 | A |
| 115 | A | 116 | A | 117 | A |
| 118 | A | 119 | A | 120 | A |
| 121 | A | 122 | A | 123 | A |
| 124 | A | 125 | A | 126 | A |
| 127 | A | 128 | A | 129 | A |
| 130 | A | 131 | A | 132 | A |
| 133 | A | 134 | A | 135 | A |
| 136 | A | 137 | A | 138 | A |
| 139 | A | 140 | A | 141 | A |
| 142 | A | 143 | A | 144 | A |
| 145 | A | 146 | A | 147 | A |
| 148 | A | 149 | A | 150 | A |
| 151 | A | 152 | A | 153 | A |
| 154 | A | 155 | A | 156 | A |
| 157 | A | 158 | A | 159 | A |
| 160 | A | 161 | A | 162 | A |
| 163 | A | 164 | A | 165 | A |
| 166 | A | 167 | A | 168 | A |
| 169 | A | 170 | A | 171 | A |
| 172 | A | 173 | A | 174 | A |
| 175 | A | 176 | A | 177 | A |
| 178 | A | 179 | A | 180 | A |
| 181 | A | 182 | A | 183 | A |
| 184 | A | 185 | A | 186 | A |
| 187 | A | 188 | A | 189 | A |
| 190 | A | 191 | A | 192 | A |
| 193 | A | 194 | A | 195 | A |
| 196 | A | 197 | A | 198 | A |
| 199 | A | 200 | A | 201 | A |
| 202 | A | 203 | A | 204 | A |
| 205 | A | 206 | A | 207 | A |
| 208 | A | 209 | A | 210 | A |
| 211 | A | 212 | A | 213 | A |
| 214 | A | 215 | A | 216 | A |
| 217 | A | 218 | A | 219 | A |
| 220 | A | 221 | A | 222 | A |
| 223 | A | 224 | A | 225 | A |
| 226 | A | 227 | A | 228 | A |
| 229 | A | 230 | A | 231 | A |
| 232 | A | 233 | A | 234 | A |
| 235 | A | 236 | A | 237 | A |
| 238 | A | 239 | A | 240 | A |
| 241 | A | 242 | A | 243 | A |
| 244 | A | 245 | A | 246 | A |
| 247 | A | 248 | A | 249 | A |
| 250 | A | 251 | A | 252 | A |
| 253 | A | 254 | A | 255 | A |
| 256 | A | 257 | A | 258 | A |
| 259 | A | | | | |

| | | | | | |
|---|---|---|---|---|---|
| Note: A: DC₅₀ < 1 nM; B: 1 nM < DC₅₀; | | | | | |

The results in Table 1 show that the compounds of the present invention have BTK degradation effects.

### Example 261. BTK-C481S degradation activity test

HEK293 OE BTK-C481S cell line cells were seeded at 500,000 to 1,000,000 cells/well in a 6-well plate. The compounds were diluted to different concentrations and added to the plate, and after 24 h of incubation, the cells were harvested. After lysing the cells, the total protein concentration in each well was determined using the BCA method. The amount of BTK-C481S was determined using Western blot or Elisa method. The amount of undegraded BTKC481S protein at each drug concentration was compared with the amount of BTKC481S protein in the blank control group to calculate the degradation rate. The degradation curve was drawn based on the degradation rate at each concentration, and the half degradation concentration (DC50) was calculated by fitting. The results are shown in Table 2.

**Table 2 BTK-C481S degradation activity**

| **Compound No.** | **DC₅₀** |
|---|---|
| 123 | A |
| 130 | A |
| 131 | A |
| 146 | A |
| 255 | A |
| 257 | A |
| 259 | A |

| | |
|---|---|
| Note: A: DC₅₀ < 10 nM; B: 10 nM < DC₅₀ | |

The results in Table 2 show that the compounds of the present invention have BTK-C481S degradation effects.

### Example 262. Test of inhibitory activity on tumor cell proliferation

The anti-tumor efficacy of the compounds was tested by measuring their inhibitory effects on OCI-LY10 and Mino cell proliferation. OCI-LY10 and Mino cells were cultured in RPMI-1640 medium containing 10% fetal calf serum. OCI-LY10 and Mino cells were digested, inoculated in a 96-well plate at a cell concentration of 10000 cells/well, and incubated overnight at 37°C, 5% CO₂. Compounds of different concentrations (1000 nM, 4-fold dilution, 8 points) were added to the 96-well plate and incubated for 72 h (LY10) or 168 h (Mino) at 37°C and 5% CO₂, and then 20 uL of MTS was added to each well. After incubation for 2 h, 25 µl of 10% SDS was added to each well to terminate the reaction. The absorbance at 490 nm and 650 nm were measured with a microplate reader. IC₅₀ was calculated using GraphPad Prism 5.0.

**Table 3 Inhibitory activity on OCI-LY10 cell proliferation**

| **Compound No.** | **OCI-LY10 IC₅₀ (nM)** | **Compound No.** | **OCI-LY10 IC₅₀ (nM)** |
|---|---|---|---|
| 115 | 0.59 | 116 | 0.11 |
| 117 | 0.33 | 118 | 0.27 |
| 119 | 0.38 | 120 | 0.21 |
| 121 | 0.74 | 122 | 0.72 |
| 123 | 0.20 | 124 | < 0.26 |
| 125 | 1.83 | 126 | < 0.06 |
| 127 | 0.39 | 128 | 0.37 |
| 129 | 0.14 | 130 | 0.28 |
| 131 | 0.90 | 132 | < 1.0 |
| 133 | 0.43 | 135 | 0.11 |
| 136 | 0.79 | 137 | 0.25 |
| 138 | 0.08 | 140 | 0.47 |
| 141 | 2.19 | 143 | 0.33 |
| 144 | 3.49 | 145 | 0.56 |
| 146 | 1.39 | 147 | 1.48 |
| 148 | 3.21 | 149 | 2.60 |
| 166 | < 1.0 | 167 | 0.45 |
| 168 | 0.79 | 170 | 1.25 |
| 171 | 2.65 | 172 | 2.39 |
| 174 | 1.03 | 180 | < 1.0 |
| 181 | 0.78 | 184 | 1.56 |
| 185 | 1.36 | 195 | 1.79 |
| 196 | 0.52 | 202 | 1.05 |
| 203 | 2.65 | 205 | 0.57 |
| 207 | 0.23 | 223 | 2.55 |
| 224 | 2.25 | 227 | 0.27 |
| 232 | 0.83 | 234 | 1.78 |
| 236 | 0.63 | 254 | 0.89 |
| 255 | 1.50 | 256 | 1.60 |
| 257 | 0.12 | 258 | 0.96 |
| 259 | 0.61 | Compound a | 4.17 |

Compound a:

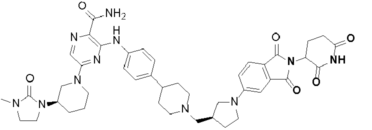

As can be seen from Table 3, the compounds of the present invention have obvious inhibitory activity on OCI-LY10 cell proliferation.

**Table 4 Inhibitory activity on Mino cell proliferation**

| **Compound No.** | **Mino IC₅₀ (nM)** | **Compound No.** | **Mino IC₅₀ (nM)** |
|---|---|---|---|
| 115 | 5.97 | 116 | 1.34 |
| 117 | 3.08 | 118 | 10.71 |
| 119 | 8.09 | 120 | 1.76 |
| 121 | 3.75 | 122 | 4.98 |
| 123 | 2.11 | 124 | 4.95 |
| 126 | 2.99 | 127 | 2.04 |
| 128 | 1.93 | 129 | 3.09 |
| 130 | 2.41 | 131 | 3.61 |
| 132 | 2.55 | 133 | 3.91 |
| 134 | 1.72 | 135 | 1.36 |
| 136 | 1.61 | 137 | 3.92 |
| 138 | 3.11 | 140 | 3.46 |
| 141 | 3.92 | 143 | 3.08 |
| 144 | 3.49 | 145 | 4.15 |
| 146 | 1.73 | 147 | 0.54 |
| 148 | 7.79 | 149 | 8.89 |
| 150 | 1.91 | 152 | 2.80 |
| 166 | 6.85 | 167 | 2.42 |
| 168 | 3.77 | 170 | 4.64 |
| 171 | 3.43 | 172 | 6.20 |
| 177 | 2.99 | 179 | 1.47 |
| 180 | 1.07 | 182 | 3.19 |
| 183 | 4.85 | 186 | 2.26 |
| 187 | 2.07 | 188 | 5.55 |
| 189 | 5.45 | 191 | 8.47 |
| 192 | 2.41 | 195 | 8.53 |
| 196 | 3.77 | 202 | 6.83 |
| 203 | 1.25 | 209 | 3.42 |
| 210 | 2.33 | 213 | 7.64 |
| 219 | 2.74 | 222 | 4.15 |
| 255 | < 0.26 | 256 | 1.87 |
| 257 | < 0.26 | 258 | 5.31 |
| 259 | 5.58 | Compound a | 14.03 |

As can be seen from the results in Table 4, the compounds of the present invention have obvious inhibitory activity on Mino cell proliferation.

### Example 263. Liver microparticle test

### Experimental procedure

### 1. Drug formulation

Compound to be tested: the compound to be tested (1 mg) was dissolved in DMSO (10 mL) to formulate to 0.1 mg/mL.

Internal standard compound: the internal standard compound (1 mg) was dissolved in DMSO (10 mL), 10 µL was taken and diluted to 10 mL to formulate to 100 ng/mL.

NADPH solution: NADPH (2.08 mg) was dissolved in PBS (50 µL) buffer to formulate to 50 mM.

### 2. Test Methods

Under the condition of ice bath, 1 mg of human liver microparticles was add to PBS (1 mL) buffer, then NADPH (20 µL) was added, and incubated at 37°C for 5 min. 10 µL of the compound to be tested was added, and then samples were taken at 0, 15, 30, 60 and 120 min respectively. 10 µL of the solution was taken with a pipette each time and added to 490 µL of the solution containing the internal standard compound. The resulting solution was vortexed and then placed on ice, and then sampling at another time point continues. All samples were centrifuged at 10,000 rpm for 10 min, and the supernatant was taken for LC/MS testing. The metabolism of the compound in liver microsomes was calculated based on the peak area.

**Table 5 Stability of compounds in liver microsome**

| No. | Human liver microsome T_{1/2} (min) | No. | Human liver microsome T_{1/2} (min) |
|---|---|---|---|
| 126 | 55.6 | 171 | > 30 |
| 128 | 43.2 | 172 | > 30 |
| 130 | 32.6 | 173 | > 30 |
| 131 | > 30 | 174 | > 30 |
| 132 | > 30 | 177 | > 30 |
| 133 | 31.7 | 179 | > 30 |
| 134 | > 30 | 181 | > 30 |
| 135 | 25.0 | 184 | > 30 |
| 136 | 24.0 | 195 | > 30 |
| 137 | 20.0 | 195 | > 30 |
| 140 | 26.3 | 205 | > 30 |
| 143 | 39.1 | 218 | > 30 |
| 144 | 146 | 222 | > 30 |
| 145 | > 30 | 232 | > 30 |
| 146 | > 30 | 255 | > 30 |
| 147 | > 30 | 256 | > 30 |
| 148 | > 30 | 257 | > 30 |
| 149 | > 30 | 258 | > 30 |
| 150 | > 30 | 259 | > 30 |
| 163 | > 30 | 254 | > 30 |
| Compound a | 9.14 | Compound b | 5.18 |

As can be seen from the results, the compounds of the present invention are metabolically stable in human liver microsomes.
Compound b:

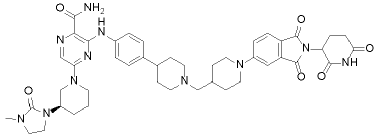

### Example 264. Mouse absorption test

Experimental method: mice were used as experimental animals, and drugs were administered intragastrically at 30 mg/kg (5% DMSO + 5% Solutol + 90% normal saline) . The blood collection time points for intragastric administration were 0.25, 0.5, 1, 2, 4, 6, 8, and 24 h. 0.5 mL of blood was taken with a capillary tube from the orbit, and placed in an EP tube containing EDTA anticoagulant. The EP tube was shaken gently for mixing uniformly, and centrifuged at 3500 rpm and 4°C for 10 min. The supernatant, i.e., plasma, was taken. Analysis was performed using LC-MS/MS.

**Table 6 Pharmacokinetic properties of compounds in mice**

| No. | Dosage mg/kg | Cmax (ng/ml) | DNAUC (ng/ml*h/mpk) |
|---|---|---|---|
| 123 | 30 | A | 1920 |
| 130 | 30 | A | 3809 |
| 131 | 30 | 6190 | 3614 |
| 132 | 30 | 7801 | 2430 |
| 145 | 30 | 13814 | 2704 |
| 254 | 30 | A | 2536 |
| 255 | 30 | A | 1955 |
| 259 | 30 | A | 2223 |
| Compound a | 100 | 5662 | 1088 |
| Compound b | 100 | 4031 | 631 |

| | | | |
|---|---|---|---|
| Note: A: Cmax > 6000 ng/mL B: Cmax < 6 000 ng/ml; DNAUC represents: AUC/Dose | | | |

According to the results, it can be seen that the compounds of the present invention have good oral absorption properties in mice.

### Example 265. Beagle dog absorption test

Beagle dogs were used as experimental animals, and drugs were administered intragastrically at10 mg/kg. The blood collection time points for intragastric administration were 0, 0.25, 0.5, 1, 2, 4, 6, 8, 10, 12, and 24 h. 1.0 mL of blood was collected from the jugular vein, placed in an EDTAK2 centrifuge tube, and placed on an ice bath, centrifuged at 5000 rpm and 4°C for 10 min to obtain the supernatant, i.e., plasma. 0.1 ml of the plasma was taken for analysis by LC-MS/MS.

**Table 7 PK of compounds in dogs**

| No. | Dosage (mg/kg) | AUC (h.ng.mL⁻¹) |
|---|---|---|
| 132 | 10 | 1687 |
| 257 | 10 | 6593 |
| Compound c | 10 | 246 |

According to the results, it can be seen that the compounds of the present invention have good oral absorption properties in dogs.

Compound c is selected from WO 2021113557:

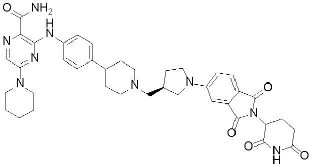

### Example 266 Chronic efficacy test of drugs

NOD/SCID mice were subcutaneously inoculated with OCL-LY10 cells to establish a tumor model. When the tumors grew to about 100 mm³, the mice were administered with drugs in groups. The mode of administration was as follows: the test compound was administered intragastrically at 10 mg/kg; The changes in body weight and tumor size of the mice were measured twice per week. After 3 weeks, the mice were sacrificed by cervical dislocation, and the efficacy of the compounds on transplanted tumors in OCL-LY10 mice was examined.

**Table 8 Effects of compounds of the present invention on transplanted tumors in OCL-LY10 mice**

| No. | Dosage (mg/kg) | TGI (%) |
|---|---|---|
| 123 | 10 | 95.66 |
| 131 | 10 | 96.67 |
| 257 | 10 | 98.74 |
| Ibrutinib | 10 | 50.72 |
| Compound c | 10 | 67.59 |

The results in Table 8 show that the compounds of the present invention have the obvious inhibitory effect on the transplanted tumors in OCL-LY10 mice.

### Example 267 Test of inhibition of B cell activation

### Experimental procedure

### 1. Cell plating

The PBMC cell cryopreservation liquid was resuspended in 1640 complete culture medium to an appropriate concentration, and inoculated into a 96-well round-bottom plate at 15 × 104 cells/90 µL/well, and the culture plate was placed in a CO2 incubator for incubation.

### 2. Formulation and addition of compounds:

The compounds to be tested were formulated into 10 mM stock solutions which were then fold-serially diluted with DMSO, and then diluted with 1640 complete culture liquid to make the concentration 100 times of the final concentration. The compound diluent was added into the cell solution in each well at 1 µL/well and shaken gently. 2 duplicate wells were set up for each concentration. The dosed cell plate was placed in a 37°C incubator with 5% carbon dioxide and incubated for 1 h.
3. The dosed cell culture plate was taken and 10 µL of Anti-Human IgM-UNLB was added into the well corresponding to the compound to be tested; in 2 groups of cell blank wells, only 10 µL of Anti-Human IgM-UNLB was added; and in 3 groups of cell blank wells, no reagents and antibodies were added. The final concentration of Anti-Human IgM-UNLB was 50 µg/mL.
4. The loaded cell culture plate was placed in a 37°C incubator with 5% carbon dioxide and incubated for 18 h. Total system: 90 µL PBMC + 1 µL compound + 10 µL Anti-Human IgM-UNLB
5. Samples were collected and stained, and the cells were analyzed using flow cytometry to obtain data. (When analyzed using flow cytometry, lymphocytes were gated).

**Table 9 Test of inhibition on B cells by the compounds of the present invention**

| No. | Concentration (nM) | Inhibition ratio% |
|---|---|---|
| 131 | 0.16 | 60.8 |
| | 0.80 | 100 |
| 257 | 0.16 | 100 |
| | 0.80 | 100 |
| Compound c | 0.16 | 10.2 |
| | 0.80 | 16.3 |

As can be seen from Table 9, the compounds of the present invention have inhibitory effects on B cell activation.

## Claims

1. A compound, **characterized in that**, the compound has a structure shown in the general formula (I): 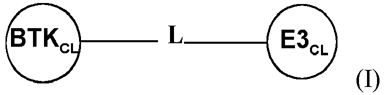
or its stereoisomer thereof, or its stereoisomer mixture thereof, or its pharmaceutically acceptable salt thereof;
wherein:
BTK_{CL} represents a chemical ligand that can bind to BTK kinase;
E3_{CL} represents a chemical ligand that can bind to E3 ubiquitin ligase;
and L represents a chemical group or a chemical bond connecting the BTK_{CL} and the E3_{CL}.

2. The compound of claim 1, **characterized in that**:
E3_{CL} is selected from: chemical fragments that can bind to CRBN E3 ubiquitin ligase.

3. The compound of claim 2, **characterized in that**:
L has the following structure:
-X1-L1-X2-Cyc1-X3-L2-X4-Cyc2-X5-L3-X6-Cyc3-X7-L4-X8-
wherein:
X1, X2, X3, X4, X5, X6, X7 and X8 are each independently selected from: absent, -O-, -S-, -N(Ra)-;
L1, L2, L3, and L4 are each independently selected from: absent, chemical bonds, C1-C4 alkyl, C2-C4 alkenyl, C2-C4 alkynyl, wherein the alkyl, alkenyl, and alkynyl can be substituted with oxo, alkyl, halogen, cyano, haloalkyl;
Cyc1, Cyc2, and Cyc3 are each independently selected from: absent, 3-12 membered heterocyclic ring, 5-12 membered aromatic ring, 5-12 membered heteroaromatic ring, 3-12 membered cycloalkane, 4-12 membered cycloalkyne, 3-12 membered cycloalkene, wherein the heterocyclic ring, aromatic ring, heteroaromatic ring, cycloalkane, cycloalkyne, and cycloalkene can be substituted with oxo, alkyl, halogen, cyano, haloalkyl;
Ra is selected from: H, C1-C4 alkyl.

4. The compound of claim 3, **characterized in that**:
BTK_{CL} is selected from the following structures: 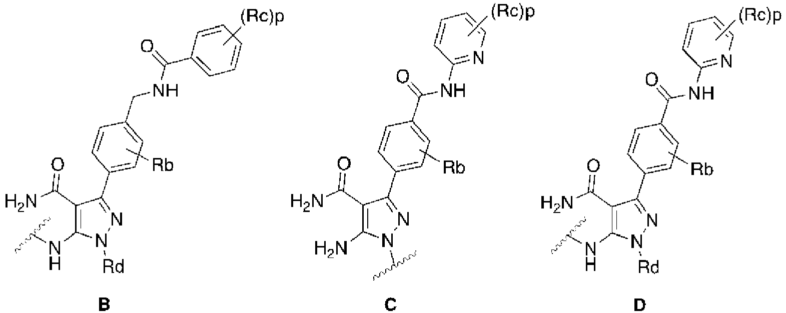 wherein:
when BTK_{CL} is selected from structure B, C, D,
Rb is selected from: H, halogen, cyano, methyl, -CF₃, C1-C3 alkoxy;
one or more Rcs are independently selected from: H, CN, halogen, C1-C4 alkyl, C3-C8 cycloalkyl, C1-C4 haloalkyl, C1-C4 alkoxy, C1-C4 alkylamino;
Rd is selected from: C1-C6 alkyl, C1-C6 haloalkyl, C3-C8 cycloalkyl, C3-C8 heterocyclyl, wherein the alkyl, cycloalkyl, and heterocyclyl can be further substituted with one or more substituents selected from: halogen, C1-C4 alkyl, C1-C4 haloalkyl, C1-C4 alkoxy, cyano, oxo, carboxyl, ester group, amido, hydroxyl;
and p is selected from: an integer of 0-4.

5. The compound of claim 3, **characterized in that**:
BTK_{CL} is selected from the following structures: 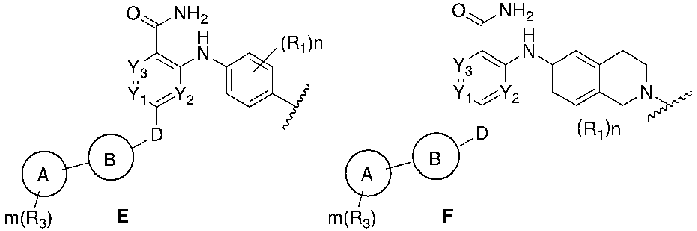
Yi is selected from: N, CR₂;
Y₂ and Y₃ are each independently selected from: N, CH;
ring A is selected from: 3-12 membered heterocyclic ring;
ring B is selected from: 5-6 membered heteroaromatic ring, 5-10 membered heterocyclic ring, wherein the heteroaromatic ring and the heterocyclic ring can be further substituted with one or more substituents selected from halogen, C1-C4 alkyl, C1-C4 haloalkyl, C1-C4 alkoxy, cyano, oxo, carboxyl, ester group, amido, hydroxyl, wherein two substituents occurring simultaneously on ring B can be connected to form a bridged ring, a spiro ring, a fused ring;
D is selected from: chemical bonds, C1-C3 alkyl, -O-, -NH-, -S-;
one or more R₁ₛ are each independently selected from: H, halogen, C1-C6 alkyl, C1-C6 haloalkyl, -CN, -COOH, -NH₂;
R₂ is selected from: hydrogen, cyano, halogen, C1-C4 alkyl, C3-C8 cycloalkyl, C1-C4 haloalkyl, C1-C4 alkoxy;
one or more R₃ₛ are selected from: 3-12 membered heterocyclyl, 5-12 membered heteroaryl, 5-12 membered aryl, 3-12 membered cycloalkyl, halogen, C1-C4 alkyl, C1-C4 haloalkyl, C1-C4 alkoxy, cyano, oxo, carboxyl, ester group, amido, hydroxyl, wherein the heterocyclyl, heteroaryl, aryl and cycloalkyl can be further substituted with substituents selected from: halogen, C1-C4 alkyl, C1-C4 haloalkyl, C1-C4 alkoxy, cyano, oxo, carboxyl, ester group, amido, hydroxyl; two R₃ₛ on ring A can be connected to form a spiro ring, a bridged ring, a fused ring;
m is selected from: an integer of 0-3;
n is selected from: an integer of 0-2; 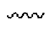
represents that the atom connected thereto is connected to L;
when Yi is selected from CH, ring A is a monocyclic ring, and ring B is a benzene ring, a 5-6 membered heteroaromatic ring, a 4-6 membered alkane heterocyclic ring or an 8-10 membered spiro ring, R₃ is not C1-C4 alkyl, C1-C4 haloalkyl.

6. The compound of claim 4, **characterized in that**, the compound has a structure shown in the general formula lib: 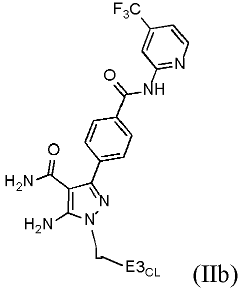 or its stereoisomer thereof, or its stereoisomer mixture thereof, or its pharmaceutically acceptable salt thereof.

7. The compound of claim 5, **characterized in that**, the compound has a structure shown in the general formula III(a), III(b): 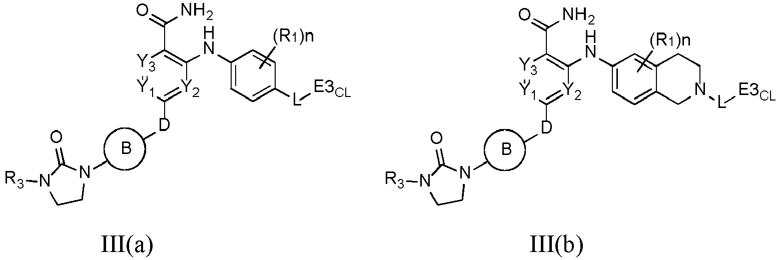
or its stereoisomer thereof, or its stereoisomer mixture thereof, or its pharmaceutically acceptable salt thereof;
ring B is not a bridged ring;
R₃ is selected from: C1-C4 alkyl, C1-C4 haloalkyl, 3-6 membered cycloalkyl, 3-6 membered heterocyclyl, 5-6 membered aryl, 5-6 membered heteroaryl, wherein the cycloalkyl, heterocyclyl, aryl, and heteroaryl can be further substituted with one or more substituents selected from: halogen, C1-C4 alkyl, C1-C4 haloalkyl, C1-C4 alkoxy, cyano, oxo, carboxyl, ester group, amido, hydroxyl, two substituents on the cycloalkyl, heterocyclyl, aryl and heteroaryl can be connected to form a fused ring, a bridged ring, or a spiro ring;
when Yi is selected from CH, R₃ is not C1-C4 alkyl, C1-C4 haloalkyl.

8. The compound of claim 5, **characterized in that**, the compound has a structure shown in the general formula IV(a), IV(b), IV(c), or IV(d): 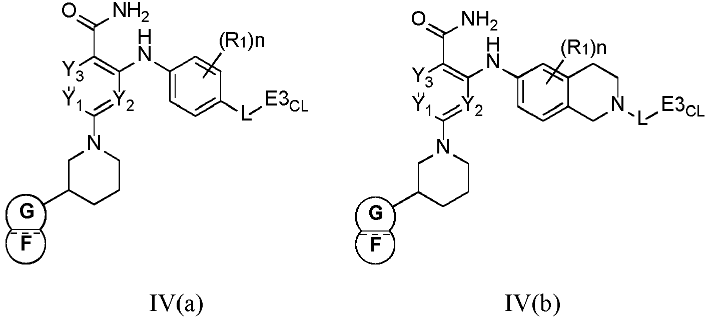 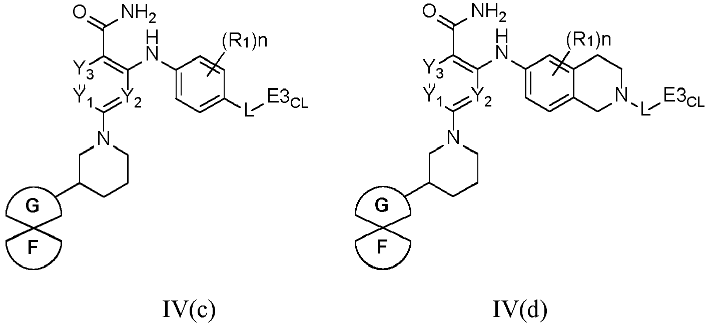
or its stereoisomer thereof, or its stereoisomer mixture thereof, or its pharmaceutically acceptable salt thereof;
wherein the fused ring or spiro ring structure composed of ring G and ring F is selected from: 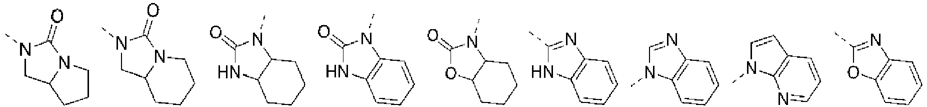 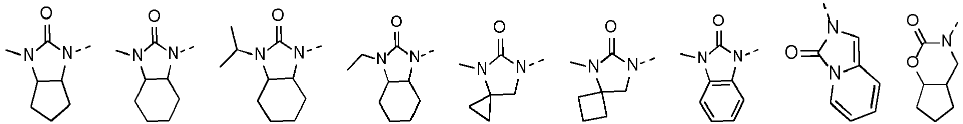 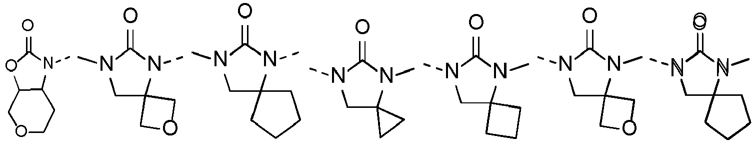
the above-mentioned fused ring, spiro ring structure can be further substituted with one or more substituents selected from: halogen, C1-C4 alkyl, C1-C4 haloalkyl, C1-C4 alkoxy, cyano, oxo, carboxyl, ester group, amido, hydroxyl.

9. The compound of claim 5, **characterized in that**, the compound has a structure shown in the general formula V(a) or V(b): 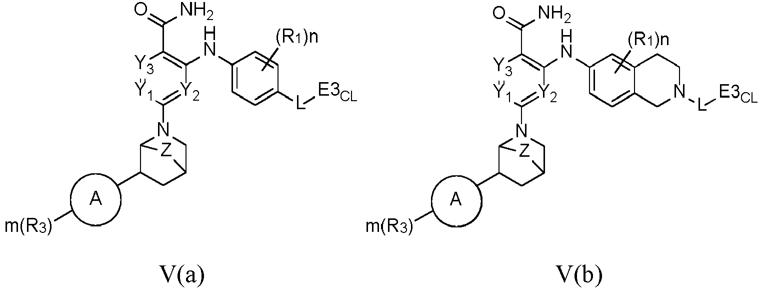
or its stereoisomer thereof, or its stereoisomer mixture thereof, or its pharmaceutically acceptable salt thereof;
Z is selected from: -CH₂-, -CH₂CH₂-, -O-.

10. The compound of claim 7, **characterized in that**:
R₃ is preferably selected from: C1-C4 alkyl, ring D;
ring D is selected from: 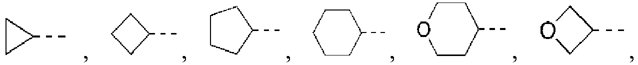 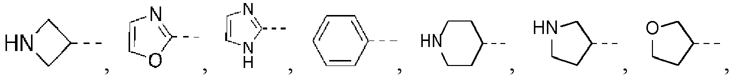 ring D can be further substituted with one or more substituents, wherein the substituents can be on C atom or N atom, and the substituents are selected from: halogen, C1-C4 alkyl, C1-C4 haloalkyl, C1-C4 alkoxy, cyano, oxo, carboxyl, ester group, amido, hydroxyl;
when Yi is CH, R₃ is selected from ring D.

11. The compound of claim 5, **characterized in that**, the compound has a structure shown in the general formula (VI): 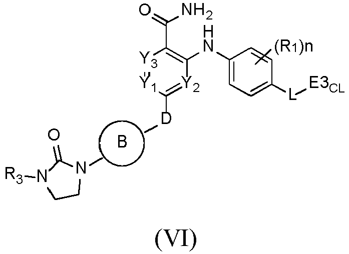
or its stereoisomer thereof, or its stereoisomer mixture thereof, or its pharmaceutically acceptable salt, prodrug thereof;
wherein:
L represents a chemical group or a chemical bond connecting the benzene ring and E3_{CL};
E3_{CL} represents a chemical ligand that can bind to E₃ ubiquitin ligase;
Yi is selected from: N, CR₂;
Y₂ and Y₃ are each independently selected from: N, CH;
D is selected from: chemical bonds;
one or more R₁ₛ are each selected from: H, halogen, C₁-C₆ alkyl, C₁-C₆ haloalkyl, -CN, -COOH, -NH₂;
R₂ is selected from: H, cyano, halogen, C₁-C₄ alkyl, C₃-C₈ cycloalkyl, C₁-C₄ haloalkyl, C₁-C₄ alkoxy;
ring B is selected from: 5-6 membered heteroaryl, 5-10 membered heterocyclyl, wherein the heteroaryl and the heterocyclyl can be further substituted with one or more substituents selected from: halogen, C₁-C₄ alkyl, C₁-C₄ haloalkyl, C₁-C₄ alkoxy, cyano, oxo, carboxyl, ester group, amido, hydroxyl, wherein two substituents occurring simultaneously on ring B can be connected to form a bridged ring, a spiro ring, a fused ring;
R₃ is selected from: C₁-C₄ alkyl, C₁-C₄ haloalkyl, 3-6 membered cycloalkyl, 3-6 membered heterocyclyl, 5-6 membered aryl, 5-6 membered heteroaryl, wherein the cycloalkyl, the heterocyclyl, the aryl, and the heteroaryl can be further substituted with one or more substituents selected from: halogen, C₁-C₄ alkyl, C₁-C₄ haloalkyl, C₁-C₄ alkoxy, cyano, oxo, carboxyl, ester group, amido, hydroxyl, two substituents on the cycloalkyl, heterocyclyl, aryl and heteroaryl can be connected to form a fused ring, a bridged ring, or a spiro ring;
n is selected from: an integer of 0-2;
when Yi is selected from CH, and ring B is a benzene ring, a 5-6 membered heteroaromatic ring, a 4-6 membered heterocyclic ring, or an 8-10 membered spiro ring, R₃ is not C₁-C₄ alkyl, C₁-C₄ haloalkyl.

12. The compound of any one of claims 1-11, **characterized in that**,
L is selected from: 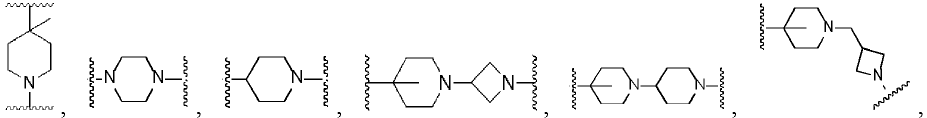 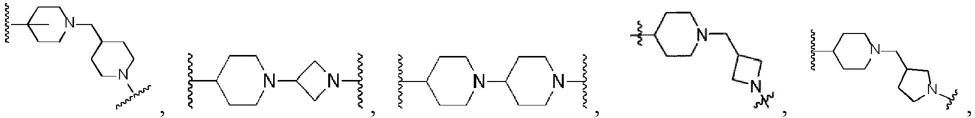 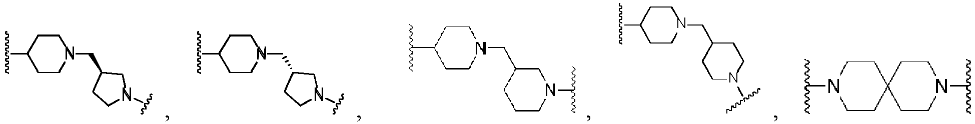 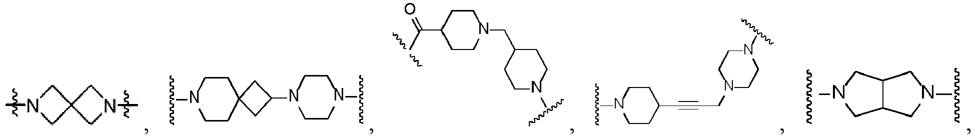 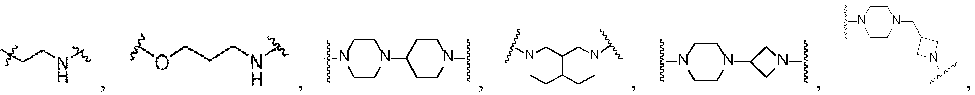 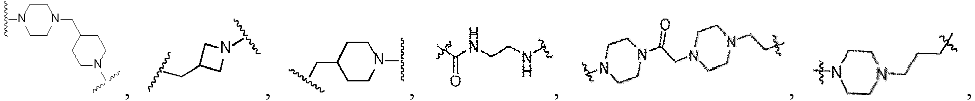 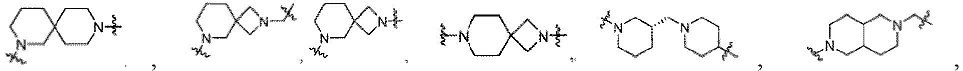 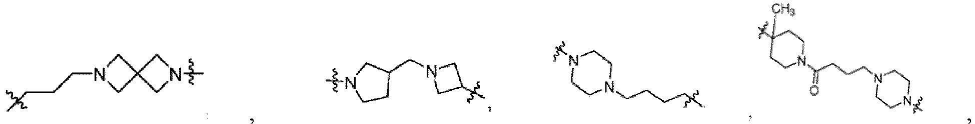 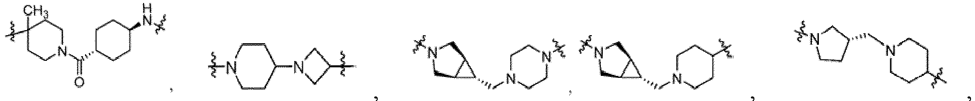 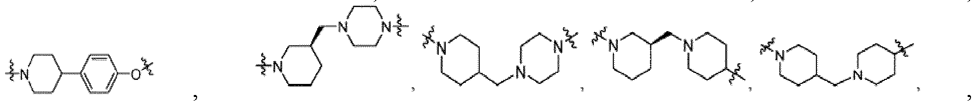 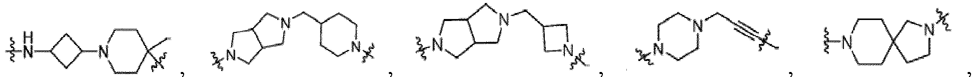 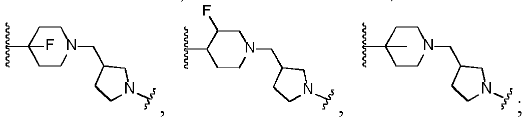 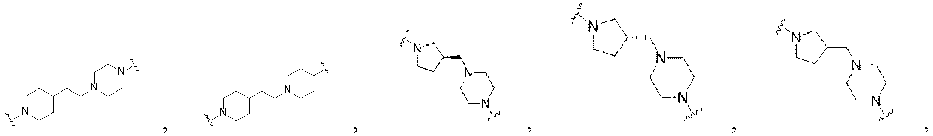 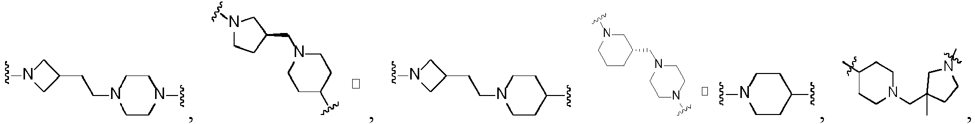 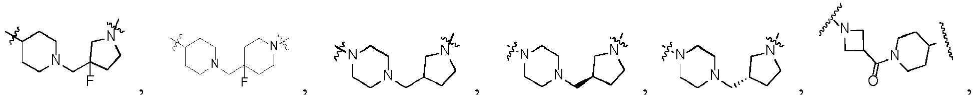 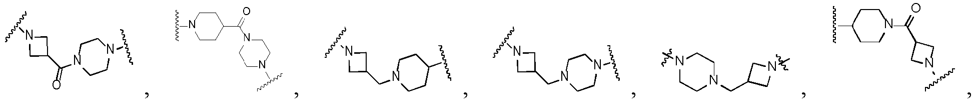 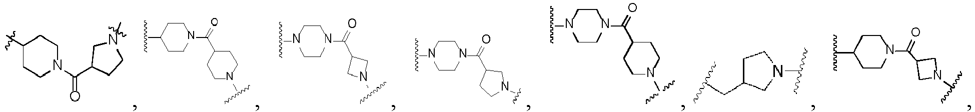 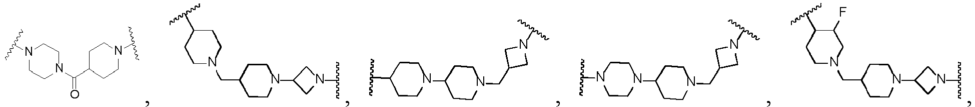 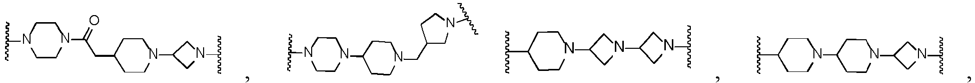 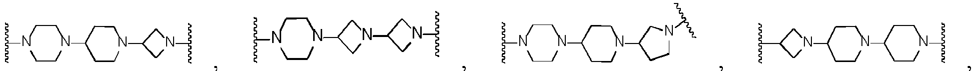 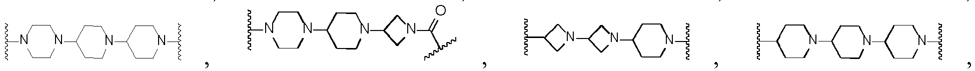 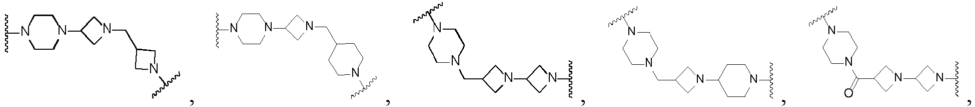 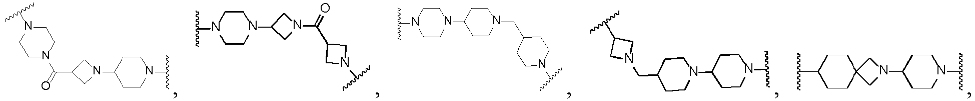 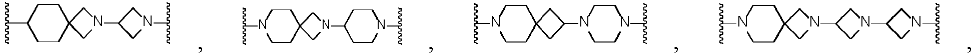 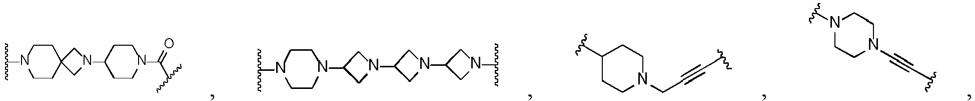 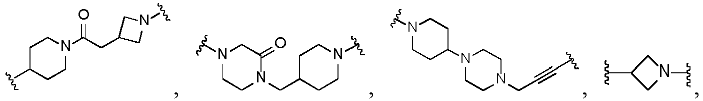
E3_{CL} is selected from: 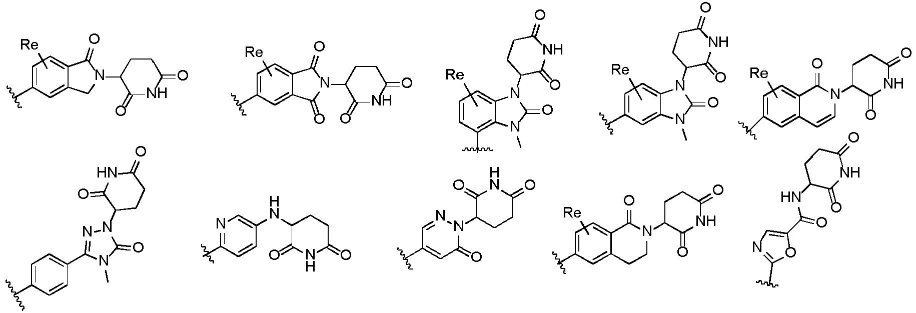 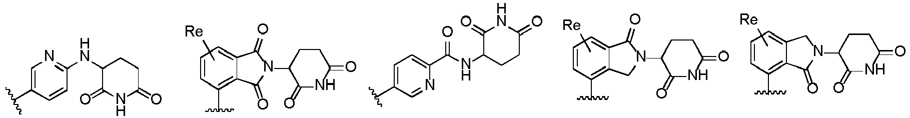 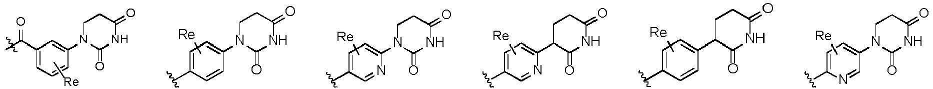 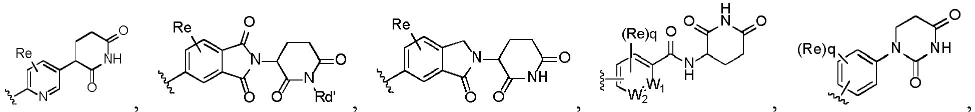 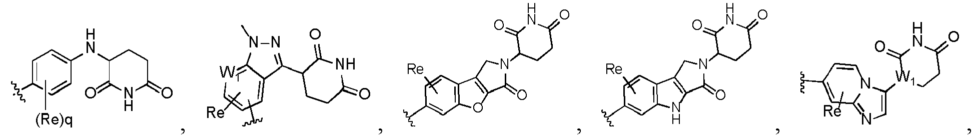 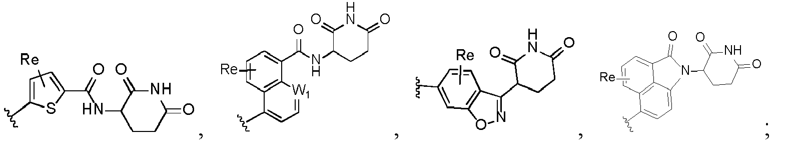
Re is selected from: H, F, Cl, -CH₃, -OMe, -CN, -CF₃, -CHF₂, -CH(CH₃)₂, -cyclopropyl, -C(O)NH₂, -S(O)₂Rf, -P(O)(Rf)₂;
Rd' is selected from: H, -Rf OCORg, -RfOCOORg, -RfOCONRgRh, -COORf, -CONRfRg;
Rf, Rg, and Rh are each independently selected from: H, Ci-Cs alkyl, 3-8 membered cycloalkyl, 3-8 membered heterocyclyl, C1-C6 alkyl, 3-8 membered cycloalkyl, C1-C6 alkyl, 3-8 membered heterocyclyl;
or two adjacent Res on the benzene ring form a 7-10 membered benzo ring together with the benzene ring; the C atom on the benzo ring can be further substituted with one or more heteroatoms selected from N, O, S; the benzo ring is optionally substituted with H, F, Cl, -CH₃, -OMe, -CN, -CF₃, -CHF₂, -CH(CH₃)₂, -cyclopropyl, -C(O)NH₂;
W₁ and W₂ are selected from: CH, N;
q is selected from: 0, 1, 2, 3.

13. The compound of claim 12, **characterized in that**,
E3_{CL} is selected from:

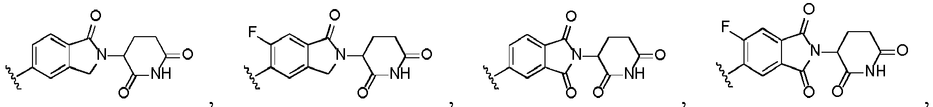

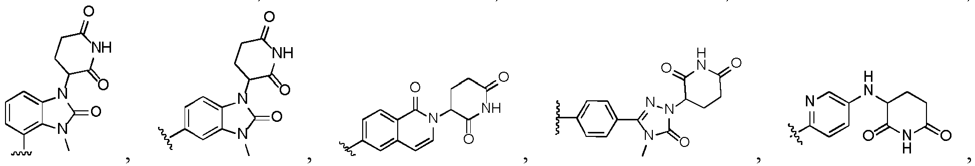

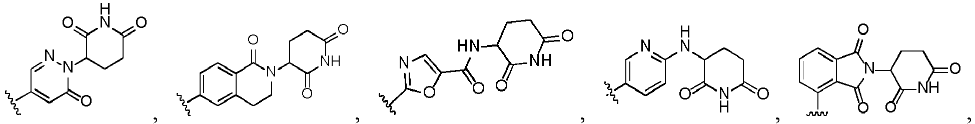

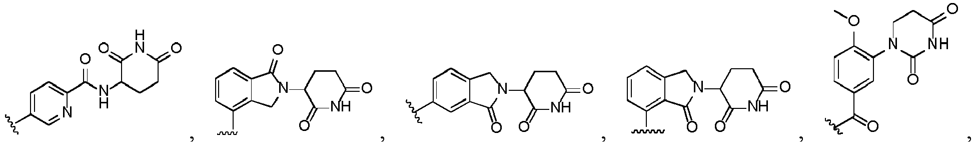

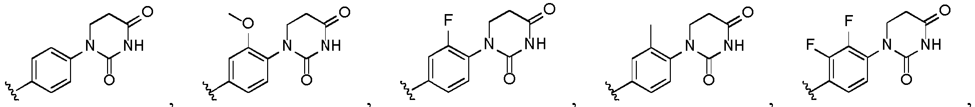

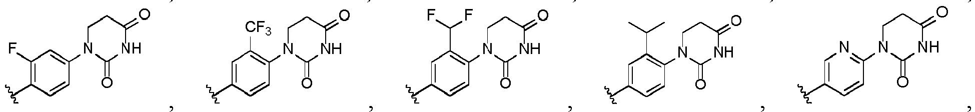

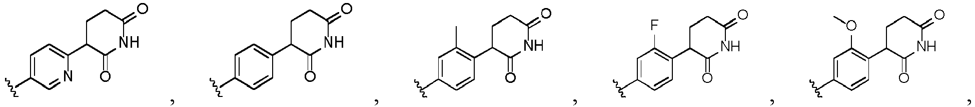

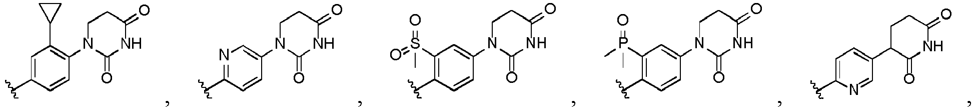

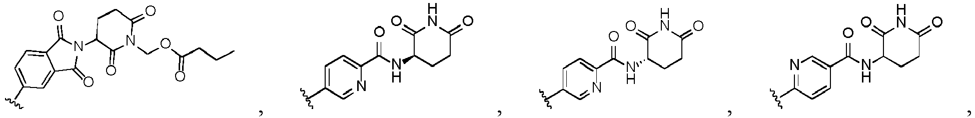

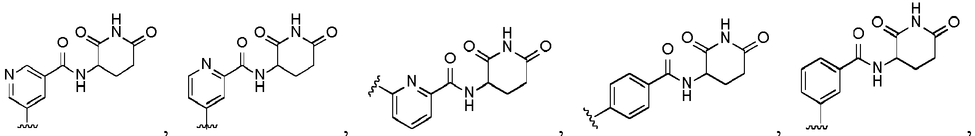

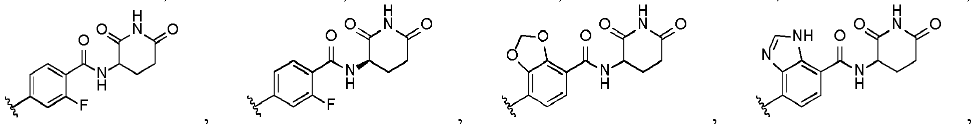

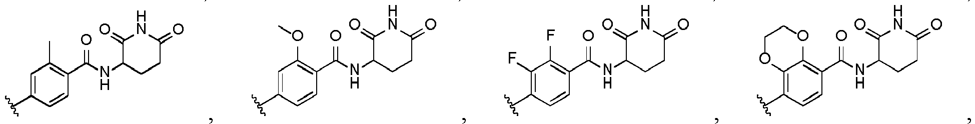

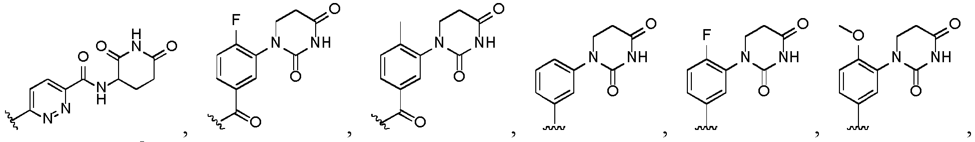

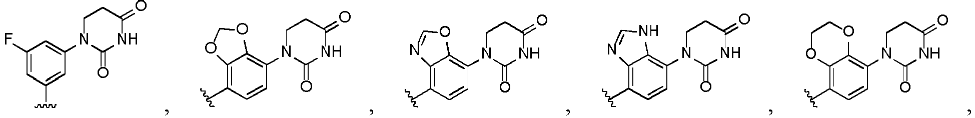

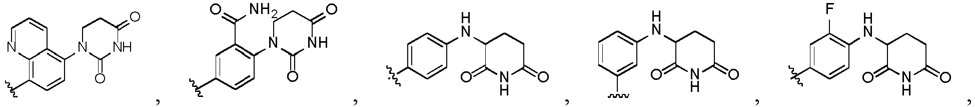

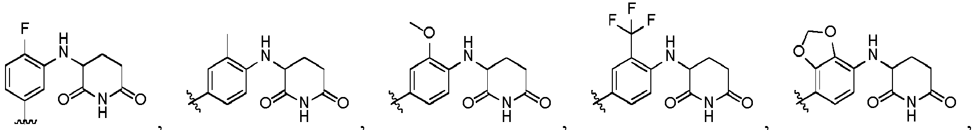

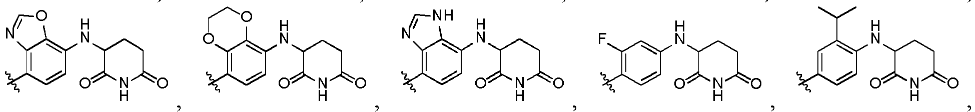

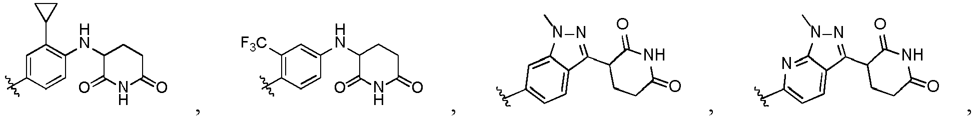

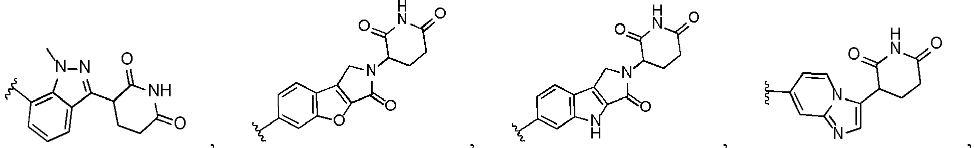

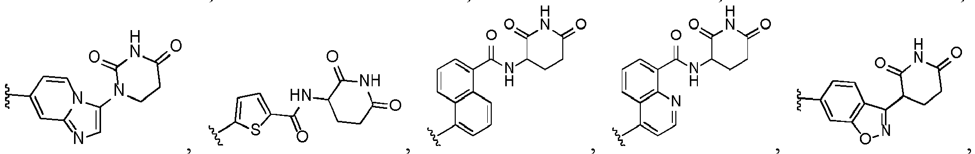

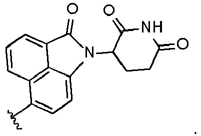

14. The compound of claim 1, **characterized in that**, the compound is selected from the following compounds:
| No. | Structure | No. | Structure |
|---|---|---|---|
| 001 | 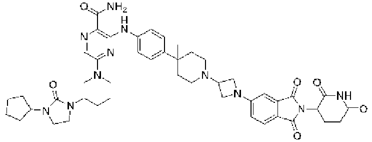 | 002 | 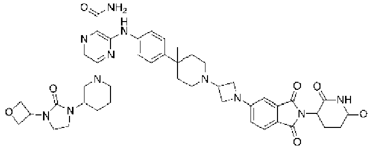 |
| 003 | 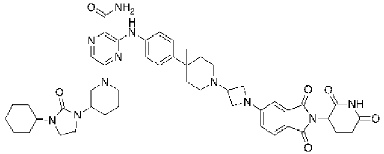 | 004 | 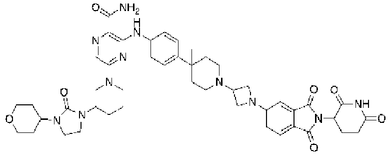 |
| 005 | 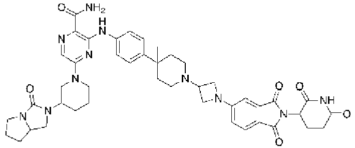 | 006 | 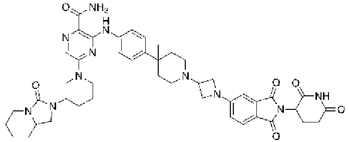 |
| 007 | 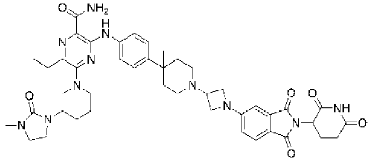 | 008 | 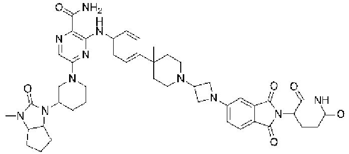 |
| 009 | 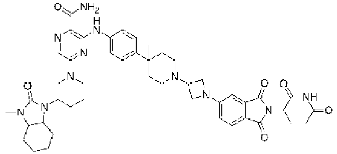 | 010 | 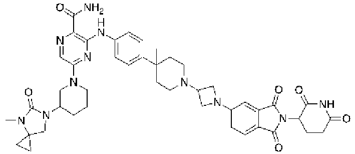 |
| 011 | 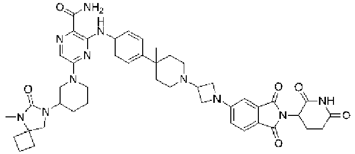 | 012 | 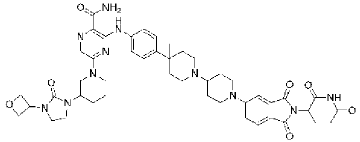 |
| 013 | 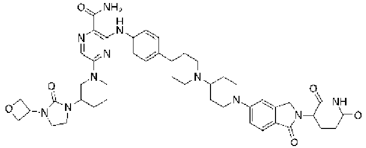 | 014 | 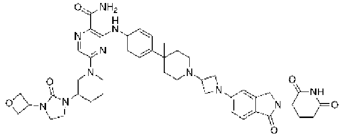 |
| 015 | 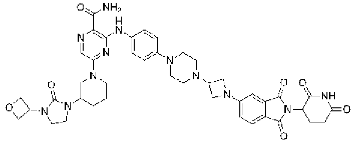 | 016 | 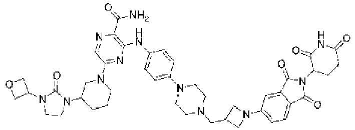 |
| 017 | 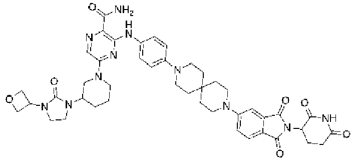 | 018 | 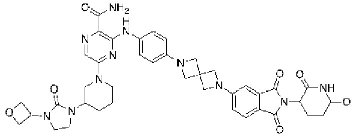 |
| 019 | 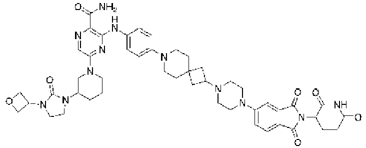 | 020 | 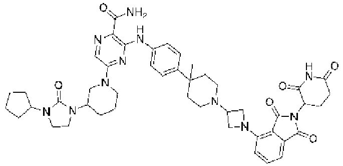 |
| 021 | 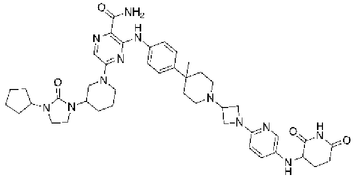 | 022 | 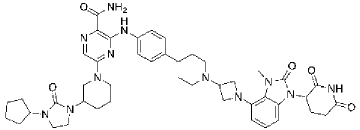 |
| 023 | 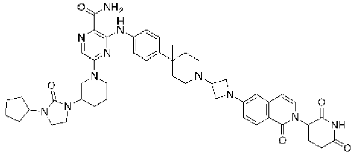 | 024 | 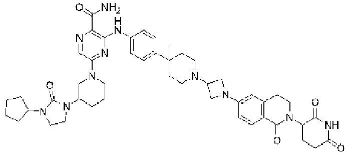 |
| 025 | 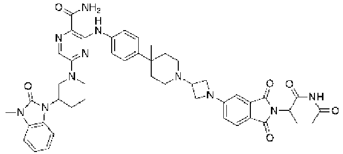 | 02 6 | 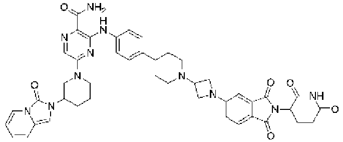 |
| 027 | 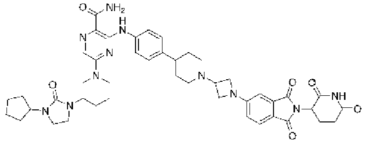 | 028 | 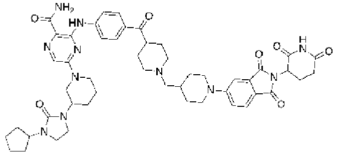 |
| 029 | 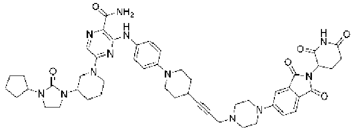 | 030 | 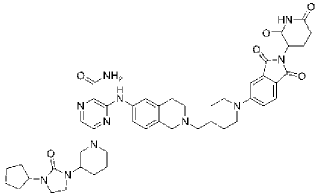 |
| 031 | 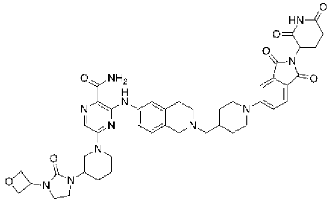 | 032 | 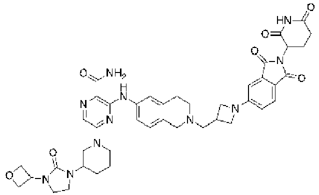 |
| 033 | 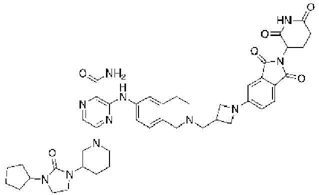 | 034 | 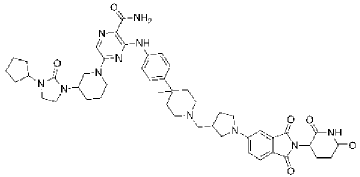 |
| 035 | 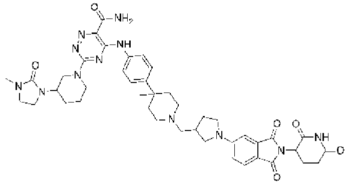 | 036 | 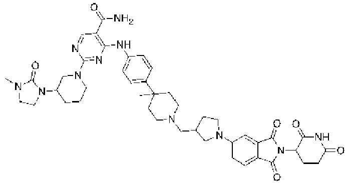 |
| 037 | 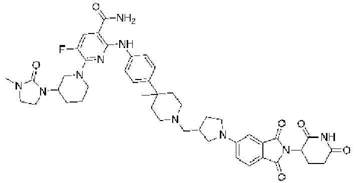 | 038 | 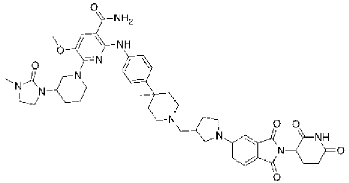 |
| 039 | 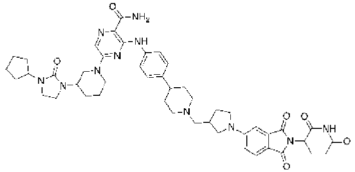 | 040 | 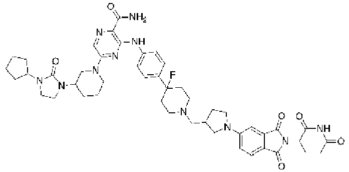 |
| 041 | 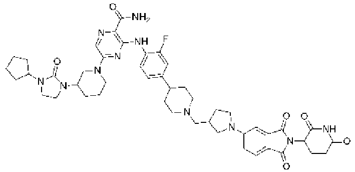 | 042 | 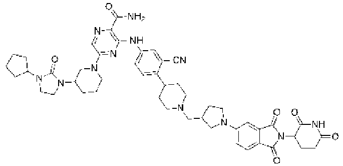 |
| 043 | 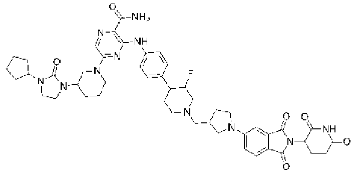 | 044 | 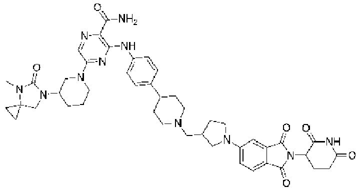 |
| 045 | 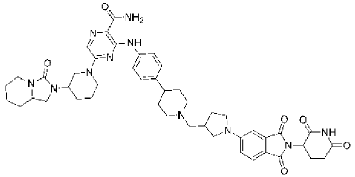 | 046 | 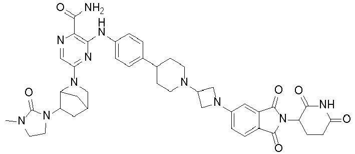 |
| 047 | 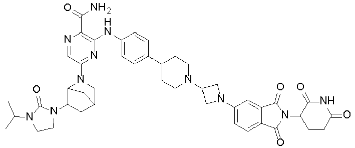 | 048 | 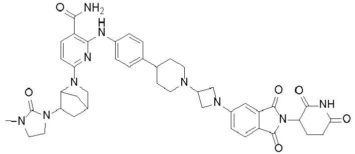 |
| 049 | 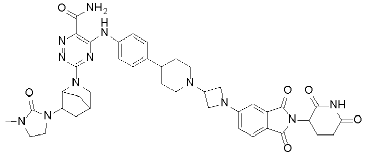 | 050 | 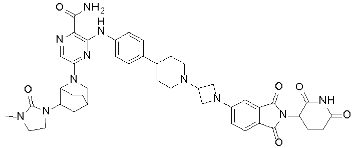 |
| 051 | 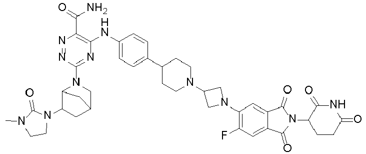 | 052 | 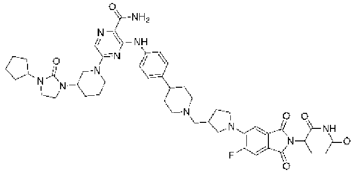 |
| 053 | 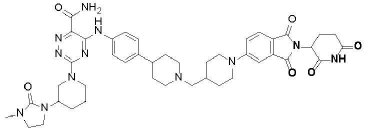 | 054 | 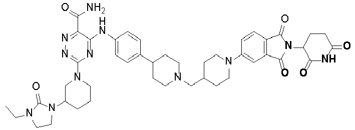 |
| 055 | 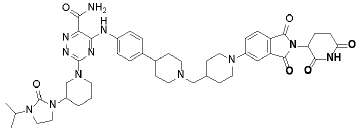 | 056 | 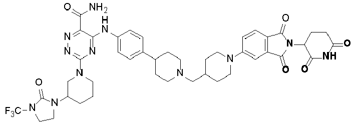 |
| 057 | 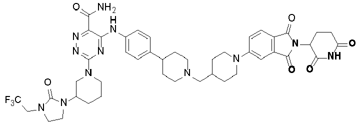 | 058 | 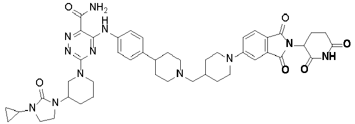 |
| 059 | 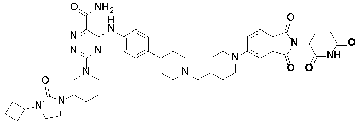 | 060 | 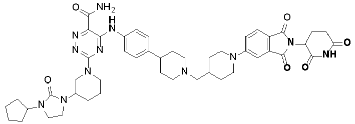 |
| 061 | 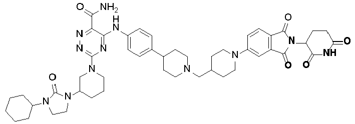 | 062 | 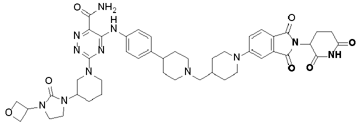 |
| 063 | 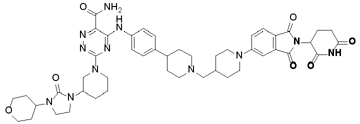 | 064 | 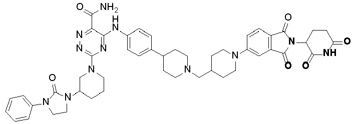 |
| 065 | 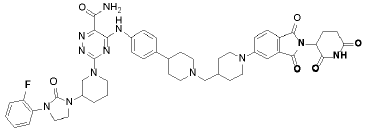 | 066 | 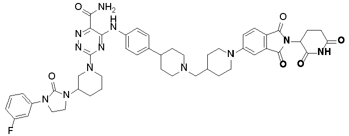 |
| 067 | 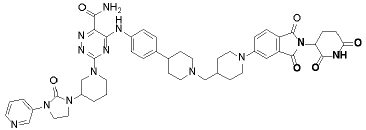 | 068 | 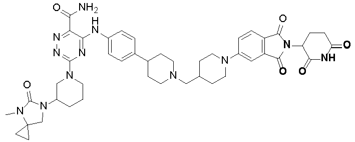 |
| 069 | 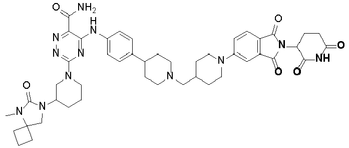 | 070 | 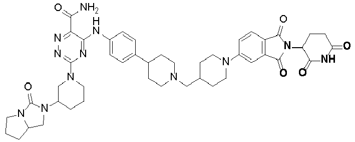 |
| 071 | 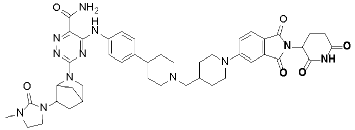 | 072 | 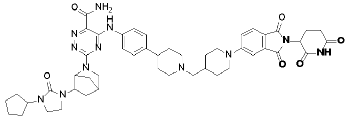 |
| 073 | 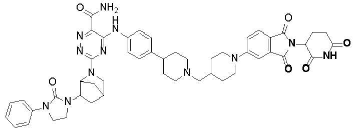 | 074 | 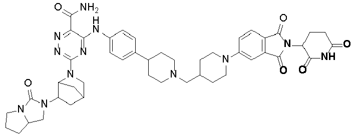 |
| 075 | 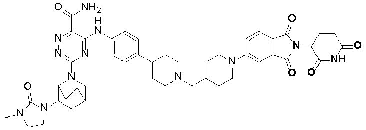 | 076 | 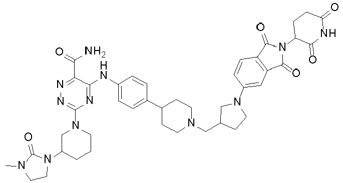 |
| 077 | 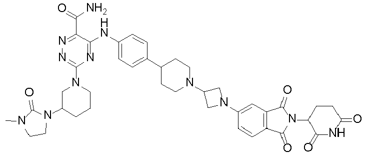 | 078 | 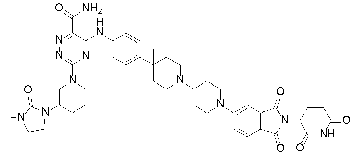 |
| 079 | 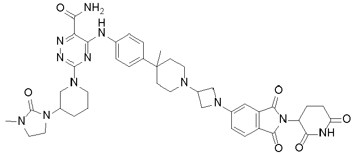 | 080 | 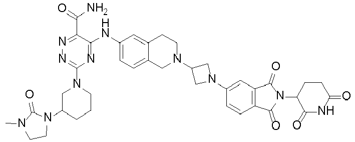 |
| 081 | 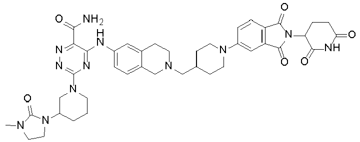 | 082 | 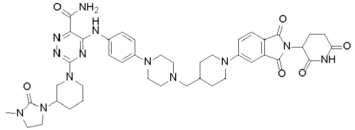 |
| 083 | 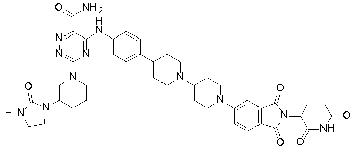 | 084 | 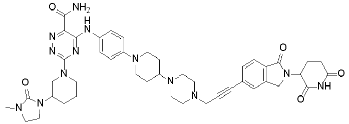 |
| 085 | 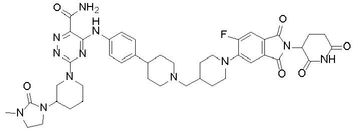 | 086 | 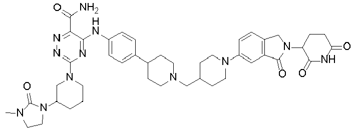 |
| 087 | 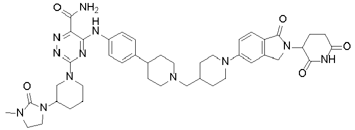 | 088 | 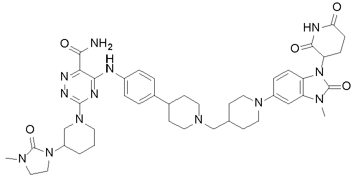 |
| 089 | 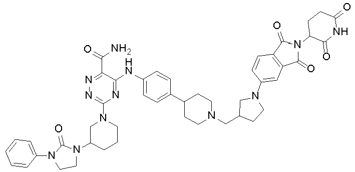 | 090 | 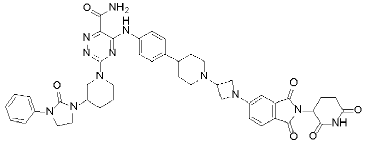 |
| 091 | 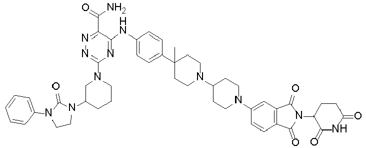 | 092 | 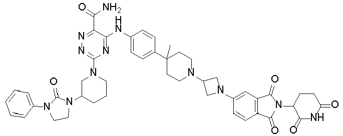 |
| 093 | 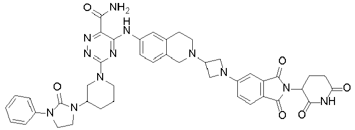 | 094 | 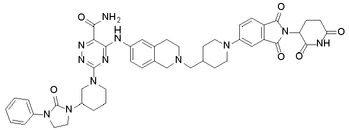 |
| 095 | 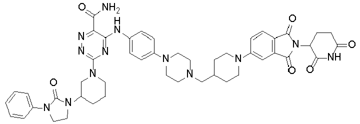 | 096 | 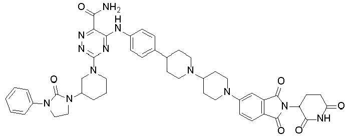 |
| 097 | 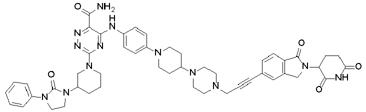 | 098 | 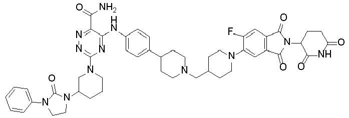 |
| 099 | 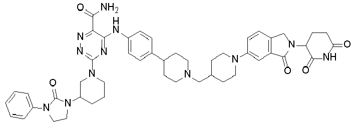 | 100 | 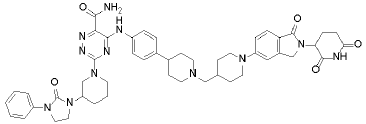 |
| 101 | 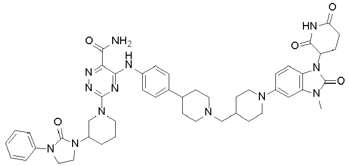 | 102 | 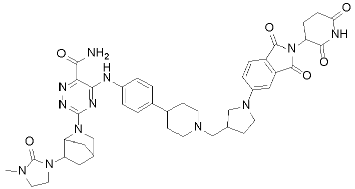 |
| 103 | 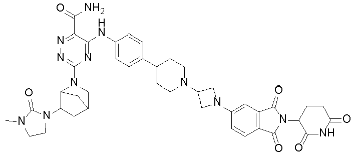 | 104 | 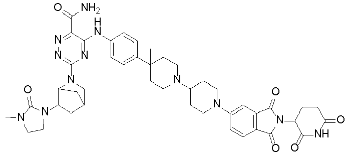 |
| 105 | 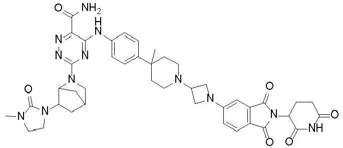 | 106 | 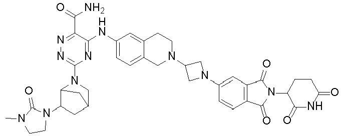 |
| 107 | 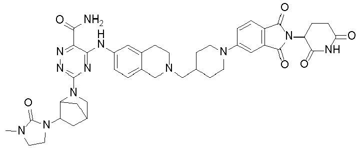 | 108 | 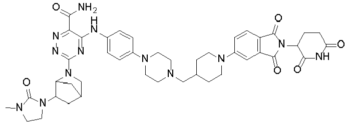 |
| 109 | 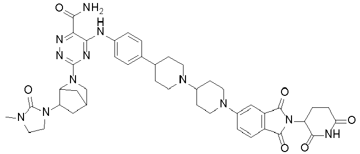 | 110 | 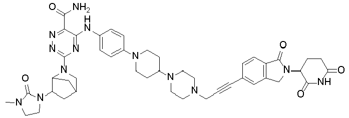 |
| 111 | 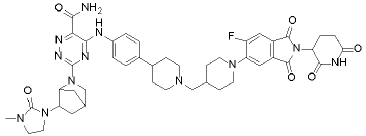 | 112 | 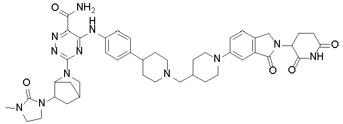 |
| 113 | 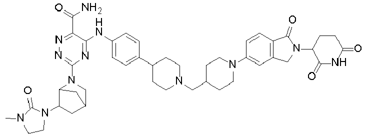 | 114 | 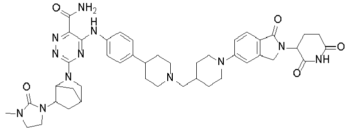 |
| 115 | 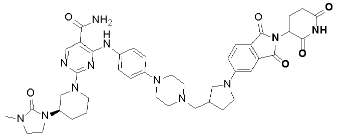 | 116 | 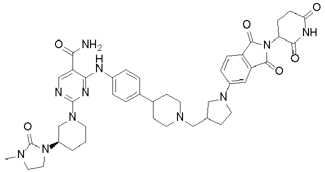 |
| 117 | 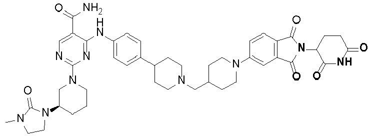 | 118 | 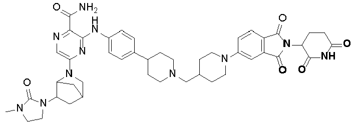 |
| 119 | 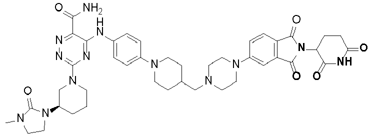 | 120 | 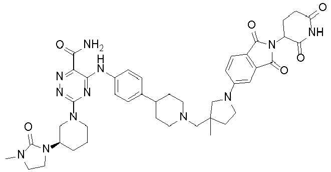 |
| 121 | 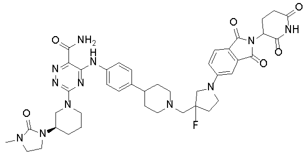 | 122 | 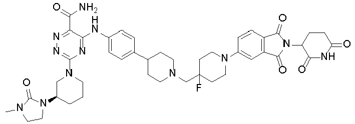 |
| 123 | 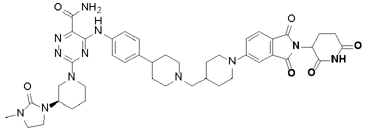 | 124 | 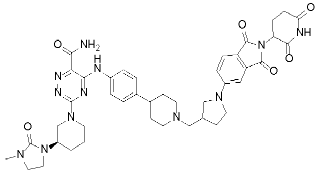 |
| 125 | 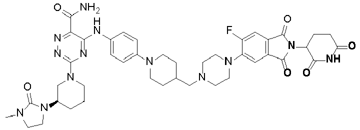 | 126 | 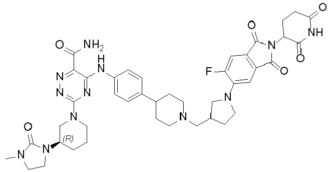 |
| 127 | 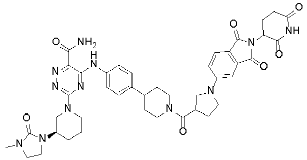 | 128 | 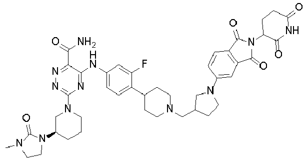 |
| 129 | 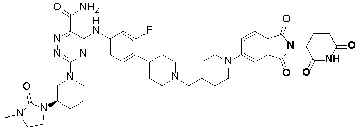 | 130 | 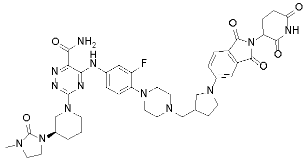 |
| 131 | 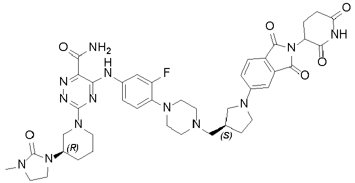 | 132 | 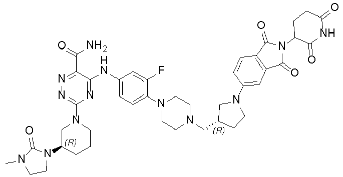 |
| 133 | 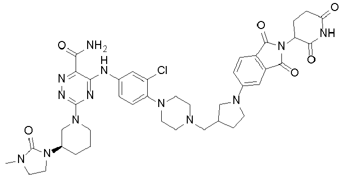 | 134 | 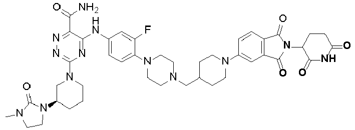 |
| 135 | 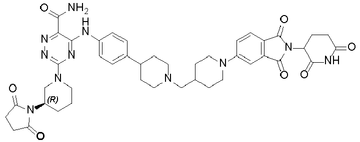 | 136 | 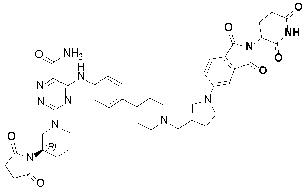 |
| 137 | 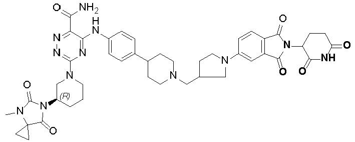 | 138 | 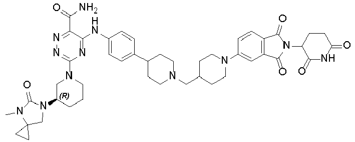 |
| 139 | 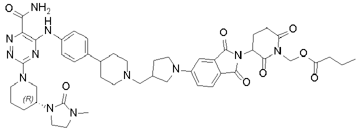 | 140 | 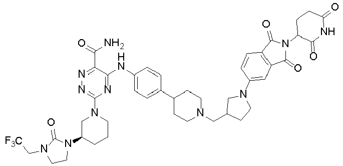 |
| 141 | 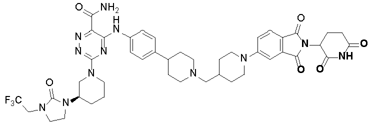 | 142 | 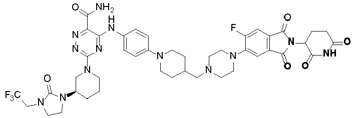 |
| 143 | 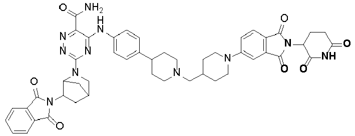 | 144 | 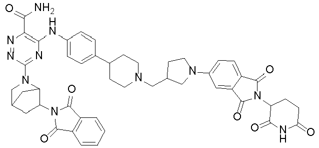 |
| 145 | 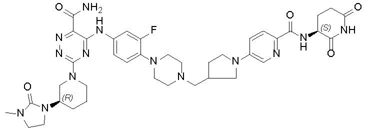 | 146 | 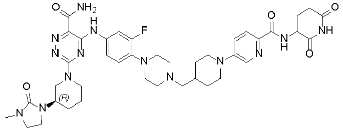 |
| 147 | 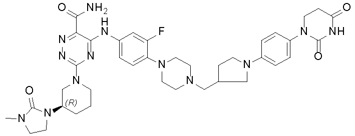 | 148 | 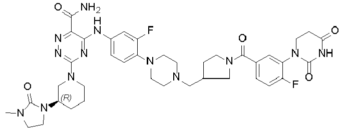 |
| 149 | 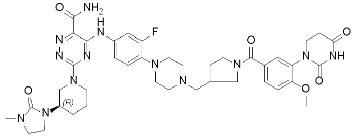 | 150 | 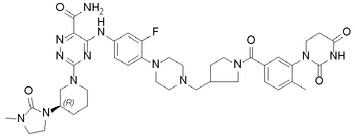 |
| 151 | 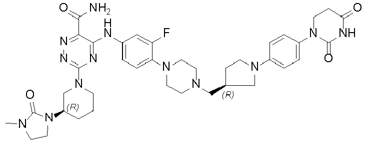 | 152 | 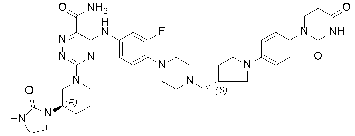 |
| 153 | 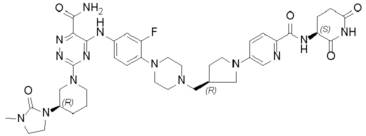 | 154 | 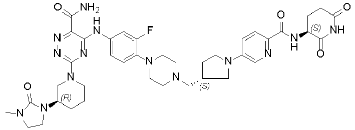 |
| 155 | 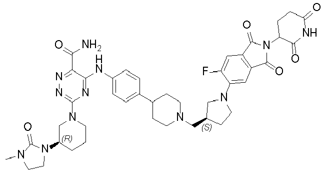 | 156 | 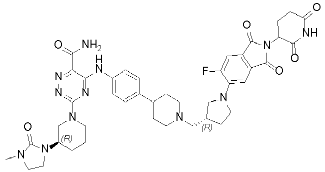 |
| 157 | 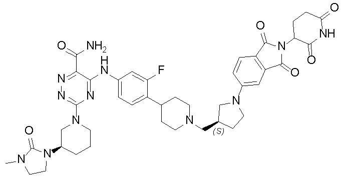 | 158 | 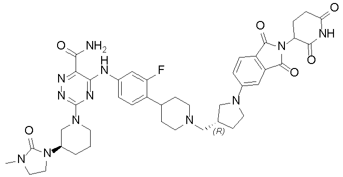 |
| 159 | 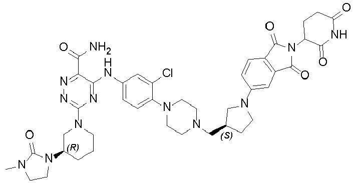 | 160 | 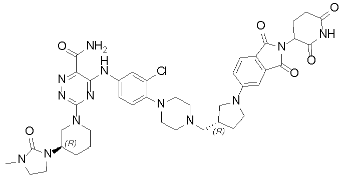 |
| 161 | 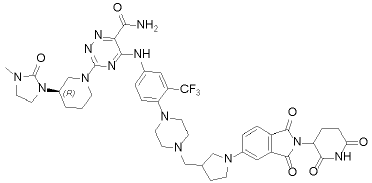 | 162 | 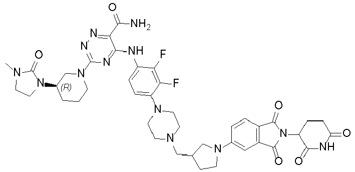 |
| 163 | 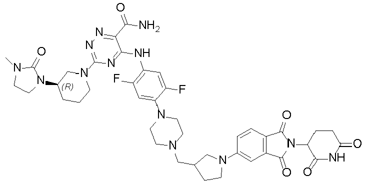 | 164 | 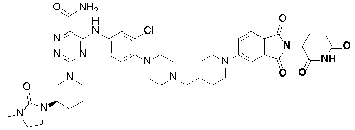 |
| 165 | 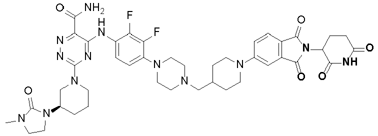 | 166 | 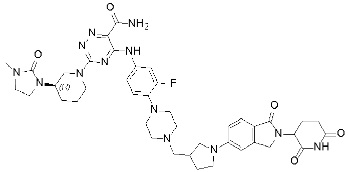 |
| 167 | 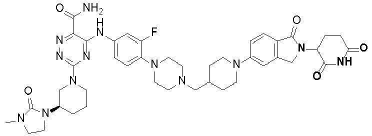 | 168 | 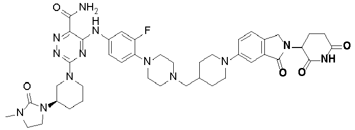 |
| 169 | 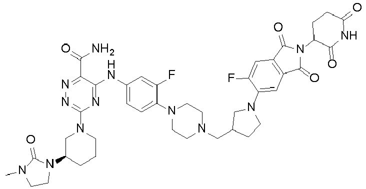 | 170 | 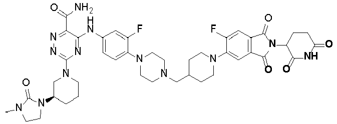 |
| 171 | 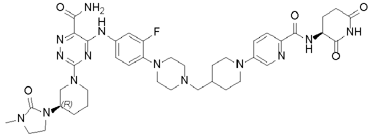 | 172 | 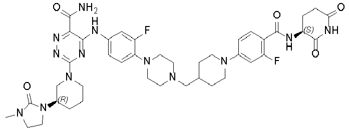 |
| 173 | 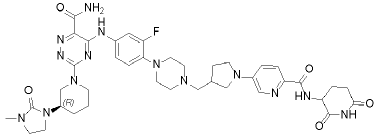 | 174 | 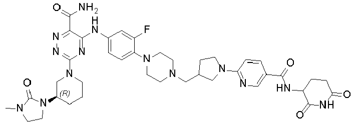 |
| 175 | 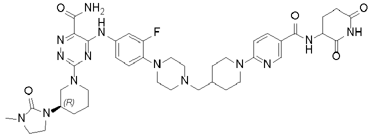 | 176 | 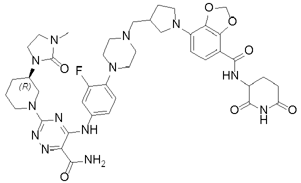 |
| 177 | 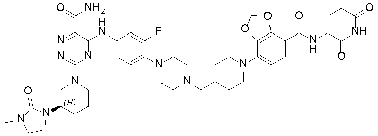 | 178 | 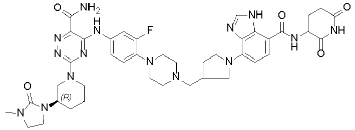 |
| 179 | 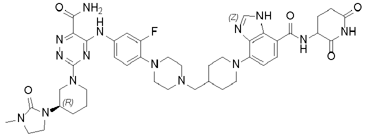 | 180 | 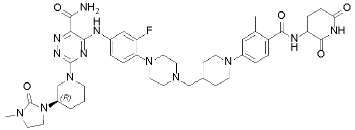 |
| 181 | 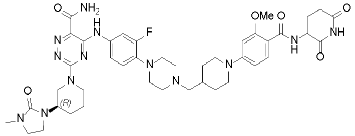 | 182 | 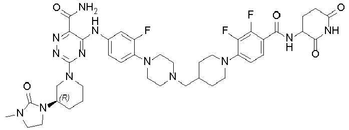 |
| 183 | 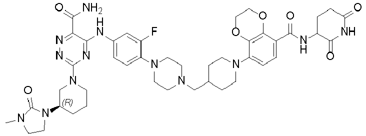 | 184 | 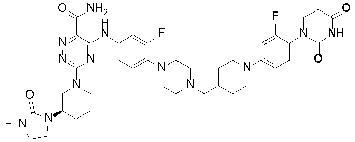 |
| 185 | 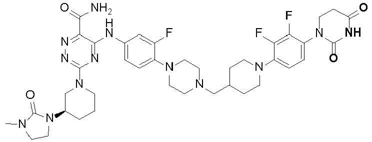 | 186 | 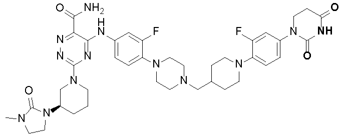 |
| 187 | 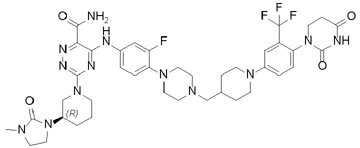 | 188 | 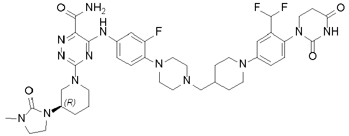 |
| 189 | 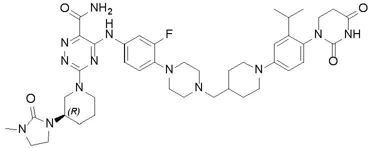 | 190 | 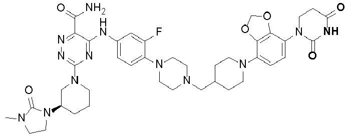 |
| 191 | 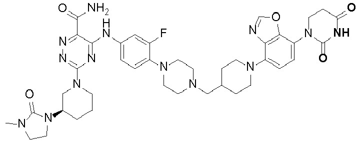 | 192 | 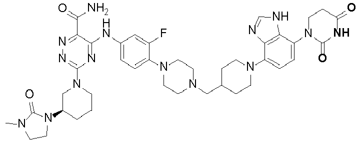 |
| 193 | 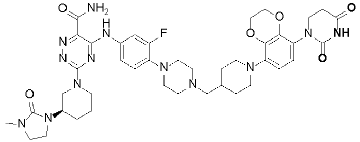 | 194 | 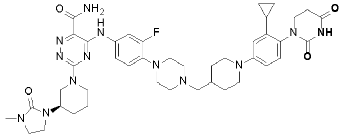 |
| 195 | 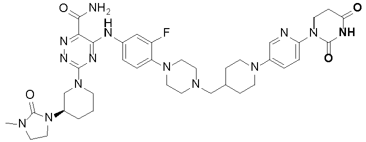 | 196 | 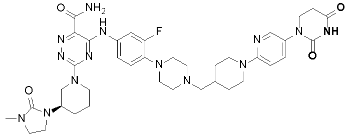 |
| 197 | 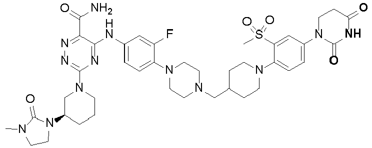 | 198 | 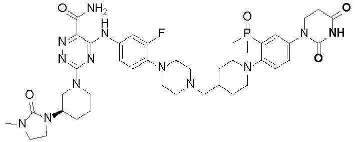 |
| 199 | 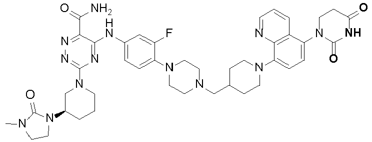 | 200 | 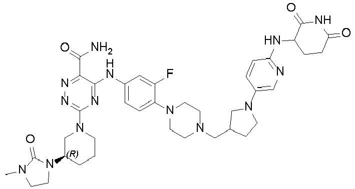 |
| 201 | 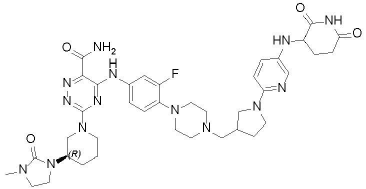 | 202 | 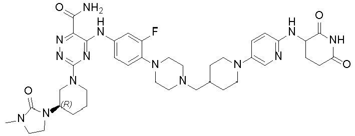 |
| 203 | 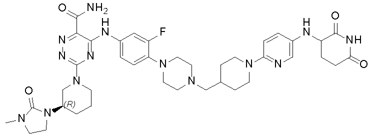 | 204 | 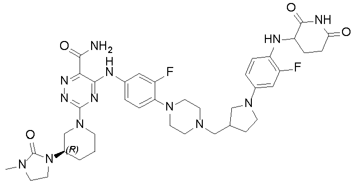 |
| 205 | 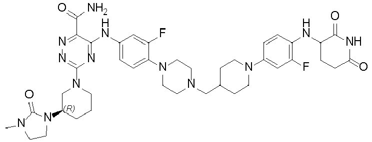 | 206 | 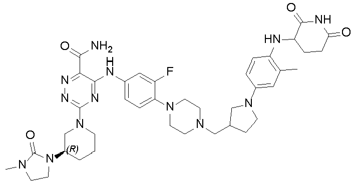 |
| 207 | 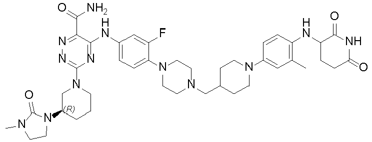 | 208 | 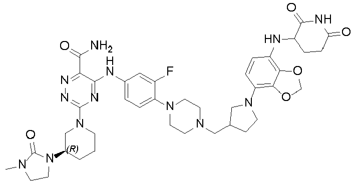 |
| 209 | 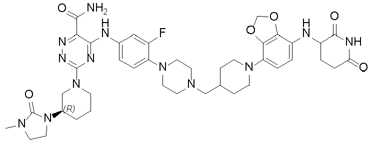 | 210 | 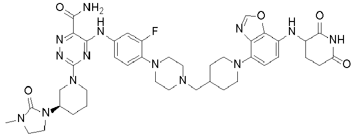 |
| 211 | 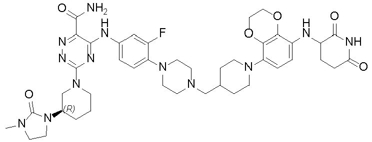 | 212 | 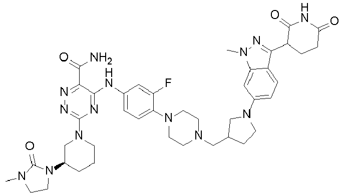 |
| 213 | 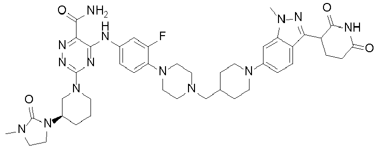 | 214 | 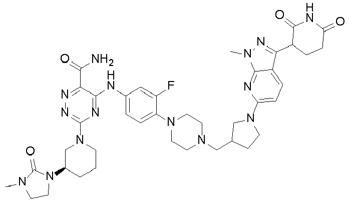 |
| 215 | 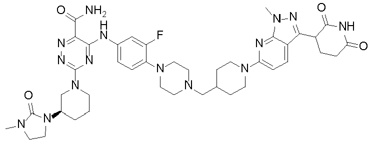 | 216 | 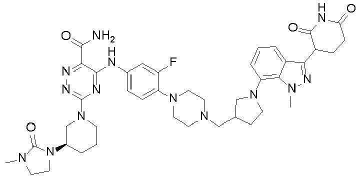 |
| 217 | 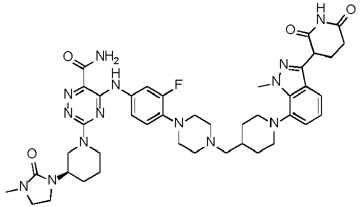 | 218 | 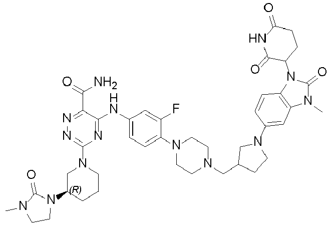 |
| 219 | 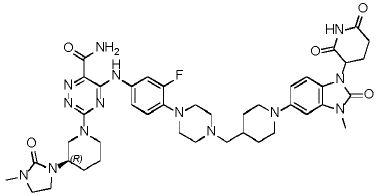 | 220 | 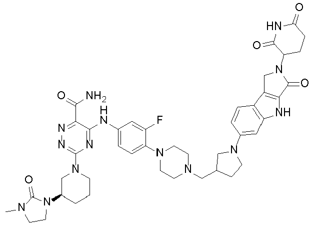 |
| 221 | 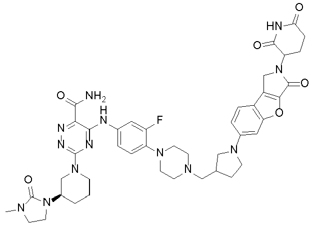 | 222 | 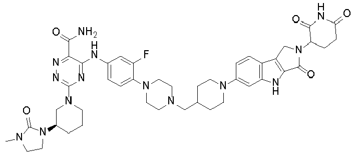 |
| 223 | 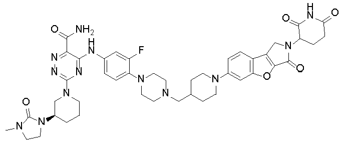 | 224 | 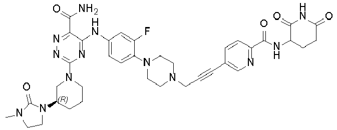 |
| 225 | 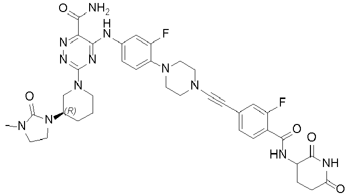 | 226 | 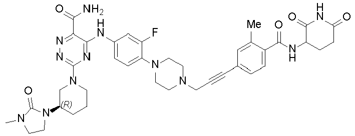 |
| 227 | 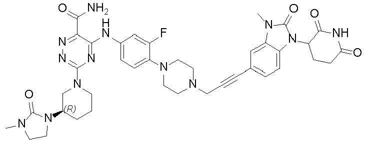 | 228 | 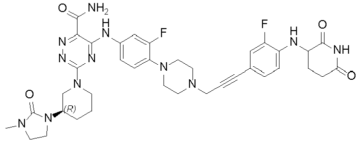 |
| 229 | 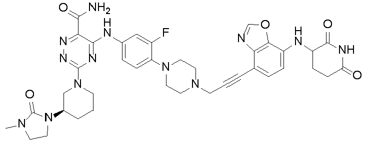 | 230 | 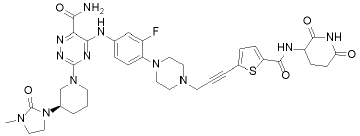 |
| 231 | 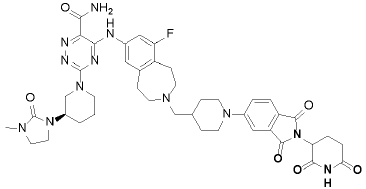 | 232 | 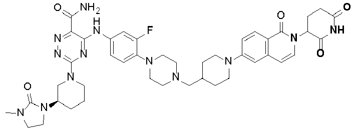 |
| 233 | 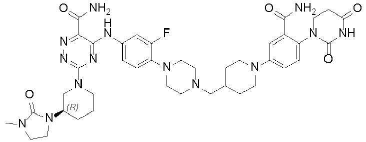 | 234 | 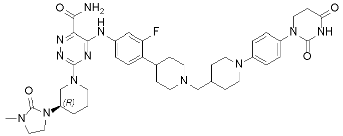 |
| 235 | 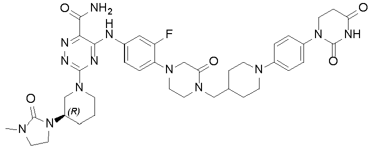 | 236 | 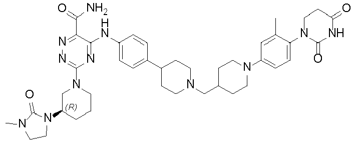 |
| 237 | 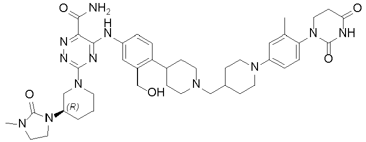 | 238 | 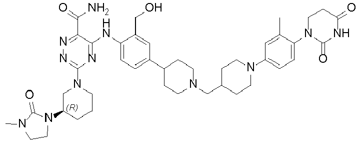 |
| 239 | 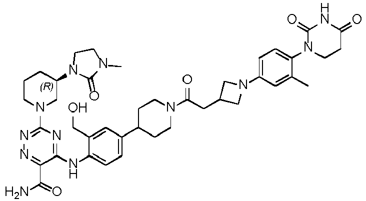 | 240 | 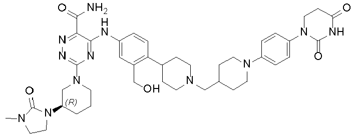 |
| 241 | 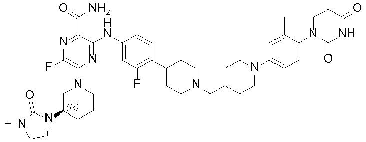 | 242 | 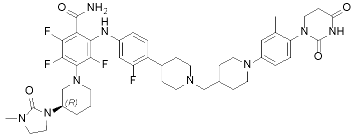 |
| 243 | 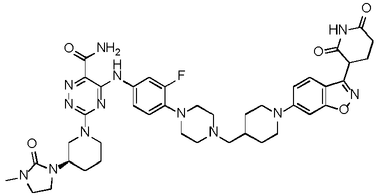 | 244 | 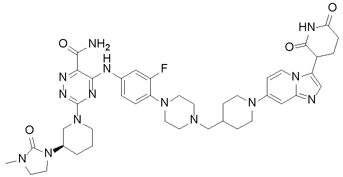 |
| 245 | 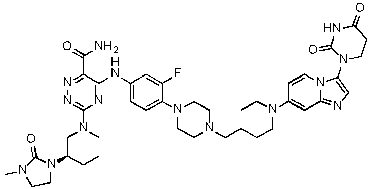 | 246 | 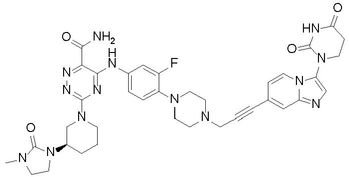 |
| 247 | 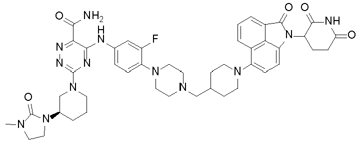 | 248 | 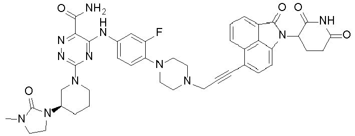 |
| 249 | 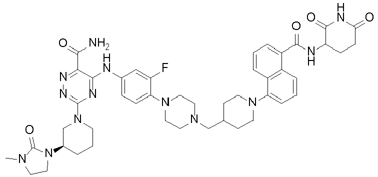 | 250 | 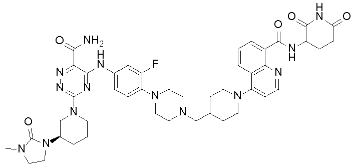 |
| 251 | 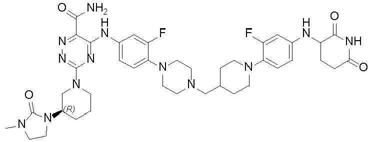 | 252 | 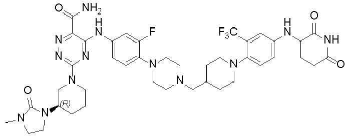 |
| 253 | 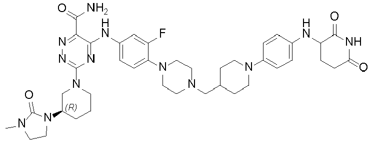 | 254 | 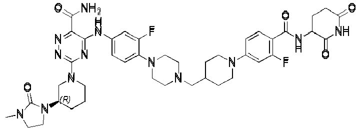 |
| 255 | 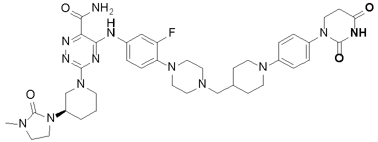 | 256 | 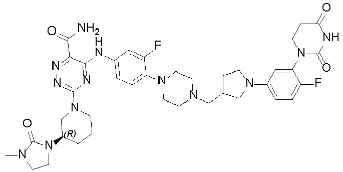 |
| 257 | 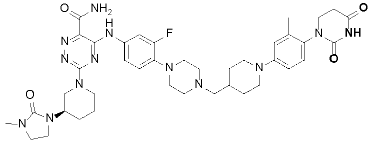 | 258 | 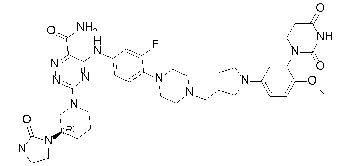 |
| 259 | 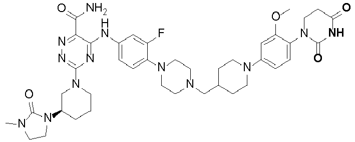 | | |
or its stereoisomer thereof, or its stereoisomer mixture thereof, or its pharmaceutically acceptable salt thereof.

15. A pharmaceutical composition, **characterized in that**, the pharmaceutical composition comprises one or more of the compounds of any one of claims 1 to 14.

16. Use of the compound of any one of claims 1 to 15 in the preparation of a drug alone or a drug that is used in combination with other drugs for treating a disease, a disorder or a condition that would benefit from the inhibition or degradation of Bruton's tyrosine kinase activity.

17. The use of claim 16, **characterized in that**, the disease, disorder or condition is that one that would benefit from the inhibition or degradation of Bruton's tyrosine kinase mutation.

18. The use of claim 16, **characterized in that**, the disease is selected from one or more of B cell or plasma cell proliferative diseases, and autoimmune diseases.

19. The use of claim 18, **characterized in that**, the B cell or plasma cell proliferative diseases include diffuse large B cell lymphoma, follicular lymphoma, chronic lymphocytic leukemia, small lymphocytic lymphoma, B cell prolymphocytic leukemia, lymphoplasmacytic lymphoma/Waldenström macroglobulinemia, splenic marginal zone lymphoma, plasma cell myeloma, plasmacytoma, extranodal marginal zone B cell lymphoma, lymph node marginal zone B cell lymphoma, mantle cell lymphoma, mediastinum (thymus) large B cell lymphoma, intravascular large B cell lymphoma, primary effusion lymphoma, Burkitt lymphoma/leukemia or lymphomatoid granulomatosis, multiple myeloma.

20. The use of claim 18, **characterized in that**, the autoimmune diseases include inflammatory bowel disease, arthritis, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, multiple sclerosis, Guillain-Barré syndrome, acute disseminated encephalomyelitis, Addison's disease, optic opsoclonus-myoclonus syndrome, ankylosing spondylitis, antiphospholipid antibody syndrome, aplastic anemia, autoimmune hepatitis, coeliac disease, Goodpasture's syndrome, immune thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behcet's disease, chronic fatigue, familial dysautonomia, endometriosis, interstitial cystitis, neuromyotonia, scleroderma or vulvodynia, and chronic graft versus host.
